(19) **Europäisches Patentamt** / **European Patent Office** / **Office européen des brevets**

(11) **EP 2 593 472 B1**

(12) **EUROPEAN PATENT SPECIFICATION**

(45) Date of publication and mention of the grant of the patent:
**16.05.2018 Bulletin 2018/20**

(21) Application number: **11807607.4**

(22) Date of filing: **15.07.2011**

(51) Int Cl.:
*C07K 14/195* (2006.01)   *C12N 9/02* (2006.01)
*C12N 15/10* (2006.01)

(86) International application number:
**PCT/US2011/044275**

(87) International publication number:
**WO 2012/009693 (19.01.2012 Gazette 2012/03)**

(54) **ANCESTRAL PROTEINS**

STAMMPROTEINE

PROTÉINES ANCESTRALES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.07.2011 US 201113044084**
**15.07.2010 US 364640 P**

(43) Date of publication of application:
**22.05.2013 Bulletin 2013/21**

(73) Proprietors:
 • **The Trustees of Columbia University in the City of New York**
   **New York, New York 10027 (US)**
 • **Georgia Tech Research Corporation**
   **Atlanta, GA 30332-0415 (US)**

(72) Inventors:
 • **FERNANDEZ, Julio, M.**
   **New York, NY 10027 (US)**

 • **PEREZ-JIMENEZ, Raul**
   **New York, NY 10027 (US)**
 • **GAUCHER, Eric**
   **Atlanta, GA 30332-0363 (US)**
 • **KOSURI, Pallav**
   **New York, NY 10036 (US)**

(74) Representative: **Barker Brettell LLP**
   **100 Hagley Road**
   **Edgbaston**
   **Birmingham B16 8QQ (GB)**

(56) References cited:
   **WO-A2-2008/021543    US-A1- 2004 052 810**
   **US-A1- 2009 092 582**

 • **COPLEY SHELLEY D ET AL: "Divergence of function in the thioredoxin fold suprafamily: Evidence for evolution of peroxiredoxins from a thioredoxin-like ancestor", BIOCHEMISTRY, vol. 43, no. 44, 9 November 2004 (2004-11-09), pages 13981-13995, XP002716965, ISSN: 0006-2960**

## Description

This application claims priority to U.S. Provisional Application No. 61/364,640, filed on July 15, 2010, and also claims priority to PCT/US11/44084, filed on July 14, 2011.

This patent disclosure contains material that is subject to copyright protection. The copyright owner has no objection to the facsimile reproduction by anyone of the patent document or the patent disclosure as it appears in the U.S. Patent and Trademark Office patent file or records, but otherwise reserves any and all copyright rights.

This invention was made with government support under HL66030 and HL61228 awarded by NIH. The government has certain rights in the invention.

To conform to the requirements for International Patent Applications, many of the figures presented herein are black and white representations of images originally created in color. The original color versions can be viewed in Perez-Jimenez et al., 2011, Nat Struct Mol Biol., 18(5):592-6 (including the accompanying Supplementary Information available in the on-line version of the manuscript available on the Nature Structural & Molecular Biology web site) and Perez-Jimenez, et al., 2009, Nat Struct Mol Biol 16: 890-6, and Alegre-Cebollada et al., 2010, J Biol Chem, 285(25):18961-6.

## BACKGROUND OF THE INVENTION

The market for industrial enzymes has exploded in the past decades, with applications now including biotech, pharma, detergents, textile production, food processing, wine making, paper manufacturing, beauty products and many other areas. This has created an increasing need for enzymes that are stable at a wider range of temperatures and pH. As of today, there is no reliable method to achieve this while not simultaneously affecting the activity. A common practice nowadays is to randomly insert mutations in existing enzymes and screen for variants that exhibit the desired characteristics. However, due to the enormous combinatorial possibilities, this often becomes a costly and work-intense endeavor, and never guarantees success. Still, this has been the preferred method to discover most of the presently used industrial enzymes, many of which are patented.

Little is known about how the chemistry of primitive enzymes arose and how the environmental conditions affected the evolution of their chemistry (Zalatan et al., Nat. Chem. Biol., 5:516-520 (2009)); however since these organisms lived on the primordial earth and in an environment that was much hotter and more acidic than today, their enzymes would have been optimized to have a higher thermal and acidic stability than their modern counterparts. Experimental paleogenetics and paleobiochemistry (e.g. the study of resurrected proteins) can reveal valuable information regarding the adaptation of extinct forms of life to climatic, ecological and physiological alterations (Thornton, Science 301, 1714-7 (2003); Thomson et al, Nat Genet 37, 630-5 (2005); Boussau et al, Nature 456, 942-5 (2008); Chang et al, Mol Biol Evol 19, 1483-9 (2002)). Unfortunately, previous reconstruction and resurrection provide a journey back in time on the order of a only few millions years (Myr) (Benner et al, Adv Enzymol Relat Areas Mol Biol 75, 1-132, xi (2007); Thornton, Nat Rev Genet 5, 366-75 (2004); Gaucher et al, Nature 425, 285-8 (2003)). Consequently, many hypotheses about ancient life remain untested and cannot be directly answered by examining fossil records (Nisbet and Sleep, Nature 409, 1083-91 (2001)). There is a need for reliable methods for optimizing the pH and temperature stabilities of existing enzymes. There is also a need for methods useful for developing enzymes in a predictable and cost effective manner that are more effective and work in a wider range of environments. This invention addresses these needs.

## SUMMARY OF THE INVENTION

In one aspect, the invention relates to an isolated polypeptide in accordance with claim 1 herein. In one embodiment, the isolated polypeptide does not have 100% identity with any extant polypeptide. In another embodiment, the variant has at least about 85.5%, at least about 90.5%, at least about 92.5%, at least about 95%, about 96%, about 96.5%, about 97%, about 97.5%, about 98%, about 98.5%, about 99%, about 99.5% or about 99.9% amino acid sequence identity to SEQ ID NO: 1.

In still a further embodiment, the isolated polypeptide has enzymatic activity. In still another embodiment, the isolated polypeptide has thioredoxin activity.

In yet another embodiment, the isolated polypeptide is labeled. In one embodiment, the label is colorimetric, radioactive, chemiluminescent, or fluorescent. In still a further embodiment, the isolated polypeptide is chemically modified. In one embodiment, the chemical modification comprises covalent modification of an amino acid. In another embodiment, the covalent modification comprises methylation, acetylation, phosphorylation, ubiquitination, sumoylation, citrullination, or ADP ribosylation.

In one aspect, the invention relates to an isolated antibody that specifically binds to a polypeptide of SEQ ID NO: 1.

In another aspect, the invention relates to an isolated nucleic acid comprising a nucleic acid sequence which encodes a polypeptide having a sequence of SEQ ID NO: 1. In another aspect, the invention relates to an isolated nucleic acid comprising a nucleic acid sequence which encodes a polypeptide having at least about 75% identity to SEQ ID NO: 1.

In one embodiment, the nucleic acid sequence is optimized for expression in a mammalian expression system. In another embodiment, the nucleic acid sequence is optimized for expression in a bacterial expression system. In one embodiment, the baterial expression system is E. coli. In another embodiment, the isolated nucleic acid is operably linked to one or more control sequences that direct the production of the polypeptide in a suitable expression host.

In another aspect, the invention relates to a recombinant expression vector comprising an isolated nucleic acid comprising a nucleic acid sequence which encodes a polypeptide having a sequence of SEQ ID NO: 1.

In another aspect, the invention relates to a recombinant expression vector comprising an isolated nucleic acid comprising a nucleic acid sequence which encodes a polypeptide having at least about 75% identity to SEQ ID NO: 1.

In still a further aspect, the invention relates to a method for producing a polypeptide having a sequence of SEQ ID NO: 1, the method comprising cultivating a host cell comprising a nucleic acid construct comprising a polynucleotide encoding the polypeptide under conditions suitable for production of the polypeptide; and recovering the polypeptide.

In still a further aspect, the invention relates to a method for producing a polypeptide having at least about 75% identity to SEQ ID NO: 1, the method comprising cultivating a host cell comprising a nucleic acid construct comprising a polynucleotide encoding the polypeptide under conditions suitable for production of the polypeptide; and recovering the polypeptide.

In one embodiment, the extant polypeptide is a thioredoxin polypeptide.

In still a further aspect, the invention relates to a method for producing a polypeptide comprising at least about 10, at least about 20, at least about 30, at least about 50 at least about 60, at least about 70, at least about 80, at least about 90 or at least about 100 consecutive amino acids of SEQ ID NO: 1, the method comprising cultivating a host cell comprising a nucleic acid construct comprising a polynucleotide encoding the polypeptide under conditions suitable for production of the polypeptide; and recovering the polypeptide.

In one embodiment, the extant polypeptide is a thioredoxin polypeptide.

In another aspect, the invention relates to a polypeptide produced according to the methods described herein.

## BRIEF DESCRIPTION OF THE FIGURES

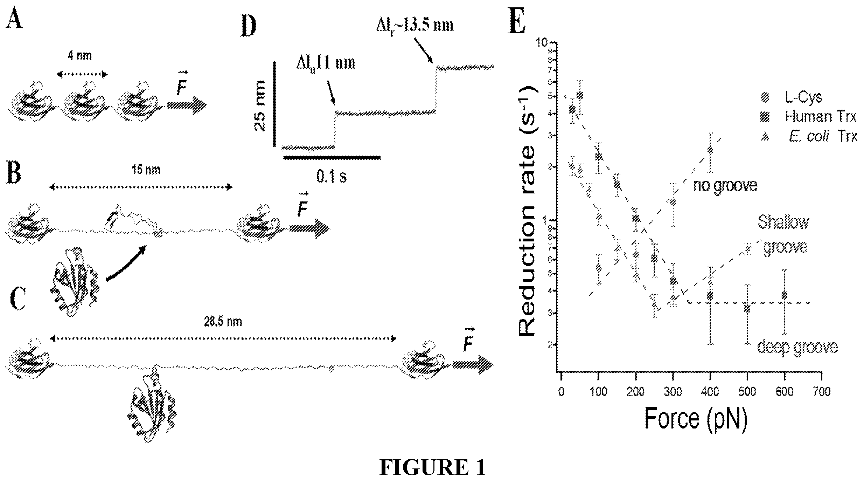

**Figure 1.** Single molecule assay of Trx catalysis. **Fig. 1A** A pair of vicinal cysteines (positions 32 and 75 in the sequence; yellow) are engineered into the 127 protein structure, dividing the protein mechanically in two parts. The two cysteines spontaneously form a disulfide bond. A polypeptide made of eight repeats of such engineered 127 proteins, $(I27_{s-s})_8$, is mechanically stretched at constant force. **Fig. IB** Unfolding of a single protein in the chain causes a step elongation by ∼11 nm. Unfolding also removes the steric constraints on the disulfide bond exposing it to a nucleophilic attack by a Trx enzyme present in the surrounding solution. **Fig. 1C** A successful nucleophilic attack reduces the disulfide bond and allows for a further extension of the protein by ∼14 nm. **Fig. ID** Experimental force clamp trace showing the stepwise elongation of a $(I27_{s-s})_8$ polypeptide at a constant force of 100 pN. The first step marks the unfolding of a single $I27_{s-s}$ module in the chain and the second the reduction of its disulfide bond. The rate of reduction at any given force is easily measured from a collection of such traces. **Fig. IE** Force dependency of the rate of reduction of disulfide bonds by different reducing agents. Human Trx shows a negative force dependency that reaches a force independent minimum. By contrast, L-cysteine shows a simple exponential increase in the rate of reduction with the applied force. Bacterial thioredoxins show a combination of mechanisms giving a characteristic V shaped force dependency.

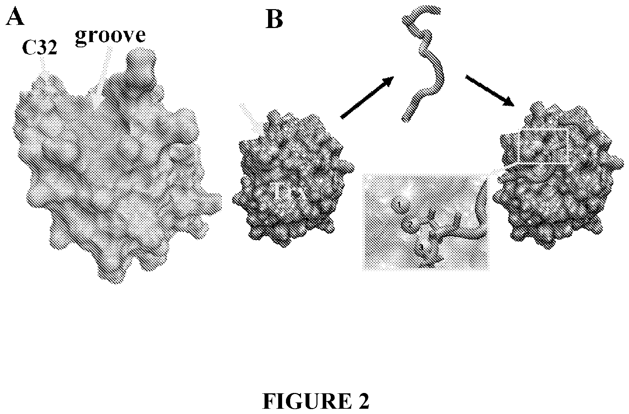

**Figure 2.** Molecular mechanisms of Trx catalysis. **Fig. 2A** Trx enzymes main structural features are a prominent binding groove marked by the shaded light green area, and the catalytic cysteine located on the rim of the groove (human; PDB code 3Trx). **Fig. 2B** A Trx enzyme collides and binds a substrate protein that contains a disulfide bond. Once the disulfide bonded substrate binds to the groove, the sulfur atoms of the catalytic cysteine (#1, inset) and the substrate disulfide (#2,3, inset) must align 180° from each other in order to acquire the correct $S_N2$ geometry for disulfide bond reduction to occur. This alignment takes place inside the binding groove.

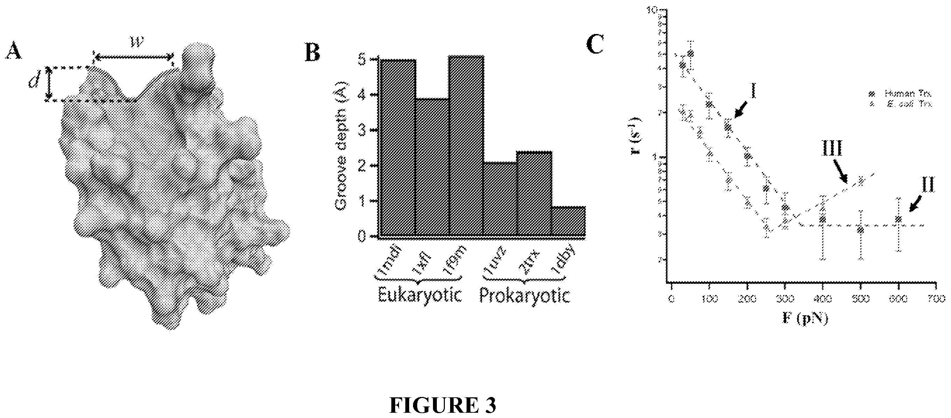

**Figure 3.** Structural characteristics of the binding groove in Trx enzymes. **Fig. 3A** Geometric characteristics of the peptide-binding groove in human Trx. **Fig. 3B** A clear structural difference can be observed when comparing bacterial and eukaryotic origin Trxs. In the case of eukaryotic Trxs the binding groove is much deeper and hindered than in the case of bacterial Trxs. **Fig. 3C** Comparison of the force dependency of the reduction rate for human and E. coli Trx enzymes. Human Trx (10 μM, red squares) shows two distinct mechanisms. A first mechanism is exponentially inhibited by force (I), and a second mechanism is force independent (II). A third mechanism is apparent in E. coli

Trx (10 μM, green triangles) whereby at high forces, the rate of catalysis increases exponentially (III).

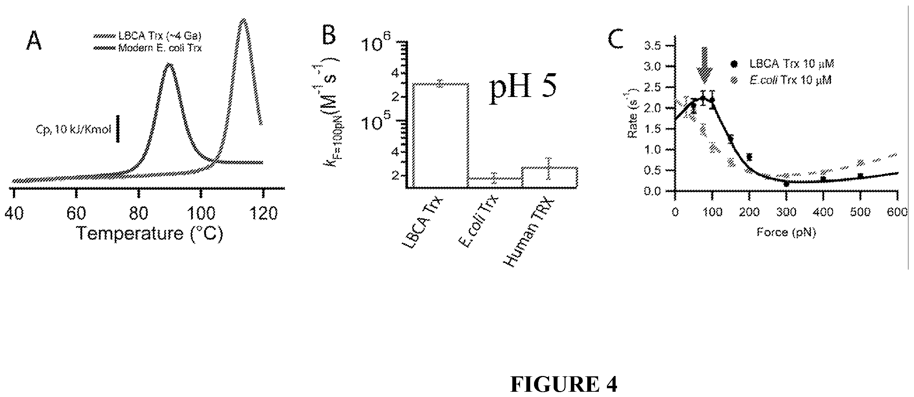

**Figure 4.** Resurrected Trx from the Last Bacterial Common Ancestor (LBCA). **Fig. 4A** Differential scanning calorimetry measure the melting temperatures of LBCA (113 °C) and modern E. Coli Trx (87 °C). **Fig. 4B** LBCA is active at pH 5, by contrast modern E. coli and human thioredoxin show ~20 fold lower rates at this pH. **Fig. 4C** The rate of reduction of LBCA shows a maximum at 100 pN, suggesting changes in the way the substrate fits into the binding groove. By contrast, all extant Trx enzymes show a maximal rate at zero force.

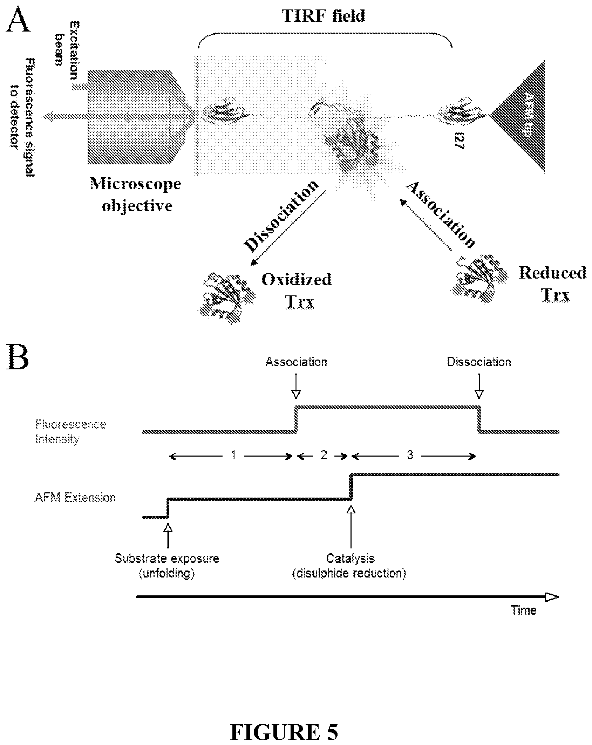

**Figure 5.** Schematic of the combined TIRF-AFM (Total Internal Reflection Fluorescence- Atomic Force Microscope) experiment. **Fig. 5A** A fluorescently labeled Trx enzyme binds to an exposed disulfide bond in an unfolded polypeptide. When bound, the enzyme is localized in the TIRF field and can consequently be detected as a bright fluorescence spot localized exactly underneath of the AFM tip. The catalysis event is independently detected by the AFM as a stepwise extension of the substrate. The final dissociation event is detected as the disappearance of the fluorescent spot from the base of the AFM cantilever. **Fig. 5B** Schematic drawing showing the expected data from a combined TIRF-AFM experiment. The fluorescence intensity data comes from the pixels on the CCD corresponding to the area under the tip of the AFM. The extension trace shows the surface-tip distance for the AFM during force-clamp. Three relevant dwell times to be measured are marked 1, 2 and 3 respectively. The force dependency of all three dwell times will be measured.

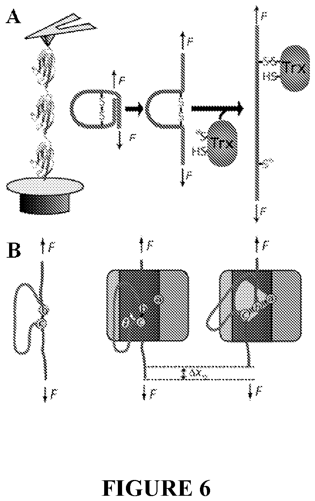

**Figure 6.** Force spectroscopy reveals the dynamic rearrangement of the substrate during Trx catalysis. **Fig. 6A** An Atomic Force Microscopy (AFM) based assay of Trx catalysis. A disulfide bonded polypeptide is picked up by an AFM cantilever and mechanically stretched at constant force. The cartoons on the right show the detection scheme. The polypeptide is first extended by unfolding, right up to the disulfide bond. The exposed disulfide then undergoes a nucleophilic attack by the Trx enzyme. Reduction of the substrate disulfide bond allows for an extra extension that is easily detected by the AFM. The rate of reduction is measured from the kinetics of the step increases in length that mark each reduction event. **Fig. 6B** A key observation made using the single molecule assay was that a sufficiently high mechanical force applied to the substrate disulfide bond inhibited the enzymatic reaction. The sequence of cartoons explains the effect of a pulling force in inhibiting the rotation of the disulfide bond that is needed to acquire the configuration for the $S_N2$ reaction.

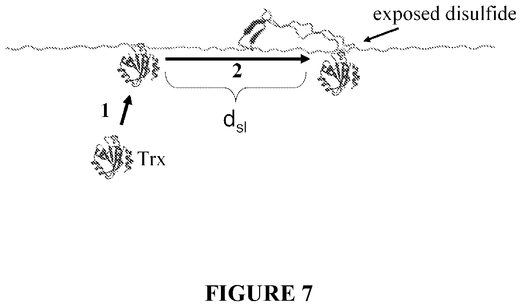

**Figure 7.** A putative search mechanism for Trx enzymes. (1) A Trx enzyme undergoing a 3-D diffusion search randomly binds the exposed polypeptide. (2) The enzyme then undergoes a 1-D diffusion search for the exposed disulfide, over a sliding distance $d_{sl}$. This mechanism greatly reduces the time necessary for finding the target.

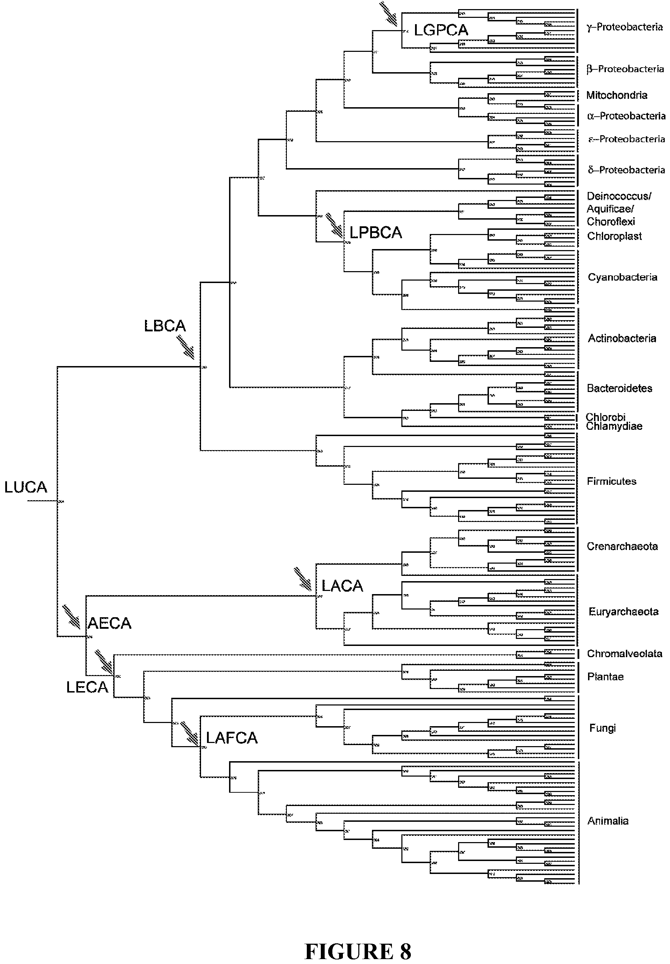

**Figure 8.** Phylogenetic Tree used for the ancestral sequence reconstruction of Trx enzymes. A total of 203 sequences were used (see Table 1). The nodes of interest are indicated with red arrows. Last bacterial common ancestors (LCBA), last archaeal common ancestor (LACA), archaea/eukaryota common ancestor (AECA), last common ancestor cyanobacterial and deinococcus/thermus groups (LPBCA) that represents the origin of photosynthetic bacteria; last eukaryotic common ancestor (LECA), last common ancestor of γ-proteobacteria (LGPCA) and last common ancestor of animals and fungi (LAFCA).

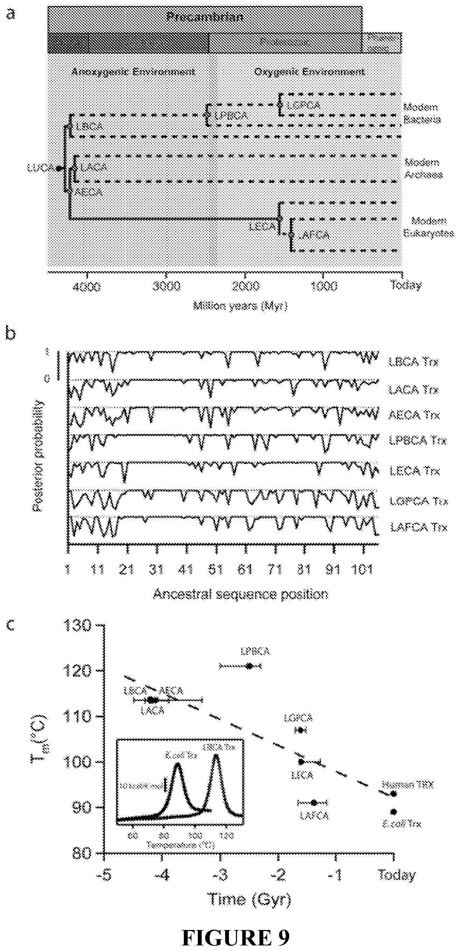

**Figure 9.** Phylogenetic analysis of Trx enzymes and ancestral sequences reconstruction. **Fig 9A** Schematic phylogenetic tree showing the geological time in which different extinct organisms lived, i.e., last bacterial common ancestors (LBCA); last archaeal common ancestor (LACA); archaea/eukaryota common ancestor (AECA) and last eukaryotic common ancestor (LECA). Other internal nodes are: the last common ancestor of photosynthetic bacteria (LPBCA), the last common ancestor of γ-proteobacteria (LGPCA), and the last common ancestor of animals and fungi (LAFCA). The dashed lines represent further bifurcations. Divergence times are compiled from multiple sources (see Hedges and Kumar, The Timetree of life, xxi, 551 p. (Oxford University Press, Oxford, 2009)). **Fig. 9B** Posterior probability distribution of the inferred amino acids across 106 sites for the interested internal nodes. The inferred amino acid at each site for the interested internal node is the residue with the highest posterior probability. **Fig. 9C** Denaturation temperatures ($T_m$) vs. geological time for ancestral Trx enzymes. Modern *E. coli* and Human Trx enzymes are also indicated. The inset shows experimental DSC thermograms for *E. coli* Trx and LBCA Trx.

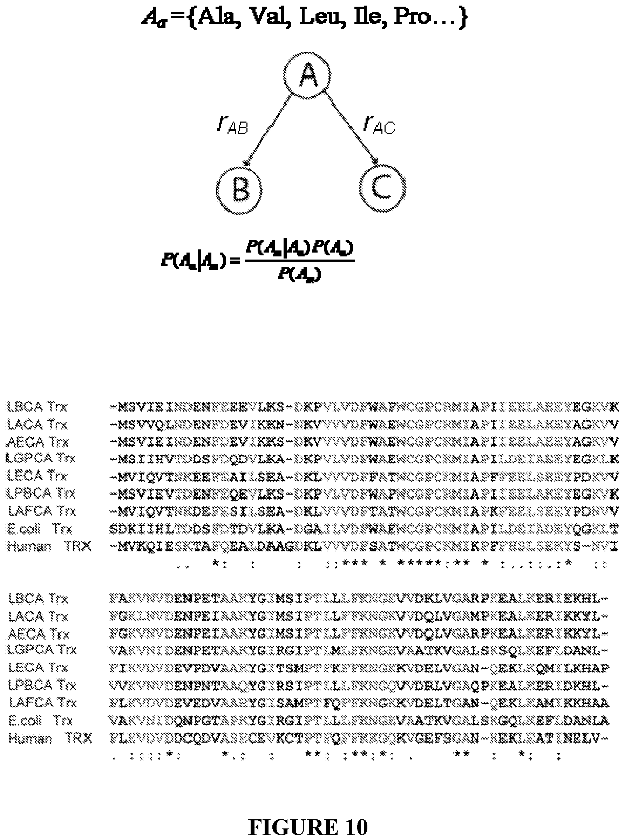

**Figure 10** M-PASs for Trx enzymes belonging to representative extinct organisms: The sequences are calculated using maximum likelihood methods. Also included are *E. coli* and human Trx sequences for comparative purposes. A high degree of conservation around the active site CGPC is observed (red residues marked with asterisks).

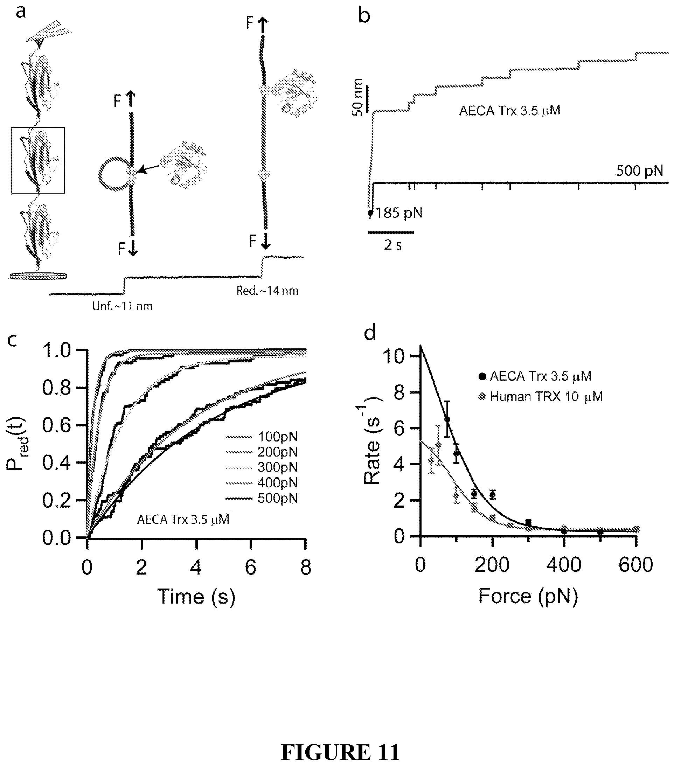

**Figure 11.** Single-molecule disulfide reduction assay. **Fig. 11A** Schematic representation of the singe-molecule disulfide reduction assay. A first pulse of force rapidly unfolds the I27$_{G32C-A75C}$ domains (Unf.). When the disulfide bond is exposed to the solvent a single Trx molecule can reduce it (Red.) **Fig. 11B** Experimental force-clamp trace showing single disulfide reductions of a (I27$_{G32C-A75C}$)$_8$ polypeptide. The unfolding pulse was set at 185 pN for 0.2 s and the test-pulse force at 500 pN. **Fig, 11C** Probability of reduction ($P_{red}$(t)) resulted from summing and normalizing the reduction test pulse at different forces for AECA Trx (3.5 μM). **Fig. 11D** Force-dependency of disulfide reduction by AECA Trx; human Trx is also shown for comparison. Both Trx enzymes show a similar pattern: a negative force-dependency of the reduction rate, from 30-200 pN, consistent with a Michaelis-Menten mechanisms and a force-independent mechanism, from 200 pN and up, described by an electron transfer reaction (Perez-Jimenez et al., Nat Struct Mol Biol 16, 890-6 (2009)). Notice the higher activity for AECA Trx (3.5 μM for AECA Trx vs. 10 μM for human TRX). The lines represent fittings to the kinetic model.

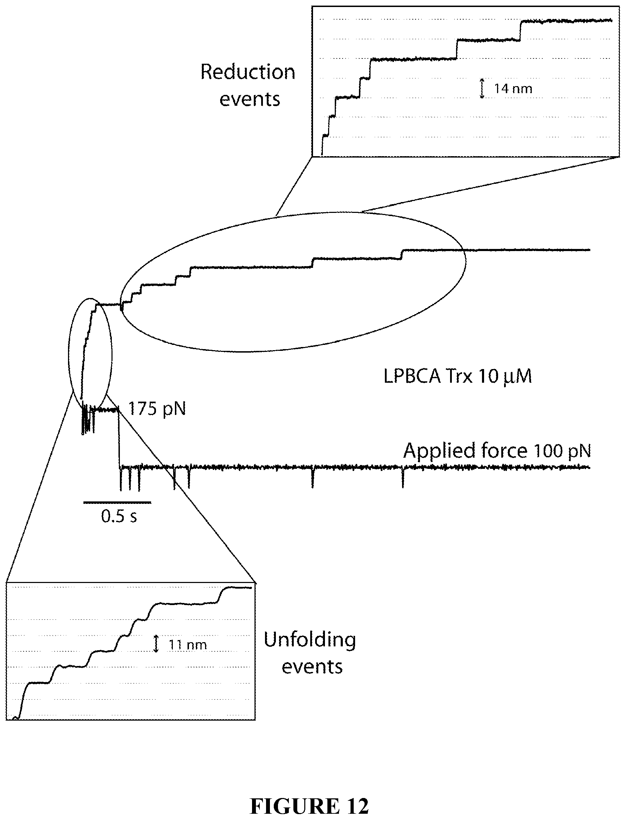

**Figure 12.** Force-clamp experiment for detection of single disulfide reduction events. A first pulse of force (175 pN, 0.3 s) unfolds the I27$_{G32C-A75C}$ domains up to the disulfide bond. The unfolding events can be monitored as a series of step of ∼11 nm per module (bottom panel). A second pulse of force (100 pN) is applied to monitor single disulfide reduction by Trx enzymes. In this case the release of the trapped residues behind the disulfide bond gives rise to a length increment of ∼14 nm per module (top panel).

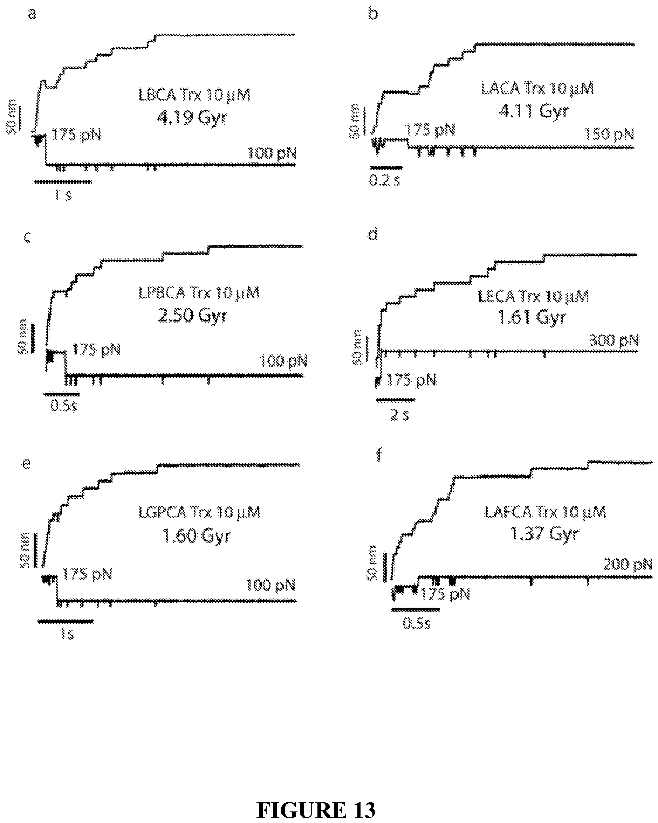

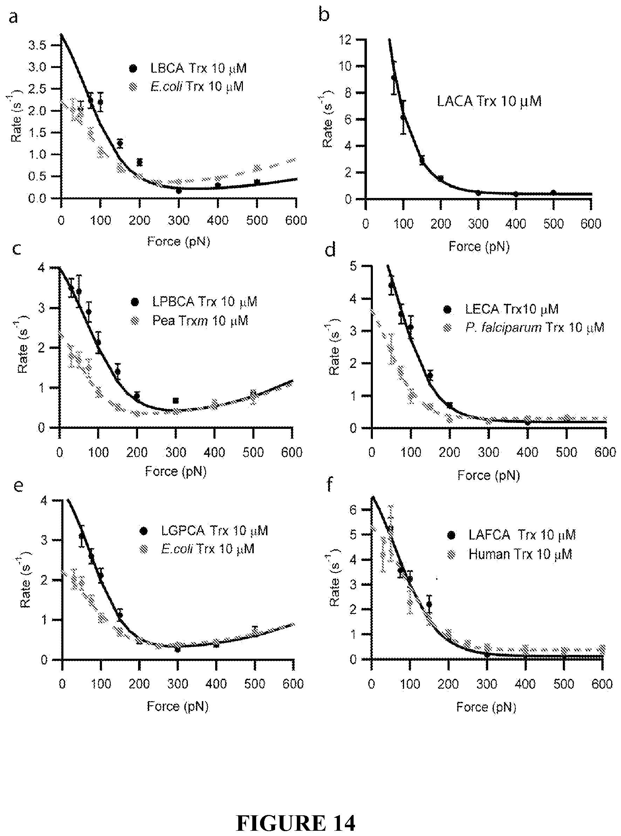

**Figure 13A-F.** Experimental traces of single disulfide reductions by ancestral Trxs. Both, the unfolding pulse (175 pN) and the test pulse at different forces are shown. Individual reduction events can be observed in the test-pulse force. Numerous traces like these (15-80) are used at every force to complete the full force-dependency of disulfide bond reduction by Trx enzymes, as shown in **Fig.14.**

**Figure 14.** Force-dependence of disulfide reduction by ancestral Trx enzymes. The reduction rate at a given force is obtained by summing, averaging and fitting to a single exponential numerous traces (15-80) like the one shown in **Fig. 11B.** The solid lines are fitting to the kinetic model. The grey circles and dashed lines represent the rate vs. force dependence for modern Trxs: Pea Trxm from chloroplast (**Fig. 14C**), *P. falciparum* Trx (**Fig. 14D**), *E. coli* Trx (**Figs. 14A** and **14E**) and Human Trx (**Fig 14F**) (all extracted from Perez-Jimenez et al. Nat Struct Mol Biol 16, 890-6 (2009)). These modern Trxs are descendants of the ancestral Trxs in the same plot.

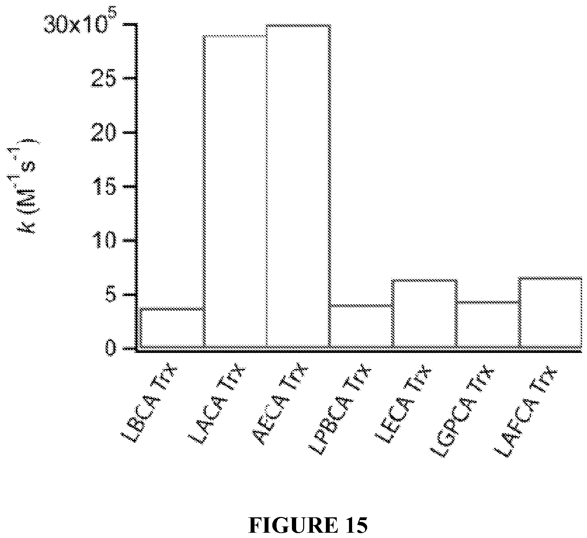

**Figure 15.** Rate constants for disulfide bond reduction by ancestral Trxs. These values are obtained by extrapolating to zero force the fitting of the reduction rate vs. force data (Fig. 8) to the three-state kinetic model described in the methods section.

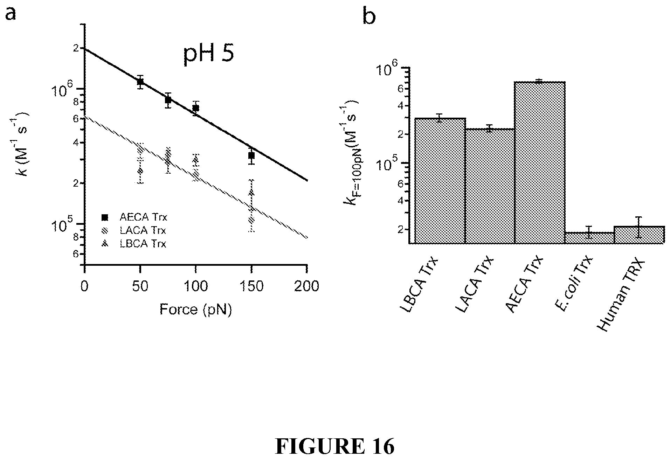

**Figure 16.** Rate constants of disulfide bond reduction at pH 5. **Fig. 16A** A high activity for AECA (black squared) and LACA (circles) Trxs can be observed at pH 5 when the substrate is pulled at low forces (50-150 pN). LBCA Trx (triangles) shows similar activity to that at pH 7.2 with a similar trend (**Fig. 14A**). The solid lines are exponential fit to the experimental data. **Fig. 16B** The rate constants for disulfide reduction by ancestral Trxs at F=100 pN are remarkably high when compared with the rate constants measured for modern Trxs, *E. coli* and human at the same force.

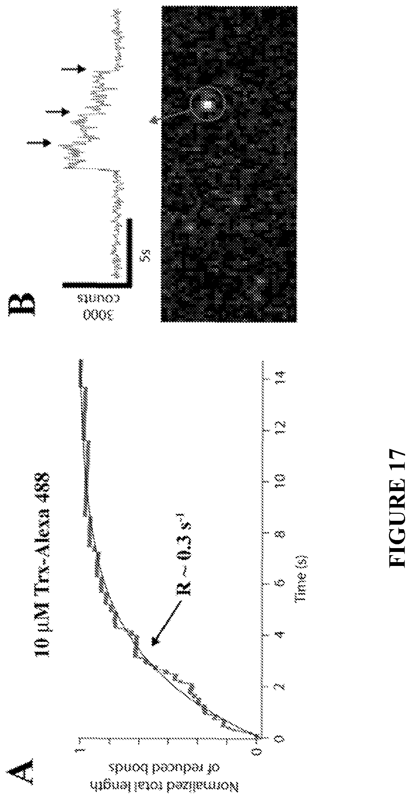

**Figure 17.** Functional assay of fluorescently labeled Trx enzymes. **Fig. 17A** Ensemble average of reduction events obtained with labeled E. coli Trx enzymes (10 μM). **Fig. 17B** TIRF image capturing a labeled enzyme entering the evanescent field. The trace shows the time course of one such visit. Stepwise bleaching events mark the multiple labels of the enzyme (arrows).

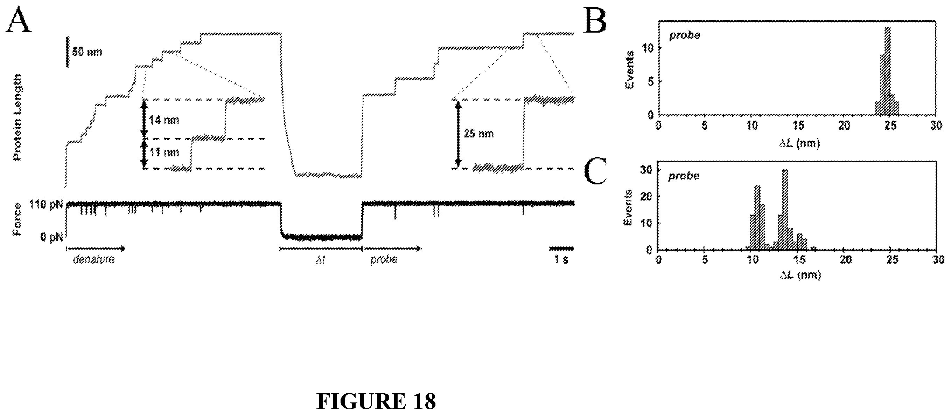

**Figure 18.** A single molecule assay for oxidative folding. **Fig. 18A** Under a *denaturing* force of 110 pN, each initial (I27$_{S-S}$)$_8$ unfolding event is measured as an 11 nm extension of the polypeptide, followed by reduction events catalyzed by human thioredoxin (10 μM wild-type hTrx), yielding additional 14 nm extensions (inset). Refolding of the fully denatured polypeptide is subsequently initiated by switching off the stretching force. After some time *Δt*, folding is stopped and the state of the substrate is *probed* by again applying a stretching force. During the probe stage we only observed 25 nm steps, indicating that while the (I27$_{S-S}$)$_8$ polypeptide had refolded, the disulfide bonds did not reoxidize. **Fig. 18B** A histogram of the step sizes observed during the probe pulse from different traces confirms the absence of reoxidized proteins. **Fig. 18C** By contrast if the exact same experiment is repeated in the presence of a mutant form of human thioredoxin (hTrx$^{C35S}$), all disulfide bonds reduced during the denature pulse, become reoxidized as demonstrated by the presence of an equal number of 11 nm and 14 nm steps during the *probe* pulse.

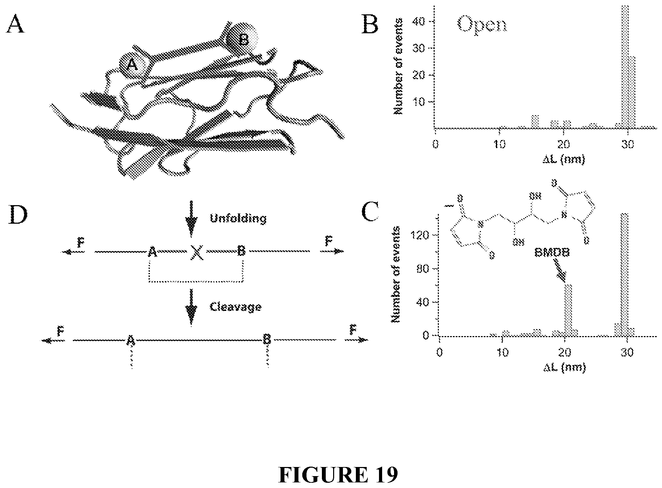

**Figure 19.** Cross-linking reaction to generate cleavable substrates. **Fig. 19A** Two distant cysteines are introduced in the I27 protein at positions A and B (positions 27 and 55). We covalently link the exposed cysteines with bifunctional molecules containing a cleavable bond (green bar). **Fig. 19B** If the I27 protein is left open, the unfolding step size is that of a full length protein with ΔL~29 nm. **Fig. 19C** If the cysteines are bridged by a bifunctional reagent (here shown with BMDB), many I27 proteins now extend by only ΔL~20 nm, limited by the covalent bridge. **Fig. 19D** Cleavage of a bridge by an enzyme will result into a further extension by ΔL~9 nm, identifying the reaction.

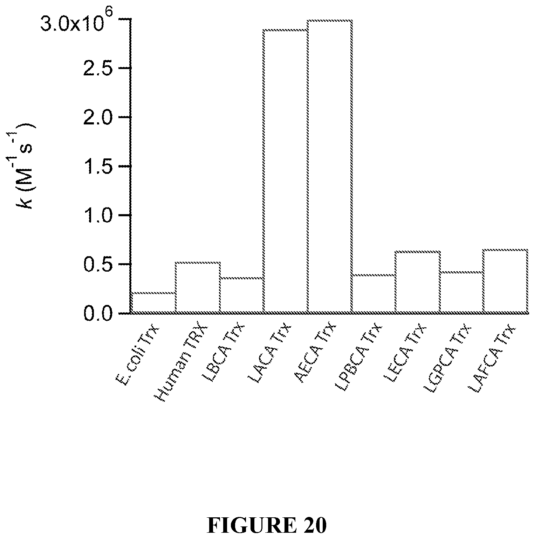

**Figure 20.** Rate constants for disulfide bond reduction by ancestral and modern Trxs enzymes. These values are obtained by extrapolating to zero force the fitting of the reduction rate vs. force data (Fig. 14) to the three-state kinetic model described herein.

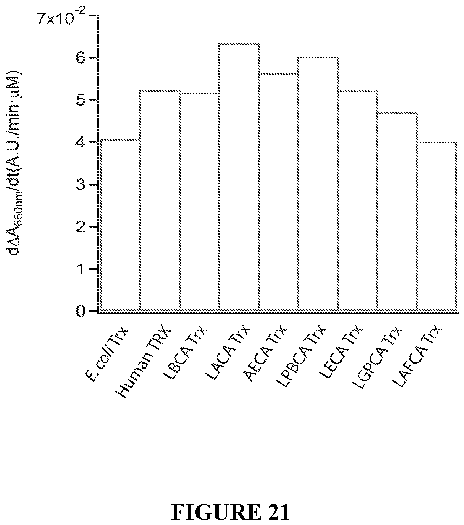

**Figure 21.** Insulin activity assay for ancestral and modern Trx enzymes. Activity determined with the turbidity insulin bulk enzymatic assay (Benner et al., Adv Enzymol Relat Areas Mol Biol 75, 1-132, xi (2007)). The turbidity assay is less sensitive in detecting differences in activity amongst the different enzymes. This assay cannot be used to probe the activity of the enzymes at pH 5 due to the precipitation of insulin at pH below 6 (Benner et al, Adv Enzymol Relat Areas Mol Biol 75, 1-132, xi (2007); Thornton, Nat Rev Genet 5, 366-75 (2004)).

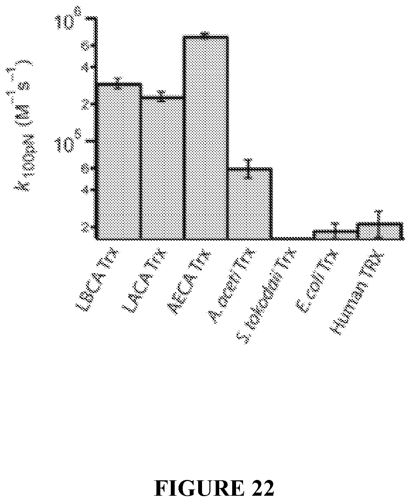

**Figure 22.** Rate constants for disulfide reduction by ancestral Trx enzymes at pH 5 are higher than for modern E. coli and human Trx. Thioredoxin from the acidophile *Acetobater aceti* shows activity at pH 5, enzymes from the thermophilic *Sulfolobus tokodaii* do not show a detectable rate of reduction at the same pH. All experiments were conducted at a pulling force of 100 pN. Error bars represent s.e.m. obtained using the bootstrap method.

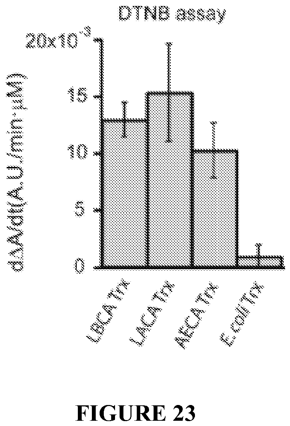

**Figure 23.** Activity of ancestral Trxs and modern *E. coli* Trx measured using DTNB as substrate at pH 5 and determined by monitoring spectrophotometrically the formation of TNB at 412 nm. Error bars represent s.d. from three different measurements.

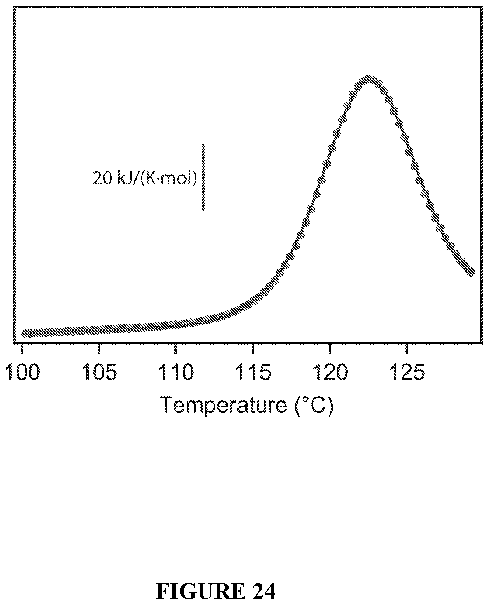

**Figure 24.** Experimental DSC thermogram for *Sulfolubus tokodaii* Trx (Archaea). The solid line represents fit to the two-state thermodynamic model (Liberies, Ancestral sequence reconstruction, xiii, 252 p. (Oxford University Press, Oxford; New York, 2007)). A $T_m$ of 122.6° C is obtained from the fit.

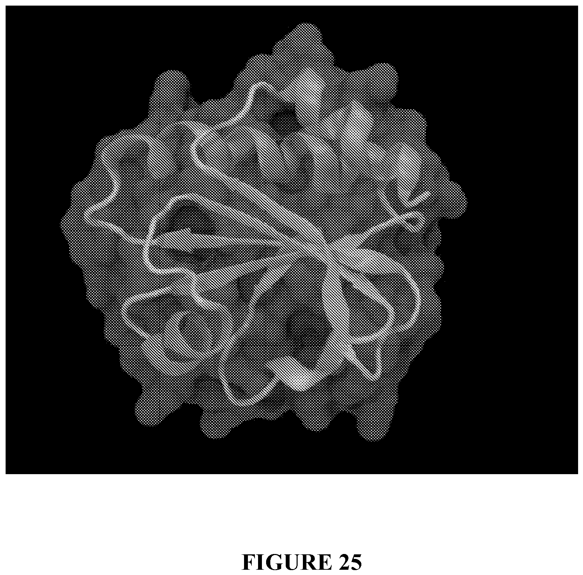

**Figure 25.** Structural representation of the ancestral enzyme thioredoxin AECA.

## DETAILED DESCRIPTION OF THE INVENTION

Industry has a large demand of pH stable and temperature polypeptides for use in a number of industrial applications. Methods to alter polypeptide pH and temperature stability without eliminating function of the polypeptide are highly needed. The methods described herein are related in part to the finding that it is possible to predict, synthesize and characterize enzymes from extinct organisms that lived on earth as long as 4 billion years ago. In certain aspects, the methods described herein are relate to the understanding that because these organisms lived on the primordial earth (i.e. in an environment that was much hotter and more acidic than today), their enzymes were necessarily optimized through selective pressure to have a higher thermal and acidic stability than their modern counterparts. In some aspects, the methods described herein are relate to the finding that because enzyme homologues exist different species, Bayesian statistics can be used to predict the ancestral gene encoding for a version of the enzyme that was present in the common ancestor of these organisms.

In certain aspects, the methods described herein can be used to substitute amino acids according to their presence in resurrected protein sequences from extinct organisms. In one embodiment, the methods described herein are useful for altering (e.g increasing) the stability of a recombinant polypeptide at low pH and/or high temperatures by making one or more conservative substitutions in the amino acid sequence of the polypeptide. In one embodiment, the methods described herein are useful for altering (e.g increasing) the activity of a recombinant polypeptide at low pH and/or high temperatures by making one or more conservative substitutions in the amino acid sequence of the polypeptide.

In certain aspects, the invention described herein relates to the finding that single molecule force-clamp spectroscopy can be used to study protein dynamics under a mechanical force. The experimental resurrection of ancestors of these universal enzymes together with the sensitivity of single-molecule techniques can be a powerful tool towards understanding the origin and evolution of life on Earth. As described herein, the force-dependency of a reaction can be a sensitive probe of substrate nanomechanics during catalysis. This type of protein spectroscopy can also be useful for obtaining details of enzyme active site dynamics. The methods described herein can also complement structural x-ray and NMR data and provide benchmarks for molecular dynamics simulations

<u>Definitions</u>

The singular forms "a," "an," and "the" include plural references unless the content clearly dictates otherwise.

As used herein, "sequence identity" means the percentage of identical nucleotide or amino acid residues at corresponding positions in two or more sequences when the sequences are aligned to maximize sequence matching, i.e., taking into account gaps and insertions. "Percent identity" in the context of two or more nucleic acids or polypeptide sequences, refers to the percentage of nucleotides or amino acids that two or more sequences or subsequences contain which are the same. A specified percentage of amino acid residues or nucleotides can be referred to such as: 60% identity, 65%, 70%, 75%, 80%, 85%, 90%, 91%, 92%, 93%, 94%, 95%, 96%, 97%, 98%, or 99% or more identity over a specified region, when compared and aligned for correspondence over a comparison window, or designated region as measured using one of the following sequence comparison algorithms or by manual alignment and visual inspection.

As used herein, the term "extant" refers to taxa (such as species, genera or families) that are still in existence (living). The term extant contrasts with extinct. As used herein, the terms "extant protein", "extant polypeptide", "extant amino acid sequence", "extant gene" and "extant nucleic acid sequence" refer to proteins, polypeptides, amino acid sequences, genes, and nucleic acid sequences from extant taxa.

Other definitions are provided throughout the specification.

A journey back in time is possible at the molecular level by resurrecting proteins from extinct organisms. Laboratory resurrection of these ancestral proteins enables exploration of aspects of ancient life that cannot be inferred from fossil records alone (Benner et al., Adv Enzymol Relat Areas Mol Biol 75, 1-132, xi (2007); Thornton, Nat Rev Genet 5, 366-75 (2004); Liberles, Ancestral sequence reconstruction, xiii, 252 p. (Oxford University Press, Oxford ; New York, 2007); Hall, Proc Natl Acad Sci U S A 103, 5431-6 (2006). Such time traveling is largely limited by the ambiguity in the historical models used for ancestral sequence inference. (Pollock and Chang, in Ancestral sequence reconstruction, pages 85-94 (ed. Liberles. D.A., Oxford University Press, Oxford ; New York, 2007); Gaucher et al., Nature 425, 285-8 (2003); Gaucher et al., Nature 451, 704-7 (2008)). For instance, uncertainties in databases, sequence alignments, failures in evolutionary theories and uncertainty in the construction of phylogenetic trees are common sources of ambiguity.

Understanding the molecular mechanisms of enzyme function presents unique challenges in biophysics. In certain aspects, the invention described herein relates to computational methods for resuscitating ancestral genes. In some embodiments, the methods described herein can be used to reconstruct the amino acid sequence of ancient proteins. Reconstructed proteins can be expressed in an expression system and, in certain applications, examined for their activity, pH stability or thermal stability (Gaucher et al. Nature, 2008. 451(7179): p. 704-U2; Gaucher et al, Nature, 2003. 425(6955): p. 285-8).

The pH and temperature stability of polypeptides can depend in part on the distribution of amino acid residues throughout the three dimensional structure of the polypeptide. In one aspect, the methods described herein are relate to findings from the resurrection of seven Precambrian thioredoxin enzymes (Trx), dating back between ~1.4 and ~4 billion years ago (Gyr). These findings relate to the evolution of enzymatic reactions of thioredoxin enzymes (Trx) from extinct organisms that lived in the Precambrian. Their mechanism of reduction was probed using single molecule force-spectroscopy which can readily distinguish simple nucleophiles from the more complex chemistry of the active site of Trx enzymes. As described herein, differential scanning calorimetry (DSC) showed that these resurrected enzymes have melting temperatures up to ~32 °C higher than those of extant Trx, following a trend with a slope of ~ 6 K/Gyr. From the force-dependency of the rate of reduction of an engineered substrate can be used to determine whether the ancient Trxs utilized chemical mechanisms of reduction similar to those of modern enzymes. As described herein, the most ancient enzymes showed high activity at low pH, where the extant Trxs became inactive under in low pH environments. The results described herein show that, while Trx enzymes have maintained their reductase chemistry unchanged, they have adapted over a 4 Gyr time span to the changes in temperature and ocean acidity that characterize the evolution of the environment from ancient to modern Earth.

The results described herein also show that the chemical mechanisms observed in modern Trx enzymes were already present in Trxs from Precambrian organisms. Ancestral Trx enzymes from LBCA, AECA and LACA that lived in the mid-to-late Hadean were highly resistant to temperature and active in relatively acidic conditions. These findings are consistent with the hypothesis that in early life Trx enzymes were present in hot environments and these environments have progressively cooled from 4 to 0.5 Gyr (Nisbet and Sleep, Nature 409, 1083-91 (2001); Gaucher et al., Nature 451, 704-7 (2008); Knauth et al., Geo. Soc. Am. Bull., 115: 566-580 (2003); Schulte, M., Oceanography 20, 42-49 (2007)). However, it is also possible that a much cooler early Earth was populated by psychrophiles, mesophiles and thermophiles and that the latter could have been the only survivors of cataclysmic events (e.g., the late heavy bombardment or global glaciations on Early Earth (Nisbet and Sleep, Nature 409, 1083-91 (2001); Gogarten-Boekels et al., Orig. Life Evol. Biosph., 25: 251-264 (1995)). Thus, these findings indicate that important biochemical pathways in the modern biosphere were already established by 3.5 Gyr ago (Nisbet and Sleep, Nature 409, 1083-91 (2001)). For instance, metabolism is one of the most conserved cellular processes. Important pathways like energy production, sugar degradation, cofactor biosynthesis or amino acids processing are highly conserved from bacteria to human and were likely present in LUCA (Peregrin- Alvarez et al, Genome Res 13, 422-7 (2003)). Thus, in some aspects, the present invention is directed to a nucleic acid, according to claim 9 herein, encoding a recombinant thioredoxin or to recombinant thioredoxin amino acid sequences, such as for example a thioredoxin polypeptide optimized to have greater stability and/or activity at high temperature and/or low pH, that has been modified to change amino acids where the one or more modified are pH optimizing or temperature optimizing modifications.

Evolution operates at multiple levels of biological organization; however, enzymatic mechanisms accompanying adaptive changes seem to be highly conserved. The ability of enzymes to maintain specific chemical reactivities and mechanisms in disparate environments is necessary for the diversification of life. While this ability is exemplified by Trx enzymes, it can also be universal to all proteins (e.g., ubiquitin, RNase, ATPase or other metabolic enzymes that have been maintained in nearly all organisms throughout the history of life). Thus, although some of compositions and methods described herein relate to the activity of resurrected thioredoxin, the paleoenzymological methods described herein can be used to generate polypeptides optimized to have greater stability and/or activity at high temperature and/or low pH. The experimental resurrection of ancestors of these universal proteins together with the sensitivity of single-molecule techniques can be a powerful tool towards understanding the origin and evolution of life on Earth.

Described are computational methods for determining ancestral sequences. Such methods can be used, for example, to determine ancestral sequences for an extant polypeptide (e.g. thioredoxin). Described are methods for increasing the stability and/or activity of a polypeptide (e.g. a thioredoxin) at low pH or at elevated temperature. Methods for determining ancestral sequences can be based on amino acid sequences or on nucleic acid sequences encoding (or predicted to encode) proteins.

In some embodiments, the computational methods described herein are based on the principle of maximum likelihood. The sequences of polypeptides used in the methods described herein can be selected on the basis of a common feature (e.g. a threshold sequence identity, common enzymatic activity, or common modular domain architecture). The methods may involve the construction of a phylogeny using an evolutionary model of the probabilities of amino acid or nucleic acid substitutions polypeptide among different organisms.

Where the sequences differ (e.g. due to mutation), the maximum likelihood methodology can be used to assigns an amino acid or nucleic acid residue to the node a phylogenetic trees (i.e., the branch point of the lineages). Generally, a model of sequence substitutions and then a maximum likelihood phylogeny can be determined for multiple data sets. The sequence at the base node of the maximum likelihood phylogeny is referred to as the ancestral sequence (or most recent common ancestor).

Described are methods for generating an ancestral polypeptide (e.g. thioredoxin) sequences through reconstruction of phylogenetic trees. The ancestral polypeptide sequence may be any polypeptide sequence which contains at least homolog in another organism.

Described is a method for increasing the temperature stability of a recombinant polypeptide produced from a nucleic acid in an expression system, the method comprising replacing one or more temperature stability decreasing amino acids of the recombinant polypeptide with one or more temperature stability increasing amino acids. Also described is a method for increasing the pH stability of a recombinant polypeptide produced from a nucleic acid in an expression system, the method comprising replacing one or more temperature pH decreasing amino acids of the recombinant polypeptide with one or more pH stability increasing amino acids.

In certain aspects, the present invention relates to the finding that it is possible to predict, synthesize and characterize polypeptides from extinct organisms. Thus, one embodiment the stability of a extant polypeptide at low pH (e.g. a pH lower than the pH at which the extant polypeptide is expressed in an organism, or the pH at which the polypeptide displays its greatest stability and/or activity) can be increased by reconstructing an ancestral polypeptide of the extant polypeptide by (a) aligning a plurality of sequences corresponding homologues of the extant polypeptide, (b) generating a phylogenetic tree of the plurality of sequences corresponding homologues of the extant polypeptide, (c) using Bayesian statistical analysis to generate inferred sequences of one or more ancestral genes encoding a version of the polypeptide that was present in a common ancestor of at least two or more organisms in the phylogenetic tree, (d) calculating posterior probabilities for all 20 amino acids in each inferred sequence, (e) generating a reconstructed ancestral polypeptide sequence by assigning to each position in the inferred sequence the amino acid residue having the highest posterior probability for that position.

Thus, one embodiment the stability of a extant polypeptide at high temperature (e.g. a temperature higher than the temperature at which the extant polypeptide is expressed in an organism, or the temperature at which the polypeptide displays its greatest stability and/or activity) can be increased by reconstructing an ancestral polypeptide of the extant polypeptide by (a) aligning a plurality of sequences corresponding homologues of the extant polypeptide, (b) generating a phylogenetic tree of the plurality of sequences corresponding homologues of the extant polypeptide, (c) using Bayesian statistical analysis to generate inferred sequences of one or more ancestral genes encoding a version of the polypeptide that was present in a common ancestor of at least two or more organisms in the phylogenetic tree, (d) calculating posterior probabilities for all 20 amino acids in each inferred sequence, (e) generating a reconstructed ancestral polypeptide sequence by assigning to each position in the inferred sequence the amino acid residue having the highest posterior probability for that position.

In another embodiment the activity of a extant polypeptide at low pH (e.g. a pH lower than the pH at which the extant polypeptide is expressed in an organism, or the pH at which the polypeptide displays its greatest stability and/or activity) can be increased by reconstructing an ancestral polypeptide of the extant polypeptide by (a) aligning a plurality of sequences corresponding homologues of the extant polypeptide, (b) generating a phylogenetic tree of the plurality of sequences corresponding homologues of the extant polypeptide, (c) using Bayesian statistical analysis to generate inferred sequences of one or more ancestral genes encoding a version of the polypeptide that was present in a common ancestor of at least two or more organisms in the phylogenetic tree, (d) calculating posterior probabilities for all 20 amino acids in each inferred sequence, (e) generating a reconstructed ancestral polypeptide sequence by assigning to each position in the inferred sequence the amino acid residue having the highest posterior probability for that position.

In another embodiment the activity of a extant polypeptide at high temperature (e.g. a temperature higher than the temperature at which the extant polypeptide is expressed in an organism, or the temperature at which the polypeptide displays its greatest stability and/or activity) can be increased by reconstructing an ancestral polypeptide of the extant polypeptide by (a) aligning a plurality of sequences corresponding homologues of the extant polypeptide, (b) generating a phylogenetic tree of the plurality of sequences corresponding homologues of the extant polypeptide, (c) using Bayesian statistical analysis to generate inferred sequences of one or more ancestral genes encoding a version of the polypeptide that was present in a common ancestor of at least two or more organisms in the phylogenetic tree, (d) calculating posterior probabilities for all 20 amino acids in each inferred sequence, (e) generating a reconstructed ancestral polypeptide sequence by assigning to each position in the inferred sequence the amino acid residue having the highest posterior probability for that position.

In another embodiment the melting temperature of a extant polypeptide can be increased by reconstructing an ancestral polypeptide of the extant polypeptide by (a) aligning a plurality of sequences corresponding homologues of the extant polypeptide, (b) generating a phylogenetic tree of the plurality of sequences corresponding homologues of the extant polypeptide, (c) using Bayesian statistical analysis to generate inferred sequences of one or more ancestral genes encoding a version of the polypeptide that was present in a common ancestor of at least two or more organisms in the phylogenetic tree, (d) calculating posterior probabilities for all 20 amino acids in each inferred sequence, (e) generating a reconstructed ancestral polypeptide sequence by assigning to each position in the inferred sequence the amino acid residue having the highest posterior probability for that position.

In one embodiment, the sequence of a reconstructed protein can be generated by contracting a phylogenetic tree from a plurality of extant (modern) sequences of the enzyme to be reconstructed. The phylogenetic tree can be used to predict the sequences corresponding to every node of the tree. In one embodiment, the enzyme to be reconstructed can be a thioredoxin enzyme and the extant enzymes of a plurality of extant thioredoxin enzymes can be used to construct a phylogenetic tree and predict the sequences of every node of the tree.

Generally, polypeptide sequences corresponding homologues of the extant polypeptide can be obtained from publicly available databases (e.g., GenBank). Sequence comparison and alignment can be performed according to different analytical parameters. For example, in some cases, one sequence can be used are a reference against which all other sequences are compared. In the case of sequence comparison algorithms, test and reference sequences can be input into a computer and sequence algorithm program parameters can be designate for analysis. Alignment of the sequences can be performed using any method, algorithm or program known in the art. Examples of suitable alignment programs include, but are not limited to, MUSCLE (Edgar, Nucleic Acids Res 32, 1792-7 (2004)), Clustal W, the BioEdit program available from North Carolina State University (available at http://www mbio.ncsu.edu/BioEdit/bioedit.html), and the SegEd program.

The terms "homologous" or "homologue" refer to related sequences that share a common ancestor or arise from gene duplication and are determined based on degree of sequence identity. Alternatively, a related sequence may be a sequence having homology, which has arisen by convergent evolution. These terms describe the relationship between a gene found in one species, subspecies, variety, cultivar or strain and the corresponding or equivalent gene in another species, subspecies, variety, cultivar or strain or, in the case of paralogous genes, two related sequences within a species, subspecies, variety, cultivar or strain. "Homologous sequences" are thought, believed, or known to be functionally related. A functional relationship may be indicated in a number of ways, including, but not limited to: (a) the degree of sequence identity; and/or (b) the same or similar biological function. Homology can be determined using software programs readily available in the art, such as those discussed in Current Protocols in Molecular Biology (F. M. Ausubel et al., eds., 1987).

The term "homolog" is also used to refer to proteins with amino acid sequences sharing at least about 60%, 70%, 80%, 90% or more identity with the amino acid sequences of an ancestral protein, such as the ancestral Trx proteins described herein. The term "homolog" is also used to refer to gene sequences with nucleic acid sequences sharing at least about 60%, 70%, 80%, 90% or more identity with nucleic acid sequences capable of encoding an ancestral protein, such as the ancestral Trx proteins described herein.

In certain embodiments of the methods described herein, the sequences and/or sequence alignments can be further subjected to manual correction. Other suitable alignment algorithms include, but are not limited to the local homology algorithm of Smith and Waterman (Adv. Appl. Math. 2:482 (1981)), by the homology alignment algorithm of Needleman and Wunsch (J. Mol. Biol. 48:443 (1970)), by the search for identity method of Pearson and Lipman (Proc. Natl. Acad. Sci. USA 85:2444 (1988)), by the progressive alignment method of Feng and Doolittle (J. Mol. Evol. 35:351-60 (1987)) (e.g. PILUP), by the CLUSTAL method described by Higgins and Sharp (Gene 73:237-44 (1988); CABIOS 5:151-53 (1989)), by computerized implementations of these algorithms (GAP, BESTFIT, FASTA, and TFASTA in the Wisconsin Genetics Software Package, Genetics Computer Group, 575 Science Dr., Madison, Wis.), or by visual inspection (see, generally Ausubel et al., Current Protocols in Molecular Biology, John Wiley and Sons, New York (1996)). Analysis of the percent sequence identity between the test sequence(s) and the reference sequence can be performed on the basis of designated program parameters. For example, a reference sequence can be compared to other test sequences to determine the percent sequence identity relationship using the following parameters different gap weights, different gap length weights, and weighted end gaps. Appropriate parameters can be identified by one skilled in the art. In some embodiments, the number of sequences can also be reduced by treating conservative substitutions occupying a position in a sequence as being identical to a single residue occupying that position. The choice of residue representing the members of one or more conservative substitution groups may be selected based on the physio-chemical properties of the amino acid, the frequency of occurrence in the sequence alignment or any other criteria known in the art.

A "conservative substitution," when describing a protein, refers to a change in the amino acid composition of the protein that is less likely to substantially alter the protein's activity. Thus, "conservatively modified variations" of a particular amino acid sequence refers to amino acid substitutions of those amino acids that are less likely to be critical for protein activity or substitution of amino acids with other amino acids having similar properties (e.g., acidic, basic, positively or negatively charged, polar or non-polar, etc.) such that the substitutions of even critical amino acids do not substantially alter activity. Conservative substitution tables providing amino acids that are often functionally similar are well known in the art (see, e.g., Creighton, Proteins, W. H. Freeman and Company (1984)). Conservative amino acid substitutions can be made at one or more non-essential amino acid residues. A conservative amino acid substitution can be a substitution in which an amino acid residue is replaced with an amino acid residue having a similar side chain. Families of amino acid residues having similar side chains have been defined in the art, including basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine), aromatic side chains (e.g., tyrosine, phenylalanine, tryptophan, histidine), aliphatic side chains (e.g., glycine, alanine, valine, leucine, isoleucine), and sulfur-containing side chains (methionine, cysteine). Substitutions can also be made between acidic amino acids and their respective amides (e.g., asparagine and aspartic acid, or glutamine and glutamic acid).

Conservative amino acid substitutions can be utilized in making variants of the Trx enzymes described herein. For example, replacement of a leucine with an isoleucine or valine, an aspartate with a glutamate, a threonine with a serine, or a similar replacement of an amino acid with a structurally related amino acid, may not have a major effect on the properties of the resulting polypeptide or fusion polypeptide. Whether an amino acid change results in a functional polypeptide or fusion polypeptide can readily be determined by assaying the specific activity of the polypeptide or fusion polypeptide.

One skilled in the art will also be able to remove sequences below a particular size cut-off, subject the sequences to split decomposition analysis to remove any phylogenetic noise. A phylogenetic tree can then be constructed by heuristic search using a maximum likelihood (ML) approach. In one embodiment, one or more phylogenetic trees can be generated a suitable program known in the art. Examples of suitable programs include, but are not limited to PAUP (e.g. PAUP 4.0 beta) and PHYML. In one embodiment, the phylogenetic analysis and the phylogenetic tree can be performed using PAUP by the minimum evolution distance criterion with 1000 bootstrap replicates. Once phylogenetic trees are generated, one skilled in the art will appreciate that such tree can be rooted according to different parameters. In certain embodiments, the phylogenetic tree can be used to predict the sequences corresponding to every node of the tree. Parameters suitable for use with the methods described herein include, but are not limited to, strict or relaxed molecular clock model (Lai, Microbiol. Rev., 56:61-79, 1992; Lee et al., J. Virol., 73:11-18, 1999), non-reversible models of substitution, midpoint rooting, and/or outgroup criterion (Gao et al., J. Virol., 79:1154-1163, 2005; Higgins and Sharp, Gene, 73:237-244, 1988; Lai, Microbiol. Rev., 56:61-79, 1992; Lee et al., J. Virol., 73:11-18, 1999; Logvinoff et al., Proc. Natl. Acad. Sci. USA, 101:10149-10154, 2004; Mink et al., Virology, 200:246-255, 1994). The rooted tree can then be used as a template to simulate an *ancestral* sequence. Simulation of *ancestral* sequences at internal nodes as well as at common ancestor can be inferred using a reconstruction program using Bayesian statistical analysis. An exemplary reconstruction program for Bayesian statistical analysis is PAML (e.g. PAML version 3.14). In one embodiment, the Bayesian statistical analysis is performed using PAML and the gamma distribution for variable replacement rates across sites is incorporated (Yang, Comput Appl Biosci 13, 555-556 (1997)). In another embodiment, the Bayesian statistical analysis is performed using MrBayes (mrbayes csit.fsu.edu). For each site of the inferred sequences, posterior probabilities can be calculated for all 20 amino acids and the amino acid residue with the highest posterior probability can be assigned at each site of an inferred sequence.

Sequences corresponding homologues of the recombinant polypeptide can be nucleic acid sequences, amino acid sequences, confirmed sequences, predicted sequences or hypothetical sequences. Where conversion of nucleic acid sequences to amino acid sequences is required (e.g. for alignment purposes), one skilled in the art will readily be able to convert the nucleic acid sequences to amino acid sequences using appropriate codon translation tables and/or algorithms for identifying protein coding regions in nucleic acids. In certain embodiments, the sequences corresponding homologues of the recombinant polypeptide can be selected such that at least one sequence is from an organism of the archaea domain, at least one sequence is from an organism of the bacteria domain and at least one sequence is from an organism of the eukarya domain.

Phylogenetically related sequences may be divided according to any criteria known to a person of skill in the art. Exemplary subdivisions include, but are not limited to subdivisions according to phylogenetic distance, function, motif organization, or the like.

The methods can be performed using a computer. In one embodiment, the invention involves the use of a computer system which is adapted to allow input of one or more sequences and which includes computer code for performing one or more of the steps of the various methods described herein. For example, described is a computer program that includes code for performing one or more of generating protein sequences, generating gene sequences, aligning gene or polypeptide sequences, generating phylogenetic relationships, performing maximum likelihood and/or Bayesian statistical analysis and for computing any of the methods described herein sequentially or simultaneously.

The computer systems can comprise a means for inputting data such as the sequence of proteins, a processor for performing the various calculations described herein, and a means for outputting or displaying the result of the calculations.

One of skill in the art can readily create computer code for executing the methods of the invention, using any suitable computer code language or system known in the art, such as "C" for example.

Thioredoxins belong to a broad family of oxidoreductase enzymes ubiquitous in all living organisms (Holmgren, Thioredoxin. Annu Rev Biochem 54, 237-71 (1985)). In one aspect, the methods described herein relate to the evolution of thioredoxin (Trx) enzymes. In certain aspects, the methods and compositions described herein relate to the finding that the chemical mechanisms of reduction by thioredoxin enzymes have evolved over time and where the earliest forms thioredoxin enzymes had capabilities that were only comparable to those of simple reducing agents like glutathione or cysteine (Figure IE) (Ainavarapu et al., Journal of the American Chemical Society, 2008. 130(20): p. 6479-6487). Such evolutionary pressures can have driven the enzymes towards developing unique and efficient mechanisms of reduction (Wiita, A.P., et al., Nature, 2007. 450(7166): p. 124-7).

The archetypical active site (CXXC) and the Trx fold are well conserved throughout evolution, indicating that Trxs enzymes were present in primitive forms of life. By using single molecule force-clamp spectroscopy the chemical mechanisms of disulfide reduction by Trx enzymes can be examined in detail at the sub-Angstrom scale (Wiita et al., Nature 450, 124-7 (2007); Perez-Jimenez et al., Nat Struct Mol Biol 16, 890-6 (2009)). Hence, the combination of single-molecule force spectroscopy and the resurrection of ancestral proteins can reveal novel insights into the reductase activity of these sulfur-based enzymes. Thioredoxin (Trx) enzymes reduce disulfide bonds in a myriad of target proteins in both intracellular and extracellular compartments (Arner and Holmgren, Eur J Biochem, 2000. 267(20): p. 6102-9; Kumar et al., Proc Natl Acad Sci USA, 2004. 101(11): p. 3759-64; Powis and Montfort, Annu Rev Biophys Biomol Struct, 2001. 30: p. 421-55). In addition to its role as an important cellular antioxidant, the reduction of disulfide bonds by Trx can activate signaling cascades by triggering conformational changes in transcription factors (e.g. NF-κB) (Lillig and Holmgren, Antioxid Redox Signal, 2007. 9(1): p. 25-47) or ion channel activation (Xu et al., TRPC channel activation by extracellular thioredoxin. Nature, 2008. 451(7174): p. 69-72). Trx plays essential roles in the life cycle of viruses (Holmgren, A., Thioredoxin and glutaredoxin systems. J Biol Chem, 1989. 264(24): p. 13963-6) and can be an activator of viral entry into cells. Trx catalyzes the reduction of disulfide bonds in the second domain of the extracellular receptor CD4 as an important step in HIV entry into cells (Matthias, et al., Nat Immunol, 2002. 3(8): p. 727-32; Matthias and Hogg, Antioxid Redox Signal, 2003. 5(1): p. 133-8). Trx is also involved in DNA replication and repair by keeping the essential enzyme ribonucleotide reductase in its reduced state (Avval and Holmgren, J Biol Chem, 2009. 284(13): p. 8233-40). Trx enzymes share a highly conserved amino acid motif, Cys-X-X-Cys, in their active sites as well as a characteristic structural motif called the Trx fold (Figure 2). There are over 5,000 known DNA sequences that contain this motif and are classified as Trxs by Pfam database (http:// pfam.sanger.ac.uk/).

Thioredoxin enzymes have structural features that help positioning the participating sulfur atoms, such that an attack through an $S_N2$ reaction is favored, resulting in disulfide bond reduction. An important structural feature in the Trx family of enzymes is the presence of a hydrophobic binding groove that abuts the active site of the enzyme (Figure 2A).

The mode of action of Trx catalysis occurs through two conserved cysteine residues of the active site which play complementary roles during the reduction of a target disulfide bond. First, the catalytic Cys32 attacks the target disulfide bond resulting in a mixed disulfide between the enzyme and the substrate. Catalysis is resolved by a subsequent nucleophilic attack by Cys35 (Carvalho, et al., J Phys Chem B, 2008. 112(8): p. 2511-23; Chivers and Raines, Biochemistry, 1997. 36(50): p. 15810-6). After this cycle, the two cysteines in the active site are disulfide bonded and the enzyme is rendered inactive. Another enzyme called Trx reductase (TrxR) draws electrons from NADPH to reduce and reactivate Trx, allowing this cycle to be repeated indefinitely (Williams et al., Eur J Biochem, 2000. 267(20): p. 6110-7; Mustacich, Powis, Biochem J, 2000. 346 Pt 1: p. 1-8). The catalytic activity of Trx enzymes relies on an active cysteine thiolate (Figure 2; Cys32) that reduces target disulfide bonds by acting as a potent nucleophile.

A structural feature of thioredoxin enzymes is a polypeptide binding groove adjacent to the active site of the enzyme. The groove also serves to orient the substrate with respect to the catalytic cysteine, creating signatures that can be detected by force-clamp spectroscopy. The target binds into the binding groove and the target is then reduced by the exposed thiol of the catalytic cysteine. At least four different types of force-dependent reactions can be distinguished. As described herein, a variety of extant and ancient thioredoxins with different groove characteristics, like depth and width, can be used to examine how groove characteristics determine the force-dependency of the reaction. In certain embodiments, the methods described herein can be used to identify groove-free forms of thioredoxin by using evolutionary trees to resuscitate ancient forms of the enzyme and study their catalytic mechanisms. As described herein, molecular dynamics simulations can be used to examine the relationship between the groove characteristics and the mechanisms observed.

A fundamental step in the evolution of thioredoxin chemistry may have been the formation of this binding groove. Thus, by resurrecting ancient forms of thioredoxins, the methods described herein can be used to identify early versions of these enzymes where groove binding was either absent or shallow and poorly evolved (Figure 4C). Such findings can be used to establish a detailed correlate between the binding groove and the observed force-dependent catalysis.

Several structural features of the binding groove can be directly measured from X-ray or NMR structures of Trx enzymes and by correlating them with observed chemical mechanisms of action. For example, structural axes can be defined to measure the depth and width of the binding groove in the region surrounding the catalytic cysteine (Figure 3A) (Perez-Jimenez, et al., Nature Structural & Molecular Biology, 2009. 16(8): p. 890-U120). Figure 3B shows the depth of the groove of three Eukaryotic Trx: spinach Trxf (PDB code:1f9m) (Capitani et al., J Mol Biol, 2000. 302: p. 135-154), human Trx (1mdi) (Qin et al., Structure, 1995. 3: p. 289-297), A. thaliana Trxh1 (1xfl) (Peterson et al., Protein Sci., 2005. 14: p. 2195-2200); and three bacterial-origin Trx: human Trx2 (1uvz) (Smeets et al., Protein Sci., 2005. 14: p. 2610-2621), C. reinhardtii Trxm (1dby) (Lancelin et al., Proteins 2000. 41: p. 334-349), and E. coli Trx (2trx) (Katti et al., J Mol Biol, 1990. 212(1): p. 167-84). A difference in the structural characteristics of the groove is apparent between these selected prokaryotic and eukaryotic Trx enzymes. Trx enzymes with deeper grooves may limit the mobility of the substrate, and thereby restrict the type of chemical mechanisms available for reduction of the substrate, resulting in different force dependencies of catalysis (Figure 3C).

The binding groove becomes evident by studying mixed disulfide complexes between a mutant form of Trx lacking C35 and disulfide bonded target such as Nf-kB and Ref-1 derived polypeptides (Figure 2B) (Qin et al., Structure, 1995. 3: p. 289-297; Qin et al., Structure 1996. 4: p. 613-620). The enzyme can be prevented from resolving the mixed disulfide stage by mutating C35 and the substrate gets trapped in the groove, disulfide bonded to the catalytic cysteine. The structure of such mixed disulfide complexes indicates that both van der Waals contacts and specific intermolecular hydrogen bonds play roles in the recognition and binding of substrates in the Trx groove (Maeda et al. Structure, 2006. 14(11): p. 1701-10).

As described herein, ancient thioredoxin enzymes can be reconstructed that are functional and show greatly altered properties. Further, as described herein, Trx enzymes from different kingdoms can be reconstructed to identify thioredoxin enzymes showing unique features in their force-dependent rate of catalysis. Such findings can be related to their binding groove. Many x-ray structures of Trx enzymes are known (e.g. PDB: 1ZZY, 2FCH, 2FD3, etc). Similarly, x-ray structures of resurrected enzymes can also be resolved (e.g. LBCA; Figure 4) and the characteristics of the groove can be correlated with observed force-dependent catalysis data. The methods described herein can also be used to develop detailed molecular models for the substrate-enzyme interactions for the thioredoxin family. These models can be tested by completing molecular dynamic simulations of the studied enzyme-substrate complexes (Wiita, A.P., et al, Nature, 2007. 450(7166): p. 124-7). Such analysis can be used to gain information about the mobility of the substrate disulfide related to the different chemical mechanisms (Perez- Jimenez, et al., Nature Structural & Molecular Biology, 2009. 16(8): p. 890-U120).

Described are Trx ancestral proteins having the Trx amino acid sequence of SEQ ID NO: 1-7. Such ancestor proteins include, for example, full- length protein, polypeptides, fragments, derivatives and analogs thereof. Also described are amino acid sequences of ancestor proteins in SEQ ID NOs: 1-7. In some embodiments, the ancestor protein is functionally active.

Also described is a last bacterial common ancestor (LBCA) Trx amino acid having the sequence

MSVIEINDENFEEEVLKSDKPVLVDFWAPWCGPCRMIAPIIEELAEEYEGKVKFAKVN VDENPETAAKYGIMSIPTLLLFKNGEVVDKLVGARPKEALKERIEKHL (SEQ ID NO: 1).

Also described is a last archaeal common ancestor (LACA) Trx amino acid having the sequence

MSVVQLNDENFDEVIKKNNKVVVVDFWAEWCGPCRMIAPIIEELAKEYAGKVVFGK LNVDENPETAAKYGIMSIPTLLFFKNGKVVDQLVGAMPKEALKERIKKYL (SEQ ID NO: 2).

Also described is an archaeal/eukaryotic common ancestor (AECA) Trx amino acid having the sequence

MSVIEINDENFDEVIKSDKVVVVDFWAEWCGPCRMIAPIIEELAEEYAGKVVFGKV NVDENPEIAAKYGIMSIPTLLFFKNGKVVDQLVGARPKEALKERIKKYL (SEQ ID NO: 3).

Also described is a last eukaryotic common ancestor (LECA) Trx amino acid having the sequence

MVIQVTNKEEFEAILSEADKLVVVDFFATWCGPCKMIAPFFEELSEEYPDKVVFIKVD VDEVPDVAAKYGITSMPTFKFFKNGKKVDELVGANQEKLKQMILKHAP (SEQ ID NO: 4).

Also described is a last common ancestor of cyanobacterial and deinococcus/thermus groups (LPBCA) Trx amino acid having the sequence

MSVIEVTDENFEQEVLKSDKPVLVDFWAPWCGPCRMIAPIIEELAKEYEGKVKVVKV NVDENPNTAAQYGIRSIPTLLLFKNGQVVDRLVGAQPKEALKERIDKHL (SEQ ID NO: 5).

Also described is the last common ancestor of γ-proteobacteria, -1.61 Gyr old (LGPCA) Trx amino acid having the sequence

MSIIHVTDDSFDQDVLKADKPVLVDFWAEWCGPCKMIAPILDEIAEEYEGKLKVAKV NIDENPETAAKYGIRGIPTLMLFKNGEVAATKVGALSKSQLKEFLDANL (SEQ ID NO: 6).

Also described is the last common ancestor of animals and fungi (LAFCA) Trx amino acid having the sequence

MVIQVTNKDEFESILSEADKLVVVDFTATWCGPCKMIAPKFEELSEEYPDNVVFLKV DVDEVEDVAAEYGISAMPTFQFFKNGKKVDELTGANQEKLKAMIKKHAA (SEQ ID NO: 7).

A specific disclosure relates to an ancestor protein, fragment, derivative or analog that can be bound by an antibody. Such ancestor proteins, fragments, derivatives or analogs can be tested for the desired immunogenicity by procedures known in the art. (See e.g., Harlow and Lane).

Described is a polypeptide which consists of or comprises a fragment that has at least 10 contiguous amino acids of the Trx amino acid sequence as provided in SEQ ID NO: 1. In other embodiments, the fragment comprises at least 20 or 50 contiguous amino acids of the Trx amino acid sequence as provided in SEQ ID NO: 1. Disclosed variants of SEQ ID NO: 1 include but are not limited to polypeptide sequences having at least from about 75.1% to about 80% identity to that of SEQ ID NO: 1. Disclosed variants of SEQ ID NO: 1 include but are not limited to polypeptide sequences having at least from about 80.1% to about 85% identity to that of SEQ ID NO: 1. Disclosed variants of SEQ ID NO: 1 include but are not limited to polypeptide sequences having at least from about 85.1% to about 90% identity to that of SEQ ID NO: 1. Disclosed variant of SEQ ID NO: 1 include but are not limited to polypeptide sequences having at least from about 90.1% to about 95% identity to that of SEQ ID NO: 1. Disclosed variants of SEQ ID NO: 1 include but are not limited to polypeptide sequences having at least from about 95.1% to about 97% identity to that of SEQ ID NO: 1. Disclosed variant of SEQ ID NO: 1 include but are not limited to polypeptide sequences having at least from about 97.1% to about 99% identity to that of SEQ ID NO: 1.

In certain aspects, the invention is directed to a Trx amino acid sequence as provided in SEQ ID NO: 1. In another embodiment of the above aspect of the invention, the nucleic acid comprises consecutive nucleotides having a sequence substantially identical to SEQ ID NO: 1.

In certain aspects, the invention is directed to an isolated nucleic acid encoding, or capable of encoding, a Trx amino acid sequence as provided in SEQ ID NO: 1. In certain aspects, the invention is directed to an isolated nucleic acid complementary to an isolated nucleic acid encoding, or capable of encoding, Trx amino acid sequences as provided in SEQ ID NO: 1.

In certain aspects, the invention is directed to isolated amino acid sequence variants of SEQ ID NO: 1. Variants of SEQ ID NO: 1 include, amino acid sequences having at least from about 75.1% to about 80% identity to that of SEQ ID NO: 1. Variants of SEQ ID NO: 1 include, amino acid sequences having at least from about 80.1% to about 85% identity to that of SEQ ID NO: 1. Variants of SEQ ID NO: 1 include, amino acid sequences having at least from about 85.1% to about 90% identity to that of SEQ ID NO: 1. Variants of SEQ ID NO: 1 include, amino acid sequences having at least from about 90.1% to about 95% identity to that of SEQ ID NO: 1. Variants of SEQ ID NO: 1 include, amino acid sequences having at least from about 95.1% to about 97% identity to that of SEQ ID NO: 1. Variants of SEQ ID NO: 1 include, amino acid sequences having at least from about 97.1% to about 99% identity to that of SEQ ID NO: 1.

Described is a polypeptide sequence comprising from about 10 to about 50 consecutive amino acids from SEQ ID NO: 1. Also described is a polypeptide sequence comprising from about 10 to about 15 consecutive amino acids from SEQ ID NO: 1. Also described is a polypeptide sequence comprising from about 10 to about 20 consecutive amino acids from SEQ ID NO: 1. Also described is a polypeptide sequence comprising from about 10 to about 25 consecutive amino acids from SEQ ID NO: 1. Also described is a polypeptide sequence comprising from about 10 to about 30 consecutive amino acids from SEQ ID NO: 1. Also described is a polypeptide sequence comprising from about 10 to about 35 consecutive amino acids from SEQ ID NO: 1. Also described is a polypeptide sequence comprising from about 10 to about 40 consecutive amino acids from SEQ ID NO: 1. Also described is a polypeptide sequence comprising from about 10 to about 45 consecutive amino acids from SEQ ID NO: 1. Also described is a polypeptide sequence comprising from about 10 to about 50 consecutive amino acids from SEQ ID NO: 1. Also described is a polypeptide sequence comprising from about 10 to about 55 consecutive amino acids from SEQ ID NO: 1.

Also described is a polypeptide sequence comprising from about 10 to about 60 consecutive amino acids from SEQ ID NO: 1. Also described is a polypeptide sequence comprising from about 10 to about 65 consecutive amino acids from SEQ ID NO: 1. Also described is a polypeptide sequence comprising from about 10 to about 70 consecutive amino acids from SEQ ID NO: 1.

Described is an isolated nucleic acid sequence comprising from about 10 to about 50 consecutive nucelotides of a nucleic acid encoding, or capable of encoding SEQ ID NO: 1. Described is an isolated nucleic acid sequence comprising from about 10 to about 100 consecutive nucleotides of a nucleic acid encoding, or capable of encoding SEQ ID NO: 1.Described is an isolated nucleic acid sequence comprising from about 10 to about 200 consecutive nucleotides of a nucleic acid encoding, or capable of encoding SEQ ID NO: 1. Described is an isolated nucleic acid sequence comprising from about 10 to about 300 consecutive nucleotides of a nucleic acid encoding, or capable of encoding SEQ ID NO: 1Described is an isolated nucleic acid sequence comprising from about 10 to about 320 consecutive nucleotides of a nucleic acid encoding, or capable of encoding SEQ ID NO: 1.

Also described are isolated nucleic acid sequences such as primers and probes, comprising nucleic acid sequences derived from a nucleic acid encoding, or capable of encoding SEQ ID NO: 1. The isolated nucleic acids which can be used as a primer and/probes are sufficient length to allow hybridization with, i.e. formation of duplex with a corresponding target nucleic acid sequence, or a nucleic acid encoding, or capable of encoding SEQ ID NO: 1, or a variant thereof.

Described is an isolated nucleic acid sequence comprising from about 10 to about 50 consecutive nucleotides of a nucleic acid encoding, or capable of encoding SEQ ID NO: 1. Described is an isolated nucleic acid sequence comprising from about 10 to about 100 consecutive nucleotides of a nucleic acid encoding, or capable of encoding SEQ ID NO: 1. Described is an isolated nucleic acid sequence comprising from about 10 to about 200 consecutive nucleotides of a nucleic acid encoding, or capable of encoding SEQ ID NO: 1. Described is an isolated nucleic acid sequence comprising from about 10 to about 300 consecutive nucleotides of a nucleic acid encoding, or capable of encoding SEQ ID NO: 1. Described is an isolated nucleic acid sequence comprising from about 10 to about 320 consecutive nucleotides of a nucleic acid encoding, or capable of encoding SEQ ID NO: 1.

Also described are isolated nucleic acid sequences such as primers and probes, comprising nucleic acid sequences derived from of a nucleic acid encoding, or capable of encoding SEQ ID NO: 1. The isolated nucleic acids which can be used as primer and/probes are of sufficient length to allow hybridization with, i.e. formation of duplex with a corresponding target nucleic acid sequence, or a nucleic acid encoding, or capable of encoding SEQ ID NO: 1, or a variant thereof.

The ancestral polypeptides described herein can be produced in a host expression system. Exemplary host expression systems include but not limited to, eukaryotic expression systems, prokaryotic expression systems, plant expression systems, animal expression systems, bacterial expression systems, yeast cell expression systems, insect cell expression systems, mammalian cell expression systems, primate cell expression systems, human cell expression systems, hamster cell expression systems, mouse cell expression systems, goat cell expression systems, sheep cell expression systems, bird cell expression systems, chicken cell expression systems, and the like. The host expression system may also be any cell line suitable for recombinant protein expression, including, but not limited to, Chinese hamster ovary (CHO) cells, mouse myeloma NS0 cells, baby hamster kidney cells (BHK), human embryo kidney 293 cells (HEK-293), human C6 cells, Madin-Darby canine kidney cells (MDCK) and Sf9 insect cells. The expression system may also be an entire organism, such as a transgenic plant or animal. For example, the expression system may be a transgenic sheep or cow that capable of expression of recombinant proteins that are secreted into the milk, or a recombinant plant capable of expressing recombinant proteins. Any suitable host system for recombinant protein expression known in the art can be used in accordance with the methodsherein. Expression of nucleic acid sequences can be regulated by a second nucleic acid sequence so that the encoded nucleic acid is expressed in a host transformed with the recombinant DNA molecule. For example, expression of an ancestral sequence can be controlled by any suitable promoter/enhancer element known in the art. Suitable promoters include, for example, the SV40 early promoter region (Benoist and Chambon, Nature 290:304-10 (1981)), the promoter contained in the 3' long terminal repeat of Rous sarcoma virus (Yamamoto et al, Cell 22:787-97 (1980)), the herpes thymidine kinase promoter (Wagner et al, Proc. Natl. Acad. Sci. USA 78: 1441-45 (1981)), the Cytomegalovirus promoter, the translational elongation factor EF-1. alpha, promoter, the regulatory sequences of the metallothionein gene (Brinster et al., Nature 296:39-42 (1982)), prokaryotic promoters such as, for example, the .beta. -lactamase promoter (Villa-Komaroff et al., Proc. Natl. Acad. Sci. USA 75:3727-31 (1978)) or the tac promoter (deBoer et al., Proc. Natl. Acad. Sci. USA 80:21-25 (1983)), plant expression vectors including the cauliflower mosaic virus 35S RNA promoter (Gardner et al., Nucl. Acids Res. 9:2871-88 (1981)), and the promoter of the photosynthetic enzyme ribulose biphosphate carboxylase (Herrera-Estrella et al., Nature 310:115-20 (1984)), promoter elements from yeast or other fungi such as the GAL7 and GAL4 promoters, the ADH (alcohol dehydrogenase) promoter, the PGK (phosphoglycerol kinase) promoter, the alkaline phosphatase promoter, and the like.

In a specific embodiment, a vector is used that comprises a promoter operably linked to the ancestral sequence encoding nucleic acid, one or more origins of replication, and, optionally, one or more selectable markers (e.g., an antibiotic resistance gene). Suitable selectable markers include, for example, those conferring resistance to ampicillin, tetracycline, neomycin, G418, and the like. An expression construct can be made, for example, by subcloning a nucleic acid encoding an ancestral sequence into a restriction site of the pRSECT expression vector. Such a construct allows for the expression of the ancestral sequence under the control of the T7 promoter with a histidine amino terminal flag sequence for affinity purification of the expressed polypeptide.

Expression systems suitable for use with the methods described herein include, but are not limited to in-vitro expression systems and in vivo expression systems. Exemplary in vitro expression systems include, but are not limited to, cell-free transcription/translation systems (e.g. ribosome based protein expression systems). Several such systems are known in the art (see, for example, Tymms (1995) In vitro Transcription and Translation Protocols: Methods in Molecular Biology Volume 37, Garland Publishing, NY).

Exemplary in vivo expression systems include, but are not limited to prokaryotic expression systems such as bacteria (e.g., E. coli and B. subtilis), yeast expression systems (e.g., Saccharomyces cerevisiae), worm expression systems (e.g. Caenorhabditis elegans), insect expression systems (e.g. Sf9 cells), plant expression systems, and amphibian expression systems (e.g. melanophore cells).

Manipulations of the ancestral sequence can also be made at the protein level. Included within the scope of the invention are ancestor protein fragments, derivatives or analogs that are differentially modified during or after synthesis (e.g., in vivo or in vitro translation). Such modifications include conservative substitution, glycosylation, acetylation, phosphorylation, amidation, derivatization by known protecting/blocking groups, proteolytic cleavage, linkage to an antibody molecule or other cellular ligand, and the like. Any of numerous chemical modifications can be carried out by known techniques, including, but not limited to, specific chemical cleavage (e.g., by cyanogen bromide); enzymatic cleavage (e.g., by trypsin, chymotrypsin, papain, V8 protease, and the like); modification by, for example, NaBH.sub.4 acetylation, formylation, oxidation and reduction; metabolic synthesis in the presence of tunicamycin; and the like. Amino acids can be modified, for example, co-translationally or post-translationally during recombinant production (e.g., N-linked glycosylation at N-X-S/T motifs during expression in mammalian cells) or modified by synthetic means. Examples of modified amino acids suitable for use with the methods described herein include, but are not limited to, glycosylated amino acids, sulfated amino acids, prenlyated (e.g., farnesylated, geranylgeranylated) amino acids, acetylated amino acids, PEG-ylated amino acids, biotinylated amino acids, carboxylated amino acids, phosphorylated amino acids, and the like. Exemplary protocol and additional amino acids can be found in Walker (1998) Protein Protocols on CD-ROM Human Press, Towata, N.J.

In addition, fragments, derivatives and analogs of ancestor proteins can be chemically synthesized. For example, a peptide corresponding to a portion, or fragment, of an ancestor protein, which comprises a desired domain, can be synthesized by use of chemical synthetic methods using, for example, an automated peptide synthesizer. (See also Hunkapiller et al., Nature 310:105-11 (1984); Stewart and Young, Solid Phase Peptide Synthesis, 2nd ed., Pierce Chemical Co., Rockford, Ill., (1984).) Furthermore, if desired, nonclassical amino acids or chemical amino acid analogs can be introduced as a substitution or addition into the polypeptide sequence. Non-classical amino acids include, but are not limited to, the D-isomers of the common amino acids, .alpha.-amino isobutyric acid, 4-aminobutyric acid, 2-amino butyric acid, 6-amino hexanoic acid, 2-amino isobutyric acid, 3-amino propionic acid, ornithine, norleucine, norvaline, hydroxyproline, sarcosine, citrulline, cysteic acid, t-butylglycine, t-butylalanine, phenylglycine, cyclohexylalanine, .beta.-alanine, selenocysteine, fluoro-amino acids, designer amino acids such as .beta.-methyl amino acids, C .alpha.-methyl amino acids, N .alpha.-methyl amino acids, and other amino acid analogs. Furthermore, the amino acid can be D (dextrorotary) or L (levorotary).

The ancestral protein, fragment, derivative or analog can also be a chimeric, or fusion, protein-comprising an ancestor protein, fragment, derivative or analog thereof (typically consisting of at least a domain or motif of the ancestor protein, or at least 10 contiguous amino acids of the ancestor protein) joined at its amino- or carboxy-terminus via a peptide bond to an amino acid sequence of a different protein. In one embodiment, such a chimeric protein is produced by recombinant expression of nucleic acid encoding the chimeric protein. The chimeric nucleic acid can be made by ligating the appropriate nucleic acid sequences to each other in the proper reading frame and expressing the chimeric product by methods commonly known in the art. Alternatively, the chimeric protein can be made by protein synthetic techniques (e.g., by use of an automated peptide synthesizer).

The nucleic acids encoding ancestral sequences can be inserted into an appropriate expression vector (i.e., a vector which contains the necessary elements for the transcription and translation of the inserted polypeptide-coding sequence). A variety of host-vector systems can be utilized to express the polypeptide-coding sequence(s). These include, for example, mammalian cell systems infected with virus (e.g., vaccinia virus, adenovirus, sindbis virus, Venezuelan equine encephalitis (VEE) virus, and the like), insect cell systems infected with virus (e.g., baculovirus), microorganisms such as yeast containing yeast vectors, or bacteria transformed with bacteriophage DNA, plasmid DNA, or cosmid DNA. The expression elements of vectors vary in their strengths and specificities. Depending on the host-vector system utilized, any one of a number of suitable transcription and translation elements can be used. In specific embodiments, the ancestral sequence is expressed in human cells, other mammalian cells, yeast or bacteria. In yet another embodiment, a fragment of an ancestral sequence comprising an immunologically active region of the sequence is expressed. In one embodiment, the ancestral genes can be cloned into a pQE80L vector and transformed in *E. coli* BL21 (DE3) cells. For expression, the cells can be incubated overnight in LB medium at 37 °C and protein expression can be induced with ImM IPTG. Expressed protein can be recovered by pelleting and sonicated the cells.

Upon expression, ancestral proteins can be isolated and purified by standard methods including chromatography (e.g., ion exchange, affinity, sizing column chromatography, high pressure liquid chromatography), centrifugation, differential solubility, or by any other standard technique for the purification of proteins. In one embodiment, the ancestral proteins can be His 6-tagged. Upon recovery, the proteins can be purified by loading cell lysates onto a *His GraviTrap* affinity column. The purified protein can be verified by SDS-PAGE. The proteins can then loaded into PD-10 desalting column and finally dialyzed against a buffer (e.g. 50 mM HEPES, pH 7.0 buffer).

Conditions for Trx enzymatic activity can vary according to the Trx enzyme because thioredoxins are in a reduced state to be active. Reduced state Trx enzymes can be generated by any method known in the art, including but not limited to the use of a complementary bacterial or eukaryotic Trx reductase (TrxR) enzyme. Where Trx enzymes are from extant sources or are resurrected enzymes, their accompanying reductases may be unknown or unavailable. In such cases small amounts of dithiothreitol (DTT) (e.g. 50-100 $\mu$M) or Tris(2-carboxyethyl)phosphine HCl (TCEP hydrochloride) can be used to maintain the enzymes in the reduced state. The amount of DTT of TCEP can be selected such that it is sufficient to maintain the enzymes in the reduced state but low enough as to not trigger the reduction of disulfide bonds by themselves. Such conditions can to be established for each individual enzyme.

Enzymes can be exceptional catalysts useful for accelerating chemical reaction rates by several orders of magnitude. The mechanisms of numerous enzymatic reactions can be studied using any number of protein biochemistry as well as structural biology approaches, including, but not limited to X-ray crystallography and NMR. Such studies can be used to identify structural features and conformational changes necessary for the catalytic activity of enzymes. Single molecule techniques can also be useful for studying enzyme dynamics in solution at the Angstrom scale. In certain aspects, single molecule techniques are useful where observation of rearrangements in the participating atoms necessary for catalysis is important. Such approaches generate data that, combined together with structural information as well as molecular dynamics simulations, can provide a more complete view of enzyme dynamics.

Several methods, some of which are based on spectrophotometry, can be used to determine the activity of Trx enzymes. Exemplary methods include, but are not limited to monitoring the oxidation of NADPH in the presence of Trx reductase or ribonucleotide reductase (Holmgren, J Biol Chem, 1979. 254(18): p. 9113-9; Holmgren, J Biol Chem, 1979. 254(19): p. 9627-32); the observation of the turbidity of solutions containing insulin, which readily aggregates after reduction of its disulfide bonds (Holmgren, J Biol Chem, 1979. 254(19): p. 9627-32) or the use of Ellman's reagent (DTNB), where upon reduction by thiol groups generates products that can be easily detected with a spectrophotometer (Holmgren, Thioredoxin. Annu Rev Biochem, 1985. 54: p. 237-71). Changes in tryptophan fluorescence have also been used to measure rates of Trx oxidation and reduction (Holmgren, J Biol Chem, 1972. 247(7): p. 1992-8). Although effective in monitoring the overall activity of thioredoxin, these methods are not sensitive enough to probe the substrate-enzyme interactions that take place in the binding groove of the enzyme. Such methods can be important because binding grooves are common in enzymes and enzymatic reactions. In such cases, examination of the enzymatic mechanisms and/or activity can be facilitated by single molecule techniques.

Described herein is a force-clamp spectrometer built on top of a "through the lens" Total Internal Reflection Fluorescence (TIRF) microscope. This experimental setup enables the application of force to a single protein while at the same time measuring a fluorescent signal. The force-spectrometer can be either an AFM (Sarkar et al., Proc Natl Acad Sci USA, 2004. 101(35): p. 12882-6), or an electromagnet (Liu et al., Biophysical Journal, 2009. 96(9): p. 3810-3821). Both of these can readily pick up and stretch a single engineered polypeptide. The design takes advantage of the stability and high spatial sensitivity of the evanescent field of the TIRF microscope. As a result of total internal reflection, an evanescent wave is formed on the surface of the microscope slide. The amplitude of the evanescent wave decays exponentially, with a space constant that can be set to be as short as ~90 nm and up to ~300 nm. The evanescent wave can excite any fluorophore that enters this field, and its fluorescence can readily be measured by a high performance CCD camera. The rapidly decaying evanescent field on the surface of the microscope slide can be used either to measure displacement in the z direction or to capture single molecule fluorescence without any background emanating from the solution buffer. The combined AFM/TIRF microscope to can be used to demonstrate that a calibrated evanescent field can be used to track the mechanical unfolding of a single polypeptide with sub-nanometer resolution (Sarkar et al., Proc Natl Acad Sci USA, 2004. 101(35): p. 12882-6). The same TIRF microscope equipped with magnetic tweezers can track the unfolding of a polypeptide at very low forces and for very long periods of time (Liu et al., Biophysical Journal, 2009. 96(9): p. 3810-3821). However, the simplest application of the AFM/TIRF microscope is in detecting fluorescence over a very short distance of a mechanically stretched protein, without interference from the bulk. This technique has been demonstrated by mechanically stretching and unfolding the protein talin, a key player in coupling the cytoskeleton of a cell to the extracellular matrix (del Rio et al., Science, 2009. 323(5914): p. 638-41). These experiments demonstrate the versatility of combining force-spectroscopy with TIRF microscopy. As described herein, this technique can be used to monitor the association/dissociation reactions of single thioredoxin enzymes as they reduce disulfide bonds in substrate proteins. Trx enzymes can be labeled while remaining active, for example, exposed lysines of Trx enzymes can be labeled with Alexa Fluor 488 fluorophore such to allow monitoring when the enzyme binds to the exposed disulfide bond. The experimental design is shown in Figure 5. This approach can be used to measure the time course of association and dissociation of fluorescently labeled thioredoxins, while simultaneously observing the reduction of the substrate and to characterize the dynamics of the enzyme-substrate interactions at the single molecule level and develop kinetic models for catalysis (Wiita, A.P., et al, Nature, 2007. 450(7166): p. 124-7).

The association and dissociation of fluorescently labeled thioredoxin enzymes can be measured while simultaneously monitoring reduction events using force- spectroscopy/TIRF instrumentation. The force dependency of association and dissociation can also be measured as can the dwell times between association and reduction. These data can be used to examine the mechanisms by which thioredoxin enzymes find their target disulfide bonds. As described herein, the single molecule AFM detection of disulfide bond reduction can be combined with simultaneous Total Internal Reflection (TIRF) detection of fluorescently labeled thioredoxin enzymes to follow them as they bind and unbind to the disulfide bond being reduced. This instrument enables real time visualization of the entire association, reduction and dissociation cycle of a single enzyme as it catalyzes the reduction of its target. The combined AFM/TIRF instrument can be used to study the search mechanism, and to measure association and dissociation rates as a function of the mechanical force applied to the substrate.

Described is the use of single molecule force-clamp spectroscopy techniques for investigating the chemical mechanisms of catalysis of thioredoxins, a broad class of enzymes that specialize in reducing disulfide bonds and that can also function as oxidases and isomerases. Thioredoxin enzymes are present in all known organisms from bacteria to human and play crucial roles in a wide variety of cellular functions. Thioredoxins have been implicated in pathological processes such as vascular damage caused by oxidative injury, virus entry into cells, and a wide variety of immune related disorders, but also have found practical use in biotechnology.

The single molecule assay for the reduction of disulfide bonds by thioredoxin can be performed by detecting the step elongation of a protein under force, which results from the cleavage of a covalent bond (Figure 6). This scheme can be generalized to other types of enzymes that catalyze the cleavage of covalent bonds such as proteases. Proteases are a vast group of proteins that efficiently catalyze the hydrolysis of peptide bonds (Beynon and Bond, Proteolytic enzymes: a practical approach. 2001, New York: Oxford University Press). Alterations in their physiological activities are responsible for the occurrence or exacerbation of numerous pathologies, such as cancer or inflammatory and cardiovascular diseases (Lopez-Otin and Bond, J Biol Chem, 2008. 283(45): p. 30433-7). Proteases are regarded as potential drug targets or biomarkers by the pharmaceutical industry (Turk, Nat Rev Drug Discov, 2006. 5(9): p. 785-99). Pharmacological interventions on protease activity benefit from detailed knowledge of their mechanism of catalysis (Walker and Lynas Cell Mol Life Sci, 2001. 58(4): p. 596-624). Single molecule techniques to study protease enzymes and uncover substrate dynamics during proteolysis, thereby enabling pharmacological intervention on protease activity from detailed knowledge of their mechanism of catalysis.

By applying a calibrated force the conformations of a disulfide bond substrate can be controlled, and the effect of this restriction on the activity of thioredoxin enzymes can be measured. This assay is a highly sensitive probe of the sub-Angstrom level rearrangement of the sulfur atoms at the catalytic center of Trx enzymes (Wiita, A.P., et al., Nature, 2007. 450(7166): p. 124-7). By combining this new form of spectroscopy together with structural data and molecular dynamics simulations we obtain novel insights into catalysis. These studies can be generalized and understood in relation to the structure of other enzymes to evaluate of the range of chemical mechanisms available to thioredoxin as well as other enzymes and how such mechanisms can be controlled by structural features such as binding grooves.

Single molecule assays can also be used to detect the oxidase activity of thioredoxin enzymes. For example, if the stretching force is quenched after a substrate disulfide bond has been reduced, the substrate protein folds, however the disulfide bond does not reform spontaneously. By introducing a mutant form of thioredoxin, efficient re-oxidation can be obtained during folding.

Force spectroscopy can also be used to examine other covalent bond cleaving enzymes. For example, proteases share structural features in common with thioredoxins such as a binding groove adjacent to the catalytic nucleophile. A steric-switch approach, where a bond cleavage event is translated into an easily identified stepwise elongation of the substrate protein, can be adapted to detect the activity of proteases, and study their catalytic mechanisms.

As described herein, single molecule force-spectroscopy experiments demonstrate that the application of a mechanical force to a substrate disulfide bond can regulate the catalytic activity of thioredoxin enzymes, thereby revealing distinct chemical mechanisms of reduction that can be distinguished by their sensitivity to an applied force. Thus, single molecule assay of thioredoxin catalysis provides with a novel and useful new approach to study the chemical mechanisms of catalysis in this important class of enzymes.

One advantage of the single molecule approach is that individual conformations, which can otherwise be averaged out in bulk experiments, can be observed directly and then correlated with the known structural features of the molecule. This approach can also be used for ion channels, where it was possible to provide a detailed account of the structure-function relationship for this class of membrane proteins. As described herein, single molecule assays for substrate dynamics in thioredoxin and protease catalysis can be used to study enzyme dynamics.

In single molecule force clamp spectroscopy experiments, a mechanical force is applied to a substrate protein containing a target disulfide bond, and the effect of the resulting stiffening on the rate of reduction or oxidation by thioredoxin enzymes is measured. The applied force restricts the movement of the enzymatic substrate in the binding groove of the enzyme, acting as a form of spectroscopy that can be used to investigate the types of substrate motions that occur during enzymatic catalysis. As described herein, this form of spectroscopy can be used to study the catalytic mechanisms of enzymes, including, but not limited to thioredoxin enzymes and proteases.

The application of force to a substrate disulfide bond can be used to modulate conformational dynamics in the binding groove of Trx (Figure 6), thereby regulating the catalytic activity of the enzyme (Wiita, A.P., et al., Nature, 2007. 450(7166): p. 124-7). This form of molecular spectroscopy can resolve substrate motions in the active site of the Trx enzyme with sub-Angstrom resolution. Force-spectroscopy of Trx catalysis indicates that the chemical mechanism of reduction is characterized by its rapid inhibition by a force applied to the substrate disulfide bond. When compared with other reducing agents, this chemical mechanism is specific to Trx enzymes. After binding to the enzymatic groove, the reaction occurs by rotation of the target disulfide bond against the pulling force in order to acquire the correct geometry for the $S_N2$ chemical reaction to occur (Figure 6B). Other chemical mechanisms of reduction also operate simultaneously (Perez-Jimenez, et al., Nature Structural & Molecular Biology, 2009. 16(8): p. 890-U120). The force-clamp spectroscopy approach is validated by the fact that the rates of reduction extrapolated to zero force agree with those measured using spectrophotometric methods (Perez-Jimenez, et al., Nature Structural & Molecular Biology, 2009. 16(8): p. 890-U120). Hence, force spectroscopy of Trx catalysis can be used to study the dynamics of a substrate in the binding groove of an enzyme. Indeed, the single molecule reduction assay (as shown in Figure 6A) is readily able to distinguish the chemistry of simple nucleophiles, such as cysteine and glutathione, from more elaborate pathways for the reduction of disulfide bonds, which are unique to groove based thioredoxin enzymes (Figure IE) (Wiita, A.P., et al., Nature, 2007. 450(7166): p. 124-7; Perez-Jimenez, et al., Nature Structural & Molecular Biology, 2009. 16(8): p. 890-U120; Ainavarapu et al., Journal of the American Chemical Society, 2008. 130(20): p. 6479-6487). Furthermore, the force-clamp spectroscopy assay is able to combine the observation of protein folding, together with reduction-oxidation cycles.

During protein disulfide bond reduction, thioredoxin binds to the substrate in a catalytically favorable configuration (Qin et al., Structure, 1995. 3: p. 289-297). The mechanisms by which thioredoxin finds a substrate disulfide bond can be examined by measuring the association and dissociation of single enzymes as they find and reduce a disulfide bond. Thioredoxin enzymes may find and position the two bonded sulfur atoms out of the thousands of atoms of the host protein by utilizing a "reduced dimensionality" approach (Adam and Delbruck, Structural Chemistry and Molecular Biology, ed. A. Rich and N. Davidson. 1968, New York: W. H. Freeman and Co. 198-215; von Hippel and Berg, J Biol Chem, 1989. 264(2): p. 675-8), similar to enzymes that target DNA (Gorman et al., Mol Cell, 2007. 28(3): p. 359-70; Stanford et al., Embo J, 2000. 19(23): p. 6546-57). A reduced dimensionality search consists of at least two distinct steps: a nonspecific association with the substrate macromolecule followed by some form of processivity along the coordinates of the substrate (Riggs, et al, Lac Repressor-Operator Interaction .3. Kinetic Studies. Journal of Molecular Biology, 1970. 53(3): p. 401-7).

In the case of DNA binding enzymes, the principle of reduced dimensionality has been well established as a widespread mechanism (Halford et al., Nucleic Acids Res, 2004. 32(10): p. 3040-52). For enzymes acting on macromolecular substrates, reduced dimensionality may be important for facilitating the target search (Adam and Delbruck, Structural Chemistry and Molecular Biology, ed. A. Rich and N. Davidson. 1968, New York: W. H. Freeman and Co. 198-215; Riggs, et al, Lac Repressor-Operator Interaction .3. Kinetic Studies. Journal of Molecular Biology, 1970. 53(3): p. 401-7; Berg and Blomberg, Biophysical Chemistry, 1978. 8(4): p. 271-280; Berg et al., Biochemistry, 1981. 20(24): p. 6929-6948; von Hippel and Berg, J Biol Chem, 1989. 264(2): p. 675-8). In the case of Trx enzymes, Trx enzymes may first bind to a substrate and then diffusing along the extended polypeptide until finding the disulfide bond. The polypeptide stays loosely bound to the enzymatic groove, and slides randomly towards the disulfide. The simplest

$$\langle t \rangle \approx \frac{\langle d_{sl}^{2} \rangle}{2D},$$

expression for the mean time to target is given by where $D$ is the diffusion coefficient for the enzyme sliding along the polypeptide and $d_{sl}$ is the sliding distance between the place where Trx was first bound to the polypeptide and the exposed disulfide bond (Figure 7). This simple scenario can be examined by directly measuring the distribution of dwell times between binding and reduction. The time to target can depend on the square of the sliding distance $d_{sl}$, which we will vary using protein engineering (Stanford et al., Embo Journal, 2000. 19(23): p. 6546-6557; Halford et al., Nucleic Acids Research, 2004. 32(10): p. 3040-3052).

Although several different ancestral Trx polypeptides are described herein, one of skill in the art will recognize that other types of ancestral polypeptides can also be produced using the methods described herein. Ancestral sequences can be generated for any polypeptide using the methods described herein, including, but not limited to therapeutic proteins and proteins susceptible to industrial use.

The stability and/or activity of any polypeptide at low pH or elevated temperature can be modified according to the methods described herein. Polypeptides having increased stability and/or activity of any polypeptide at low pH or elevated temperature that can be produced according to the methods described herein can be from any source or origin and can include a polypeptide found in prokaryotes, viruses, and eukaryotes, including fungi, plants, yeasts, insects, and animals, including mammals (e.g. humans). Polypeptides having increased stability and/or activity of any polypeptide at low pH or elevated temperature that can be produced according to the methods described herein include, but are not limited to any polypeptide sequences, known or hypothetical or unknown, which can be identified using common sequence repositories. Example of such sequence repositories include, but are not limited to GenBank EMBL, DDBJ and the NCBI. Other repositories can easily be identified by searching on the internet. Polypeptides that can be produced using the methods described herein also include polypeptides have at least about 60%, 70%, 75%, 80%, 90%, 95%, or at least about 99% or more identity to any known or available polypeptide (e.g., a therapeutic polypeptide, a diagnostic polypeptide, an industrial enzyme, or portion thereof, and the like).

Polypeptides having increased stability and/or activity of any polypeptide at low pH or elevated temperature that can be produced according to the methods described herein also include polypeptides comprising one or more non-natural amino acids. As used herein, a non-natural amino acid can be, but is not limited to, an amino acid comprising a moiety where a chemical moiety is attached, such as an aldehyde- or keto-derivatized amino acid, or a non-natural amino acid that includes a chemical moiety. A non-natural amino acid can also be an amino acid comprising a moiety where a saccharide moiety can be attached, or an amino acid that includes a saccharide moiety.

Polypeptides having increased stability and/or activity of any polypeptide at low pH or elevated temperature can also comprise peptide derivatives (for example, that contain one or more non-naturally occurring amino acids). In specific embodiments, the library members contain one or more non-natural or non-classical amino acids or cyclic peptides. Non-classical amino acids include but are not limited to the D-isomers of the common amino acids, -amino isobutyric acid, 4-aminobutyric acid, Abu, 2-amino butyric acid;. -Abu, -Ahx, 6-amino hexanoic acid; Aib, 2-amino isobutyric acid; 3-amino propionic acid; omithine; norleucine; norvaline, hydroxyproline, sarcosine, citrulline, cysteic acid, t-butylglycine, t-butylalanine, phenylglycine, cyclohexylalanine, .beta.-alanine, designer amino acids such as .beta.-methyl amino acids, C-methyl amino acids, N-methyl amino acids, fluoro-amino acids and amino acid analogs in general. Furthermore, the amino acid can be D (dextrorotary) or L (levorotary).

Also inclusive are derivative polypeptides having an amino acid sequence selected from the group consisting of a polypeptide of SEQ ID NOs: 1-7 and which has been acetylated, carboxylated, phosphorylated, glycosylated, ubiquitinated or other post-translational modifications. In another embodiment, the derivative has been labeled with, e.g., radioactive isotopes such as $^{125}$I, $^{32}$P, $^{35}$S, and $^{3}$H. In another embodiment, the derivative has been labeled with fluorophores, chemiluminescent agents, enzymes, and antiligands that can serve as specific binding pair members for a labeled ligand.

Polypeptide modifications are well known to those of skill and have been described in detail in the scientific literature. Several common modifications, such as glycosylation, lipid attachment, sulfation, gamma-carboxylation of glutamic acid residues, hydroxylation and ADP-ribosylation, for instance, are described in most basic texts, such as, for instance Creighton, Protein Structure and Molecular Properties, 2nd ed., W. H. Freeman and Company (1993). Many detailed reviews are available on this subject, such as, for example, those provided by Wold, in Johnson (ed.), Post-translational Covalent Modification of Proteins, pgs. 1-12, Academic Press (1983); Seifter et al., Meth. Enzymol. 182: 626-646 (1990) and Rattan et al., Ann. N. Y Acad. Sci. 663: 48-62 (1992).

One can determine whether a polypeptide of the invention will be post-translationally modified by analyzing the sequence of the polypeptide to determine if there are peptide motifs indicative of sites for post-translational modification. There are a number of computer programs that permit prediction of post-translational modifications. See, e.g., expasy with the extension .org of the world wide web (accessed Nov. 11, 2002), which includes PSORT, for prediction of protein sorting signals and localization sites, SignalP, for prediction of signal peptide cleavage sites, MITOPROT and Predotar, for prediction of mitochondrial targeting sequences, NetOGlyc, for prediction of type O-glycosylation sites in mammalian proteins, big-PI Predictor and DGPI, for prediction of prenylation-anchor and cleavage sites, and NetPhos, for prediction of Ser, Thr and Tyr phosphorylation sites in eukaryotic proteins. Other computer programs, such as those included in GCG, also can be used to determine post-translational modification peptide motifs.

Examples of types of post-translational modifications include, but are not limited to: (Z)-dehydrobutyrine; 1-chondroitin sulfate-L-aspartic acid ester; 1'-glycosyl-L-tryptophan; 1'-phospho-L-histidine; 1-thioglycine; 2'-(S-L-cysteinyl)-L-histidine; 2'-[3-carboxamido (trimethylammonio)propyl]-L-histidine; 2'-alpha-mannosyl-L-tryptophan; 2-methyl-L-glutamine; 2-oxobutanoic acid; 2-pyrrolidone carboxylic acid; 3'-(1'-L-histidyl)-L-tyrosine; 3'-(8alpha-FAD)-L-histidine; 3'-(S-L-cysteinyl)-L-tyrosine; 3', 3'', 5'-triiodo-L-thyronine; 3'-4'-phospho-L-tyrosine; 3-hydroxy-L-proline; 3'-methyl-L-histidine; 3-methyl-L-lanthionine; 3'-phospho-L-histidine; 4'-(L-tryptophan)-L-tryptophyl quinone; 42 N-cysteinyl-glycosylphosphatidylinositolethanolamine; 43-(T-L-histidyl)-L-tyrosine; 4-hydroxy-L-arginine; 4-hydroxy-L-lysine; 4-hydroxy-L-proline; 5'-(N6-L-lysine)-L-topaquinone; 5-hydroxy-L-lysine; 5-methyl-L-arginine; alpha-1-microglobulin-Ig alpha complex chromophore; bis-L-cysteinyl bis-L-histidino diiron disulfide; bis-L-cysteinyl-L-N3'-histidino-L-serinyl tetrairon' tetrasulfide; chondroitin sulfate D-glucuronyl-D-galactosyl-D-galactosyl-D-xylosyl-L-serine; D-alanine; D-allo-isoleucine; D-asparagine; dehydroalanine; dehydrotyrosine; dermatan 4-sulfate D-glucuronyl-D-galactosyl-D-galactosyl-D-xylosyl-L-serine; D-glucuronyl-N-glycine; dipyrrolylmethanemethyl-L-cysteine; D-leucine; D-methionine; D-phenylalanine; D-serine; D-tryptophan; glycine amide; glycine oxazolecarboxylic acid; glycine thiazolecarboxylic acid; heme P450-bis-L-cysteine-L-tyrosine; heme-bis-L-cysteine; hemediol-L-aspartyl ester-L-glutamyl ester; hemediol-L-aspartyl ester-L-glutamyl ester-L-methionine sulfonium; heme-L-cysteine; heme-L-histidine; heparan sulfate D-glucuronyl-D-galactosyl-D-galactosyl-D-xylosyl-L-serine; heme P450-bis-L-cysteine-L-lysine; hexakis-L-cysteinyl hexairon hexasulfide; keratan sulfate D-glucuronyl-D-galactosyl-D-galactosyl-D-xylosyl-L-threonine; L oxoalanine- lactic acid; L phenyllactic acid; 1'-(8alpha-FAD)-L-histidine; L-2'.4',5'-topaquinone; L-3',4'-dihydroxyphenylalanine; L-3'.4'.5'-trihydroxyphenylalanine; L-4'-bromophenylalanine; L-6'-bromotryptophan; L-alanine amide; L-alanyl imidazolinone glycine; L-allysine; L-arginine amide; L-asparagine amide; L-aspartic 4-phosphoric anhydride; L-aspartic acid 1-amide; L-beta-methylthioaspartic acid; L-bromohistidine; L-citrulline; L-cysteine amide; L-cysteine glutathione disulfide; L-cysteine methyl disulfide; L-cysteine methyl ester; L-cysteine oxazolecarboxylic acid; L-cysteine oxazolinecarboxylic acid; L-cysteine persulfide; L-cysteine sulfenic acid; L-cysteine sulfinic acid; L-cysteine thiazolecarboxylic acid; L-cysteinyl homocitryl molybdenum-heptairon-nonasulfide; L-cysteinyl imidazolinone glycine; L-cysteinyl molybdopterin; L-cysteinyl molybdopterin guanine dinucleotide; L-cystine; L-erythro-beta-hydroxyasparagine; L-erythro-beta-hydroxyaspartic acid; L-gamma-carboxyglutarnic acid; L-glutamic acid 1-amide; L-glutamic acid 5-methyl ester; L-glutamine amide; L-glutamyl 5-glycerylphosphorylethanolarnine; L-histidine amide; L-isoglutamyl-polyglutamic acid; L-isoglutamyl-polyglycine; L-isoleucine amide; L-lanthionine; L-leucine amide; L-lysine amide; L-lysine thiazolecarboxylic acid; L-lysinoalanine; L-methionine amide; L-methionine sulfone; L-phenyalanine thiazolecarboxylic acid; L-phenylalanine amide; L-proline amide; L-selenocysteine; L-selenocysteinyl molybdopterin guanine dinucleotide; L-serine amide; L-serine thiazolecarboxylic acid; L-seryl imidazolinone glycine; L-T-bromophenylalanine; L-T-bromophenylalanine; L-threonine amide; L-thyroxine; L-tryptophan amide; L-tryptophyl quinone; L-tyrosine amide; L-valine amide; meso-lanthionine; N-(L-glutamyl)-L-tyrosine; N-(L-isoaspartyl)-glycine; N-(L-isoaspartyl)-cysteine; N,N,N-trimethyl-L-alanine; N,N-dimethyl-L-proline; N2-acetyl-L-lysine; N2-succinyl-L-tryptophan; N4-(ADP-ribosyl)-L-asparagine; N4-glycosyl-L-asparagine; N4-hydroxymethyl-L-asparagine; N4-methyl-L-asparagine; N5-methyl-L-glutamine; N6-1-carboxyethyl-L-lysine; N6-(4-amino hydroxybutyl)-L-lysine; N6-(L-isoglutamyl)-L-lysine; N6-(phospho-5'-adenosine)-L-lysine; N6-(phospho-5'-guanosine)-L-lysine; N6,N6,N6-trimethyl-L-lysine; N6,N6-dimethyl-L-lysine; N6-acetyl-L-lysine; N6-biotinyl-L-lysine; N6-carboxy-L-lysine; N6-formyl-L-lysine; N6-glycyl-L-lysine; N6-lipoyl-L-lysine; N6-methyl-L-lysine; N6-methyl-N6-poly(N-methylpropylamine)-L-lysine; N6-mureinyl-L-lysine; N6-myristoyl-L-lysine; N6-palmitoyl-L-lysine; N6-pyridoxal phosphate-L-lysine; N6-pyruvic acid 2-iminyl-L-lysine; N6-retinal-L-lysine; N-acetylglycine; N-acetyl-L-glutamine; N-acetyl-L-alanine; N-acetyl-L-aspartic acid; N-acetyl-L-cysteine; N-acetyl-L-glutamic acid; N-acetyl-L-isoleucine; N-acetyl-L-methionine; N-acetyl-L-proline; N-acetyl-L-serine; N-acetyl-L-threonine; N-acetyl-L-tyrosine; N-acetyl-L-valine; N-alanyl-glycosylphosphatidylinositolethanolamine; N-asparaginyl-glycosylphosphatidylinositolethanolamine; N-aspartyl-glycosylphosphatidylinositolethanolamine; N-formylglycine; N-formyl-L-methionine; N-glycyl-glycosylphosphatidylinositolethanolamine; N-L-glutamyl-poly-L-glutamic acid; N-methylglycine; N-methyl-L-alanine; N-methyl-L-methionine; N-methyl-L-phenylalanine; N-myristoyl-glycine; N-palmitoyl-L-cysteine; N-pyruvic acid 2-iminyl-L-cysteine; N-pyruvic acid 2-iminyl-L-valine; N-seryl-glycosylphosphatidylinositolethanolamine; N-seryl-glycosyOSPhingolipidinositolethanolamine; O-(ADP-ribosyl)-L-serine; O-(phospho-5'-adenosine)-L-threonine; O-(phospho-5'-DNA)-L-serine; O-(phospho-5'-DNA)-L-threonine; O-(phospho-5rRNA)-L-serine; O-(phosphoribosyl dephospho-coenzyme A)-L-serine; O-(sn-1-glycerophosphoryl)-L-serine; O4'-(8alpha-FAD)-L-tyrosine; O4'-(phospho-5'-adenosine)-L-tyrosine; O4'-(phospho-5'-DNA)-L-tyrosine; O4'-(phospho-5'-RNA)-L-tyrosine; O4'-(phospho-5'-uridine)-L-tyrosine; O4-glycosyl-L-hydroxyproline; O4'-glycosyl-L-tyrosine; O4'-sulfo-L-tyrosine; O5-glycosyl-L-hydroxylysine; O-glycosyl-L-serine; O-glycosyl-L-threonine; omega-N-(ADP-ribosyl)-L-arginine; omega-N-omega-N'-dimethyl-L-arginine; omega-N-methyl-L-arginine; omega-N-omega-N-dimethyl-L-arginine; omega-N-phospho-L-arginine; O'octanoyl-L-serine; O-palmitoyl-L-serine; O-palmitoyl-L-threonine; O-phospho-L-serine; O-phospho-L-threonine; O-phosphopantetheine-L-serine; phycoerythrobilin-bis-L-cysteine; phycourobilin-bis-L-cysteine; pyrroloquinoline quinone; pyruvic acid; S hydroxy cinnamyl-L-cysteine; S-(2-aminovinyl)methyl-D-cysteine; S-(2-aminovinyl)-D-cysteine; S-(6-FW-L-cysteine; S-(8alpha-FAD)-L-cysteine; S-(ADP-ribosyl)-L-cysteine; S-(L-isoglutamyl)-L-cysteine; S-12-hydroxyfamesyl-L-cysteine; S-acetyl-L-cysteine; S-diacylglycerol-L-cysteine; S-diphytanylglycerot diether-L-cysteine; S-famesyl-L-cysteine; S-geranylgeranyl-L-cysteine; S-glycosyl-L-cysteine; S-glycyl-L-cysteine; S-methyl-L-cysteine; S-nitrosyl-L-cysteine; S-palmitoyl-L-cysteine; S-phospho-L-cysteine; S-phycobiliviolin-L-cysteine; S-phycocyanobilin-L-cysteine; S-phycoerythrobilin-L-cysteine; S-phytochromobilin-L-cysteine; S-selenyl-L-cysteine; S-sulfo-L-cysteine; tetrakis-L-cysteinyl diiron disulfide; tetrakis-L-cysteinyl iron; tetrakis-L-cysteinyl tetrairon tetrasulfide; trans-2,3-cis 4-dihydroxy-L-proline; tris-L-cysteinyl triiron tetrasulfide; tris-L-cysteinyl triiron trisulfide; tris-L-cysteinyl-L-aspartato tetrairon tetrasulfide; tris-L-cysteinyl-L-cysteine persulfido-bis-L-glutamato-L-histidino tetrairon disulfide trioxide; tris-L-cysteinyl-L-N3'-histidino tetrairon tetrasulfide; tris-L-cysteinyl-L-NM'-histidino tetrairon tetrasulfide; and tris-L-cysteinyl-L-serinyl tetrairon tetrasulfide.

Additional examples of post translational modifictions can be found in web sites such as the Delta Mass database based on Krishna, R. G. and F. Wold (1998). Posttranslational Modifications. Proteins--Analysis and Design. R. H. Angeletti. San Diego, Academic Press. 1 : 121-206.; Methods in Enzymo logy, 193, J. A. McClosky (ed) (1990), pages 647-660; Methods in Protein Sequence Analysis edited by Kazutomo Imahori and Fumio Sakiyama, Plenum Press, (1993) "Post-translational modifications of proteins" R. G. Krishna and F. Wold pages 167-172; "GlycoSuiteDB: a new curated relational database of glycoprotein glycan structures and their biological sources" Cooper et al. Nucleic Acids Res. 29; 332-335 (2001) "O-GLYCBASE version 4.0: a revised database of O-glycosylated proteins" Gupta et al. Nucleic Acids Research, 27: 370-372 (1999); and "PhosphoBase, a database of phosphorylation sites: release 2.O.", Kreegipuu et al. Nucleic Acids Res 27(1):237-239 (1999) see also, WO 02/21139A2.

Exemplary polypeptides having increased stability and/or activity of any polypeptide at low pH or elevated temperature that can be produced according to the methods described herein include but are not limited to, cytokines, inflammatory molecules, growth factors, their receptors, and oncogene products or portions thereof. Examples of cytokines, inflammatory molecules, growth factors, their receptors, and oncogene products include, but are not limited to e.g., alpha-1 antitrypsin, Angiostatin, Antihemolytic factor, antibodies (including an antibody or a functional fragment or derivative thereof selected from: Fab, Fab', F(ab)2, Fd, Fv, ScFv, diabody, tribody, tetrabody, dimer, trimer or minibody), angiogenic molecules, angiostatic molecules, Apolipopolypeptide, Apopolypeptide, Asparaginase, Adenosine deaminase, Atrial natriuretic factor, Atrial natriuretic polypeptide, Atrial peptides, Angiotensin family members, Bone Morphogenic Polypeptide (BMP-1, BMP-2, BMP-3, BMP-4, BMP-5, BMP-6, BMP-7, BMP-8a, BMP-8b, BMP-10, BMP-15, etc.); C--X--C chemokines (e.g., T39765, NAP-2, ENA-78, Gro-a, Gro-b, Gro-c, IP-10, GCP-2, NAP-4, SDF-1, PF4, MIG), Calcitonin, CC chemokines (e.g., Monocyte chemoattractant polypeptide-1, Monocyte chemoattractant polypeptide-2, Monocyte chemoattractant polypeptide-3, Monocyte inflammatory polypeptide-1 alpha, Monocyte inflammatory polypeptide-1 beta, RANTES, 1309, R83915, R91733, HCC1, T58847, D31065, T64262), CD40 ligand, C-kit Ligand, Ciliary Neurotrophic Factor, Collagen, Colony stimulating factor (CSF), Complement factor 5a, Complement inhibitor, Complement receptor 1, cytokines, (e.g., epithelial Neutrophil Activating Peptide-78, GRO alpha/MGSA, GRO beta, GRO gamma , MIP-1 alpha , MIP-1 delta, MCP-1), deoxyribonucleic acids, Epidermal Growth Factor (EGF), Erythropoietin ("EPO", representing a preferred target for modification by the incorporation of one or more non-natural amino acid), Exfoliating toxins A and B, Factor IX, Factor VII, Factor VIII, Factor X, Fibroblast Growth Factor (FGF), Fibrinogen, Fibronectin, G-CSF, GM-CSF, Glucocerebrosidase, Gonadotropin, growth factors, Hedgehog polypeptides (e.g., Sonic, Indian, Desert), Hemoglobin, Hepatocyte Growth Factor (HGF), Hepatitis viruses, Hirudin, Human serum albumin, Hyalurin-CD44, Insulin, Insulin-like Growth Factor (IGF-I, IGF-II), interferons (e.g., interferon-alpha, interferon-beta, interferon-gamma, interferon-epsilon, interferon-zeta, interferon-eta, interferon-kappa, interferon-lambda, interferon-T, interferon-zeta, interferon-omega), glucagon-like peptide (GLP-1), GLP-2, GLP receptors, glucagon, other agonists of the GLP-1R, natriuretic peptides (ANP, BNP, and CNP), Fuzeon and other inhibitors of HIV fusion, Hurudin and related anticoagulant peptides, Prokineticins and related agonists including analogs of black mamba snake venom, TRAIL, RANK ligand and its antagonists, calcitonin, amylin and other glucoregulatory peptide hormones, and Fc fragments, exendins (including exendin-4), exendin receptors, interleukins (e.g., IL-1, IL-2, IL-3, IL-4, IL-5, IL-6, IL-7, IL-8, IL-9, IL-10, IL-11, IL-12, etc.), I-CAM-1/LFA-1, Keratinocyte Growth Factor (KGF), Lactoferrin, leukemia inhibitory factor, Luciferase, Neurturin, Neutrophil inhibitory factor (NIF), oncostatin M, Osteogenic polypeptide, Parathyroid hormone, PD-ECSF, PDGF, peptide hormones (e.g., Human Growth Hormone), Oncogene products (Mos, Rel, Ras, Raf, Met, etc.), Pleiotropin, Polypeptide A, Polypeptide G, Pyrogenic exotoxins A, B, and C, Relaxin, Renin, ribonucleic acids, SCF/c-kit, Signal transcriptional activators and suppressors (p53, Tat, Fos, Myc, Jun, Myb, etc.), Soluble complement receptor 1, Soluble I-CAM 1, Soluble interleukin receptors (IL-1, 2, 3, 4, 5, 6, 7, 9, 10, 11, 12, 13, 14, 15), soluble adhesion molecules, Soluble TNF receptor, Somatomedin, Somatostatin, Somatotropin, Streptokinase, Superantigens, i.e., Staphylococcal enterotoxins (SEA, SEB, SEC1, SEC2, SEC3, SED, SEE), Steroid hormone receptors (such as those for estrogen, progesterone, testosterone, aldosterone, LDL receptor ligand and corticosterone), Superoxide dismutase (SOD), Toll-like receptors (such as Flagellin), Toxic shock syndrome toxin (TSST-1), Thymosin a 1, Tissue plasminogen activator, transforming growth factor (TGF- alpha, TGF- beta), Tumor necrosis factor beta (TNF beta), Tumor necrosis factor receptor (TNFR), Tumor necrosis factor-alpha (TNF alpha), transcriptional modulators (for example, genes and transcriptional modular polypeptides that regulate cell growth, differentiation and/or cell regulation), Vascular Endothelial Growth Factor (VEGF), virus-like particle, VLA-4NCAM-1, Urokinase, signal transduction molecules, estrogen, progesterone, testosterone, aldosterone, LDL, corticosterone.

Additional polypeptides having increased stability and/or activity of any polypeptide at low pH or elevated temperature that can be produced according to the methods described herein include but are not limited to enzymes (e.g., industrial enzymes) or portions thereof. Examples of enzymes include, but are not limited to amidases, amino acid racemases, acylases, dehalogenases, dioxygenases, diarylpropane peroxidases, epimerases, epoxide hydrolases, esterases, isomerases, kinases, glucose isomerases, glycosidases, glycosyl transferases, haloperoxidases, monooxygenases (e.g., p450s), lipases, lignin peroxidases, nitrile hydratases, nitrilases, proteases, phosphatases, subtilisins, transaminase, and nucleases.

Other polypeptides having increased stability and/or activity of any polypeptide at low pH or elevated temperature that can be produced according to the methods described herein include, but are not limited to, agriculturally related polypeptides such as insect resistance polypeptides (e.g., Cry polypeptides), starch and lipid production enzymes, plant and insect toxins, toxin-resistance polypeptides, Mycotoxin detoxification polypeptides, plant growth enzymes (e.g., Ribulose 1,5-Bisphosphate Carboxylase/Oxygenase), lipoxygenase, and Phosphoenolpyruvate carboxylase.

Polypeptides having increased stability and/or activity of any polypeptide at low pH or elevated temperature that can be produced according to the methods described herein include, but are not limited to, antibodies, immunoglobulin domains of antibodies and their fragments. Examples of antibodies include, but are not limited to antibodies, antibody fragments, antibody derivatives, Fab fragments, Fab' fragments, F(ab)2 fragments, Fd fragments, Fv fragments, single-chain Fv fragments (scFv), diabodies, tribodies, tetrabodies, dimers, trimers, and minibodies.

Also described is a composition comprising a recombinant polypeptide having increased stability and/or activity of any polypeptide at low pH or elevated temperature produced according to the methods described herein, and an additional component selected from the group consisting of pharmaceutically acceptable diluents, carriers, excipients and adjuvants.

Polypeptides having increased stability and/or activity of any polypeptide at low pH or elevated temperature that can be produced according to the methods described herein can also further comprise a chemical moiety selected from the group consisting of: cytotoxins, pharmaceutical drugs, dyes or fluorescent labels, a nucleophilic or electrophilic group, a ketone or aldehyde, azide or alkyne compounds, photocaged groups, tags, a peptide, a polypeptide, a polypeptide, an oligosaccharide, polyethylene glycol with any molecular weight and in any geometry, polyvinyl alcohol, metals, metal complexes, polyamines, imidizoles, carbohydrates, lipids, biopolymers, particles, solid supports, a polymer, a targeting agent, an affinity group, any agent to which a complementary reactive chemical group can be attached, biophysical or biochemical probes, isotypically-labeled probes, spin-label amino acids, fluorophores, aryl iodides and bromides.

Also described is mutating nucleotide sequences to add/create or remove/disrupt sequences. Such mutations can me made using any suitable mutagenesis method known in the art, including, but not limited to, site-directed mutagenesis, oligonucleotide-directed mutagenesis, positive antibiotic selection methods, unique restriction site elimination (USE), deoxyuridine incorporation, phosphorothioate incorporation, and PCR-based mutagenesis methods. Details of such methods can be found in, for example, Lewis et al. (1990) Nucl. Acids Res. 18, p3439; Bohnsack et al. (1996) Meth. Mol. Biol. 57, p1; Vavra et al. (1996) Promega Notes 58, 30; Altered SitesII in vitro Mutagenesis Systems Technical Manual #TM001, Promega Corporation; Deng et al.. (1992) Anal. Biochem. 200, p81; Kunkel et al. (1985) Proc. Natl. Acad. Sci. USA 82, p488; Kunke et al. (1987) Meth. Enzymol. 154, p367; Taylor et al. (1985) Nucl. Acids Res. 13, p8764; Nakamaye et al. (1986) Nucl. Acids Res. 14, p9679; Higuchi et al. (1988) Nucl. Acids Res. 16, p7351; Shimada et al. (1996) Meth. Mol. Biol. 57, p157; Ho et al. (1989) Gene 77, p51; Horton et al. (1989) Gene 77, p61; and Sarkar et al. (1990) BioTechniques 8, p404. Numerous kits for performing site-directed mutagenesis are commercially available, such as the QuikChange II Site-Directed Mutagenesis Kit and the Altered Sites II in vitro mutagenesis system. Such commercially available kits may also be used to optimize sequences. Other techniques that can be used to generate modified nucleic acid sequences are well known to those of skill in the art. See for example Sambrook et al. (2001) Molecular Cloning: A Laboratory Manual, 3rd Ed., Cold Spring Harbor Laboratory, Cold Spring Harbor, N.Y

The following examples illustrate the present invention, and are set forth to aid in the understanding of the invention, and should not be construed to limit in any way the scope of the invention as defined in the claims which follow thereafter.

## EXAMPLES

### Example 1: Paleoenzymology at the single-molecule level: probing the chemistry of resurrected enzymes

A highly articulated phylogenetic tree encompassing over 200 diverse Trx sequences from the three domains of life was constructed (**Fig. 8**). Several biologically relevant nodes for sequence reconstruction and laboratory resurrection were sampled from this tree. Divergence dates estimates were applied to nodes in the tree assuming the root of the tree lies between bacteria and the common ancestor of archaea/eukaryotes (Hedges and Kumar, The Timetree of life, xxi, 551 p. (Oxford University Press, Oxford, 2009)). In particular, Trx enzymes belonging to the last bacterial common ancestor (LBCA in **Fig. 9**), the last archaeal common ancestor (LACA) and the archaeal/eukaryotic common ancestor (AECA) (**Fig. 9**) were resurrected. These organisms are thought to have inhabited Earth 4.2-3.5 Gyr ago (**Fig. 9A**) after diverging from the last universal common ancestor (LUCA) (Boussau et al., Nature 456, 942-5 (2008); Hedges and Kumar, The Timetree of life, xxi, 551 p. (Oxford University Press, Oxford, 2009)). A node corresponding to the last eukaryotic common ancestor (LECA) that lived in the Proterozoic, ~1.60 Gyr ago was also selected. Two other internal nodes in the bacterial lineages were selected; the last common ancestor of cyanobacterial and deinococcus/thermus groups (LPBCA) which existed ~2.50 Gyr ago and represents the origin of photosynthetic bacteria, and the last common ancestor of y-proteobacteria, ~1.61 Gyr old (LGPCA). Finally, the last common ancestor of animals and fungi (LAFCA) that lived ~1.37 Gyr ago (**Fig. 9A**) was also chosen.

The sequences of the ancestral Trx enzymes were reconstructed using statistical methods based on maximum likelihood (Liberles, Ancestral sequence reconstruction, xiii, 252 p. (Oxford University Press, Oxford ; New York, 2007; Gaucher et al., Nature 425, 285-8 (2003)). For a given node in the tree, the posterior probability values for all 20 amino acids were calculated considering each site of the inferred sequence. These values represent the probability that a certain residue occupied a specific position in the sequence at a particular point in the phylogeny. The posterior probabilities were calculated on the basis of an amino acid replacement matrix (Yang et al., Genetics 141, 1641-50 (1995)). The most probabilistic ancestral sequence (M-PAS) at a specific node was then reconstructed by assigning to each site the residue with the highest posterior probability. **Fig. 9B** shows the posterior probability distribution of the inferred amino acids across 106 sites for the selected sequences. The M-PASs of interest are summarized in **Fig. 10.** The genes encoding these sequences were synthesized and the proteins were expressed and purified from *E. coli* cells.

Table 1

| List of Thioredoxin sequences used for ancestral sequences reconstruction. The following GI numbers were accessed from GenBank. The names of the hosting organisms are also provided: | | | |
|---|---|---|---|
| 57164261 Ovis | 1620905 Fagopyrum | 15894825 Clostridium | 15807833 Deinococcus |
| 27806783 Bos | 46226985 Cryptosporidium | 15896334 Clostridium | 46199687 Thermus |
| 47523692 Sus | 68350806 Theileria | 20807685 Thermoanaerobacter | 15805968 Deinococcus |
| 126352340 Equus | 148804689 Plasmodium | 76789276 Chlamydia | 147669275 Dehalococcoides |

(continued)

| List of Thioredoxin sequences used for ancestral sequences reconstruction. The following GI numbers were accessed from GenBank. The names of the hosting organisms are also provided: | | | |
|---|---|---|---|
| 6755911 Mus | 11498883 Archaeoglobus | 15836191 Chlamydophila | 118047160 Chloroflexus |
| 16758644 Rattus | 116754023 Methanosaeta | 119357517 Chlorobium | 118048687 Chloroflexus |
| 146291083 Rabbit | 91773622 Methanococcoides | 119357012 Chlorobium | 118046691 Chloroflexus |
| 135773 Human | 154149646 Candidatus | 29345629 Bacteroides | 15606934 Aquifex |
| 67461921 Ponab | 88603734 Methanospirillum | 150024368 Flavobacterium | 42521808 Bdellovibrio |
| 267126 Macmu | 48477193 Picrophilus | 34539910 Porphyromonas | 39998535 Geobacter |
| 13560979 Callithrix | 150401020 Methanococcus | 29347639 Bacteroides | 42523902 Bdellovibrio |
| 126339826 Monodelphis | 124485138 Methanocorpusculum | 29346087 Bacteroides | 120602368 Desulfovibrio |
| 149412981 Ornithorhynchus | 116754438 Methanosaeta | 34540117 Porphyromonas | 39998370 Geobacter |
| 45382053 Gallus | 76802488 Natronomonas | 29346866 Bacteroides | 116619824 Solibacter |
| 29373131 Melopsittacus | 110667588 Haloquadratum | 29345628 Bacteroides | 116619449 Solibacter |
| 12958636 Ophiophagus | 55380304 Haloarcula | 32477354 Rhodopirellula | 94970094 Acidobacteria |
| 194332745 Xenopus | 76802694 Natronomonas | 32476401 Rhodopirellula | 34556879 Wolinella |
| 47215756 Tetraodon | 16120325 Halobacterium | 15608608 Mycobacterium | 15645443 Helicobacter |
| 9837585 Ictalurus | 11499727 Archaeoglobus | 57116870 Mycobacterium | 57237155 Campylobacter |
| 50539990 Danio | 13541608 Thermoplasma | 62391823 Corynebacterium | 15646067 Helicobacter |
| 194160556 Drosophila | 119720035 Thermofilum | 72163169 Thermobifida | 34557886 Wolinella |
| 17648013 Drosophila | 159040636 Caldivirga | 21219405 Streptomyces | 34556999 Wolinella |
| 194141429 Drosophila | 70607552 Sulfolobus | 72160576 Thermobifida | 159184127 Agrobacterium |
| 48104680 Apis | 15899007 Sulfolobus | 15607956 Mycobacterium | 150398433 Sinorhizobium |
| 91084205 Tribolium | 15922449 Sulfolobus | 21219599 Streptomyces | 17988305 Brucella |

(continued)

| List of Thioredoxin sequences used for ancestral sequences reconstruction. The following GI numbers were accessed from GenBank. The names of the hosting organisms are also provided: | | | |
|---|---|---|---|
| 148298796 Bombyx | 124027987 Hyperthermus | 62391938 Corynebacterium | 15603883 Rickettsia |
| 90819972 Graphocephala | 118431868 Aeropyrum | 21223797 Streptomyces | 108935910 Bovin Mitochondrio |
| 169639275 Litopenaeus | 146304377 Metallosphaera | 15611050 Mycobacterium | 194226778 Equus |
| 30580603 Geocy | 70607229 Sulfolobus | 72163508 Thermobifida | 21361403 Homo Mitochondrion |
| 115401922 Aspergillus | 15897303 Sulfolobus | 21222296 Streptomyces | 16758038 Rattus Mitochondrio |
| 119479067 Neosartorya | 126465005 Staphylothermus | 16329883 Synechocystis | 9903609 Mus Mitochondrion |
| 40746887 Aspergillus | 118431901 Aeropyrum | 17229833 Nostoc | 74318624 Thiobacillus |
| 115401518 Aspergillus | 15894111 Clostridium | 17229385 Nostoc | 121635072 Neisseria |
| 150951554 Pichia | 20808289 Thermoanaerobacter | 16331440 Synechocystis | 74316054 Thiobacillus |
| 46441186 Candida | 16079205 Bacillus | 22299829 Thermosynechococcus | 126454139 Burkholderia |
| 126213085 Pichia | 16077522 Bacillus | 22297898 Thermosynechococcus | 33602206 Bordetella |
| 50309357 Kluyveromyces | 15901736 Streptococcus | 16329237 Synechocystis | 74318419 Thiobacillus |
| 151943486 Saccharomyces | 29377495 Enterococcus | 22299630 Thermosynechococcus | 74316241 Thiobacillus |
| 50291653 Candida | 153181008 Listeria | 17229697 Nostoc | 33602001 Bordetella |
| 151941211 Saccharomyces | 28377165 Lactobacillus | 17229859 Nostoc | 66043570 Pseudomonas |
| 19114764 Schizosaccharomyces | 28379765 Lactobacillus | 22298354 Thermosynechococcus | 27364380 Vibrio |
| 167537844 Monosiga | 150393692 Staphylococcus | 17229358 Nostoc | 16124003 Yersinia |
| 67479051 Entamoeba | 138896249 Geobacillus | 126696505 Prochlorococcus | 16767191 Salmonella |
| 165988451 Dictyostelium | 30264587 Bacillus | 16331825 Synechocystis | 30064924 Shigella |
| 15236327 Arabidopsis | 16079902 Bacillus | 17227548 Nostoc1 | 67005950 Escherichia |
| 15232567 Arabidopsis | 28378864 Lactobacillus | 1351239 Pea Chloroplast | 16130507 Escherichia |

(continued)

| List of Thioredoxin sequences used for ancestral sequences reconstruction. The following GI numbers were accessed from GenBank. The names of the hosting organisms are also provided: | | | |
|---|---|---|---|
| 154721452 Limonium | 153179313 Listeria | 2507458 Spiol Chloroplast | 30063983 Shigella |
| 162461510 Zea | 29375972 Enterococcus | 11135474 Wheat Chloroplast | 16765969 Salmonella |
| 157335070 Vitis | 15901605 Streptococcus | 15594012 Pisum Chloroplast | 16123427 Yersinia |
| 145351136 Ostreococcus | 110798962 Clostridium | 11135407 Brana Chloroplast | 27366792 Vibrio |
| 53801490 Helicosporidium | 110800418 Clostridium | 46199419 Thermus | |

Thermal stability of ancient Trx enzymes

As a first step toward investigating the physico-chemical properties of these resurrected enzymes, differential scanning calorimetry (DSC) was used to measure their thermal stabilities. The denaturation temperature ($T_m$) can provide an idea about the temperature range in which the proteins are operative. **Fig. 9C** shows a plot of the $T_m$ of the resurrected enzymes against geological time. A $T_m$ of ~113 °C was measured for LBCA, AECA and LACA Trx. As observed in **Fig. 9C** (inset), LBCA Trx maintains a highly populated native state up to ~105 °C, where the thermal transition begins. By contrast, a $T_m$ for modern *E. coli* and human Trxs of 88.8 and 93.3 °C respectively, was determined. The $\Delta T_m$ between the oldest and modern Trx is ~25 °C, a similar value than that determined for bacterial EF (Gaucher et al., Nature 451, 704-7 (2008)), which corroborates the hypothesis of the thermophilic nature of LBCA, AECA and LACA (Boussau et al., Nature 456, 942-5 (2008)). In **Fig. 9C** shows a paleotemperature trend yielding a decrease in the $T_m$ of 5.8 ±1.8 K/Gyr. These results show that, in early life, Trx enzymes functioned in hot environments and that these environments have progressively cooled from 4 to 0.5 Gyr ago (Nisbet and Sleep, Nature 409, 1083-91 (2001); Gaucher et al., Nature 451, 704-7 (2008); Knauth and Lowe, Geol. Soc. Am. Bull. 115, 566-580 (2003); Schulte, M. The Emergence of Life on Earth. Oceanography 20, 42-49 (2007)). Although the thermodynamic denaturation temperatures determined for the ancestral Trxs follow a similar cooling trend that the ancient oceans, the actual values are about 50 degrees higher than the ocean temperatures inferred from maximum $\delta^{18}O$ (Gaucher et al., Nature 451, 704-7 (2008)). Accordingly, Trx evolution may operate primarily on *kinetic* stability and this could be reflected in thermodynamic stability (Godoy-Ruiz et al., J Mol Biol 362, 966-78 (2006)). However, other than loss of function upon denaturation, the particular way in which the value of $T_m$ is related to Trx enzyme fitness is still unknown.

Force-dependent chemical kinetics of disulfide reduction

It is also of great interest to examine the chemical mechanisms of disulfide bond reduction utilized by the resurrected enzymes. Given the ancient origin of the resurrected thioredoxin enzymes, with some of them predating the buildup of atmospheric oxygen, it can be assumed that chemical mechanisms of disulfide bond reduction utilized by the resurrected enzymes are closer to that of simple sulfur based molecules. Simple sulfur based molecules utilize a straightforward collision-driven substitution nucleophilic bimolecular ($S_N2$) mechanism of reduction (Kice et al., Progress in Inorganic Chemistry (ed. Edwards, J.O.) 147-206 (2007)). By contrast, Trx enzymes utilize a complex mixture of chemical mechanisms including a critical substrate binding and rearrangement reaction that accounts for the vast increase in the efficiency of Trx over the simpler sulfur compounds that were available in early geochemistry (Wiita et al., Nature 450, 124-7 (2007); Perez-Jimenez et al., Nat Struct Mol Biol 16, 890-6 (2009)).

A single molecule force-spectroscopy based assay can be used to measure the effect of applying a well-controlled force to a disulfide bonded substrate, on its rate of reduction by a nucleophile. This assay can be used to distinguish the simple $S_N2$ chemistry of nucleophiles (e.g. hydroxide, glutathione and L-Cys), from the more complex reduction chemistry of the Trx enzymes (Wiita et al., Nature 450, 124-7 (2007); Perez-Jimenez et al., Nat Struct Mol Biol 16, 890-6 (2009); Wiita et al., Proc Natl Acad Sci U S A 103, 7222-7 (2006); Koti Ainavarapu et al., J Am Chem Soc 130, 6479-87 (2008); Garcia-Manyes et al., Nature Chemistry 1, 236-242 (2009); Liang and Fernandez, Mechanochemistry: One Bond at a Time. ACS Nano (2009)). This feature makes this assay a good system to probe the chemistry of the resurrected enzymes.

This approach is described in **Figure 11.** Although different types of substrates can be used in this approach, in one embodiment, the substrate is an engineered polypeptide made of eight repeats of the I27 immunoglobulin-like protein modified by mutating to Cys positions 32nd and 75th(I27$_{G32C-A75C}$)$_8$. The cysteines oxidize spontaneously, forming disulfide bonds that are hidden within each folded I27 protein in the chain. Single polypeptides are picked up and stretched in solutions containing the desired nucleophile using an AFM. In a typical experiment, a constant force is applied to the polypeptide (175-185 pN, 0.2-0.3 s). This rapidly unfolds the I27$_{G32C-A75C}$ modules up to the disulfide bond. The unfolding events result in a stepwise increase in the length of the polypeptide where each module contributes with ~11 nm in length (**Fig. 11A, Fig. 12**). After unfolding, every disulfide bond becomes exposed to the solvent. If active Trx enzymes are present in the solution, single reduction events of ~14 nm per module can be observed (**Fig. 11A,, B; Fig. 12, Fig. 13**). All the ancestral enzymes resurrected using the methods described herein were able to trigger staircases of reduction events (**Fig 11B** and **Fig. 12, Fig. 13**) indicating that they were all active. In order to measure the reduction rate, 15 to 80 reduction staircases similar to the one shown in **Fig. 11B** can be summed and the resulting average can be fit with a single exponential. This procedure can be fitted for different pulling forces (**Fig. 11C**). The resulting set of data measures the force-dependency of the rate of reduction of the disulfide bond (**Fig. 11D**).

The chemical mechanisms of disulfide reduction can be distinguished by their sensitivity to the force applied to the substrate (Perez-Jimenez et al., Nat Struct Mol Biol 16, 890-6 (2009)). Simple thiol reducing agents show a force-dependency where the rate always increased exponentially with the applied force (Wiita et al., Proc Natl Acad Sci U S A 103, 7222-7 (2006); Koti Ainavarapu et al., J Am Chem Soc 130, 6479-87 (2008)). By contrast, modern Trx enzymes show a negative force dependency in the range of 30-200 pN (Perez-Jimenez et al., Nat Struct Mol Biol 16, 890-6 (2009)). This mechanism is consistent with a Michaelis-Menten binding reaction followed by a force-inhibited reorientation of the substrate disulfide bond, necessary for an $S_N2$ reaction to occur (Wiita et al., Nature 450, 124-7 (2007)). In a second mechanism, the rate of reduction increases exponentially at forces above 200 pN. This mechanism can be described by a simple $S_N2$ reaction and is found only in Trx enzymes of bacterial origin. Present in all thioredoxin enzymes, there is a force-independent mechanism of reduction that can be ascribed to single electron transfer reaction (Perez-Jimenez et al., Nat Struct Mol Biol 16, 890-6 (2009)).

Surprisingly, the same three reduction mechanisms can be observed in the ancient enzymes with similar patterns to those found in extant Trxs (**Fig. 11D, Fig. 14**). Indeed, the force-dependency of the reduction rate measured from the resurrected enzymes can be fit using the three-state kinetic model used with modern Trxs (Wiita et al., Nature 450, 124-7 (2007); Perez-Jimenez et al., Nat Struct Mol Biol 16, 890-6 (2009)) (Table 2).

**Table 2. Kinetic parameters for Ancestral Trxs.**

| Enzyme | $a_0$ (μM$^{-1}$·s$^{-1}$) | $\beta_0$ (s$^{-1}$) | $\gamma_0$ (μM$^{-1}$·s$^{-1}$) | $k_{10}$ (s$^{-1}$) | $\Delta x_{12}$ (Å) | $\Delta x_{02}$ (Å) | $\lambda_0$(s$^{-1}$) |
|---|---|---|---|---|---|---|---|
| LBCA Trx | 0.47±0.08 | 30±2 | 0.004±0.001 | 5.8±0.7 | -0.74±0.06 | 0.19±0.02 | 0.09±0.02 |
| LACA Trx | 8.2±0.2 | 43±3 | - | 3.8±1 | -0.76±0.04 | - | 0.38±0.05 |
| AECA Trx | 4.2±0.3 | 25±2 | 0.019±0.004 | 3.8±0.6 | -0.84±0.05 | 0.19±0.02 | 0.21±0.04 |
| LPBCA Trx | 0.47±0.04 | 30±3 | 0.017±0.002 | 4.9±0.5 | -0.71±0.01 | 0.17±0.02 | 0.19±0.02 |
| LECA Trx | 0.76±0.08 | 38±2 | - | 4.2±0.7 | -0.80±0.03 | - | 0.18±0.01 |
| LGPCA Trx | 0.48±0.02 | 34±2 | 0.012±0.002 | 3.8±0.4 | -0.83±0.02 | 0.17±0.02 | 0.35±0.02 |
| LAFCA Trx | 0.81±0.10 | 37±3 | - | 4.6±0.8 | -0.74±0.03 | - | 0.06±0.02 |
| *E. coli* Trx1* | 0.25±0.02 | 24±2 | 0.012±0.002 | 4.7±0.5 | -0.74±0.05 | 0.16±0.01 | 0.08±0.02 |
| Human Trx1* | 0.52±0.05 | 33±2 | - | 3.1±0.9 | -0.71±0.05 | - | 0.35±0.02 |
| The parameters were obtained using the kinetic model previously described (see methods section and references 14 and 15 in the main text). They are the result of numeric optimization of the global fit using the downhill simplex method. The errors correspond to the standard deviation. *E. coli* and human Trxs are also included, obtained from refs 14 and 15 (*). | | | | | | | |

One might expect that Trx enzymes from primitive forms of life should have less-developed chemical mechanisms. For instance, one of the main factors controlling the chemistry of Trx catalysis is the geometry of the binding groove. In the case of modern bacterial-origin Trxs, the binding groove is less pronounced than in eukaryotic Trxs (Perez-Jimenez et al., Nat Struct Mol Biol 16, 890-6 (2009)). This structural difference is responsible for the different chemical behavior observed in eukaryotic *versus* bacterial Trxs. If ancient enzymes had a less-structured groove, it could make their chemistry more similar to that of simple reducing agents like L-Cys or TCEP (Ainavarapu et al., J Am Chem Soc 130, 436-7 (2008)). However, the chemistry of Trx enzymes seems to have been established very early in evolution, about 4 Gyr ago, in the same manner that it is observed today. This observation shows that the step from simple reducing compounds to well-structured and functional enzymes occurred early in molecular evolution (Nisbet and Sleep, Nature 409, 1083-91 (2001)).

Nevertheless, several aspects of the catalytic mechanisms of some ancestral Trxs are intriguing. For example, high activity is observed for AECA and LACA Trxs when the substrate is pulled at forces below 200 pN (**Fig 11D** and **14B**). From the fitting of the reduction rate *versus* force data to the three-state kinetic model, an extrapolation to zero force yields rate constants of $30\times10^5$ $M^{-1}$ $s^{-1}$ for AECA Trx and $29\times10^5$ $M^{-1}$ $s^{-1}$ for LACA Trx. The extrapolation to zero force in the rest of ancestral Trxs predicts rate constants ranging from $3.7\times10^5$ $M^{-1}$ $s^{-1}$ to $6.6\times10^5$ $M^{-1}$ $s^{-1}$ (**Fig. 15**). These latter values are similar to those found in extant Trx enzymes (Perez-Jimenez et al., Nat Struct Mol Biol 16, 890-6 (2009)). Another interesting feature is the small upward slope observed at low forces for LBCA Trx with a maximum at ~100 pN (**Fig. 14A**). Although structural information would be needed to fully address this point, it seems possible that the binding between substrate and enzyme is not optimum at zero force. A better conformation can be achieved by applying force to the substrate.

Activity of ancestral Trxs in acidic conditions (pH 5)

LBCA, AECA and LACA lived in an anoxygenic environment likely rich in sulfur compounds and $CO_2$ whereas LPBCA, LECA, LGPCA and LAFCA lived in an oxygenic environment (Nisbet and Sleep, Nature 409, 1083-91 (2001)) (**Fig. 9A**). The high level of $CO_2$ in the Hadean was partly responsible for the proposed low pH of the ancient oceans (~5.5) (Walker, Nature 302, 518-520 (1983); Russell and Hall, J Geol Soc Lond 154, 377-402 (1997)). Therefore, following the hypothesis that early life lived in seawater, the natural habitat in which LBCA, AECA and LACA lived was likely to have been acidic in addition to hot. This is especially important given that the reactivity of modern Trx enzymes is due, in part, to the low $pK_a$ value of the reactive Cys: 6.7 vs. 8.0 for L-Cys (Holmgren, Thioredoxin. Annu Rev Biochem 54, 237-71 (1985)). This low $pK_a$ is needed to maintain the reactive thiolate anion form of the catalytic cysteine in the active site of the enzyme (Holmgren, Thioredoxin. Annu Rev Biochem 54, 237-71 (1985)) and is a consequence of complex electrostatic interactions between several residues that stabilize the deprotonated form of the reactive cysteine (Dyson, H.J. et al., Biochemistry 36, 2622-36 (1997). Thus, Trx activity is highly sensitive to pH and modern enzymes would not work well at low pH because the catalytic thiol would be protonated and inactive. To examine these considerations the reactivity of LACA, AECA and LBCA enzymes were compared with the extant human and *E. coli* Trx enzymes at pH 5. This analysis showed that the resurrected enzymes operate in low pH environments. The force dependency of reduction for AECA, LACA and LBCA at pH 5 was measured, over the 50-150 pN force range (**Fig.16A**). For AECA Trx, an extrapolation to zero force gives a reduction rate constant of $19\times10^5$ $M^{-1}$ $s^{-1}$ (**Fig. 16A,** solid line); similarly for LACA, a rate constant of $6.2\times10^5$ $M^{-1}$ $s^{-1}$ is estimated, whereas for LBCA Trx the reduction rates observed at pH 5 are strikingly similar to those measured at pH 7.2 (**Fig. 16A**). These are very high values similar to those measured for some modern Trx enzymes at neutral pH (Perez-Jimenez et al., Nat Struct Mol Biol 16, 890-6 (2009)). **Fig. 16B** shows a comparison of the rate constants of reduction measured at 100 pN for LBCA, LACA and AECA with modern *E. coli* and human Trxs also measured at pH 5. It is clear from these data that ancient Trx enzymes were well adapted to function under acidic conditions and that Trx enzymes were able to maintain similar reduction rate constants as they evolved into more alkaline environments.

Methods Summary

Thioredoxin sequences were retrieved from GenBank. Phylogenetic analysis and sequence reconstructions were performed using MrBayes, PAUP and PAML as previously described (Gaucher et al., Nature 451, 704-7 (2008)). The reconstructed sequences were synthesized, cloned into pQE80L vector and expressed in *E. coli* cells. Protein engineering and purification was carried as described in Wiita et al., Nature 450, 124-7 (2007). Thermal stabilities were measured using a VP-Capillary DSC calorimeter from MicroCal. The heat capacity vs. temperature profiles were analyzed following the two-state thermodynamic model (Ibarra-Molero et al., Biochemistry 38, 8138-49 (1999)). AFM experiments were performed in a custom-made apparatus in its force-clamp mode (Fernandez and Li, Science 303, 1674-8 (2004)). Silicon nitride cantilevers were used with a typical spring constant of 0.02 N/m. The buffer used in the experiments contained 10mM HEPES, 150 mM NaCl, 1 mM EDTA, 2 mM NADPH, pH 7.2. Individual $(I27_{G32C-A75C})_8$ proteins are stretched at a constant force of 175-185 pN during 0.2-0.3 s. This pulse unfolds the modules up to the disulfide bond. The test-pulse force is then applied during several seconds to allow capturing all the possible reduction events. Trx reductase 50 nM (eukaryotic or bacterial) or DTE 200 $\mu$M was used to keep Trx enzymes in their reduced state. The traces containing reduction events are summated, normalized and fitted with a single exponential obtaining thus the reduction rate ($r = 1/\tau$). A kinetic model containing two force-dependent rate constants was applied. The kinetic parameters were solved using matrix analysis and the errors were estimated using the bootstrap method. Igor software was used for data collection and analysis.

Phylogenetic analysis and ancestral sequence reconstruction.

A total of 203 thioredoxin sequences from the three domains of life were retrieved from GenBank (Table 1). Sequences were aligned using MUSCLE (Edgar, Nucleic Acids Res 32, 1792-7 (2004)) and further corrected manually. The phylogenetic analysis was carried out by the minimum evolution distance criterion with 1000 bootstrap replicates using PAUP* 4.0 beta. Ancestral sequences were reconstructed using PAML version 3.14 and incorporated the gamma distribution for variable replacement rates across sites (Yang, Comput Appl Biosci 13, 555-556 (1997)). For each site of the inferred sequences, posterior probabilities were calculated for all 20 amino acids. The amino acid residue with the highest posterior probability was then assigned at each site.

Protein Expression and Purification.

Genes encoding the ancestral Trxs enzymes were synthesized and codon-optimized for expression in *E. coli* cells. The genes were cloned into pQE80L vector (Qiagen) and transformed in *E. coli* BL21 (DE3) cells (Invitrogen). Cells were incubated overnight in LB medium at 37 °C and protein expression was induced with 1mM IPTG. Cell pellets were sonicated and the His 6-tagged proteins were loaded onto *His GraviTrap* affinity column (GE Healthcare). The purified protein was verified by SDS-PAGE. The proteins were then loaded into PD-10 desalting column (GE Healthcare) and finally dialyzed against 50 mM HEPES, pH 7.0 buffer. The preparation of $(I27_{G32C-A75C})_8$ was carried out as follows: mutations Gly32Cys and Ala75Cys are introduced into the I27 module using the QuickChange site-directed mutagenesis protocol. Multi-step cloning was performed to produce an N-C-linked eight-domain polypeptide. The gene encoding the polypeptide was cloned into a pQE80L and the protein was expressed at 37 °C for 4 hours in *E. coli* BLR (DE3) cells. Cell pellet was lysed using a French press. The polypeptide with a His 6-tagged was purified using Talon-Co$^{2+}$ resin. The protein was further purified by size exclusion chromatography on a Superdex 200 HR 10/30 column. The protein was eluted in 10 mM HEPES, 150 mM NaCl, 1 mM EDTA, pH 7.2.

DSC experiments

Thermal stabilities of ancestral and modern Trx enzymes were measured with a VP-Capillary DSC (MicroCal). Protein solutions were dialyzed into a buffer of 50 mM HEPES, pH 7. The scan speed was set to 1.5 K/min. Several buffer-buffer baselines were first obtained for proper equilibration of the calorimeter. Concentrations were 0.3-0.7 mg/mL and were determined spectrophotometrically at 280 nm using theoretical extinction coefficients and molecular weights. The experimental traces were analyzed following the two-state thermodynamic model (Ibarra-Molero et al., Biochemistry 38, 8138-49 (1999)).

AFM experiments

The atomic force microscope used is a custom-made design (Fernandez and Li, Science 303, 1674-8 (2004)). Data acquisition is controlled by two PCI cards 6052E and 6703 (National Instruments). Cantilever model MLCT of silicon nitride were used. We calibrate the cantilever using the equipartition theorem (Florin et al., Biosensors & Bioelectronics 10, 895-901 (1995)) giving rise to a typical spring constant of 0.02 N/m. The AFM works in the force-clamp mode with length resolution of 0.5 nm. The feedback response can reach 5 ms. The buffer used in the experiment is 10 mM HEPES, pH 7.2, 150 mM NaCl, 1 mM EDTA, 2mM NADPH. Trx enzymes are added to a desired concentration. The buffer also contains Trx reductase 50nM (prokaryotic or eukaryotic) to keep Trx enzymes in their reduced state. *E. coli* Trx reductase works well with bacterial-origin Trx enzymes whereas eukaryotic Trx reductase works with Archaea/Eukaryote Trx enzymes. Similar results are obtained when using DTE 200 $\mu$M to keep Trx enzymes reduced, thus demonstrating that modern Trx reductases maintain fully reduced ancestral Trx enzymes. For the experiments at pH 5, 20 mM sodium acetate buffer and 200 $\mu$M DTE was used.

To perform the experiment 3-6 $\mu$l of substrate at ~0.1 mg/mL was deposited on a gold-covered coverslide. A drop of ~100 $\mu$l containing the Trx solution was then added. The force-clamp protocol consists of three pulses of force. In the first pulse the cantilever tip was pressed against the surface at 800 pN for 2s. In the second pulse the attached $(I27_{G32C-A75C})_8$ is stretched at 175-185 pN for 0.2-0.3s. The third pulse is the test force where the reduction events are captured. This pulse is applied at different forces 30-500 pN time enough to capture all the possible reduction events.

The traces were collected and analyzed using custom-written software in Igor Pro 6.03. The traces containing the reduction events at each force were summated, normalized and fitted with a single exponential. From the fitting we can obtain a time constant, $\tau$, and thus the reduction rate at a given force (r = 1/$\tau$). Bootstrapping method was used to obtain the error of the reduction rates. The bootstrapping was run 1000 times for each reduction rate obtaining a distribution from where the s.e.m. can be calculated.

AFM data analysis

The data were fitted following a three-state kinetic model previously described (Wiita et al., Nature 450, 124-7 (2007); Perez-Jimenez et al., Nat Struct Mol Biol 16, 890-6 (2009)). In this model three different chemical mechanisms are taken into account. The rate constants used in the kinetic model are:

$$k_{01} = \alpha_0[\text{Trx}]$$

$$k_{12} = \beta_0 \exp(F\Delta x_{12}/k_B T) + \lambda_0$$

$$k_{02} = \gamma_0 [\text{Trx}] \exp(F\Delta x_{02}/k_B T) + \lambda_0$$

$$k_{10} = \delta_0$$

Rate constants $k_{01}$ and $k_{02}$ depend on Trx concentration in a linear manner. $k_{12}$ and $k_{02}$ exponentially depend on force. The kinetic model is solved using matrix analysis and parameters $\alpha_0$, $\beta_0$, $\Delta x_{12}$, $\gamma_0$, $\Delta x_{02}$, $\lambda$ and $\delta_0$ can be obtained for each ancestral enzyme. The optimal kinetic parameters are calculated by numerical optimization using the downhill simplex method (Nelder and Mead, Computer Journal 7, 308-313 (1965) (Table 2).

A brief explanation of the different chemical mechanisms is as follows: when the substrate is stretched at low force (below 200 pN) $k_{01}$ and $k_{12}$ dominate. The negative force dependence observed in all Trx enzymes (ancestral and modern) gives rise to a negative value of $\Delta x_{12}$. This is consistent with a shortening of the polypeptide chain. This shortening was explained by a force-inhibited rotation of the disulfide bond necessary for the correct alignment of the S-S bond (180°) for an $S_N2$ reaction to occur. This mechanism is similar to a Michaelis-Menten reaction in which the formation of an enzyme-substrate complex is crucial. A second reduction mechanism occurs at forces over 200 pN where $k_{02}$ dominates. In the case of bacterial-origin Trxs, the rate of reduction is exponentially accelerated. This is consistent with a simple $S_N2$ reaction with an elongation of the disulfide bond at the transition state, $\Delta x_{02}$. This elongation, ~0.18 Å, is only observed in bacterial-origin Trxs. In the case of eukaryotic-origin Trxs the rate of disulfide bond reduction when the substrate is pulled at forces over 200 pN is essentially force-independent. In this case $k_{02} = \lambda_0$. This force-independent mechanism is explained by a single-electron transfer reaction accounted for the parameter $\lambda_0$ in the kinetic model. This mechanism seems to be ubiquitous to all Trx enzymes but is certainly remarkable in eukaryotic-origin Trxs. The origin of this diversity of chemical mechanisms was explained on the basis of the structural features of the binding groove (Perez-Jimenez et al., Nat Struct Mol Biol 16, 890-6 (2009)).

**Example 2: Reconstruction of Ancient Thioredoxin Enzymes**

Described herein is data demonstrating the feasibility of reconstructing ancient thioredoxin enzymes from predicted nodes. For example, the predicted DNA sequence of a Trx enzyme from the node corresponding to the Last Bacterial Common Ancestor, dated about 4 billion years ago, was selected for gene synthesis and protein expression in our laboratory (Figure 4).

The resuscitated LBCA Trx showed a 26 °C higher denaturation temperature than that of modern E. coli Trx. Higher denaturation temperatures have also been reported for resuscitated elongation factor proteins (Gaucher et al., Nature, 2008. 451(7179): p. 704-U2; Gaucher et al., Nature, 2003. 425(6955): p. 285-8). The LBCA thioredoxin enzyme also showed a high rate of catalysis at pH 5, where extant enzymes are largely inactive (Figure 4B). While this ancestral enzyme showed the typical biphasic force-dependent catalysis of the extant enzymes (Wiita, A.P., et al., Nature, 2007. 450(7166): p. 124-7), its peak activity was measured at 100 pN, suggesting a less well developed binding groove (Figure 4C).

Results with the last bacterial common ancestor Trx enzyme show the feasibility of resurrecting active enzymes that disappeared from Earth millions of years ago. This approach can be used to uncover variations in the chemical mechanisms of thioredoxin catalysis (Figure 4) and correlate them with the structure of these enzymes. These methods can also be used to generate one or more thioredoxin enzymes with characteristics not present in the extant enzymes (e.g. the absence of a binding groove).

The force-dependent rate of reduction shows that human Trx, which has a much deeper groove than that of *E. coli,* excludes the force accelerated mechanism of reduction (type III in Figure 3C). In addition to depth and length, another characteristic of the binding groove that can be examined is the mean hydrophobicity per residue of a Trx enzyme. These parameters can be measured directly from over one hundred Trx structures currently available in PDB. Extreme examples of each groove parameter can be identified. These specific Trxs can be expressed to complete the force-spectroscopy experiments. The relative amplitude of each chemical mechanism of reduction measured using force spectroscopy, and the measured features of the binding groove can be correlated and calculated from the structure.

**Example 3: Single Molecule Assays to Examine Other Bond Cleaving Enzymes**

Any enzyme that cleaves covalent bonds can be investigated using the single molecule force spectroscopy assay described herein. Exemplary molecules that can be examined using the methods described herein include but are not limited to proteases. Proteases are a vast group of proteins with highly important physiological functions (Lopez-Otin and Bond, J Biol Chem, 2008. 283(45): p. 30433-7). The fact that their catalytic mechanisms have been thoroughly studied by traditional techniques facilitates interpretation of the single-molecule results (Frey and Hegeman, Enzymatic reaction mechanisms. 2007, Oxford: Oxford University Press). The high substrate specificity shown by some proteases can be used to design substrates suitable for single-molecule force spectroscopy. The proteolysis of those substrates can be studied under force. The catalytic activity of proteases will a complex force dependency because proteases have substrate-binding grooves that are similar to those found in thioredoxin enzymes and because the chemical mechanism of proteolysis can involve geometric rearrangements at the transition state (Frey and Hegeman, Enzymatic reaction mechanisms. 2007, Oxford: Oxford University Press). As in the case of thioredoxins, the molecular interpretation of the force dependency of proteases will shed light into the sub-Ångström contortions of the substrate atoms as they are cleaved by the protease during catalysis.

To determine the force-dependency of protease catalysis, an appropriate substrate that can detect single protease cleavage events will be constructed. Because simply cleaving the backbone of a mechanically stretched protein would be the end the experiment because the polypeptide would loose its mechanical continuity, a substrate which retains its mechanical integrity upon cleavage and which also extends sufficiently to provide an unmistakable fingerprint will be constructed.

An exemplary substrate, as set forth in Figure 19, can be designed by introducing two cysteines in a given protein (e.g. the 127 protein). The cysteines can be placed at a distance from one another so that they do not form a disulfide bond (residues A and B, Figure 19A). The free cysteines can be used as specific conjugation points for a polypeptide containing the protease recognition sequence. The use of cysteines to specifically label proteins is commonplace in modern molecular biology (Wynn et al., Methods Enzymol, 1995. 251: p. 351-6; Crankshaw and Grant, Curr Protoc Protein Sci, 2001. Chapter 15: p. Unit15 1; Corey, Methods Mol Biol, 2004. 283: p. 197-206). Typically, maleimide (Ji, Methods Enzymol, 1983. 91: p. 580-609) or sulfhydryl reagents (Cecconi et al., Eur Biophys J, 2008. 37(6): p. 729-38)) are employed. Indeed, a variety of bifunctional reagents (Green et al., Protein Sci, 2001. 10(7): p. 1293-304) can be employed to trap proteins in specific conformations (Milanesi et al., Biochemistry, 2008. 47(51): p. 13620-34; Cipriano et al., Proteins, 2008. 73(2): p. 458-67). Cysteine residues were introduced in positions 27 and 55 of the 127 protein (Figure 19A), and bridged with the bifunctional reagent BMDB to creating a covalent bridge between positions 27 and 55 of the 127 protein. Mechanical unfolding of an 127 protein gives a normal extension of $\Delta L=29$ nm (Figure 19B). When mutant 127 proteins are reacted with the bifunctional reagent BMDB, the unfolding is now limited to only $\Delta L=20$ nm due to the presence of a covalent bridge formed by the BMDB (Figure 19C). Bifunctional bridges with short polypeptides that can be cleaved by a protease (e.g. enterokinase) can be created. For example, 127 polypeptides that serve as substrates for the enzyme enterokinase can be generated. This enzyme is readily available and of wide commercial use and specifically cleaves the sequence Asp-Asp-Asp-Asp-Lys-X after the Lys, as long as X is not a proline (Light and H. Janska Trends Biochem Sci, 1989. 14(3): p. 110-2). In such cases, cleavage of the covalent bridge will result into a further extension by $\Delta L=9$ nm which uniquely identifies the cleavage reaction (Figure 19D). As in the case of thioredoxin activity, the rate of appearance of the 9 nm steps measures the rate of catalysis at different forces.

Although the covalent bridge design works (Figure 19A, B,C), the efficiency of the bridging reaction is in the range of 30-40%, leaving open the remaining 127 proteins of a polypeptide. This limits the number of cleavage events that can be detected per polypeptide. A variety of additional bifunctional enterokinase substrates either with thiols or maleimides can be constructed and those that have the highest bridging efficiency can be selected for additional analysis.

Short polypeptides containing a cleavage sequence and terminated by either thiols or maleimides (to covalently link the short polypeptide to the exposed cysteines) can also be generated. Because the intra-molecular conjugation scheme described herein is also dependent on the distance between the reactive groups, the position of the exposed cysteines conjugating bifunctional reagents (recognition sequences) can be varied among different lengths until optimal constructs are identified. The force dependency of the catalytic activity of enterokinase can be studied using these substrates. Given that enterokinase contains a substrate-binding groove (Lu et al., J Mol Biol, 1999. 292(2): p. 361-73), and that the chemistry of proteolysis involves structural rearrangements of the participating atoms (e.g. formation of a tetrahedral intermediate), these substrates can be used to determine whether force exerts a complex effect on enterokinase activity. Once the force-dependency of protease catalysis is measured, kinetic models can be developed to explain the data. In particular, the measured force dependency can be used to formulate activity models as a series of chemical mechanisms that require bond rotations/elongation of the recognition sequence. The effect of width, depth and hydrophobicity of the binding groove can be studies as functions of the measured force dependent mechanisms. This approach can also be extended to study other specific proteases such as factor Xa and thrombin as well as the role of substrate conformations in enzymatic catalysis. This approach can also be important for the development of drug targets given the medical importance of protease inhibitors.

**Example 4 : A single molecule assay for thioredoxin catalysis**

An octamer of the I27 module can be mutated to incorporate two cysteine residues (G32C, A75C; Figure 1, gold labeled residues). The two cysteine residues spontaneously form a stable disulfide bond that is buried in the $\beta$-sandwich fold of the I27 protein. This is polypeptide $(I27_{S-S})_8$. The disulfide bond mechanically separates the I27 protein into two parts (Figure 1A). The unsequestered amino acids that readily unfold and extend under a stretching force are depicted in red. The blue region marks 43 amino acids which are trapped behind the disulfide bond (Figure 1B) and can be extended if the disulfide bond is reduced by a nucleophile such as the enzyme Trx (Figure 1C). Force-clamp AFM can be used to extend single $(I27_{S-S})_8$ polypeptides. The constant force causes individual I27 proteins in the chain to unfold, resulting in stepwise increases in length of the molecule following each unfolding event. After unfolding, the stretching force is directly applied to the now solvent exposed disulfide bond, and if a reducing agent is present in the bathing solution, the bond can be chemically reduced giving rise to a new stepwise increase in length of the polypeptide (Figure 1D). The size of the step increases in length observed during these force clamp experiments corresponds to the number of amino acids released, serving as a precise fingerprint to identify the reduction events. The rate of disulfide bond reduction can be measured at a given force by fitting a single exponential to an ensemble average of many reduction traces (Wiita, A.P., et al., Nature, 2007. 450(7166): p. 124-7; Perez-Jimenez, et al., Nature Structural & Molecular Biology, 2009. 16(8): p. 890-U120; Ainavarapu et al., Journal of the American Chemical Society, 2008. 130(20): p. 6479-6487). Figure IE shows a plot of the rate of reduction as a function of force for experiments done in the presence of human Trx, E. Coli Trx and the simpler nucleophile L-Cysteine. From these data, at least three different types of force-dependencies can be distinguished. These force dependencies may be related to the particular arrangement of the substrate in the binding groove of the enzymes (Perez-Jimenez, et al., Nature Structural & Molecular Biology, 2009. 16(8): p. 890-U120). In the case of L-Cys, the force dependency can arise from the much simpler $S_N2$ arrangement of a simple nucleophile (Ainavarapu et al., Journal of the American Chemical Society, 2008. 130(20): p. 6479-6487; Wiita et al., Proc Natl Acad Sci U S A, 2006. 103(19): p. 7222-7). The classical assays for disulfide bond reduction would only show bulk rates at zero force. The increased detail observed in the enzymatic mechanisms can now be interpreted at the molecular/atomic level (Perez-Jimenez, et al., Nature Structural & Molecular Biology, 2009. 16(8): p. 890-U120).

**Example 5: Simultaneous Measurement of Association/Dissociation and Reduction Reactions in Single Thioredoxin Enzymes.**

The methods described herein can be used to detect when the enzyme reduces a target disulfide bond. To determine when Trx enzymes bind to a substrate, how long it takes to reduce the substrate after binding and how long the enzyme remains attached to the substrate after the reduction event, force-clamp assays of disulfide bond reduction can be combined with single molecule fluorescence detection of enzyme binding to the exposed substrate using our newly developed AFM/TIRF instrument (Figure 5). To observe enzymatic binding to a mechanically extended substrate, Trx enzymes can be labeled with a fluorophore (e.g. Alexa Fluor 488 fluorophore). Fluorophores, such as Alexa Fluor 488 dye, can readily be ligated to the exposed primary amines of a protein. A Trx enzyme may contain up to 12 lysine residues with varying degrees of exposure to the solvent. Force-clamp experiments show that labeled E. coli Trx enzymes are bright and reduce the substrate disulfide bonds at a rate of 0.3 s$^{-1}$, which is only about half of the rate measured with the unlabeled enzyme (Figure 17A).

The labeled enzymes can be observed in the TIRF microscope. Figure 17B shows a labeled enzyme visiting the evanescent field of a TIRF microscope driven by Brownian motion. Single enzymes are brightly fluorescent and can be monitored as a function of time using an efficient CCD camera (Andor Technology). These capabilities can be used to follow the binding and dissociation of labeled Trx enzymes interacting with their target disulfide bonds, while at the same time assaying their reduction using force-clamp spectroscopy. Such analysis can be used to measure directly the rates of association and dissociation of single enzymes as they bind and reduce single disulfide bonds in an extended protein. Data sets, such as those shown in Figure 5B can be collected using the methods described herein. The methods described herein can also be used to determine whether the rates of association and dissociation are force-dependent and to refine simplified models of binding and reduction (Wiita, A.P., et al., Nature, 2007. 450(7166): p. 124-7).

The dissociation dwell time of the enzyme after a disulfide bond has been reduced can be measured from the combined AFM/TIRF experiments (Figure 5). Since Trx is covalently linked to the substrate immediately after the catalytic reaction (Holmgren, A., Thioredoxin and glutaredoxin systems. J Biol Chem, 1989. 264(24): p. 13963-6), this dwell time depends on both an intermolecular reduction event and the off-rate of the non-covalently bound enzyme. As a control experiment, the WT Trx can be switched for a C35A mutant Trx that is redox active but incapable of detaching from the substrate after reducing it (Wynn et al., Methods Enzymol, 1995. 251: p. 351-6). In this case, the Trx enzyme catalyzing the reaction will remain stationary and visible in the evanescent excitation field until it is photobleached. Such methods can be used to capture the association and dissociation reaction of a single thioredoxin enzyme with its target during disulfide bond reduction. Every step involved in the activity of single thioredoxin enzymes can be separated and measured independently, allowing for the development of detailed kinetic model for this enzyme and the mechanisms by which it finds its target.

**Example 6: Detecting the oxidase activity of thioredoxin enzymes.**

The single molecule assay described herein can also be used to study oxidative folding by thioredoxin enzymes. In vivo, thiol-disulfide exchange reactions are catalyzed by a number of enzymes belonging to the thioredoxin (Trx) superfamily. All of these enzymes share the thioredoxin fold and most feature a CXXC active site motif (Martin, Structure, 1995. 3(3): p. 245-50). In humans and other eukaryotes, thioredoxin catalyzes the cleavage of disulfide bonds whereas PDI enzymes catalyze their oxidation and isomerization. However the function of PDI as an oxidase is not unique given that, in S. cerevisiae, deletion strains lacking the essential gene encoding PDI can be rescued by a gene encoding for a simple thioredoxin C35S mutant (Chivers et al., EMBO J, 1996. 15(11): p. 2659-67). This thioredoxin variant has a CXXS active site, meaning that the conventional pathway for substrate reduction is not possible. In addition, PDI-like enzymes with CXXS active sites have also been shown to complement this yeast deletion strain (Tachibana et al., Mol Cell Biol, 1992. 12 (10): p. 4601-11; LaMantia et al., Cell, 1993. 74(5): p. 899-908). In certain aspects, the new single molecule oxidative folding assay described herein (e.g. Figure 18) can be used to determine whether (1) the requirements for catalysis of oxidative folding are the same as those for disulfide bond reduction, (2) whether the C-terminal cysteine functions as a switch between these processes, and (3) the binding groove play a key role in oxidative folding.

As shown in Figure 18, a protein made of eight disulfide bonded repeats (I27$_{S-S}$) can be picked up and stretched. In one embodiment, the protein is in a buffer containing 10 $\mu$M of wild type human Trx enzyme. The polypeptide is then exposed to a pulling force of 110-150 pN (denature), which results into a number of stepwise extensions. As shown in Figures 1A-1D, steps of 11 nm correspond to unfolding events where a single domain extends up to the disulfide bond. This exposes the disulfide and enables its reduction by the thioredoxin enzyme. Reduction of the disulfide in turn releases an additional 14 nm of the polypeptide chain. These precise step lengths serve as a fingerprint identifier that unambiguously verify these events. When all domains are unfolded and reduced, the force is switched off and the protein is allowed to refold for some time ($\Delta$t=5 s; Figure 18). The force is then again switched on (probe) triggering again a series of stepwise elongations if any refolding had taken place. As soon as the force is switched on, folding is abruptly stopped and the folded status of each substrate domain can be probed at a time $\Delta$t after refolding was initiated. During the probe pulse, the protein extends in steps of 25 nm. This step size corresponds to the sum of unfolding (11 nm) and reduction (14 nm) steps and thus marks the unfolding of a domain without a formed disulfide bond. While not all the domains refolded during the folding period, none of the refolded domains formed a disulfide bond, indicating that the wild type form of thioredoxin does not catalyze reoxidation (Figure 18B). By contrast if the experiment shown in Figure 18A,B is repeated in the presence of the C35S human thioredoxin mutant (hTrx$^{C35S}$), the step sizes during the probe pulse are now entirely composed of 11 and 14 nm steps, indicating the full reoxidation of all the disulfide bonds (Figure 18C).

Shown in Figure 18 is a demonstration of the sensitivity of the oxidative folding assay described herein. As shown, in Figure 18, the assay enables detection that that the replacement of a single atom in the catalytic site of the enzyme (from sulfur to oxygen) in human thioredoxin is sufficient for hTrx to gain the oxidase function, in addition to keeping intact its reductase activity. These results explain why hTrx$^{C35S}$ can rescue PDI deletion strains of S. cerevisiae (Chivers et al., EMBO J, 1996. 15(11): p. 2659-67).

To study the oxidase mechanisms of thioredoxin, the value of $\Delta$t can ne varied in order to determine the rate of reoxidation by hTrx$^{C35S}$. The force dependency of the rate of reoxidation can be measured by quenching the force to different values during the folding/reoxidation period $\Delta$t. The methods described herein may also reveal a complex force dependency from substrate-enzyme interactions during oxidative folding. To study the role played by the binding groove in the reoxidation of the substrate, the C35S mutation will be engineered into E. coli thioredoxin enzymes. E. coli thioredoxin enzymes that have a much shallower groove than human Trx and show different mechanisms in its force dependency (Figure 3). The properties of the binding groove can be an important factor in reoxidation. To determine whether the full folding of the host I27 protein is a necessary condition for reoxidation the number of 11 nm steps (unfolding of a natively folded protein) will be compared with the number of 14 nm steps (reduction of re-oxidized bonds) observed during the probe pulse (Figure 18A). For example, if folding is not necessary, then there will be more steps of 14 nm than steps of 11 nm, etc. These results can be used to determine how the association and dissociation cycles are affected by the C35S mutation in single thioredoxin enzymes and to correlate the reoxidation events with the binding/unbinding reactions of fluorescently labeled Trx[C35S] enzymes (Figure 5). The combined folding/reoxidation assay shown in Figure 18 together with experiments similar to those highlighted in Figure 5, can be used to reveal the dynamics of a single thioredoxin enzyme as it oxidizes a target disulfide bond during the folding of the host protein.

The single molecule assays described herein have the ability to identify and separate the different stages of protein folding (Garcia-Manyes et al., PNAS, 2009. 106(26): p. 10534-10539; Garcia-Manyes et al., PNAS, 2009. 106(26): p. 10540-10545), and can thus be used to determine at what stage of folding a thioredoxin enzyme is capable of oxidizing a substrate. Although the finding described herein show that the human thioredoxin mutant hTrx[C35S] gains oxidase activity, the methods described herein can also be used to determine whether the C35S mutation can have a similar effect on other members of the thioredoxin family with different groove depths.

**Example 7: Other Activities of Resurrected Enzymes**

**Fig. 20** shows the rate constants for disulfide bond reduction by ancestral and modern Trxs enzymes. Although these latter values are within the same range of those found in extant Trx enzymes using AFM (**Fig. 14** and Perez-Jimenez et al., Nat Struct Mol Biol 16, 890-6 (2009)) and bulk experiments (Holmgren et al., J Biol Chem 254, 9113-9 (1979)), there was a trend in the reconstructed enzymes to show higher reduction rates at forces below 200 pN (**Fig.14**). It is speculated that this trend may be related to substrate specificity of the enzymes. Ancient enzymes may be less substrate specific than modern ones, and therefore, might be more efficient with generic substrates such as those used herein.

The activity of the ancestral enzymes was measured using the conventional insulin assay (**Fig. 21**) The values of insulin precipitation rates obtained with this assay are similar to those previously determined for *E. coli* Trx (Suarez, M. et al., Biophys Chem 147, 13-9 (2010); Holmgren, A., J Biol Chem 254, 9627-32 (1979)).

**Fig. 22** shows a comparison of the rate of reduction measured at 100 pN for LBCA, LACA and AECA with the rates of some modern *Trx* enzymes also measured at pH 5.

Due to spontaneous precipitation of insulin at pH below 6, DTNB was used as a substrate for disulfide reduction to further verify the ability of the oldest enzymes to work at pH 5 (**Fig. 23**). This analysis of reconstructed enzymes indicated that ancient Trx enzymes were well adapted to function under acidic conditions and that Trx enzymes could maintain similar reduction rate constants as they evolved in more alkaline environments. A feature of the thioredoxin family of enzymes is that many of them are secreted to the extracellular environment where most disulfide-bonded proteins are found (Xu, S.Z. et al., Nature 451, 69-72 (2008); Windle, H.J., Fox, A., Ni Eidhin, D. & Kelleher, D., J Biol Chem 275, 5081-9 (2000). From this perspective, thioredoxin enzymes are perhaps one of the few types of enzymes for which a correlate can be established between their pH sensitivity and the environmental conditions found outside cells (Xu, S.Z. et al., Nature 451, 69-72 (2008); Windle, H.J., Fox, A., Ni Eidhin, D. & Kelleher, D., J Biol Chem 275, 5081-9 (2000). It is informative to compare the acid tolerance of the resurrected enzymes with enzymes from extant extremophiles. For example, Trx from *Sulfolobus tokodaii* (thermophilic archaea (Ming, H. et al., Proteins 69, 204-8 (2007)), with a melting temperature of 122° C (**Fig. 24**), is active at pH 7 ($0.12 \times 10^5$ M$^{-1}$ s$^{-1}$ at 50 pN), but does not show detectable activity at pH 5 (**Fig. 22**) which is not surprising given that *Sulfolobus* regulates its cytosolic pH (Baker-Austin, C. & Dopson, M., Trends Microbiol 15, 165-71 (2007). By contrast, Trx from *Acetobacter aceti* (acidophilic bacteria (Starks, C.M., Francois, J.A., MacArthur, K.M., Heard, B.Z. & Kappock, T.J. Protein Sci 16, 92-8 (2007) that grows at pH 4) is active at pH 5 ($0.6 \times 10^5$ M$^{-1}$ s$^{-1}$ at 100 pN), reflecting its acidic cytosol (Starks, C.M., Francois, J.A., MacArthur, K.M., Heard, B.Z. & Kappock, T.J., Protein Sci 16, 92-8 (2007); Menzel, U. & Gottschalk, G., Archives of Microbiology 143, 47-51 (1985).

Method Summary

Thioredoxin bulk enzymatic measurements. Bulk-solvent oxidoreductase activity for ancestral thioredoxins was determined using the insulin precipitation assay as described (Suarez, M. et al., Biophys Chem 147, 13-9 (2010); Holmgren, A., J Biol Chem 254, 9627-32 (1979); Perez-Jimenez et al., J. Biol. Chem., 283: 27121-27129 (2008)). In order to further verify the activity of ancestral Trxs enzymes at acidic pH, DTNB (5,5'-dithiobis-(2-nitrobenzoic acid)) was used as a substrate at pH 5. In this assay, Trxs enzymes were preactivated by incubation with 1 mM DTT. The reaction was initiated by adding active Trx to a final concentration of 4 $\mu$M to the cuvette containing 1mM DTNB in 20 mM sodium acetate buffer, pH 5. Change in absorbance at 412 nm due to the formation of TNB was followed during 1 min. Activity was determined from the slope $d\Delta A_{412}/dt$. A control experiment lacking Trx was registered and subtracted as baseline.

**Example 8: Crystal Structure of Ancestral Enzyme Thioredoxin AECA**

The crystal structure of the ancestral enzyme thioredoxin AECA is depicted in Figure 25.

<u>Table 3</u>: Refinement Summary of Crystal Structure of Ancestral Enzyme Thioredoxin AECA

```
REMARK ****************** REFINEMENT SUMMARY: QUICK FACTS ******************
REMARK Start: r_work = 0.3754 r_free = 0.3753 bonds = 0.001 angles = 0.295
REMARK Final: r_work = 0.2284 r_free = 0.3032 bonds = 0.009 angles = 1.278
REMARK *************************************************************************
REMARK
REMARK Rigid body refinement target: auto
REMARK Information about total rigid body shift of selected groups:
REMARK                                  rotation (deg)              translation
(A)
REMARK                          xyz          total        xyz          total
REMARK  group  1:  0.066  -0.035  0.032  0.08    0.02  -2.19  0.01   2.19


REMARK ***************** REFINEMENT STATISTICS STEP BY STEP *****************
REMARK leading digit, like 1_, means number of macro-cycle
REMARK 0   : statistics at the very beginning when nothing is done yet
REMARK 1_bss: bulk solvent correction and/or (anisotropic) scaling
REMARK 1_xyz: refinement of coordinates
REMARK 1_adp: refinement of ADPs (Atomic Displacement Parameters)
REMARK 1_sar: simulated annealing refinement of x,y,z
REMARK 1_wat: ordered solvent update (add / remove)
REMARK 1_rbr: rigid body refinement
REMARK 1_gbr: group B-factor refinement
REMARK 1_occ: refinement of occupancies
REMARK ------------------------------------------------------------------
REMARK  R-factors, x-ray target values and norm of gradient of x-ray target
REMARK  stage      r-work r-free  xray_target_w  xray_target_t
REMARK    0    :  0.4439 0.4580   3.996716e+00   4.056539e+00
REMARK    1_bss:  0.3754 0.3753   3.859379e+00   3.906067e+00
REMARK    1_rbr:  0.3755 0.3716   3.857346e+00   3.906505e+00
REMARK    1_bss:  0.3754 0.3715   3.856606e+00   3.901563e+00
REMARK    1_fit:  0.3543 0.3590   3.816897e+00   3.875086e+00
REMARK    1_xyz:  0.2973 0.3436   3.698549e+00   3.866009e+00
REMARK    1_adp:  0.2655 0.3319   3.619059e+00   3.822724e+00
REMARK    1_occ:  0.2694 0.3249   3.623934e+00   3.822419e+00
REMARK    2_bss:  0.2644 0.3224   3.616364e+00   3.822741e+00
REMARK    2_sar:  0.2463 0.3110   3.584169e+00   3.805693e+00
REMARK    2_fit:  0.2584 0.3092   3.610219e+00   3.800907e+00
REMARK    2_xyz:  0.2367 0.3132   3.560169e+00   3.782447e+00
REMARK    2_adp:  0.2325 0.3113   3.548015e+00   3.770248e+00
REMARK    2_occ:  0.2325 0.3113   3.548015e+00   3.770248e+00
REMARK    3_bss:  0.2308 0.3100   3.546772e+00   3.769120e+00
REMARK    3_fit:  0.2364 0.3034   3.546495e+00   3.757393e+00
REMARK    3_xyz:  0.2281 0.3097   3.544193e+00   3.770737e+00
```

```
REMARK      3_adp:  0.2281 0.3080    3.542285e+00    3.767369e+00
REMARK      3_occ:  0.2281 0.3080    3.542285e+00    3.767369e+00
REMARK      3_bss:  0.2284 0.3032    3.536917e+00    3.762045e+00
REMARK   --------------------------------------------------------------------
REMARK   stage      k_sol   b_sol      b11      b22      b33     b12      b13      b23
REMARK     0   :    0.000    0.000    0.000    0.000    0.000   0.000    0.000    0.000
REMARK     1_bss:   0.277   22.223   -8.643   18.065   -9.422   0.000   -7.697   -0.000
REMARK     1_rbr:   0.278   22.363   -8.344   18.129   -9.095   0.000   -7.927    0.000
REMARK     1_bss:   0.278   22.363   -8.344   18.129   -9.095   0.000   -7.927    0.000
REMARK     1_fit:   0.278   22.363   -8.344   18.129   -9.095   0.000   -7.927    0.000
REMARK     1_xyz:   0.278   22.363   -8.344   18.129   -9.095   0.000   -7.927    0.000
REMARK     1_adp:   0.278   22.363   -8.344   18.129   -9.095   0.000   -7.927    0.000
REMARK     1_occ:   0.278   22.363   -8.344   18.129   -9.095   0.000   -7.927    0.000
REMARK     2_bss:   0.288   39.551   -7.348   16.799   -9.450   0.000   -6.425    0.000
REMARK     2_sar:   0.288   39.551   -7.348   16.799   -9.450   0.000   -6.425    0.000
REMARK     2_fit:   0.288   39.551   -7.348   16.799   -9.450   0.000   -6.425    0.000
REMARK     2_xyz:   0.288   39.551   -7.348   16.799   -9.450   0.000   -6.425    0.000
REMARK     2_adp:   0.288   39.551   -7.348   16.799   -9.450   0.000   -6.425    0.000
REMARK     2_occ:   0.288   39.551   -7.348   16.799   -9.450   0.000   -6.425    0.000
REMARK     3_bss:   0.285   38.678   -7.408   16.497   -9.089   0.000   -5.848    0.000
REMARK     3_fit:   0.285   38.678   -7.408   16.497   -9.089   0.000   -5.848    0.000
REMARK     3_xyz:   0.285   38.678   -7.408   16.497   -9.089   0.000   -5.848    0.000
REMARK     3_adp:   0.285   38.678   -7.408   16.497   -9.089   0.000   -5.848    0.000
REMARK     3_occ:   0.285   38.678   -7.408   16.497   -9.089   0.000   -5.848    0.000
REMARK     3_bss:   0.288   38.710   -7.489   16.446   -8.957   0.000   -5.560    0.000
REMARK   --------------------------------------------------------------------
REMARK   stage     <pher>      fom    alpha         beta
REMARK     0   :   60.971   0.3860   0.1586     1494.490
REMARK     1_bss:  46.439   0.5699   0.2434      752.240
REMARK     1_rbr:  47.128   0.5609   0.2407      740.986
REMARK     1_bss:  46.772   0.5654   0.2416      735.017
REMARK     1_fit:  45.218   0.5844   0.2462      662.267
REMARK     1_xyz:  43.306   0.6062   0.2590      574.043
REMARK     1_adp:  42.094   0.6195   0.2520      517.705
REMARK     1_occ:  41.821   0.6230   0.2563      519.536
REMARK     2_bss:  41.744   0.6240   0.2583      520.022
REMARK     2_sar:  40.876   0.6348   0.2599      513.188
REMARK     2_fit:  41.440   0.6281   0.2520      517.930
REMARK     2_xyz:  39.351   0.6534   0.2654      487.068
REMARK     2_adp:  38.723   0.6608   0.2688      470.384
REMARK     2_occ:  38.723   0.6608   0.2688      470.384
REMARK     3_bss:  38.607   0.6623   0.2642      464.464
REMARK     3_fit:  37.684   0.6736   0.2655      444.291
REMARK     3_xyz:  38.693   0.6609   0.2638      456.285
REMARK     3_adp:  38.508   0.6631   0.2638      447.088
REMARK     3_occ:  38.508   0.6631   0.2638      447.088
REMARK     3_bss:  38.000   0.6692   0.2653      431.262
REMARK   --------------------------------------------------------------------
REMARK   stage      angl    bond    chir    dihe    plan    repu   geom_target
REMARK     0   :    0.295   0.001   0.015   6.225   0.001   4.112   1.3106e-02
REMARK     1_bss:   0.295   0.001   0.015   6.225   0.001   4.112   1.3106e-02
REMARK     1_rbr:   0.295   0.001   0.015   6.225   0.001   4.112   1.3115e-02
REMARK     1_bss:   0.295   0.001   0.015   6.225   0.001   4.112   1.3115e-02
REMARK     1_fit:   1.503   0.051   0.100  13.094   0.011   4.094   9.7914e-01
REMARK     1_xyz:   1.386   0.012   0.087  15.419   0.006   4.124   1.4517e-01
REMARK     1_adp:   1.386   0.012   0.087  15.419   0.006   4.124   1.4517e-01
REMARK     1_occ:   1.386   0.012   0.087  15.419   0.006   4.124   1.4517e-01
REMARK     2_bss:   1.386   0.012   0.087  15.419   0.006   4.124   1.4517e-01
REMARK     2_sar:   1.599   0.017   0.103  17.177   0.007   4.104   2.0875e-01
REMARK     2_fit:   1.663   0.026   0.115  17.316   0.008   4.100   3.0113e-01
REMARK     2_xyz:   1.293   0.010   0.085  18.112   0.006   4.121   1.3492e-01
REMARK     2_adp:   1.293   0.010   0.085  18.112   0.006   4.121   1.3492e-01
REMARK     2_occ:   1.293   0.010   0.085  18.112   0.006   4.121   1.3492e-01
```

```
REMARK    3_bss:   1.293  0.010  0.085 18.112  0.006  4.121  1.3492e-01
REMARK    3_fit:   1.371  0.031  0.094 18.520  0.007  4.105  4.1601e-01
REMARK    3_xyz:   1.278  0.009  0.083 18.495  0.005  4.117  1.3388e-01
REMARK    3_adp:   1.278  0.009  0.083 18.495  0.005  4.117  1.3388e-01
REMARK    3_occ:   1.278  0.009  0.083 18.495  0.005  4.117  1.3388e-01
REMARK    3_bss:   1.278  0.009  0.083 18.495  0.005  4.107  1.3469e-01
REMARK   -----------------------------------------------------------------
REMARK                   Maximal deviations:
REMARK    stage      angl   bond   chir   dihe   plan   repu      |grad|
REMARK    0     :    4.887  0.010  0.095 73.272  0.012  2.601  1.0739e-02
REMARK    1_bss:    4.887  0.010  0.095 73.272  0.012  2.601  1.0739e-02
REMARK    1_rbr:    4.887  0.010  0.095 73.272  0.012  2.601  1.0756e-02
REMARK    1_bss:    4.887  0.010  0.095 73.272  0.012  2.601  1.0756e-02
REMARK    1_fit:   26.860  1.956  0.443 89.447  0.084  0.469  7.0512e-01
REMARK    1_xyz:    9.306  0.066  0.366 89.866  0.061  2.069  8.9770e-02
REMARK    1_adp:    9.306  0.066  0.366 89.866  0.061  2.069  8.9770e-02
REMARK    1_occ:    9.306  0.066  0.366 89.866  0.061  2.069  8.9770e-02
REMARK    2_bss:    9.306  0.066  0.366 89.866  0.061  2.069  8.9770e-02
REMARK    2_sar:   12.521  0.195  0.728 83.560  0.052  2.138  2.7433e-01
REMARK    2_fit:   12.521  0.578  0.728 83.560  0.052  1.860  3.1580e-01
REMARK    2_xyz:   11.987  0.076  0.408 83.948  0.055  2.214  7.0435e-02
REMARK    2_adp:   11.987  0.076  0.408 83.948  0.055  2.214  7.0435e-02
REMARK    2_occ:   11.987  0.076  0.408 83.948  0.055  2.214  7.0435e-02
REMARK    3_bss:   11.987  0.076  0.408 83.948  0.055  2.214  7.0435e-02
REMARK    3_fit:   11.987  1.408  0.458 87.325  0.055  0.460  3.6860e-01
REMARK    3_xyz:   12.449  0.051  0.412 85.180  0.047  2.165  6.7907e-02
REMARK    3_adp:   12.449  0.051  0.412 85.180  0.047  2.165  6.7907e-02
REMARK    3_occ:   12.449  0.051  0.412 85.180  0.047  2.165  6.7907e-02
REMARK    3_bss:   12.449  0.051  0.412 85.180  0.047  2.165  6.8308e-02
REMARK   -----------------------------------------------------------------
REMARK           |-----overall-----|---macromolecule----|------solvent-------|
REMARK    stage   b_max  b_min  b_ave  b_max  b_min  b_ave  b_max  b_min  b_ave
REMARK    0     :  88.25  20.00  56.09 163.22  25.82  69.41  78.07  44.85  59.47
REMARK    1_bss:   99.53  31.28  67.37 163.22  25.82  69.41  78.07  44.85  59.47
REMARK    1_rbr:   99.53  31.28  67.37 163.22  25.82  69.41  78.07  44.85  59.47
REMARK    1_bss:   99.53  31.28  67.37 173.50  27.46  69.36  98.66  39.86  55.15
REMARK    1_fit:   99.53  31.28  67.37 173.50  27.46  69.36  98.66  39.86  55.15
REMARK    1_xyz:   99.53  31.28  67.37 172.62  26.59  68.49  97.79  38.99  54.28
REMARK    1_adp: 163.67  26.26  69.86 172.62  26.59  68.49  66.68  35.89  52.10
REMARK    1_occ: 163.67  26.26  69.86 172.62  26.59  68.49  66.68  35.89  52.10
REMARK    2_bss: 163.22  25.82  69.41 174.53  26.64  68.49  61.07  38.80  50.33
REMARK    2_sar: 163.22  25.82  69.37 174.53  26.64  68.49  61.07  38.80  50.33
REMARK    2_fit: 163.22  25.82  69.37 174.56  26.67  68.52  61.10  38.83  46.66
REMARK   -----------------------------------------------------------------
REMARK    stage       Deviation of refined
REMARK                model from start model
REMARK                  max      min     mean
REMARK    0     :    0.000    0.000    0.000
REMARK    1_bss:    0.000    0.000    0.000
REMARK    1_rbr:    2.222    2.150    2.188
REMARK    1_bss:    2.222    2.150    2.188
REMARK    1_fit:    8.290    0.420    2.307
REMARK    1_xyz:    8.288    0.507    2.324
REMARK    1_adp:    8.288    0.507    2.324
REMARK    1_occ:    8.288    0.507    2.324
REMARK    2_bss:    8.288    0.507    2.324
REMARK    2_sar:    9.001    0.493    2.411
REMARK    2_fit:    9.001    0.480    2.412
REMARK    2_xyz:    9.178    0.518    2.422
REMARK    2_adp:    9.178    0.518    2.422
REMARK    2_occ:    9.178    0.518    2.422
REMARK    3_bss:    9.178    0.518    2.422
REMARK    3_fit:    9.178    0.518    2.423
```

EP 2 593 472 B1

```
REMARK     3_xyz:     9.175    0.593    2.433
REMARK     3_adp:     9.175    0.593    2.433
REMARK     3_occ:     9.175    0.593    2.433
REMARK     3_bss:     9.175    0.593    2.433
REMARK  ----------------------------------------------------------------
REMARK  stage   number of ordered solvent
REMARK       0    :              0
REMARK       1_bss:              0
REMARK       1_rbr:              0
REMARK       1_bss:              0
REMARK       1_fit:              0
REMARK       1_xyz:              0
REMARK       1_adp:              0
REMARK       1_occ:              0
REMARK       2_bss:              0
REMARK       2_sar:             11
REMARK       2_fit:             11
REMARK       2_xyz:             11
REMARK       2_adp:             11
REMARK       2_occ:             11
REMARK       3_bss:             11
REMARK       3_fit:              8
REMARK       3_xyz:              8
REMARK       3_adp:              8
REMARK       3_occ:              8
REMARK       3_bss:              5
REMARK  ----------------------------------------------------------------
REMARK MODEL CONTENT.
REMARK ELEMENT         ATOM RECORD COUNT    OCCUPANCY SUM
REMARK          C                    1640          1621.00
REMARK          S                      12            12.00
REMARK          O                     470           464.00
REMARK          N                     402           397.00
REMARK    TOTAL                      2524          2494.00
REMARK  ----------------------------------------------------------------
REMARK r_free_flags.md5.hexdigest 130536f97c5a634b1e93427cc8887f1a
REMARK
REMARK  3 REFINEMENT.
REMARK  3   PROGRAM     : PHENIX (phenix.refine: 1.6.1_357)
REMARK  3   AUTHORS     : Adams,Afonine,Chen,Davis,Echols,Gopal,
REMARK  3               : Grosse-Kunstleve,Headd,Hung,Immormino,Ioerger,McCoy,
REMARK  3               : McKee,Moriarty,Pai,Read,Richardson,Richardson,Romo,
REMARK  3               : Sacchettini,Sauter,Smith,Storoni,Terwilliger,Zwart
REMARK  3
REMARK  3   REFINEMENT TARGET : ML
REMARK  3
REMARK  3   DATA USED IN REFINEMENT.
REMARK  3   RESOLUTION RANGE HIGH (ANGSTROMS) : 2.485
REMARK  3   RESOLUTION RANGE LOW  (ANGSTROMS) : 45.444
REMARK  3   MIN(FOBS/SIGMA_FOBS)              : 0.01
REMARK  3   COMPLETENESS FOR RANGE       (%) : 91.17
REMARK  3   NUMBER OF REFLECTIONS            : 10755
REMARK  3
REMARK  3   FIT TO DATA USED IN REFINEMENT.
REMARK  3   R VALUE      (WORKING + TEST SET) : 0.2322
REMARK  3   R VALUE            (WORKING SET) : 0.2284
REMARK  3   FREE R VALUE                     : 0.3032
REMARK  3   FREE R VALUE TEST SET SIZE   (%) : 4.71
REMARK  3   FREE R VALUE TEST SET COUNT      : 507
REMARK  3
REMARK  3   FIT TO DATA USED IN REFINEMENT (IN BINS).
REMARK  3   BIN  RESOLUTION RANGE   COMPL.    NWORK NFREE   RWORK  RFREE
REMARK  3     1 45.4520 -  3.9441    0.98      2804   151  0.1900 0.2596
```

```
REMARK   3     2  3.9441 -  3.1308     0.97      2713    132  0.2293 0.3307
REMARK   3     3  3.1308 -  2.7351     0.89      2519    110  0.2902 0.3834
REMARK   3     4  2.7351 -  2.4850     0.80      2212    114  0.3442 0.4128
REMARK   3
REMARK   3 BULK SOLVENT MODELLING.
REMARK   3  METHOD USED        : FLAT BULK SOLVENT MODEL
REMARK   3  SOLVENT RADIUS     : 1.11
REMARK   3  SHRINKAGE RADIUS   : 0.90
REMARK   3  GRID STEP FACTOR   : 4.00
REMARK   3  K_SOL             : 0.288
REMARK   3  B_SOL             : 38.710
REMARK   3
REMARK   3 ERROR ESTIMATES.
REMARK   3  COORDINATE ERROR (MAXIMUM-LIKELIHOOD BASED)    : 0.45
REMARK   3  PHASE ERROR (DEGREES, MAXIMUM-LIKELIHOOD BASED) : 38.00
REMARK   3
REMARK   3 OVERALL SCALE FACTORS.
REMARK   3  SCALE = SUM(|F_OBS|*|F_MODEL|)/SUM(|F_MODEL|**2) : 0.3065
REMARK   3  ANISOTROPIC SCALE MATRIX ELEMENTS (IN CARTESIAN BASIS).
REMARK   3   B11 : -7.4891
REMARK   3   B22 : 16.4458
REMARK   3   B33 : -8.9567
REMARK   3   B12 : 0.0000
REMARK   3   B13 : -5.5598
REMARK   3   B23 : 0.0000
REMARK   3
REMARK   3 R FACTOR FORMULA.
REMARK   3  R = SUM(||F_OBS|-SCALE*|F_MODEL||)/SUM(|F_OBS|)
REMARK   3
REMARK   3 TOTAL MODEL STRUCTURE FACTOR (F_MODEL).
REMARK   3  F_MODEL = FB_CART * (F_CALC_ATOMS + F_BULK)
REMARK   3   F_BULK = K_SOL * EXP(-B_SOL * S**2 / 4) * F_MASK
REMARK   3   F_CALC_ATOMS = ATOMIC MODEL STRUCTURE FACTORS
REMARK   3   FB_CART = EXP(-H(t) * A(-1) * B * A(-1t) * H)
REMARK   3    A = orthogonalization matrix, H = MILLER INDEX
REMARK   3    (t) = TRANSPOSE, (-1) = INVERSE
REMARK   3
REMARK   3 STRUCTURE FACTORS CALCULATION ALGORITHM : FFT
REMARK   3
REMARK   3 DEVIATIONS FROM IDEAL VALUES.
REMARK   3              RMSD     MAX   COUNT
REMARK   3  BOND      :  0.009   0.051   2574
REMARK   3  ANGLE     :  1.278  12.449   3478
REMARK   3  CHIRALITY :  0.083   0.412    388
REMARK   3  PLANARITY :  0.005   0.047    446
REMARK   3  DIHEDRAL  : 18.495  85.180    982
REMARK   3  MIN NONBONDED DISTANCE : 2.165
REMARK   3
REMARK   3 ATOMIC DISPLACEMENT PARAMETERS.
REMARK   3  WILSON B : None
REMARK   3  RMS(B_ISO_OR_EQUIVALENT_BONDED) : 7.48
REMARK   3  ATOMS            NUMBER OF ATOMS
REMARK   3                    ISO.  ANISO.
REMARK   3   ALL          :  2524       0
REMARK   3   ALL (NO H)   :  2524       0
REMARK   3   SOLVENT      :     5       0
REMARK   3   NON-SOLVENT  :  2519       0
REMARK   3   HYDROGENS    :     0       0
REMARK   3
```

Table 4: Atomic Coordinates for Residues of a Crystal Structure of Ancestral Enzyme Thioredoxin AECA

```
CRYST1   37.573    48.783    91.033  90.00    93.22    90.00 P 1 21 1
SCALE1      0.026615  0.000000  0.001500        0.00000
SCALE2      0.000000  0.020499  0.000000        0.00000
SCALE3      0.000000  0.000000  0.011002        0.00000
ATOM      1   N   SER A   1      18.325  18.563  30.461  1.00 93.03          N
ATOM      2   CA  SER A   1      17.742  17.660  31.452  1.00 95.56          C
ATOM      3   CB  SER A   1      16.427  17.069  30.946  1.00 89.37          C
ATOM      4   OG  SER A   1      15.755  16.394  31.990  1.00 98.90          O
ATOM      5   C   SER A   1      18.726  16.551  31.847  1.00 93.26          C
ATOM      6   O   SER A   1      19.647  16.804  32.621  1.00 94.14          O
ATOM      7   N   VAL A   2      18.536  15.335  31.331  1.00 63.44          N
ATOM      8   CA  VAL A   2      19.472  14.237  31.601  1.00 64.27          C
ATOM      9   CB  VAL A   2      19.418  13.136  30.509  1.00 54.51          C
ATOM     10   CG1 VAL A   2      20.456  12.086  30.797  1.00 54.85          C
ATOM     11   CG2 VAL A   2      18.056  12.486  30.449  1.00 57.38          C
ATOM     12   C   VAL A   2      20.923  14.719  31.729  1.00 60.52          C
ATOM     13   O   VAL A   2      21.687  14.680  30.769  1.00 61.25          O
ATOM     14   N   ILE A   3      21.297  15.182  32.914  1.00 76.52          N
ATOM     15   CA  ILE A   3      22.631  15.739  33.111  1.00 82.49          C
ATOM     16   CB  ILE A   3      22.777  16.448  34.488  1.00 75.29          C
ATOM     17   CG1 ILE A   3      24.249  16.775  34.797  1.00 81.94          C
ATOM     18   CD1 ILE A   3      24.866  17.886  33.926  1.00 82.01          C
ATOM     19   CG2 ILE A   3      22.182  15.603  35.588  1.00 74.23          C
ATOM     20   C   ILE A   3      23.693  14.664  32.953  1.00 77.76          C
ATOM     21   O   ILE A   3      23.445  13.493  33.221  1.00 79.68          O
ATOM     22   N   GLU A   4      24.869  15.072  32.497  1.00 68.06          N
ATOM     23   CA  GLU A   4      25.980  14.160  32.314  1.00 67.25          C
ATOM     24   CB  GLU A   4      26.657  14.414  30.970  1.00 78.88          C
ATOM     25   CG  GLU A   4      26.798  15.892  30.611  1.00 88.69          C
ATOM     26   CD  GLU A   4      25.528  16.489  30.011  1.00 87.15          C
ATOM     27   OE1 GLU A   4      25.146  16.067  28.896  1.00 82.96          O
ATOM     28   OE2 GLU A   4      24.915  17.378  30.651  1.00 79.79          O
ATOM     29   C   GLU A   4      26.950  14.411  33.433  1.00 78.75          C
ATOM     30   O   GLU A   4      27.749  15.343  33.380  1.00 89.72          O
ATOM     31   N   ILE A   5      26.876  13.589  34.465  1.00 61.04          N
ATOM     32   CA  ILE A   5      27.680  13.846  35.641  1.00 58.02          C
ATOM     33   CB  ILE A   5      26.984  13.325  36.915  1.00 59.29          C
ATOM     34   CG1 ILE A   5      27.476  11.924  37.269  1.00 61.82          C
ATOM     35   CD1 ILE A   5      28.652  11.900  38.232  1.00 65.42          C
ATOM     36   CG2 ILE A   5      25.474  13.315  36.751  1.00 54.50          C
ATOM     37   C   ILE A   5      29.083  13.236  35.512  1.00 61.60          C
ATOM     38   O   ILE A   5      29.248  12.142  34.960  1.00 49.59          O
ATOM     39   N   ASN A   6      30.079  13.960  36.029  1.00 73.16          N
ATOM     40   CA  ASN A   6      31.471  13.506  36.097  1.00 71.38          C
ATOM     41   CB  ASN A   6      32.326  14.337  35.156  1.00 72.01          C
ATOM     42   CG  ASN A   6      32.189  15.818  35.425  1.00 77.59          C
ATOM     43   OD1 ASN A   6      32.898  16.370  36.270  1.00 81.58          O
ATOM     44   ND2 ASN A   6      31.259  16.470  34.725  1.00 69.66          N
ATOM     45   C   ASN A   6      32.026  13.649  37.510  1.00 74.67          C
ATOM     46   O   ASN A   6      31.438  14.338  38.349  1.00 72.89          O
ATOM     47   N   ASP A   7      33.175  13.022  37.758  1.00 96.40          N
ATOM     48   CA  ASP A   7      33.815  12.995  39.090  1.00102.57          C
ATOM     49   CB  ASP A   7      35.270  12.508  38.978  1.00 90.00          C
ATOM     50   CG  ASP A   7      35.411  11.225  38.150  1.00 96.84          C
ATOM     51   OD1 ASP A   7      35.570  11.312  36.908  1.00 93.02          O
ATOM     52   OD2 ASP A   7      35.387  10.125  38.744  1.00 96.83          O
ATOM     53   C   ASP A   7      33.788  14.302  39.910  1.00 98.35          C
```

```
ATOM    54  O    ASP A   7     33.919  14.266  41.132  1.00  99.35           O
ATOM    55  N    GLU A   8     33.624  15.439  39.240  1.00  94.34           N
ATOM    56  CA   GLU A   8     33.701  16.747  39.892  1.00  93.93           C
ATOM    57  CB   GLU A   8     34.539  17.708  39.045  1.00 104.22           C
ATOM    58  CG   GLU A   8     35.984  17.849  39.531  1.00 109.88           C
ATOM    59  CD   GLU A   8     36.678  16.502  39.713  1.00 112.71           C
ATOM    60  OE1  GLU A   8     36.910  16.108  40.878  1.00 110.85           O
ATOM    61  OE2  GLU A   8     36.992  15.846  38.691  1.00 115.09           O
ATOM    62  C    GLU A   8     32.355  17.374  40.243  1.00  89.65           C
ATOM    63  O    GLU A   8     32.095  17.682  41.410  1.00  84.85           O
ATOM    64  N    ASN A   9     31.504  17.569  39.238  1.00  70.09           N
ATOM    65  CA   ASN A   9     30.125  17.996  39.496  1.00  68.14           C
ATOM    66  CB   ASN A   9     29.423  18.422  38.203  1.00  69.98           C
ATOM    67  CG   ASN A   9     28.974  17.231  37.340  1.00  70.02           C
ATOM    68  OD1  ASN A   9     28.025  17.342  36.557  1.00  65.59           O
ATOM    69  ND2  ASN A   9     29.652  16.097  37.482  1.00  65.99           N
ATOM    70  C    ASN A   9     29.277  16.953  40.248  1.00  62.48           C
ATOM    71  O    ASN A   9     28.146  17.229  40.618  1.00  62.41           O
ATOM    72  N    PHE A  10     29.834  15.770  40.498  1.00  73.47           N
ATOM    73  CA   PHE A  10     29.079  14.673  41.116  1.00  71.62           C
ATOM    74  CB   PHE A  10     29.983  13.519  41.562  1.00  70.83           C
ATOM    75  CG   PHE A  10     29.223  12.402  42.240  1.00  74.73           C
ATOM    76  CD2  PHE A  10     28.534  11.456  41.484  1.00  75.00           C
ATOM    77  CE2  PHE A  10     27.816  10.435  42.086  1.00  66.17           C
ATOM    78  CZ   PHE A  10     27.767  10.352  43.473  1.00  72.89           C
ATOM    79  CE1  PHE A  10     28.434  11.289  44.243  1.00  68.64           C
ATOM    80  CD1  PHE A  10     29.154  12.319  43.623  1.00  71.79           C
ATOM    81  C    PHE A  10     28.210  15.053  42.301  1.00  76.07           C
ATOM    82  O    PHE A  10     27.230  14.376  42.602  1.00  81.66           O
ATOM    83  N    ASP A  11     28.577  16.113  43.001  1.00  93.76           N
ATOM    84  CA   ASP A  11     27.897  16.415  44.250  1.00  86.23           C
ATOM    85  CB   ASP A  11     28.820  17.152  45.203  1.00  90.97           C
ATOM    86  CG   ASP A  11     30.026  16.311  45.582  1.00  96.54           C
ATOM    87  OD1  ASP A  11     30.474  15.501  44.738  1.00  95.75           O
ATOM    88  OD2  ASP A  11     30.523  16.442  46.718  1.00 103.21           O
ATOM    89  C    ASP A  11     26.579  17.129  44.034  1.00  89.66           C
ATOM    90  O    ASP A  11     26.232  18.057  44.755  1.00  87.34           O
ATOM    91  N    AGLU A  12    25.859  16.671  43.011  1.00  95.44           N
ATOM    92  CA   AGLU A  12    24.486  17.080  42.746  1.00  96.77           C
ATOM    93  CB   AGLU A  12    24.349  17.496  41.292  1.00  93.86           C
ATOM    94  CG   AGLU A  12    25.441  18.483  40.919  1.00  99.84           C
ATOM    95  CD   AGLU A  12    25.920  19.307  42.128  1.00 102.33           C
ATOM    96  OE1 AGLU A  12     27.153  19.363  42.381  1.00  96.27           O
ATOM    97  OE2 AGLU A  12     25.056  19.889  42.830  1.00  98.47           O
ATOM    98  C   AGLU A  12     23.618  15.891  43.081  1.00  92.75           C
ATOM    99  O   AGLU A  12     22.412  15.878  42.872  1.00  87.89           O
ATOM   100  N   BGLU A  12     25.846  16.698  43.014  0.00  95.31           N
ATOM   101  CA  BGLU A  12     24.475  17.142  42.827  0.00  96.51           C
ATOM   102  CB  BGLU A  12     24.205  17.518  41.368  0.00  93.89           C
ATOM   103  CG  BGLU A  12     23.983  16.344  40.436  0.00  90.85           C
ATOM   104  CD  BGLU A  12     25.275  15.800  39.866  0.00  91.65           C
ATOM   105  OE1 BGLU A  12     25.838  16.450  38.962  0.00  92.17           O
ATOM   106  OE2 BGLU A  12     25.728  14.727  40.317  0.00  92.30           O
ATOM   107  C   BGLU A  12     23.609  15.975  43.272  0.00  92.78           C
ATOM   108  O   BGLU A  12     22.383  16.053  43.301  0.00  89.36           O
ATOM   109  N    VAL A  13     24.291  14.889  43.624  1.00  82.34           N
ATOM   110  CA   VAL A  13     23.678  13.726  44.207  1.00  82.59           C
ATOM   111  CB   VAL A  13     24.518  12.487  43.847  1.00  78.66           C
ATOM   112  CG1  VAL A  13     24.043  11.276  44.614  1.00  74.13           C
ATOM   113  CG2  VAL A  13     24.488  12.238  42.346  1.00  77.77           C
ATOM   114  C    VAL A  13     23.709  13.934  45.729  1.00  85.18           C
ATOM   115  O    VAL A  13     22.894  13.369  46.474  1.00  77.97           O
ATOM   116  N    ILE A  14     24.654  14.768  46.171  1.00  80.06           N
```

41

| ATOM | 117 | CA  | ILE | A | 14 | 24.880 | 15.035 | 47.597 | 1.00 | 84.88 | C |
|------|-----|-----|-----|---|----|--------|--------|--------|------|-------|---|
| ATOM | 118 | CB  | ILE | A | 14 | 26.403 | 14.894 | 48.015 | 1.00 | 80.37 | C |
| ATOM | 119 | CG1 | ILE | A | 14 | 27.000 | 13.556 | 47.575 | 1.00 | 69.92 | C |
| ATOM | 120 | CD1 | ILE | A | 14 | 28.332 | 13.228 | 48.239 | 1.00 | 64.35 | C |
| ATOM | 121 | CG2 | ILE | A | 14 | 26.571 | 14.985 | 49.514 | 1.00 | 91.17 | C |
| ATOM | 122 | C   | ILE | A | 14 | 24.339 | 16.409 | 48.045 | 1.00 | 88.80 | C |
| ATOM | 123 | O   | ILE | A | 14 | 24.547 | 16.825 | 49.189 | 1.00 | 93.22 | O |
| ATOM | 124 | N   | LYS | A | 15 | 23.641 | 17.115 | 47.159 | 1.00 | 84.90 | N |
| ATOM | 125 | CA  | LYS | A | 15 | 23.075 | 18.413 | 47.537 | 1.00 | 84.96 | C |
| ATOM | 126 | CB  | LYS | A | 15 | 24.014 | 19.548 | 47.117 | 1.00 | 92.62 | C |
| ATOM | 127 | CG  | LYS | A | 15 | 25.493 | 19.251 | 47.346 | 1.00 | 91.34 | C |
| ATOM | 128 | CD  | LYS | A | 15 | 26.381 | 20.084 | 46.441 | 1.00 | 83.09 | C |
| ATOM | 129 | CE  | LYS | A | 15 | 26.775 | 21.400 | 47.069 | 1.00 | 87.11 | C |
| ATOM | 130 | NZ  | LYS | A | 15 | 27.702 | 22.143 | 46.153 | 1.00 | 74.02 | N |
| ATOM | 131 | C   | LYS | A | 15 | 21.667 | 18.645 | 46.967 | 1.00 | 82.89 | C |
| ATOM | 132 | O   | LYS | A | 15 | 21.323 | 19.759 | 46.552 | 1.00 | 77.76 | O |
| ATOM | 133 | N   | LYS | A | 16 | 20.852 | 17.596 | 46.956 | 1.00 | 80.48 | N |
| ATOM | 134 | CA  | LYS | A | 16 | 19.491 | 17.713 | 46.456 | 1.00 | 81.00 | C |
| ATOM | 135 | CB  | LYS | A | 16 | 19.392 | 17.217 | 45.007 | 1.00 | 79.63 | C |
| ATOM | 136 | CG  | LYS | A | 16 | 18.033 | 17.485 | 44.339 | 1.00 | 76.94 | C |
| ATOM | 137 | CD  | LYS | A | 16 | 17.866 | 18.938 | 43.819 | 1.00 | 78.71 | C |
| ATOM | 138 | CE  | LYS | A | 16 | 17.916 | 20.013 | 44.931 | 1.00 | 80.92 | C |
| ATOM | 139 | NZ  | LYS | A | 16 | 16.731 | 20.030 | 45.865 | 1.00 | 78.45 | N |
| ATOM | 140 | C   | LYS | A | 16 | 18.509 | 16.944 | 47.317 | 1.00 | 78.57 | C |
| ATOM | 141 | O   | LYS | A | 16 | 18.801 | 15.837 | 47.778 | 1.00 | 73.23 | O |
| ATOM | 142 | N   | ASP | A | 17 | 17.336 | 17.533 | 47.518 | 1.00 | 68.38 | N |
| ATOM | 143 | CA  | ASP | A | 17 | 16.276 | 16.859 | 48.248 | 1.00 | 62.15 | C |
| ATOM | 144 | CB  | ASP | A | 17 | 15.275 | 17.883 | 48.797 | 1.00 | 68.54 | C |
| ATOM | 145 | CG  | ASP | A | 17 | 15.684 | 18.464 | 50.148 | 1.00 | 67.94 | C |
| ATOM | 146 | OD1 | ASP | A | 17 | 14.774 | 18.876 | 50.905 | 1.00 | 58.95 | O |
| ATOM | 147 | OD2 | ASP | A | 17 | 16.898 | 18.513 | 50.447 | 1.00 | 76.04 | O |
| ATOM | 148 | C   | ASP | A | 17 | 15.557 | 15.875 | 47.323 | 1.00 | 73.48 | C |
| ATOM | 149 | O   | ASP | A | 17 | 14.738 | 15.073 | 47.779 | 1.00 | 72.90 | O |
| ATOM | 150 | N   | LYS | A | 18 | 15.877 | 15.947 | 46.025 | 1.00 | 72.08 | N |
| ATOM | 151 | CA  | LYS | A | 18 | 15.144 | 15.237 | 44.963 | 1.00 | 64.54 | C |
| ATOM | 152 | CB  | LYS | A | 18 | 14.957 | 16.153 | 43.739 | 1.00 | 57.20 | C |
| ATOM | 153 | CG  | LYS | A | 18 | 13.611 | 16.856 | 43.685 | 1.00 | 59.65 | C |
| ATOM | 154 | CD  | LYS | A | 18 | 13.261 | 17.513 | 45.023 | 1.00 | 76.14 | C |
| ATOM | 155 | CE  | LYS | A | 18 | 11.780 | 17.941 | 45.115 | 1.00 | 68.61 | C |
| ATOM | 156 | NZ  | LYS | A | 18 | 11.439 | 18.690 | 46.391 | 1.00 | 46.87 | N |
| ATOM | 157 | C   | LYS | A | 18 | 15.819 | 13.936 | 44.531 | 1.00 | 58.93 | C |
| ATOM | 158 | O   | LYS | A | 18 | 17.034 | 13.892 | 44.352 | 1.00 | 64.80 | O |
| ATOM | 159 | N   | VAL | A | 19 | 15.030 | 12.881 | 44.361 | 1.00 | 46.98 | N |
| ATOM | 160 | CA  | VAL | A | 19 | 15.556 | 11.609 | 43.882 | 1.00 | 48.15 | C |
| ATOM | 161 | CB  | VAL | A | 19 | 14.424 | 10.613 | 43.613 | 1.00 | 51.03 | C |
| ATOM | 162 | CG1 | VAL | A | 19 | 14.988 | 9.218  | 43.324 | 1.00 | 44.51 | C |
| ATOM | 163 | CG2 | VAL | A | 19 | 13.493 | 10.565 | 44.788 | 1.00 | 54.70 | C |
| ATOM | 164 | C   | VAL | A | 19 | 16.390 | 11.760 | 42.607 | 1.00 | 47.76 | C |
| ATOM | 165 | O   | VAL | A | 19 | 15.907 | 12.203 | 41.568 | 1.00 | 52.65 | O |
| ATOM | 166 | N   | VAL | A | 20 | 17.649 | 11.372 | 42.692 | 1.00 | 42.79 | N |
| ATOM | 167 | CA  | VAL | A | 20 | 18.532 | 11.390 | 41.542 | 1.00 | 38.05 | C |
| ATOM | 168 | CB  | VAL | A | 20 | 19.954 | 11.887 | 41.911 | 1.00 | 38.95 | C |
| ATOM | 169 | CG1 | VAL | A | 20 | 20.823 | 11.924 | 40.690 | 1.00 | 43.85 | C |
| ATOM | 170 | CG2 | VAL | A | 20 | 19.901 | 13.273 | 42.532 | 1.00 | 34.16 | C |
| ATOM | 171 | C   | VAL | A | 20 | 18.617 | 9.974  | 40.995 | 1.00 | 32.43 | C |
| ATOM | 172 | O   | VAL | A | 20 | 18.954 | 9.016  | 41.704 | 1.00 | 29.51 | O |
| ATOM | 173 | N   | VAL | A | 21 | 18.272 | 9.830  | 39.730 | 1.00 | 45.32 | N |
| ATOM | 174 | CA  | VAL | A | 21 | 18.428 | 8.552  | 39.080 | 1.00 | 46.21 | C |
| ATOM | 175 | CB  | VAL | A | 21 | 17.229 | 8.203  | 38.223 | 1.00 | 43.66 | C |
| ATOM | 176 | CG1 | VAL | A | 21 | 17.588 | 7.049  | 37.299 | 1.00 | 36.41 | C |
| ATOM | 177 | CG2 | VAL | A | 21 | 16.049 | 7.872  | 39.114 | 1.00 | 43.66 | C |
| ATOM | 178 | C   | VAL | A | 21 | 19.634 | 8.658  | 38.193 | 1.00 | 40.45 | C |
| ATOM | 179 | O   | VAL | A | 21 | 19.712 | 9.548  | 37.349 | 1.00 | 43.66 | O |

| ATOM | 180 | N   | VAL A | 22 | 20.584 | 7.760  | 38.390 | 1.00 | 34.03 | N |
|------|-----|-----|-------|----|--------|--------|--------|------|-------|---|
| ATOM | 181 | CA  | VAL A | 22 | 21.810 | 7.815  | 37.612 | 1.00 | 42.77 | C |
| ATOM | 182 | CB  | VAL A | 22 | 23.049 | 8.363  | 38.417 | 1.00 | 36.79 | C |
| ATOM | 183 | CG1 | VAL A | 22 | 23.152 | 7.715  | 39.732 | 1.00 | 37.85 | C |
| ATOM | 184 | CG2 | VAL A | 22 | 24.357 | 8.161  | 37.636 | 1.00 | 38.48 | C |
| ATOM | 185 | C   | VAL A | 22 | 22.088 | 6.501  | 36.894 | 1.00 | 34.67 | C |
| ATOM | 186 | O   | VAL A | 22 | 21.996 | 5.416  | 37.462 | 1.00 | 36.22 | O |
| ATOM | 187 | N   | ASP A | 23 | 22.437 | 6.646  | 35.626 | 1.00 | 27.78 | N |
| ATOM | 188 | CA  | ASP A | 23 | 22.554 | 5.555  | 34.701 | 1.00 | 29.29 | C |
| ATOM | 189 | CB  | ASP A | 23 | 21.702 | 5.905  | 33.474 | 1.00 | 40.54 | C |
| ATOM | 190 | CG  | ASP A | 23 | 21.840 | 4.910  | 32.346 | 1.00 | 46.95 | C |
| ATOM | 191 | OD1 | ASP A | 23 | 22.114 | 3.709  | 32.595 | 1.00 | 50.35 | O |
| ATOM | 192 | OD2 | ASP A | 23 | 21.648 | 5.332  | 31.187 | 1.00 | 59.45 | O |
| ATOM | 193 | C   | ASP A | 23 | 24.020 | 5.400  | 34.336 | 1.00 | 32.65 | C |
| ATOM | 194 | O   | ASP A | 23 | 24.677 | 6.354  | 33.930 | 1.00 | 34.36 | O |
| ATOM | 195 | N   | PHE A | 24 | 24.543 | 4.195  | 34.495 | 1.00 | 40.40 | N |
| ATOM | 196 | CA  | PHE A | 24 | 25.948 | 3.955  | 34.225 | 1.00 | 41.68 | C |
| ATOM | 197 | CB  | PHE A | 24 | 26.553 | 3.093  | 35.335 | 1.00 | 47.07 | C |
| ATOM | 198 | CG  | PHE A | 24 | 26.608 | 3.780  | 36.668 | 1.00 | 48.84 | C |
| ATOM | 199 | CD2 | PHE A | 24 | 27.632 | 4.678  | 36.968 | 1.00 | 54.62 | C |
| ATOM | 200 | CE2 | PHE A | 24 | 27.674 | 5.329  | 38.205 | 1.00 | 52.58 | C |
| ATOM | 201 | CZ  | PHE A | 24 | 26.694 | 5.084  | 39.144 | 1.00 | 50.65 | C |
| ATOM | 202 | CE1 | PHE A | 24 | 25.669 | 4.191  | 38.859 | 1.00 | 51.01 | C |
| ATOM | 203 | CD1 | PHE A | 24 | 25.630 | 3.544  | 37.622 | 1.00 | 48.65 | C |
| ATOM | 204 | C   | PHE A | 24 | 26.043 | 3.226  | 32.913 | 1.00 | 41.92 | C |
| ATOM | 205 | O   | PHE A | 24 | 25.516 | 2.130  | 32.786 | 1.00 | 42.21 | O |
| ATOM | 206 | N   | TRP A | 25 | 26.719 | 3.818  | 31.935 | 1.00 | 40.66 | N |
| ATOM | 207 | CA  | TRP A | 25 | 26.711 | 3.270  | 30.577 | 1.00 | 43.71 | C |
| ATOM | 208 | CB  | TRP A | 25 | 25.732 | 4.069  | 29.712 | 1.00 | 39.40 | C |
| ATOM | 209 | CG  | TRP A | 25 | 26.231 | 5.477  | 29.562 | 1.00 | 38.20 | C |
| ATOM | 210 | CD1 | TRP A | 25 | 26.342 | 6.413  | 30.557 | 1.00 | 43.71 | C |
| ATOM | 211 | NE1 | TRP A | 25 | 26.876 | 7.571  | 30.055 | 1.00 | 44.68 | N |
| ATOM | 212 | CE2 | TRP A | 25 | 27.139 | 7.400  | 28.721 | 1.00 | 38.78 | C |
| ATOM | 213 | CD2 | TRP A | 25 | 26.751 | 6.086  | 28.378 | 1.00 | 33.04 | C |
| ATOM | 214 | CE3 | TRP A | 25 | 26.917 | 5.657  | 27.060 | 1.00 | 35.02 | C |
| ATOM | 215 | CZ3 | TRP A | 25 | 27.449 | 6.540  | 26.136 | 1.00 | 37.50 | C |
| ATOM | 216 | CH2 | TRP A | 25 | 27.829 | 7.845  | 26.511 | 1.00 | 43.16 | C |
| ATOM | 217 | CZ2 | TRP A | 25 | 27.683 | 8.287  | 27.799 | 1.00 | 43.79 | C |
| ATOM | 218 | C   | TRP A | 25 | 28.104 | 3.388  | 29.961 | 1.00 | 46.18 | C |
| ATOM | 219 | O   | TRP A | 25 | 29.066 | 3.811  | 30.625 | 1.00 | 40.29 | O |
| ATOM | 220 | N   | ALA A | 26 | 28.191 | 3.045  | 28.675 | 1.00 | 33.67 | N |
| ATOM | 221 | CA  | ALA A | 26 | 29.414 | 3.205  | 27.929 | 1.00 | 32.26 | C |
| ATOM | 222 | CB  | ALA A | 26 | 30.515 | 2.283  | 28.497 | 1.00 | 41.42 | C |
| ATOM | 223 | C   | ALA A | 26 | 29.198 | 2.935  | 26.465 | 1.00 | 31.60 | C |
| ATOM | 224 | O   | ALA A | 26 | 28.469 | 2.018  | 26.087 | 1.00 | 38.72 | O |
| ATOM | 225 | N   | GLU A | 27 | 29.866 | 3.723  | 25.638 | 1.00 | 34.85 | N |
| ATOM | 226 | CA  | GLU A | 27 | 29.734 | 3.614  | 24.205 | 1.00 | 36.73 | C |
| ATOM | 227 | CB  | GLU A | 27 | 30.776 | 4.481  | 23.520 | 1.00 | 38.76 | C |
| ATOM | 228 | CG  | GLU A | 27 | 30.297 | 5.037  | 22.179 | 1.00 | 52.02 | C |
| ATOM | 229 | CD  | GLU A | 27 | 29.128 | 5.996  | 22.328 | 1.00 | 59.51 | C |
| ATOM | 230 | OE1 | GLU A | 27 | 29.331 | 7.122  | 22.838 | 1.00 | 58.26 | O |
| ATOM | 231 | OE2 | GLU A | 27 | 27.995 | 5.608  | 21.957 | 1.00 | 66.32 | O |
| ATOM | 232 | C   | GLU A | 27 | 29.732 | 2.186  | 23.640 | 1.00 | 43.36 | C |
| ATOM | 233 | O   | GLU A | 27 | 29.006 | 1.891  | 22.674 | 1.00 | 47.24 | O |
| ATOM | 234 | N   | TRP A | 28 | 30.531 | 1.298  | 24.215 | 1.00 | 38.70 | N |
| ATOM | 235 | CA  | TRP A | 28 | 30.633 | -0.061 | 23.665 | 1.00 | 40.05 | C |
| ATOM | 236 | CB  | TRP A | 28 | 31.995 | -0.685 | 24.006 | 1.00 | 39.35 | C |
| ATOM | 237 | CG  | TRP A | 28 | 32.297 | -0.554 | 25.466 | 1.00 | 36.23 | C |
| ATOM | 238 | CD1 | TRP A | 28 | 33.007 | 0.453  | 26.074 | 1.00 | 36.47 | C |
| ATOM | 239 | NE1 | TRP A | 28 | 33.045 | 0.237  | 27.440 | 1.00 | 43.64 | N |
| ATOM | 240 | CE2 | TRP A | 28 | 32.344 | -0.905 | 27.734 | 1.00 | 38.86 | C |
| ATOM | 241 | CD2 | TRP A | 28 | 31.862 | -1.435 | 26.515 | 1.00 | 30.95 | C |
| ATOM | 242 | CE3 | TRP A | 28 | 31.098 | -2.608 | 26.543 | 1.00 | 42.18 | C |

| ATOM | 243 | CZ3 | TRP | A | 28 | 30.851 | -3.219 | 27.778 | 1.00 | 41.80 | C |
|------|-----|-----|-----|---|----|--------|--------|--------|------|-------|---|
| ATOM | 244 | CH2 | TRP | A | 28 | 31.348 | -2.665 | 28.967 | 1.00 | 38.31 | C |
| ATOM | 245 | CZ2 | TRP | A | 28 | 32.089 | -1.509 | 28.966 | 1.00 | 33.73 | C |
| ATOM | 246 | C   | TRP | A | 28 | 29.516 | -0.971 | 24.171 | 1.00 | 35.51 | C |
| ATOM | 247 | O   | TRP | A | 28 | 29.500 | -2.157 | 23.883 | 1.00 | 43.56 | O |
| ATOM | 248 | N   | CYS | A | 29 | 28.575 | -0.439 | 24.932 | 1.00 | 38.50 | N |
| ATOM | 249 | CA  | CYS | A | 29 | 27.582 | -1.316 | 25.552 | 1.00 | 44.43 | C |
| ATOM | 250 | CB  | CYS | A | 29 | 27.392 | -0.947 | 27.024 | 1.00 | 44.93 | C |
| ATOM | 251 | SG  | CYS | A | 29 | 26.007 | -1.798 | 27.803 | 1.00 | 50.55 | S |
| ATOM | 252 | C   | CYS | A | 29 | 26.240 | -1.364 | 24.812 | 1.00 | 44.88 | C |
| ATOM | 253 | O   | CYS | A | 29 | 25.497 | -0.370 | 24.753 | 1.00 | 43.32 | O |
| ATOM | 254 | N   | GLY | A | 30 | 25.943 | -2.532 | 24.258 | 1.00 | 52.78 | N |
| ATOM | 255 | CA  | GLY | A | 30 | 24.747 | -2.721 | 23.468 | 1.00 | 55.23 | C |
| ATOM | 256 | C   | GLY | A | 30 | 23.499 | -2.497 | 24.284 | 1.00 | 56.80 | C |
| ATOM | 257 | O   | GLY | A | 30 | 22.715 | -1.588 | 23.986 | 1.00 | 54.84 | O |
| ATOM | 258 | N   | PRO | A | 31 | 23.305 | -3.336 | 25.310 | 1.00 | 48.40 | N |
| ATOM | 259 | CA  | PRO | A | 31 | 22.186 | -3.256 | 26.253 | 1.00 | 48.15 | C |
| ATOM | 260 | CB  | PRO | A | 31 | 22.612 | -4.201 | 27.370 | 1.00 | 43.17 | C |
| ATOM | 261 | CG  | PRO | A | 31 | 23.353 | -5.277 | 26.658 | 1.00 | 42.21 | C |
| ATOM | 262 | CD  | PRO | A | 31 | 24.057 | -4.594 | 25.466 | 1.00 | 45.02 | C |
| ATOM | 263 | C   | PRO | A | 31 | 21.960 | -1.860 | 26.798 | 1.00 | 50.22 | C |
| ATOM | 264 | O   | PRO | A | 31 | 20.826 | -1.514 | 27.131 | 1.00 | 52.28 | O |
| ATOM | 265 | N   | CYS | A | 32 | 23.008 | -1.056 | 26.868 | 1.00 | 38.97 | N |
| ATOM | 266 | CA  | CYS | A | 32 | 22.851 | 0.298  | 27.377 | 1.00 | 43.30 | C |
| ATOM | 267 | CB  | CYS | A | 32 | 24.219 | 0.949  | 27.599 | 1.00 | 50.17 | C |
| ATOM | 268 | SG  | CYS | A | 32 | 25.334 | -0.040 | 28.648 | 1.00 | 58.61 | S |
| ATOM | 269 | C   | CYS | A | 32 | 22.047 | 1.136  | 26.401 | 1.00 | 47.60 | C |
| ATOM | 270 | O   | CYS | A | 32 | 21.446 | 2.146  | 26.771 | 1.00 | 49.30 | O |
| ATOM | 271 | N   | ARG | A | 33 | 22.057 | 0.718  | 25.139 | 1.00 | 51.00 | N |
| ATOM | 272 | CA  | ARG | A | 33 | 21.318 | 1.417  | 24.100 | 1.00 | 46.52 | C |
| ATOM | 273 | C   | ARG | A | 33 | 19.815 | 1.196  | 24.257 | 1.00 | 45.22 | O |
| ATOM | 274 | O   | ARG | A | 33 | 19.016 | 1.997  | 23.783 | 1.00 | 48.76 | O |
| ATOM | 275 | CB  | ARG | A | 33 | 21.782 | 0.965  | 22.723 | 1.00 | 45.56 | C |
| ATOM | 276 | CG  | ARG | A | 33 | 23.195 | 1.373  | 22.411 | 1.00 | 46.47 | C |
| ATOM | 277 | CD  | ARG | A | 33 | 23.806 | 0.573  | 21.258 | 1.00 | 39.31 | C |
| ATOM | 278 | NE  | ARG | A | 33 | 25.171 | 1.023  | 21.055 | 1.00 | 47.75 | N |
| ATOM | 279 | CZ  | ARG | A | 33 | 26.187 | 0.249  | 20.707 | 1.00 | 49.20 | C |
| ATOM | 280 | NH1 | ARG | A | 33 | 26.008 | -1.041 | 20.467 | 1.00 | 47.25 | N |
| ATOM | 281 | NH2 | ARG | A | 33 | 27.384 | 0.790  | 20.590 | 1.00 | 50.60 | N |
| ATOM | 282 | N   | MET | A | 34 | 19.422 | 0.117  | 24.921 | 1.00 | 44.44 | N |
| ATOM | 283 | CA  | MET | A | 34 | 18.007 | -0.054 | 25.222 | 1.00 | 55.68 | C |
| ATOM | 284 | C   | MET | A | 34 | 17.455 | 1.069  | 26.113 | 1.00 | 53.24 | C |
| ATOM | 285 | O   | MET | A | 34 | 16.485 | 1.723  | 25.746 | 1.00 | 53.05 | O |
| ATOM | 286 | CB  | MET | A | 34 | 17.712 | -1.456 | 25.767 | 1.00 | 51.44 | C |
| ATOM | 287 | CG  | MET | A | 34 | 17.687 | -2.508 | 24.663 | 1.00 | 43.70 | C |
| ATOM | 288 | SD  | MET | A | 34 | 18.086 | -4.175 | 25.212 | 1.00 | 62.13 | S |
| ATOM | 289 | CE  | MET | A | 34 | 16.486 | -4.717 | 25.777 | 1.00 | 57.11 | C |
| ATOM | 290 | N   | ILE | A | 35 | 18.080 | 1.338  | 27.252 | 1.00 | 41.35 | N |
| ATOM | 291 | CA  | ILE | A | 35 | 17.477 | 2.311  | 28.161 | 1.00 | 45.25 | C |
| ATOM | 292 | CB  | ILE | A | 35 | 17.625 | 1.905  | 29.617 | 1.00 | 57.49 | C |
| ATOM | 293 | CG1 | ILE | A | 35 | 19.096 | 1.858  | 30.020 | 1.00 | 48.32 | C |
| ATOM | 294 | CD1 | ILE | A | 35 | 19.306 | 0.990  | 31.288 | 1.00 | 40.60 | C |
| ATOM | 295 | CG2 | ILE | A | 35 | 16.915 | 0.559  | 29.867 | 1.00 | 60.05 | C |
| ATOM | 296 | C   | ILE | A | 35 | 17.837 | 3.784  | 28.000 | 1.00 | 47.14 | C |
| ATOM | 297 | O   | ILE | A | 35 | 17.153 | 4.661  | 28.552 | 1.00 | 40.82 | O |
| ATOM | 298 | N   | ALA | A | 36 | 18.905 | 4.054  | 27.257 | 1.00 | 47.27 | N |
| ATOM | 299 | CA  | ALA | A | 36 | 19.294 | 5.432  | 26.958 | 1.00 | 43.95 | C |
| ATOM | 300 | CB  | ALA | A | 36 | 20.377 | 5.456  | 25.916 | 1.00 | 43.77 | C |
| ATOM | 301 | C   | ALA | A | 36 | 18.104 | 6.294  | 26.505 | 1.00 | 44.39 | C |
| ATOM | 302 | O   | ALA | A | 36 | 17.889 | 7.382  | 27.032 | 1.00 | 44.41 | O |
| ATOM | 303 | N   | PRO | A | 37 | 17.326 | 5.806  | 25.523 | 1.00 | 39.47 | N |
| ATOM | 304 | CA  | PRO | A | 37 | 16.149 | 6.543  | 25.044 | 1.00 | 44.55 | C |
| ATOM | 305 | CB  | PRO | A | 37 | 15.601 | 5.632  | 23.948 | 1.00 | 40.58 | C |

| ATOM | 306 | CG | PRO | A | 37 | 16.147 | 4.254 | 24.284 | 1.00 | 42.38 | C |
|------|-----|-----|-----|---|----|--------|--------|--------|------|-------|---|
| ATOM | 307 | CD | PRO | A | 37 | 17.499 | 4.524 | 24.812 | 1.00 | 38.01 | C |
| ATOM | 308 | C | PRO | A | 37 | 15.072 | 6.723 | 26.121 | 1.00 | 42.99 | C |
| ATOM | 309 | O | PRO | A | 37 | 14.384 | 7.756 | 26.107 | 1.00 | 35.37 | O |
| ATOM | 310 | N | ILE | A | 38 | 14.938 | 5.726 | 27.005 | 1.00 | 37.72 | N |
| ATOM | 311 | CA | ILE | A | 38 | 13.970 | 5.709 | 28.111 | 1.00 | 43.02 | C |
| ATOM | 312 | CB | ILE | A | 38 | 13.783 | 4.289 | 28.656 | 1.00 | 43.66 | C |
| ATOM | 313 | CG1 | ILE | A | 38 | 13.147 | 3.382 | 27.605 | 1.00 | 42.38 | C |
| ATOM | 314 | CD1 | ILE | A | 38 | 12.943 | 1.953 | 28.095 | 1.00 | 42.09 | C |
| ATOM | 315 | CG2 | ILE | A | 38 | 12.971 | 4.307 | 29.947 | 1.00 | 35.62 | C |
| ATOM | 316 | C | ILE | A | 38 | 14.332 | 6.607 | 29.307 | 1.00 | 45.32 | C |
| ATOM | 317 | O | ILE | A | 38 | 13.458 | 7.199 | 29.965 | 1.00 | 38.85 | O |
| ATOM | 318 | N | ILE | A | 39 | 15.614 | 6.686 | 29.636 | 1.00 | 55.36 | N |
| ATOM | 319 | CA | ILE | A | 39 | 15.987 | 7.617 | 30.684 | 1.00 | 54.74 | C |
| ATOM | 320 | CB | ILE | A | 39 | 17.488 | 7.528 | 31.081 | 1.00 | 50.42 | C |
| ATOM | 321 | CG1 | ILE | A | 39 | 17.707 | 6.418 | 32.111 | 1.00 | 47.26 | C |
| ATOM | 322 | CD1 | ILE | A | 39 | 17.695 | 5.035 | 31.522 | 1.00 | 55.04 | C |
| ATOM | 323 | CG2 | ILE | A | 39 | 17.948 | 8.819 | 31.697 | 1.00 | 47.52 | C |
| ATOM | 324 | C | ILE | A | 39 | 15.602 | 8.991 | 30.163 | 1.00 | 51.78 | C |
| ATOM | 325 | O | ILE | A | 39 | 15.035 | 9.792 | 30.889 | 1.00 | 55.74 | O |
| ATOM | 326 | N | GLU | A | 40 | 15.867 | 9.231 | 28.881 | 1.00 | 51.45 | N |
| ATOM | 327 | CA | GLU | A | 40 | 15.653 | 10.546 | 28.271 | 1.00 | 57.83 | C |
| ATOM | 328 | CB | GLU | A | 40 | 16.241 | 10.588 | 26.854 | 1.00 | 58.08 | C |
| ATOM | 329 | CG | GLU | A | 40 | 17.765 | 10.442 | 26.786 | 1.00 | 68.61 | C |
| ATOM | 330 | CD | GLU | A | 40 | 18.542 | 11.730 | 27.122 | 1.00 | 80.83 | O |
| ATOM | 331 | OE1 | GLU | A | 40 | 17.926 | 12.829 | 27.167 | 1.00 | 78.79 | O |
| ATOM | 332 | OE2 | GLU | A | 40 | 19.781 | 11.630 | 27.329 | 1.00 | 71.59 | O |
| ATOM | 333 | C | GLU | A | 40 | 14.191 | 11.047 | 28.282 | 1.00 | 55.84 | C |
| ATOM | 334 | O | GLU | A | 40 | 13.945 | 12.214 | 28.577 | 1.00 | 50.91 | O |
| ATOM | 335 | N | GLU | A | 41 | 13.227 | 10.181 | 27.976 | 1.00 | 59.10 | N |
| ATOM | 336 | CA | GLU | A | 41 | 11.818 | 10.598 | 27.996 | 1.00 | 62.42 | C |
| ATOM | 337 | CB | GLU | A | 41 | 10.979 | 9.844 | 26.947 | 1.00 | 70.65 | C |
| ATOM | 338 | CG | GLU | A | 41 | 10.362 | 8.593 | 27.480 | 1.00 | 73.03 | C |
| ATOM | 339 | CD | GLU | A | 41 | 11.367 | 7.824 | 28.277 | 1.00 | 74.65 | O |
| ATOM | 340 | OE1 | GLU | A | 41 | 12.529 | 7.813 | 27.828 | 1.00 | 76.53 | O |
| ATOM | 341 | OE2 | GLU | A | 41 | 11.024 | 7.279 | 29.352 | 1.00 | 75.48 | O |
| ATOM | 342 | C | GLU | A | 41 | 11.166 | 10.520 | 29.382 | 1.00 | 68.27 | C |
| ATOM | 343 | O | GLU | A | 41 | 10.053 | 10.997 | 29.567 | 1.00 | 67.91 | O |
| ATOM | 344 | N | LEU | A | 42 | 11.856 | 9.917 | 30.350 | 1.00 | 60.11 | N |
| ATOM | 345 | CA | LEU | A | 42 | 11.400 | 9.973 | 31.734 | 1.00 | 50.89 | C |
| ATOM | 346 | CB | LEU | A | 42 | 11.890 | 8.773 | 32.546 | 1.00 | 50.48 | C |
| ATOM | 347 | CG | LEU | A | 42 | 11.289 | 7.365 | 32.359 | 1.00 | 51.90 | C |
| ATOM | 348 | CD1 | LEU | A | 42 | 12.166 | 6.317 | 33.041 | 1.00 | 51.41 | C |
| ATOM | 349 | CD2 | LEU | A | 42 | 9.882 | 7.256 | 32.891 | 1.00 | 47.33 | C |
| ATOM | 350 | C | LEU | A | 42 | 11.847 | 11.292 | 32.361 | 1.00 | 56.20 | C |
| ATOM | 351 | O | LEU | A | 42 | 11.141 | 11.866 | 33.183 | 1.00 | 61.90 | O |
| ATOM | 352 | N | ALA | A | 43 | 13.013 | 11.785 | 31.953 | 1.00 | 61.33 | N |
| ATOM | 353 | CA | ALA | A | 43 | 13.491 | 13.095 | 32.402 | 1.00 | 58.95 | C |
| ATOM | 354 | CB | ALA | A | 43 | 14.847 | 13.405 | 31.801 | 1.00 | 58.44 | C |
| ATOM | 355 | C | ALA | A | 43 | 12.489 | 14.185 | 32.037 | 1.00 | 66.39 | C |
| ATOM | 356 | O | ALA | A | 43 | 12.115 | 15.007 | 32.872 | 1.00 | 71.98 | O |
| ATOM | 357 | N | GLU | A | 44 | 12.041 | 14.169 | 30.787 | 1.00 | 102.79 | N |
| ATOM | 358 | CA | GLU | A | 44 | 11.005 | 15.087 | 30.316 | 1.00 | 104.45 | C |
| ATOM | 359 | CB | GLU | A | 44 | 10.829 | 14.949 | 28.802 | 1.00 | 101.94 | C |
| ATOM | 360 | CG | GLU | A | 44 | 9.495 | 15.455 | 28.284 | 1.00 | 104.58 | C |
| ATOM | 361 | CD | GLU | A | 44 | 9.264 | 16.932 | 28.555 | 1.00 | 114.25 | C |
| ATOM | 362 | OE1 | GLU | A | 44 | 9.492 | 17.384 | 29.701 | 1.00 | 107.52 | O |
| ATOM | 363 | OE2 | GLU | A | 44 | 8.845 | 17.640 | 27.612 | 1.00 | 113.75 | O |
| ATOM | 364 | C | GLU | A | 44 | 9.665 | 14.856 | 31.015 | 1.00 | 100.58 | C |
| ATOM | 365 | O | GLU | A | 44 | 9.031 | 15.790 | 31.496 | 1.00 | 108.77 | O |
| ATOM | 366 | N | GLU | A | 45 | 9.245 | 13.600 | 31.052 | 1.00 | 61.49 | N |
| ATOM | 367 | CA | GLU | A | 45 | 8.012 | 13.182 | 31.703 | 1.00 | 58.65 | C |
| ATOM | 368 | CB | GLU | A | 45 | 7.876 | 11.665 | 31.552 | 1.00 | 60.06 | C |

```
ATOM    369  CG   GLU A  45       6.478  11.111  31.635  1.00 68.80           C
ATOM    370  CD   GLU A  45       5.992  10.957  33.060  1.00 79.02           C
ATOM    371  OE1  GLU A  45       6.760  11.299  33.992  1.00 84.33           O
ATOM    372  OE2  GLU A  45       4.842  10.492  33.252  1.00 72.78           O
ATOM    373  C    GLU A  45       7.935  13.582  33.190  1.00 68.45           C
ATOM    374  O    GLU A  45       6.852  13.804  33.712  1.00 70.96           O
ATOM    375  N    TYR A  46       9.074  13.662  33.874  1.00 76.24           N
ATOM    376  CA   TYR A  46       9.091  13.983  35.304  1.00 76.56           C
ATOM    377  CB   TYR A  46      10.020  13.038  36.071  1.00 72.55           C
ATOM    378  CG   TYR A  46       9.428  11.708  36.466  1.00 64.06           C
ATOM    379  CD1  TYR A  46       8.379  11.633  37.357  1.00 73.18           C
ATOM    380  CE1  TYR A  46       7.849  10.411  37.734  1.00 72.99           C
ATOM    381  CZ   TYR A  46       8.377   9.253  37.223  1.00 67.54           C
ATOM    382  OH   TYR A  46       7.857   8.032  37.608  1.00 72.25           O
ATOM    383  CE2  TYR A  46       9.424   9.309  36.341  1.00 62.50           C
ATOM    384  CD2  TYR A  46       9.951  10.526  35.979  1.00 64.36           C
ATOM    385  C    TYR A  46       9.569  15.400  35.565  1.00 79.28           C
ATOM    386  O    TYR A  46       9.575  15.852  36.709  1.00 79.41           O
ATOM    387  N    ALA A  47      10.008  16.079  34.513  1.00 90.53           N
ATOM    388  CA   ALA A  47      10.575  17.424  34.632  1.00 94.35           C
ATOM    389  CB   ALA A  47      10.109  18.305  33.477  1.00 94.31           C
ATOM    390  C    ALA A  47      10.293  18.101  35.978  1.00 90.88           C
ATOM    391  O    ALA A  47       9.147  18.440  36.284  1.00 84.10           O
ATOM    392  N    GLY A  48      11.345  18.282  36.777  1.00 97.59           N
ATOM    393  CA   GLY A  48      11.248  18.998  38.036  1.00 99.33           C
ATOM    394  C    GLY A  48      10.733  18.158  39.190  1.00104.25           C
ATOM    395  O    GLY A  48      10.209  18.690  40.172  1.00108.99           O
ATOM    396  N    LYS A  49      10.867  16.842  39.068  1.00 99.69           N
ATOM    397  CA   LYS A  49      10.507  15.933  40.148  1.00 94.06           C
ATOM    398  CB   LYS A  49       9.346  15.036  39.742  1.00 94.57           C
ATOM    399  CG   LYS A  49       8.019  15.743  39.599  1.00 96.63           C
ATOM    400  CD   LYS A  49       6.983  14.780  39.051  1.00 94.95           C
ATOM    401  CE   LYS A  49       5.582  15.373  39.080  1.00100.99           C
ATOM    402  NZ   LYS A  49       4.567  14.369  38.640  1.00 96.64           N
ATOM    403  C    LYS A  49      11.711  15.067  40.444  1.00 92.39           C
ATOM    404  O    LYS A  49      12.290  15.138  41.527  1.00 99.57           O
ATOM    405  N    VAL A  50      12.078  14.247  39.467  1.00 62.39           N
ATOM    406  CA   VAL A  50      13.270  13.433  39.561  1.00 56.12           C
ATOM    407  CB   VAL A  50      12.991  11.990  39.117  1.00 52.89           C
ATOM    408  CG1  VAL A  50      14.238  11.134  39.280  1.00 47.70           C
ATOM    409  CG2  VAL A  50      11.826  11.408  39.910  1.00 55.45           C
ATOM    410  C    VAL A  50      14.327  14.026  38.660  1.00 58.14           C
ATOM    411  O    VAL A  50      14.033  14.509  37.560  1.00 54.59           O
ATOM    412  N    VAL A  51      15.556  14.001  39.151  1.00 56.06           N
ATOM    413  CA   VAL A  51      16.710  14.437  38.391  1.00 54.13           C
ATOM    414  CB   VAL A  51      17.748  15.076  39.334  1.00 54.20           C
ATOM    415  CG1  VAL A  51      18.970  15.568  38.569  1.00 44.70           C
ATOM    416  CG2  VAL A  51      17.108  16.202  40.131  1.00 55.34           C
ATOM    417  C    VAL A  51      17.314  13.196  37.751  1.00 52.68           C
ATOM    418  O    VAL A  51      17.424  12.151  38.400  1.00 49.82           O
ATOM    419  N    PHE A  52      17.709  13.298  36.487  1.00 44.22           N
ATOM    420  CA   PHE A  52      18.283  12.141  35.808  1.00 45.68           C
ATOM    421  CB   PHE A  52      17.374  11.692  34.655  1.00 41.91           C
ATOM    422  CG   PHE A  52      16.031  11.212  35.101  1.00 39.09           C
ATOM    423  CD2  PHE A  52      15.776   9.853  35.233  1.00 42.63           C
ATOM    424  CE2  PHE A  52      14.535   9.392  35.650  1.00 38.82           C
ATOM    425  CZ   PHE A  52      13.517  10.296  35.939  1.00 46.06           C
ATOM    426  CE1  PHE A  52      13.762  11.666  35.818  1.00 52.73           C
ATOM    427  CD1  PHE A  52      15.023  12.112  35.405  1.00 47.52           C
ATOM    428  C    PHE A  52      19.696  12.424  35.302  1.00 48.32           C
ATOM    429  O    PHE A  52      19.924  13.415  34.613  1.00 53.51           O
ATOM    430  N    GLY A  53      20.636  11.542  35.630  1.00 44.13           N
ATOM    431  CA   GLY A  53      22.032  11.738  35.266  1.00 46.50           C
```

| ATOM | 432 | C   | GLY | A | 53 | 22.720 | 10.505 | 34.698 | 1.00 | 47.50 | C |
| ATOM | 433 | O   | GLY | A | 53 | 22.436 | 9.374  | 35.099 | 1.00 | 46.48 | O |
| ATOM | 434 | N   | LYS | A | 54 | 23.637 | 10.725 | 33.762 | 1.00 | 43.69 | N |
| ATOM | 435 | CA  | LYS | A | 54 | 24.380 | 9.641  | 33.146 | 1.00 | 46.23 | C |
| ATOM | 436 | CB  | LYS | A | 54 | 24.305 | 9.750  | 31.619 | 1.00 | 48.25 | C |
| ATOM | 437 | CG  | LYS | A | 54 | 22.898 | 9.819  | 31.064 | 1.00 | 59.49 | C |
| ATOM | 438 | CD  | LYS | A | 54 | 22.904 | 9.871  | 29.535 | 1.00 | 62.01 | C |
| ATOM | 439 | CE  | LYS | A | 54 | 23.287 | 8.522  | 28.941 | 1.00 | 63.95 | C |
| ATOM | 440 | NZ  | LYS | A | 54 | 22.511 | 7.412  | 29.558 | 1.00 | 62.92 | N |
| ATOM | 441 | C   | LYS | A | 54 | 25.836 | 9.681  | 33.579 | 1.00 | 45.36 | C |
| ATOM | 442 | O   | LYS | A | 54 | 26.397 | 10.755 | 33.774 | 1.00 | 47.64 | O |
| ATOM | 443 | N   | VAL | A | 55 | 26.446 | 8.506  | 33.692 | 1.00 | 38.19 | N |
| ATOM | 444 | CA  | VAL | A | 55 | 27.850 | 8.386  | 34.061 | 1.00 | 33.44 | C |
| ATOM | 445 | CB  | VAL | A | 55 | 28.018 | 7.946  | 35.528 | 1.00 | 44.45 | C |
| ATOM | 446 | CG1 | VAL | A | 55 | 29.493 | 7.646  | 35.858 | 1.00 | 34.00 | C |
| ATOM | 447 | CG2 | VAL | A | 55 | 27.458 | 9.007  | 36.475 | 1.00 | 39.10 | C |
| ATOM | 448 | C   | VAL | A | 55 | 28.598 | 7.373  | 33.201 | 1.00 | 36.90 | C |
| ATOM | 449 | O   | VAL | A | 55 | 28.381 | 6.167  | 33.320 | 1.00 | 39.80 | O |
| ATOM | 450 | N   | ASN | A | 56 | 29.510 | 7.871  | 32.369 | 1.00 | 41.37 | N |
| ATOM | 451 | CA  | ASN | A | 56 | 30.322 | 7.039  | 31.488 | 1.00 | 40.98 | C |
| ATOM | 452 | CB  | ASN | A | 56 | 30.999 | 7.922  | 30.432 | 1.00 | 43.39 | C |
| ATOM | 453 | CG  | ASN | A | 56 | 31.533 | 7.129  | 29.248 | 1.00 | 56.82 | C |
| ATOM | 454 | OD1 | ASN | A | 56 | 32.252 | 6.147  | 29.422 | 1.00 | 58.12 | O |
| ATOM | 455 | ND2 | ASN | A | 56 | 31.168 | 7.552  | 28.025 | 1.00 | 55.17 | N |
| ATOM | 456 | C   | ASN | A | 56 | 31.360 | 6.209  | 32.250 | 1.00 | 47.59 | C |
| ATOM | 457 | O   | ASN | A | 56 | 32.392 | 6.699  | 32.702 | 1.00 | 56.82 | O |
| ATOM | 458 | N   | VAL | A | 57 | 31.088 | 4.931  | 32.380 | 1.00 | 35.23 | N |
| ATOM | 459 | CA  | VAL | A | 57 | 31.937 | 4.065  | 33.165 | 1.00 | 38.26 | C |
| ATOM | 460 | CB  | VAL | A | 57 | 31.213 | 2.751  | 33.344 | 1.00 | 35.80 | C |
| ATOM | 461 | CG1 | VAL | A | 57 | 32.169 | 1.599  | 33.536 | 1.00 | 49.63 | C |
| ATOM | 462 | CG2 | VAL | A | 57 | 30.221 | 2.897  | 34.503 | 1.00 | 32.54 | C |
| ATOM | 463 | C   | VAL | A | 57 | 33.392 | 3.935  | 32.645 | 1.00 | 56.19 | C |
| ATOM | 464 | O   | VAL | A | 57 | 34.306 | 3.509  | 33.373 | 1.00 | 54.69 | O |
| ATOM | 465 | N   | ASP | A | 58 | 33.610 | 4.342  | 31.401 | 1.00 | 56.01 | N |
| ATOM | 466 | CA  | ASP | A | 58 | 34.962 | 4.470  | 30.874 | 1.00 | 61.21 | C |
| ATOM | 467 | CB  | ASP | A | 58 | 34.964 | 4.525  | 29.341 | 1.00 | 59.67 | C |
| ATOM | 468 | CG  | ASP | A | 58 | 34.598 | 3.193  | 28.710 | 1.00 | 66.87 | C |
| ATOM | 469 | OD1 | ASP | A | 58 | 34.975 | 2.138  | 29.275 | 1.00 | 62.78 | O |
| ATOM | 470 | OD2 | ASP | A | 58 | 33.919 | 3.200  | 27.655 | 1.00 | 78.46 | O |
| ATOM | 471 | C   | ASP | A | 58 | 35.614 | 5.722  | 31.433 | 1.00 | 67.99 | C |
| ATOM | 472 | O   | ASP | A | 58 | 36.644 | 5.649  | 32.091 | 1.00 | 78.17 | O |
| ATOM | 473 | N   | GLU | A | 59 | 34.994 | 6.870  | 31.196 | 1.00 | 65.81 | N |
| ATOM | 474 | CA  | GLU | A | 59 | 35.532 | 8.137  | 31.676 | 1.00 | 67.13 | C |
| ATOM | 475 | CB  | GLU | A | 59 | 34.695 | 9.301  | 31.124 | 1.00 | 68.72 | C |
| ATOM | 476 | CG  | GLU | A | 59 | 34.163 | 9.059  | 29.691 | 1.00 | 71.52 | C |
| ATOM | 477 | CD  | GLU | A | 59 | 33.552 | 10.309 | 29.021 | 1.00 | 79.95 | C |
| ATOM | 478 | OE1 | GLU | A | 59 | 32.885 | 11.108 | 29.718 | 1.00 | 79.67 | O |
| ATOM | 479 | OE2 | GLU | A | 59 | 33.736 | 10.488 | 27.790 | 1.00 | 72.91 | O |
| ATOM | 480 | C   | GLU | A | 59 | 35.648 | 8.218  | 33.216 | 1.00 | 77.98 | C |
| ATOM | 481 | O   | GLU | A | 59 | 36.376 | 9.062  | 33.736 | 1.00 | 76.37 | O |
| ATOM | 482 | N   | ASN | A | 60 | 34.950 | 7.337  | 33.942 | 1.00 | 67.37 | N |
| ATOM | 483 | CA  | ASN | A | 60 | 34.921 | 7.407  | 35.412 | 1.00 | 60.51 | C |
| ATOM | 484 | CB  | ASN | A | 60 | 33.711 | 8.214  | 35.890 | 1.00 | 46.14 | C |
| ATOM | 485 | CG  | ASN | A | 60 | 33.282 | 9.282  | 34.886 | 1.00 | 60.40 | C |
| ATOM | 486 | OD1 | ASN | A | 60 | 33.563 | 10.473 | 35.052 | 1.00 | 64.54 | O |
| ATOM | 487 | ND2 | ASN | A | 60 | 32.590 | 8.854  | 33.838 | 1.00 | 53.67 | N |
| ATOM | 488 | C   | ASN | A | 60 | 34.913 | 6.042  | 36.103 | 1.00 | 59.53 | C |
| ATOM | 489 | O   | ASN | A | 60 | 33.963 | 5.710  | 36.813 | 1.00 | 52.80 | O |
| ATOM | 490 | N   | PRO | A | 61 | 35.996 | 5.265  | 35.930 | 1.00 | 72.66 | N |
| ATOM | 491 | CA  | PRO | A | 61 | 36.143 | 3.863  | 36.375 | 1.00 | 69.61 | C |
| ATOM | 492 | CB  | PRO | A | 61 | 37.554 | 3.477  | 35.898 | 1.00 | 63.03 | C |
| ATOM | 493 | CG  | PRO | A | 61 | 37.995 | 4.586  | 34.989 | 1.00 | 69.75 | C |
| ATOM | 494 | CD  | PRO | A | 61 | 37.249 | 5.808  | 35.383 | 1.00 | 69.43 | C |

| ATOM | 495 | C | PRO | A | 61 | 36.075 | 3.648 | 37.891 | 1.00 | 73.17 | C |
| ATOM | 496 | O | PRO | A | 61 | 35.811 | 2.521 | 38.340 | 1.00 | 64.06 | O |
| ATOM | 497 | N | GLU | A | 62 | 36.331 | 4.708 | 38.656 | 1.00 | 84.34 | N |
| ATOM | 498 | CA | GLU | A | 62 | 36.444 | 4.615 | 40.110 | 1.00 | 93.89 | C |
| ATOM | 499 | CB | GLU | A | 62 | 37.537 | 5.555 | 40.636 | 1.00 | 84.86 | C |
| ATOM | 500 | CG | GLU | A | 62 | 37.250 | 7.031 | 40.410 | 1.00 | 92.10 | C |
| ATOM | 501 | CD | GLU | A | 62 | 36.924 | 7.346 | 38.949 | 1.00 | 93.85 | C |
| ATOM | 502 | OE1 | GLU | A | 62 | 37.863 | 7.557 | 38.153 | 1.00 | 94.51 | O |
| ATOM | 503 | OE2 | GLU | A | 62 | 35.728 | 7.377 | 38.590 | 1.00 | 93.66 | O |
| ATOM | 504 | C | GLU | A | 62 | 35.104 | 4.874 | 40.808 | 1.00 | 88.68 | C |
| ATOM | 505 | O | GLU | A | 62 | 34.675 | 4.068 | 41.626 | 1.00 | 78.72 | O |
| ATOM | 506 | N | ILE | A | 63 | 34.451 | 5.992 | 40.486 | 1.00 | 74.77 | N |
| ATOM | 507 | CA | ILE | A | 63 | 33.091 | 6.252 | 40.969 | 1.00 | 77.65 | C |
| ATOM | 508 | CB | ILE | A | 63 | 32.531 | 7.531 | 40.355 | 1.00 | 65.68 | C |
| ATOM | 509 | CG1 | ILE | A | 63 | 31.035 | 7.633 | 40.587 | 1.00 | 65.15 | C |
| ATOM | 510 | CD1 | ILE | A | 63 | 30.475 | 8.968 | 40.147 | 1.00 | 68.07 | C |
| ATOM | 511 | CG2 | ILE | A | 63 | 32.758 | 7.515 | 38.893 | 1.00 | 68.04 | C |
| ATOM | 512 | C | ILE | A | 63 | 32.241 | 5.045 | 40.595 | 1.00 | 66.12 | C |
| ATOM | 513 | O | ILE | A | 63 | 31.450 | 4.529 | 41.386 | 1.00 | 62.36 | O |
| ATOM | 514 | N | ALA | A | 64 | 32.454 | 4.570 | 39.383 | 1.00 | 48.98 | N |
| ATOM | 515 | CA | ALA | A | 64 | 31.961 | 3.258 | 39.018 | 1.00 | 52.44 | C |
| ATOM | 516 | CB | ALA | A | 64 | 32.567 | 2.814 | 37.683 | 1.00 | 49.52 | C |
| ATOM | 517 | C | ALA | A | 64 | 32.291 | 2.259 | 40.134 | 1.00 | 47.56 | C |
| ATOM | 518 | O | ALA | A | 64 | 31.393 | 1.709 | 40.772 | 1.00 | 47.94 | O |
| ATOM | 519 | N | ALA | A | 65 | 33.582 | 2.057 | 40.390 | 1.00 | 76.11 | N |
| ATOM | 520 | CA | ALA | A | 65 | 34.043 | 1.109 | 41.414 | 1.00 | 77.00 | C |
| ATOM | 521 | CB | ALA | A | 65 | 35.537 | 0.858 | 41.267 | 1.00 | 75.70 | C |
| ATOM | 522 | C | ALA | A | 65 | 33.713 | 1.532 | 42.850 | 1.00 | 66.64 | C |
| ATOM | 523 | O | ALA | A | 65 | 33.484 | 0.678 | 43.701 | 1.00 | 61.04 | O |
| ATOM | 524 | N | LYS | A | 66 | 33.696 | 2.837 | 43.114 | 1.00 | 44.96 | N |
| ATOM | 525 | CA | LYS | A | 66 | 33.328 | 3.336 | 44.433 | 1.00 | 51.42 | C |
| ATOM | 526 | CB | LYS | A | 66 | 33.148 | 4.852 | 44.413 | 1.00 | 53.94 | C |
| ATOM | 527 | CG | LYS | A | 66 | 32.494 | 5.390 | 45.701 | 1.00 | 59.24 | C |
| ATOM | 528 | CD | LYS | A | 66 | 32.471 | 6.934 | 45.774 | 1.00 | 62.72 | C |
| ATOM | 529 | CE | LYS | A | 66 | 33.814 | 7.565 | 45.381 | 1.00 | 70.11 | C |
| ATOM | 530 | NZ | LYS | A | 66 | 34.074 | 7.548 | 43.891 | 1.00 | 73.37 | N |
| ATOM | 531 | C | LYS | A | 66 | 32.017 | 2.710 | 44.854 | 1.00 | 57.94 | C |
| ATOM | 532 | O | LYS | A | 66 | 31.956 | 1.915 | 45.797 | 1.00 | 59.93 | O |
| ATOM | 533 | N | TYR | A | 67 | 30.972 | 3.081 | 44.122 | 1.00 | 60.30 | N |
| ATOM | 534 | CA | TYR | A | 67 | 29.635 | 2.589 | 44.346 | 1.00 | 51.39 | C |
| ATOM | 535 | CB | TYR | A | 67 | 28.639 | 3.549 | 43.719 | 1.00 | 48.16 | C |
| ATOM | 536 | CG | TYR | A | 67 | 28.561 | 4.916 | 44.374 | 1.00 | 50.71 | C |
| ATOM | 537 | CD1 | TYR | A | 67 | 27.942 | 5.085 | 45.611 | 1.00 | 52.64 | C |
| ATOM | 538 | CE1 | TYR | A | 67 | 27.848 | 6.349 | 46.214 | 1.00 | 50.96 | C |
| ATOM | 539 | CZ | TYR | A | 67 | 28.372 | 7.462 | 45.570 | 1.00 | 54.02 | C |
| ATOM | 540 | OH | TYR | A | 67 | 28.277 | 8.721 | 46.157 | 1.00 | 48.49 | O |
| ATOM | 541 | CE2 | TYR | A | 67 | 28.987 | 7.313 | 44.331 | 1.00 | 49.25 | C |
| ATOM | 542 | CD2 | TYR | A | 67 | 29.078 | 6.044 | 43.746 | 1.00 | 48.30 | C |
| ATOM | 543 | C | TYR | A | 67 | 29.437 | 1.184 | 43.776 | 1.00 | 54.82 | C |
| ATOM | 544 | O | TYR | A | 67 | 28.310 | 0.766 | 43.515 | 1.00 | 56.39 | O |
| ATOM | 545 | N | GLY | A | 68 | 30.536 | 0.466 | 43.574 | 1.00 | 56.40 | N |
| ATOM | 546 | CA | GLY | A | 68 | 30.482 | -0.950 | 43.254 | 1.00 | 55.05 | C |
| ATOM | 547 | C | GLY | A | 68 | 29.645 | -1.348 | 42.056 | 1.00 | 67.16 | C |
| ATOM | 548 | O | GLY | A | 68 | 28.865 | -2.312 | 42.113 | 1.00 | 64.48 | O |
| ATOM | 549 | N | ILE | A | 69 | 29.816 | -0.617 | 40.959 | 1.00 | 60.21 | N |
| ATOM | 550 | CA | ILE | A | 69 | 29.095 | -0.923 | 39.733 | 1.00 | 64.12 | C |
| ATOM | 551 | CB | ILE | A | 69 | 29.036 | 0.286 | 38.769 | 1.00 | 65.56 | C |
| ATOM | 552 | CG1 | ILE | A | 69 | 28.344 | 1.460 | 39.460 | 1.00 | 60.89 | C |
| ATOM | 553 | CD1 | ILE | A | 69 | 26.968 | 1.108 | 39.974 | 1.00 | 52.49 | C |
| ATOM | 554 | CG2 | ILE | A | 69 | 28.291 | -0.088 | 37.491 | 1.00 | 53.30 | C |
| ATOM | 555 | C | ILE | A | 69 | 29.735 | -2.120 | 39.040 | 1.00 | 68.87 | C |
| ATOM | 556 | O | ILE | A | 69 | 30.838 | -2.029 | 38.498 | 1.00 | 56.10 | O |
| ATOM | 557 | N | MET | A | 70 | 29.025 | -3.240 | 39.062 | 1.00 | 96.40 | N |

| ATOM | 558 | CA | MET A | 70 | 29.536 | -4.486 | 38.519 | 1.00 | 91.03 | C |
| ATOM | 559 | CB | MET A | 70 | 28.636 | -5.641 | 38.952 | 1.00 | 92.70 | C |
| ATOM | 560 | CG | MET A | 70 | 28.511 | -5.796 | 40.462 | 1.00 | 110.83 | C |
| ATOM | 561 | SD | MET A | 70 | 30.035 | -6.383 | 41.252 | 1.00 | 131.58 | S |
| ATOM | 562 | CE | MET A | 70 | 30.629 | -4.890 | 42.043 | 1.00 | 107.40 | C |
| ATOM | 563 | C | MET A | 70 | 29.578 | -4.434 | 37.005 | 1.00 | 94.80 | C |
| ATOM | 564 | O | MET A | 70 | 30.609 | -4.117 | 36.413 | 1.00 | 94.51 | O |
| ATOM | 565 | N | ASER A | 71 | 28.444 | -4.755 | 36.403 | 1.00 | 66.34 | N |
| ATOM | 566 | CA | ASER A | 71 | 28.257 | -4.705 | 34.972 | 1.00 | 60.37 | C |
| ATOM | 567 | CB | ASER A | 71 | 27.592 | -5.995 | 34.517 | 1.00 | 62.84 | C |
| ATOM | 568 | OG | ASER A | 71 | 26.418 | -6.218 | 35.276 | 1.00 | 60.95 | O |
| ATOM | 569 | C | ASER A | 71 | 27.327 | -3.561 | 34.670 | 1.00 | 56.56 | C |
| ATOM | 570 | O | ASER A | 71 | 26.519 | -3.190 | 35.496 | 1.00 | 60.71 | O |
| ATOM | 571 | N | BSER A | 71 | 28.455 | -4.756 | 36.382 | 0.00 | 66.65 | N |
| ATOM | 572 | CA | BSER A | 71 | 28.339 | -4.711 | 34.932 | 0.00 | 59.78 | C |
| ATOM | 573 | CB | BSER A | 71 | 28.094 | -6.109 | 34.360 | 0.00 | 60.66 | C |
| ATOM | 574 | OG | BSER A | 71 | 29.157 | -6.993 | 34.676 | 0.00 | 61.36 | O |
| ATOM | 575 | C | BSER A | 71 | 27.208 | -3.773 | 34.530 | 0.00 | 57.10 | C |
| ATOM | 576 | O | BSER A | 71 | 26.156 | -3.743 | 35.160 | 0.00 | 56.96 | O |
| ATOM | 577 | N | ILE A | 72 | 27.437 | -3.003 | 33.476 | 1.00 | 46.79 | N |
| ATOM | 578 | CA | ILE A | 72 | 26.457 | -2.046 | 32.998 | 1.00 | 44.17 | C |
| ATOM | 579 | CB | ILE A | 72 | 27.123 | -0.807 | 32.385 | 1.00 | 39.94 | C |
| ATOM | 580 | CG1 | ILE A | 72 | 28.158 | -1.201 | 31.325 | 1.00 | 30.91 | C |
| ATOM | 581 | CD1 | ILE A | 72 | 28.853 | 0.020 | 30.721 | 1.00 | 35.85 | C |
| ATOM | 582 | CG2 | ILE A | 72 | 27.713 | 0.064 | 33.487 | 1.00 | 33.09 | C |
| ATOM | 583 | C | ILE A | 72 | 25.540 | -2.738 | 31.980 | 1.00 | 42.80 | C |
| ATOM | 584 | O | ILE A | 72 | 25.865 | -3.833 | 31.516 | 1.00 | 45.02 | O |
| ATOM | 585 | N | PRO A | 73 | 24.364 | -2.139 | 31.681 | 1.00 | 45.08 | N |
| ATOM | 586 | CA | PRO A | 73 | 23.842 | -0.898 | 32.275 | 1.00 | 42.17 | C |
| ATOM | 587 | CB | PRO A | 73 | 22.685 | -0.538 | 31.352 | 1.00 | 40.79 | C |
| ATOM | 588 | CG | PRO A | 73 | 22.118 | -1.894 | 30.988 | 1.00 | 44.40 | C |
| ATOM | 589 | CD | PRO A | 73 | 23.324 | -2.840 | 30.894 | 1.00 | 48.45 | C |
| ATOM | 590 | C | PRO A | 73 | 23.287 | -1.187 | 33.661 | 1.00 | 41.24 | C |
| ATOM | 591 | O | PRO A | 73 | 22.857 | -2.311 | 33.931 | 1.00 | 41.06 | O |
| ATOM | 592 | N | THR A | 74 | 23.272 | -0.168 | 34.506 | 1.00 | 34.88 | N |
| ATOM | 593 | CA | THR A | 74 | 22.797 | -0.297 | 35.859 | 1.00 | 36.45 | C |
| ATOM | 594 | CB | THR A | 74 | 23.935 | -0.797 | 36.765 | 1.00 | 46.44 | C |
| ATOM | 595 | OG1 | THR A | 74 | 23.949 | -2.232 | 36.738 | 1.00 | 43.67 | O |
| ATOM | 596 | CG2 | THR A | 74 | 23.781 | -0.283 | 38.210 | 1.00 | 44.52 | C |
| ATOM | 597 | C | THR A | 74 | 22.294 | 1.059 | 36.300 | 1.00 | 33.11 | C |
| ATOM | 598 | O | THR A | 74 | 22.888 | 2.086 | 35.977 | 1.00 | 34.58 | O |
| ATOM | 599 | N | LEU A | 75 | 21.178 | 1.076 | 37.011 | 1.00 | 36.36 | N |
| ATOM | 600 | CA | LEU A | 75 | 20.620 | 2.336 | 37.476 | 1.00 | 38.78 | C |
| ATOM | 601 | C | LEU A | 75 | 20.703 | 2.435 | 38.988 | 1.00 | 38.71 | C |
| ATOM | 602 | O | LEU A | 75 | 20.427 | 1.463 | 39.678 | 1.00 | 40.16 | O |
| ATOM | 603 | CB | LEU A | 75 | 19.170 | 2.431 | 37.056 | 1.00 | 33.84 | C |
| ATOM | 604 | CG | LEU A | 75 | 18.827 | 3.192 | 35.790 | 1.00 | 36.18 | C |
| ATOM | 605 | CD1 | LEU A | 75 | 19.805 | 2.879 | 34.700 | 1.00 | 44.96 | C |
| ATOM | 606 | CD2 | LEU A | 75 | 17.427 | 2.779 | 35.397 | 1.00 | 34.75 | C |
| ATOM | 607 | N | LEU A | 76 | 21.048 | 3.610 | 39.502 | 1.00 | 35.54 | N |
| ATOM | 608 | CA | LEU A | 76 | 21.203 | 3.805 | 40.942 | 1.00 | 35.11 | C |
| ATOM | 609 | CB | LEU A | 76 | 22.633 | 4.274 | 41.254 | 1.00 | 43.47 | C |
| ATOM | 610 | CG | LEU A | 76 | 23.510 | 3.459 | 42.201 | 1.00 | 39.70 | C |
| ATOM | 611 | CD1 | LEU A | 76 | 23.430 | 1.980 | 41.853 | 1.00 | 37.85 | C |
| ATOM | 612 | CD2 | LEU A | 76 | 24.945 | 3.951 | 42.112 | 1.00 | 45.25 | C |
| ATOM | 613 | C | LEU A | 76 | 20.256 | 4.888 | 41.377 | 1.00 | 35.01 | C |
| ATOM | 614 | O | LEU A | 76 | 20.291 | 5.982 | 40.830 | 1.00 | 37.73 | O |
| ATOM | 615 | N | PHE A | 77 | 19.399 | 4.611 | 42.353 | 1.00 | 48.58 | N |
| ATOM | 616 | CA | PHE A | 77 | 18.599 | 5.696 | 42.910 | 1.00 | 48.57 | C |
| ATOM | 617 | CB | PHE A | 77 | 17.170 | 5.259 | 43.240 | 1.00 | 48.66 | C |
| ATOM | 618 | CG | PHE A | 77 | 16.444 | 4.633 | 42.079 | 1.00 | 51.12 | C |
| ATOM | 619 | CD1 | PHE A | 77 | 17.073 | 4.469 | 40.857 | 1.00 | 57.06 | C |
| ATOM | 620 | CE1 | PHE A | 77 | 16.428 | 3.885 | 39.789 | 1.00 | 48.27 | C |

| ATOM | 621 | CZ | PHE | A | 77 | 15.131 | 3.466 | 39.921 | 1.00 | 55.24 | C |
|------|-----|-----|-----|---|----|--------|-------|--------|------|-------|---|
| ATOM | 622 | CE2 | PHE | A | 77 | 14.479 | 3.633 | 41.125 | 1.00 | 64.99 | C |
| ATOM | 623 | CD2 | PHE | A | 77 | 15.139 | 4.216 | 42.201 | 1.00 | 59.20 | C |
| ATOM | 624 | C | PHE | A | 77 | 19.307 | 6.247 | 44.139 | 1.00 | 50.97 | C |
| ATOM | 625 | O | PHE | A | 77 | 19.667 | 5.506 | 45.060 | 1.00 | 46.84 | O |
| ATOM | 626 | N | PHE | A | 78 | 19.521 | 7.558 | 44.122 | 1.00 | 50.78 | N |
| ATOM | 627 | CA | PHE | A | 78 | 20.211 | 8.260 | 45.182 | 1.00 | 49.34 | C |
| ATOM | 628 | CB | PHE | A | 78 | 21.293 | 9.164 | 44.597 | 1.00 | 49.00 | C |
| ATOM | 629 | CG | PHE | A | 78 | 22.602 | 8.474 | 44.329 | 1.00 | 55.83 | C |
| ATOM | 630 | CD2 | PHE | A | 78 | 22.878 | 7.942 | 43.083 | 1.00 | 57.57 | C |
| ATOM | 631 | CE2 | PHE | A | 78 | 24.099 | 7.320 | 42.834 | 1.00 | 56.85 | C |
| ATOM | 632 | CZ | PHE | A | 78 | 25.059 | 7.241 | 43.825 | 1.00 | 54.28 | C |
| ATOM | 633 | CE1 | PHE | A | 78 | 24.799 | 7.772 | 45.065 | 1.00 | 51.56 | C |
| ATOM | 634 | CD1 | PHE | A | 78 | 23.580 | 8.391 | 45.312 | 1.00 | 59.69 | C |
| ATOM | 635 | C | PHE | A | 78 | 19.208 | 9.138 | 45.893 | 1.00 | 54.17 | C |
| ATOM | 636 | O | PHE | A | 78 | 18.461 | 9.863 | 45.249 | 1.00 | 51.45 | O |
| ATOM | 637 | N | LYS | A | 79 | 19.204 | 9.097 | 47.217 | 1.00 | 63.44 | N |
| ATOM | 638 | CA | LYS | A | 79 | 18.310 | 9.947 | 47.986 | 1.00 | 64.82 | C |
| ATOM | 639 | CB | LYS | A | 79 | 17.132 | 9.125 | 48.525 | 1.00 | 62.83 | C |
| ATOM | 640 | CG | LYS | A | 79 | 16.005 | 9.963 | 49.076 | 1.00 | 57.94 | C |
| ATOM | 641 | CD | LYS | A | 79 | 15.606 | 11.011 | 48.078 | 1.00 | 64.26 | C |
| ATOM | 642 | CE | LYS | A | 79 | 14.563 | 11.945 | 48.646 | 1.00 | 64.13 | C |
| ATOM | 643 | NZ | LYS | A | 79 | 15.199 | 13.174 | 49.167 | 1.00 | 67.89 | N |
| ATOM | 644 | C | LYS | A | 79 | 19.094 | 10.592 | 49.127 | 1.00 | 60.03 | C |
| ATOM | 645 | O | LYS | A | 79 | 19.835 | 9.913 | 49.844 | 1.00 | 62.59 | O |
| ATOM | 646 | N | ASN | A | 80 | 18.941 | 11.902 | 49.284 | 1.00 | 46.36 | N |
| ATOM | 647 | CA | ASN | A | 80 | 19.639 | 12.617 | 50.347 | 1.00 | 57.29 | C |
| ATOM | 648 | CB | ASN | A | 80 | 19.017 | 12.305 | 51.721 | 1.00 | 54.52 | C |
| ATOM | 649 | CG | ASN | A | 80 | 17.672 | 12.980 | 51.922 | 1.00 | 56.75 | C |
| ATOM | 650 | OD1 | ASN | A | 80 | 17.488 | 14.149 | 51.562 | 1.00 | 55.42 | O |
| ATOM | 651 | ND2 | ASN | A | 80 | 16.720 | 12.246 | 52.498 | 1.00 | 52.91 | N |
| ATOM | 652 | C | ASN | A | 80 | 21.128 | 12.286 | 50.375 | 1.00 | 54.15 | C |
| ATOM | 653 | O | ASN | A | 80 | 21.720 | 12.210 | 51.442 | 1.00 | 59.64 | O |
| ATOM | 654 | N | GLY | A | 81 | 21.722 | 12.070 | 49.204 | 1.00 | 46.36 | N |
| ATOM | 655 | CA | GLY | A | 81 | 23.153 | 11.855 | 49.089 | 1.00 | 37.03 | C |
| ATOM | 656 | C | GLY | A | 81 | 23.558 | 10.402 | 49.131 | 1.00 | 40.71 | C |
| ATOM | 657 | O | GLY | A | 81 | 24.663 | 10.053 | 48.713 | 1.00 | 46.00 | O |
| ATOM | 658 | N | LYS | A | 82 | 22.678 | 9.543 | 49.631 | 1.00 | 40.79 | N |
| ATOM | 659 | CA | LYS | A | 82 | 23.013 | 8.121 | 49.717 | 1.00 | 45.83 | C |
| ATOM | 660 | CB | LYS | A | 82 | 22.887 | 7.605 | 51.161 | 1.00 | 51.57 | C |
| ATOM | 661 | CG | LYS | A | 82 | 21.467 | 7.575 | 51.741 | 1.00 | 51.54 | C |
| ATOM | 662 | CD | LYS | A | 82 | 21.505 | 7.293 | 53.262 | 1.00 | 47.42 | C |
| ATOM | 663 | CE | LYS | A | 82 | 20.107 | 7.116 | 53.849 | 1.00 | 58.79 | C |
| ATOM | 664 | NZ | LYS | A | 82 | 19.113 | 8.117 | 53.333 | 1.00 | 57.60 | N |
| ATOM | 665 | C | LYS | A | 82 | 22.218 | 7.239 | 48.742 | 1.00 | 53.14 | C |
| ATOM | 666 | O | LYS | A | 82 | 21.035 | 7.495 | 48.453 | 1.00 | 45.11 | O |
| ATOM | 667 | N | VAL | A | 83 | 22.882 | 6.201 | 48.238 | 1.00 | 57.57 | N |
| ATOM | 668 | CA | VAL | A | 83 | 22.258 | 5.242 | 47.325 | 1.00 | 47.88 | C |
| ATOM | 669 | CB | VAL | A | 83 | 23.292 | 4.241 | 46.806 | 1.00 | 42.21 | C |
| ATOM | 670 | CG1 | VAL | A | 83 | 24.342 | 4.002 | 47.879 | 1.00 | 72.41 | C |
| ATOM | 671 | CG2 | VAL | A | 83 | 22.618 | 2.935 | 46.380 | 1.00 | 46.97 | C |
| ATOM | 672 | C | VAL | A | 83 | 21.072 | 4.512 | 47.983 | 1.00 | 57.40 | C |
| ATOM | 673 | O | VAL | A | 83 | 21.165 | 4.056 | 49.126 | 1.00 | 52.23 | O |
| ATOM | 674 | N | VAL | A | 84 | 19.955 | 4.401 | 47.258 | 1.00 | 55.17 | N |
| ATOM | 675 | CA | VAL | A | 84 | 18.708 | 3.932 | 47.866 | 1.00 | 50.27 | C |
| ATOM | 676 | CB | VAL | A | 84 | 17.729 | 5.132 | 48.079 | 1.00 | 55.25 | C |
| ATOM | 677 | CG1 | VAL | A | 84 | 16.433 | 4.967 | 47.298 | 1.00 | 51.31 | C |
| ATOM | 678 | CG2 | VAL | A | 84 | 17.470 | 5.338 | 49.559 | 1.00 | 48.68 | C |
| ATOM | 679 | C | VAL | A | 84 | 18.047 | 2.739 | 47.157 | 1.00 | 45.69 | C |
| ATOM | 680 | O | VAL | A | 84 | 17.122 | 2.142 | 47.679 | 1.00 | 47.56 | O |
| ATOM | 681 | N | ASP | A | 85 | 18.557 | 2.381 | 45.986 | 1.00 | 48.47 | N |
| ATOM | 682 | CA | ASP | A | 85 | 18.022 | 1.284 | 45.181 | 1.00 | 49.86 | C |
| ATOM | 683 | CB | ASP | A | 85 | 16.589 | 1.590 | 44.721 | 1.00 | 49.28 | C |

```
ATOM    684   CG  ASP A  85      15.822    0.339   44.322   1.00  59.16         C
ATOM    685   OD1 ASP A  85      16.341   -0.779   44.565   1.00  58.58         O
ATOM    686   OD2 ASP A  85      14.696    0.471   43.781   1.00  62.86         O
ATOM    687   C   ASP A  85      18.933    1.083   43.968   1.00  50.64         C
ATOM    688   O   ASP A  85      19.682    1.985   43.598   1.00  41.86         O
ATOM    689   N   GLN A  86      18.856   -0.085   43.340   1.00  46.27         N
ATOM    690   CA  GLN A  86      19.793   -0.421   42.284   1.00  49.48         C
ATOM    691   CB  GLN A  86      21.074   -1.028   42.875   1.00  49.78         C
ATOM    692   CG  GLN A  86      22.111   -1.422   41.853   1.00  51.92         C
ATOM    693   CD  GLN A  86      23.285   -2.173   42.453   1.00  59.84         C
ATOM    694   OE1 GLN A  86      24.215   -1.567   42.995   1.00  58.16         O
ATOM    695   NE2 GLN A  86      23.248   -3.506   42.360   1.00  52.42         N
ATOM    696   C   GLN A  86      19.158   -1.397   41.328   1.00  52.56         C
ATOM    697   O   GLN A  86      18.871   -2.534   41.700   1.00  52.85         O
ATOM    698   N   LEU A  87      18.928   -0.943   40.097   1.00  51.65         N
ATOM    699   CA  LEU A  87      18.352   -1.793   39.063   1.00  43.49         C
ATOM    700   CB  LEU A  87      17.211   -1.095   38.310   1.00  50.83         C
ATOM    701   CG  LEU A  87      16.283   -0.033   38.925   1.00  58.10         C
ATOM    702   CD1 LEU A  87      14.878   -0.165   38.333   1.00  55.28         C
ATOM    703   CD2 LEU A  87      16.215   -0.073   40.446   1.00  56.98         C
ATOM    704   C   LEU A  87      19.456   -2.191   38.094   1.00  49.58         C
ATOM    705   O   LEU A  87      20.049   -1.337   37.428   1.00  49.69         O
ATOM    706   N   VAL A  88      19.748   -3.488   38.021   1.00  46.19         N
ATOM    707   CA  VAL A  88      20.809   -3.938   37.129   1.00  47.56         C
ATOM    708   CB  VAL A  88      21.862   -4.821   37.826   1.00  50.30         C
ATOM    709   CG1 VAL A  88      22.759   -5.486   36.782   1.00  40.02         C
ATOM    710   CG2 VAL A  88      22.689   -3.981   38.767   1.00  37.73         C
ATOM    711   C   VAL A  88      20.274   -4.678   35.930   1.00  44.65         C
ATOM    712   O   VAL A  88      19.534   -5.636   36.067   1.00  46.30         O
ATOM    713   N   GLY A  89      20.685   -4.230   34.746   1.00  47.50         N
ATOM    714   CA  GLY A  89      20.172   -4.749   33.494   1.00  38.50         C
ATOM    715   C   GLY A  89      19.080   -3.855   32.922   1.00  35.81         C
ATOM    716   O   GLY A  89      18.469   -3.057   33.636   1.00  36.36         O
ATOM    717   N   ALA A  90      18.841   -3.987   31.621   1.00  58.32         N
ATOM    718   CA  ALA A  90      17.763   -3.255   30.952   1.00  62.38         C
ATOM    719   CB  ALA A  90      17.927   -3.352   29.441   1.00  48.77         C
ATOM    720   C   ALA A  90      16.378   -3.765   31.378   1.00  51.30         C
ATOM    721   O   ALA A  90      16.197   -4.964   31.602   1.00  56.70         O
ATOM    722   N   ARG A  91      15.417   -2.850   31.508   1.00  47.07         N
ATOM    723   CA  ARG A  91      14.019   -3.212   31.763   1.00  52.53         C
ATOM    724   CB  ARG A  91      13.800   -3.542   33.244   1.00  62.63         C
ATOM    725   CG  ARG A  91      14.097   -2.384   34.191   1.00  62.42         C
ATOM    726   CD  ARG A  91      14.445   -2.868   35.591   1.00  70.13         C
ATOM    727   NE  ARG A  91      15.411   -3.967   35.569   1.00  68.83         N
ATOM    728   CZ  ARG A  91      15.690   -4.740   36.616   1.00  67.30         C
ATOM    729   NH1 ARG A  91      15.079   -4.531   37.782   1.00  46.68         N
ATOM    730   NH2 ARG A  91      16.578   -5.725   36.488   1.00  65.15         N
ATOM    731   C   ARG A  91      13.066   -2.093   31.326   1.00  57.03         C
ATOM    732   O   ARG A  91      13.449   -0.918   31.345   1.00  55.46         O
ATOM    733   N   PRO A  92      11.823   -2.461   30.925   1.00  57.44         N
ATOM    734   CA  PRO A  92      10.780   -1.584   30.364   1.00  53.47         C
ATOM    735   CB  PRO A  92       9.520   -2.441   30.476   1.00  44.62         C
ATOM    736   CG  PRO A  92      10.018   -3.806   30.212   1.00  49.42         C
ATOM    737   CD  PRO A  92      11.387   -3.871   30.892   1.00  56.99         C
ATOM    738   C   PRO A  92      10.563   -0.267   31.084   1.00  51.47         C
ATOM    739   O   PRO A  92      10.686   -0.212   32.299   1.00  58.71         O
ATOM    740   N   LYS A  93      10.219    0.773   30.326   1.00  44.84         N
ATOM    741   CA  LYS A  93       9.884    2.080   30.891   1.00  40.64         C
ATOM    742   CB  LYS A  93       9.434    3.040   29.787   1.00  34.36         C
ATOM    743   CG  LYS A  93       8.944    4.408   30.224   1.00  33.81         C
ATOM    744   CD  LYS A  93       8.691    5.313   28.991   1.00  33.35         C
ATOM    745   CE  LYS A  93       8.075    6.643   29.434   1.00  49.53         C
ATOM    746   NZ  LYS A  93       7.792    7.616   28.346   1.00  58.07         N
```

| ATOM | 747 | C | LYS | A | 93 | 8.846 | 2.000 | 32.023 | 1.00 | 52.11 | C |
|------|-----|-----|-----|---|-----|--------|--------|--------|------|-------|---|
| ATOM | 748 | O | LYS | A | 93 | 9.012 | 2.645 | 33.058 | 1.00 | 53.14 | O |
| ATOM | 749 | N | GLU | A | 94 | 7.795 | 1.202 | 31.851 | 1.00 | 59.73 | N |
| ATOM | 750 | CA | GLU | A | 94 | 6.747 | 1.121 | 32.878 | 1.00 | 61.21 | C |
| ATOM | 751 | CB | GLU | A | 94 | 5.506 | 0.370 | 32.374 | 1.00 | 58.21 | C |
| ATOM | 752 | CG | GLU | A | 94 | 5.787 | -0.599 | 31.226 | 1.00 | 69.25 | C |
| ATOM | 753 | CD | GLU | A | 94 | 5.912 | 0.080 | 29.856 | 1.00 | 64.98 | C |
| ATOM | 754 | OE1 | GLU | A | 94 | 4.948 | 0.767 | 29.453 | 1.00 | 58.73 | O |
| ATOM | 755 | OE2 | GLU | A | 94 | 6.973 | -0.068 | 29.193 | 1.00 | 56.11 | O |
| ATOM | 756 | C | GLU | A | 94 | 7.250 | 0.521 | 34.191 | 1.00 | 62.23 | C |
| ATOM | 757 | O | GLU | A | 94 | 6.772 | 0.881 | 35.267 | 1.00 | 66.11 | O |
| ATOM | 758 | N | ALA | A | 95 | 8.223 | -0.381 | 34.101 | 1.00 | 46.59 | N |
| ATOM | 759 | CA | ALA | A | 95 | 8.789 | -0.997 | 35.291 | 1.00 | 49.78 | C |
| ATOM | 760 | CB | ALA | A | 95 | 9.497 | -2.281 | 34.939 | 1.00 | 48.29 | C |
| ATOM | 761 | C | ALA | A | 95 | 9.730 | -0.039 | 36.034 | 1.00 | 54.56 | C |
| ATOM | 762 | O | ALA | A | 95 | 9.744 | -0.005 | 37.262 | 1.00 | 49.49 | O |
| ATOM | 763 | N | LEU | A | 96 | 10.512 | 0.742 | 35.296 | 1.00 | 56.16 | N |
| ATOM | 764 | CA | LEU | A | 96 | 11.303 | 1.802 | 35.916 | 1.00 | 58.31 | C |
| ATOM | 765 | CB | LEU | A | 96 | 12.123 | 2.562 | 34.870 | 1.00 | 60.94 | C |
| ATOM | 766 | CG | LEU | A | 96 | 13.495 | 1.992 | 34.509 | 1.00 | 61.66 | C |
| ATOM | 767 | CD1 | LEU | A | 96 | 13.378 | 0.533 | 34.148 | 1.00 | 64.27 | C |
| ATOM | 768 | CD2 | LEU | A | 96 | 14.138 | 2.764 | 33.364 | 1.00 | 60.70 | C |
| ATOM | 769 | C | LEU | A | 96 | 10.394 | 2.772 | 36.653 | 1.00 | 57.57 | C |
| ATOM | 770 | O | LEU | A | 96 | 10.743 | 3.269 | 37.717 | 1.00 | 56.98 | O |
| ATOM | 771 | N | LYS | A | 97 | 9.227 | 3.042 | 36.074 | 1.00 | 67.97 | N |
| ATOM | 772 | CA | LYS | A | 97 | 8.277 | 3.981 | 36.656 | 1.00 | 65.41 | C |
| ATOM | 773 | CB | LYS | A | 97 | 7.199 | 4.351 | 35.646 | 1.00 | 73.77 | C |
| ATOM | 774 | CG | LYS | A | 97 | 7.665 | 5.274 | 34.556 | 1.00 | 74.08 | C |
| ATOM | 775 | CD | LYS | A | 97 | 7.110 | 6.667 | 34.758 | 1.00 | 71.53 | C |
| ATOM | 776 | CE | LYS | A | 97 | 5.611 | 6.691 | 34.580 | 1.00 | 80.59 | C |
| ATOM | 777 | NZ | LYS | A | 97 | 5.069 | 8.053 | 34.847 | 1.00 | 91.82 | N |
| ATOM | 778 | C | LYS | A | 97 | 7.615 | 3.407 | 37.888 | 1.00 | 71.95 | C |
| ATOM | 779 | O | LYS | A | 97 | 7.095 | 4.149 | 38.712 | 1.00 | 81.45 | O |
| ATOM | 780 | N | GLU | A | 98 | 7.614 | 2.084 | 37.999 | 1.00 | 65.39 | N |
| ATOM | 781 | CA | GLU | A | 98 | 7.033 | 1.424 | 39.159 | 1.00 | 73.85 | C |
| ATOM | 782 | CB | GLU | A | 98 | 6.702 | -0.041 | 38.843 | 1.00 | 69.72 | C |
| ATOM | 783 | CG | GLU | A | 98 | 5.233 | -0.406 | 39.107 | 1.00 | 72.59 | C |
| ATOM | 784 | CD | GLU | A | 98 | 4.771 | -1.638 | 38.336 | 1.00 | 81.27 | C |
| ATOM | 785 | OE1 | GLU | A | 98 | 3.776 | -1.519 | 37.583 | 1.00 | 76.85 | O |
| ATOM | 786 | OE2 | GLU | A | 98 | 5.391 | -2.719 | 38.485 | 1.00 | 80.46 | O |
| ATOM | 787 | C | GLU | A | 98 | 8.010 | 1.522 | 40.329 | 1.00 | 71.10 | C |
| ATOM | 788 | O | GLU | A | 98 | 7.617 | 1.557 | 41.498 | 1.00 | 69.60 | O |
| ATOM | 789 | N | ARG | A | 99 | 9.290 | 1.607 | 39.987 | 1.00 | 66.73 | N |
| ATOM | 790 | CA | ARG | A | 99 | 10.376 | 1.529 | 40.956 | 1.00 | 58.23 | C |
| ATOM | 791 | CB | ARG | A | 99 | 11.594 | 0.897 | 40.287 | 1.00 | 52.06 | C |
| ATOM | 792 | CG | ARG | A | 99 | 12.685 | 0.471 | 41.226 | 1.00 | 73.40 | C |
| ATOM | 793 | CD | ARG | A | 99 | 12.531 | -0.973 | 41.685 | 1.00 | 73.80 | C |
| ATOM | 794 | NE | ARG | A | 99 | 13.804 | -1.481 | 42.202 | 1.00 | 71.07 | N |
| ATOM | 795 | CZ | ARG | A | 99 | 14.251 | -2.718 | 42.017 | 1.00 | 72.57 | C |
| ATOM | 796 | NH1 | ARG | A | 99 | 13.521 | -3.600 | 41.342 | 1.00 | 74.25 | N |
| ATOM | 797 | NH2 | ARG | A | 99 | 15.428 | -3.072 | 42.511 | 1.00 | 72.33 | N |
| ATOM | 798 | C | ARG | A | 99 | 10.702 | 2.919 | 41.504 | 1.00 | 62.05 | C |
| ATOM | 799 | O | ARG | A | 99 | 11.247 | 3.058 | 42.595 | 1.00 | 63.69 | O |
| ATOM | 800 | N | ILE | A | 100 | 10.319 | 3.946 | 40.752 | 1.00 | 56.12 | N |
| ATOM | 801 | CA | ILE | A | 100 | 10.591 | 5.336 | 41.118 | 1.00 | 52.23 | C |
| ATOM | 802 | CB | ILE | A | 100 | 10.609 | 6.250 | 39.870 | 1.00 | 47.29 | C |
| ATOM | 803 | CG1 | ILE | A | 100 | 11.885 | 6.013 | 39.075 | 1.00 | 45.49 | C |
| ATOM | 804 | CD1 | ILE | A | 100 | 11.943 | 6.842 | 37.826 | 1.00 | 47.64 | C |
| ATOM | 805 | CG2 | ILE | A | 100 | 10.570 | 7.710 | 40.244 | 1.00 | 43.48 | C |
| ATOM | 806 | C | ILE | A | 100 | 9.607 | 5.915 | 42.129 | 1.00 | 59.82 | C |
| ATOM | 807 | O | ILE | A | 100 | 9.983 | 6.741 | 42.959 | 1.00 | 59.68 | O |
| ATOM | 808 | N | LYS | A | 101 | 8.344 | 5.509 | 42.046 | 1.00 | 79.70 | N |
| ATOM | 809 | CA | LYS | A | 101 | 7.317 | 6.043 | 42.940 | 1.00 | 76.96 | C |

52

| | | | | | | | | | | |
|---|---|---|---|---|---|---|---|---|---|---|
| ATOM | 810 | CB | LYS A 101 | 5.938 | 5.598 | 42.465 | 1.00 | 82.89 | | C |
| ATOM | 811 | CG | LYS A 101 | 5.803 | 4.091 | 42.343 | 1.00 | 80.32 | | C |
| ATOM | 812 | CD | LYS A 101 | 4.356 | 3.683 | 42.139 | 1.00 | 86.67 | | C |
| ATOM | 813 | CE | LYS A 101 | 4.218 | 2.172 | 42.011 | 1.00 | 87.56 | | C |
| ATOM | 814 | NZ | LYS A 101 | 4.712 | 1.456 | 43.229 | 1.00 | 87.65 | | N |
| ATOM | 815 | C | LYS A 101 | 7.537 | 5.607 | 44.388 | 1.00 | 74.25 | | C |
| ATOM | 816 | O | LYS A 101 | 7.328 | 6.377 | 45.325 | 1.00 | 71.03 | | O |
| ATOM | 817 | N | LYS A 102 | 7.959 | 4.361 | 44.562 | 1.00 | 77.52 | | N |
| ATOM | 818 | CA | LYS A 102 | 8.247 | 3.833 | 45.884 | 1.00 | 77.12 | | C |
| ATOM | 819 | C | LYS A 102 | 9.395 | 4.604 | 46.531 | 1.00 | 80.30 | | C |
| ATOM | 820 | O | LYS A 102 | 9.802 | 4.300 | 47.650 | 1.00 | 85.37 | | O |
| ATOM | 821 | CB | LYS A 102 | 8.572 | 2.338 | 45.808 | 1.00 | 86.43 | | C |
| ATOM | 822 | CG | LYS A 102 | 9.894 | 1.982 | 45.119 | 1.00 | 88.05 | | C |
| ATOM | 823 | CD | LYS A 102 | 10.096 | 0.464 | 45.091 | 1.00 | 89.96 | | C |
| ATOM | 824 | CE | LYS A 102 | 11.524 | 0.062 | 45.477 | 1.00 | 92.22 | | C |
| ATOM | 825 | NZ | LYS A 102 | 11.553 | −1.174 | 46.334 | 1.00 | 94.05 | | N |
| ATOM | 826 | N | TYR A 103 | 9.911 | 5.591 | 45.801 | 1.00 | 79.35 | | N |
| ATOM | 827 | CA | TYR A 103 | 10.954 | 6.497 | 46.270 | 1.00 | 85.72 | | C |
| ATOM | 828 | CB | TYR A 103 | 12.336 | 6.078 | 45.758 | 1.00 | 82.75 | | C |
| ATOM | 829 | CG | TYR A 103 | 12.917 | 4.897 | 46.481 | 1.00 | 76.37 | | C |
| ATOM | 830 | CD1 | TYR A 103 | 13.259 | 4.988 | 47.817 | 1.00 | 76.14 | | C |
| ATOM | 831 | CE1 | TYR A 103 | 13.787 | 3.901 | 48.496 | 1.00 | 81.23 | | C |
| ATOM | 832 | CZ | TYR A 103 | 13.978 | 2.709 | 47.826 | 1.00 | 85.28 | | C |
| ATOM | 833 | OH | TYR A 103 | 14.502 | 1.620 | 48.494 | 1.00 | 77.89 | | O |
| ATOM | 834 | CE2 | TYR A 103 | 13.637 | 2.602 | 46.489 | 1.00 | 80.80 | | C |
| ATOM | 835 | CD2 | TYR A 103 | 13.115 | 3.690 | 45.830 | 1.00 | 73.41 | | C |
| ATOM | 836 | C | TYR A 103 | 10.652 | 7.864 | 45.717 | 1.00 | 87.35 | | C |
| ATOM | 837 | O | TYR A 103 | 11.529 | 8.518 | 45.163 | 1.00 | 91.19 | | O |
| ATOM | 838 | N | LEU A 104 | 9.402 | 8.287 | 45.834 | 1.00 | 99.12 | | N |
| ATOM | 839 | CA | LEU A 104 | 8.986 | 9.539 | 45.220 | 1.00 | 104.27 | | C |
| ATOM | 840 | CB | LEU A 104 | 7.620 | 9.379 | 44.541 | 1.00 | 95.39 | | C |
| ATOM | 841 | CG | LEU A 104 | 7.483 | 10.111 | 43.204 | 1.00 | 101.70 | | C |
| ATOM | 842 | CD1 | LEU A 104 | 8.861 | 10.305 | 42.566 | 1.00 | 92.92 | | C |
| ATOM | 843 | CD2 | LEU A 104 | 6.530 | 9.362 | 42.268 | 1.00 | 87.83 | | C |
| ATOM | 844 | C | LEU A 104 | 8.972 | 10.683 | 46.234 | 1.00 | 114.63 | | C |
| ATOM | 845 | O | LEU A 104 | 9.117 | 11.851 | 45.868 | 1.00 | 109.80 | | O |
| ATOM | 846 | OXT | LEU A 104 | 8.828 | 10.466 | 47.442 | 1.00 | 117.02 | | O |
| TER | | | | | | | | | | |
| ATOM | 847 | N | SER B 1 | −0.577 | −26.412 | 26.761 | 1.00 | 105.17 | | N |
| ATOM | 848 | CA | SER B 1 | −0.553 | −25.853 | 25.415 | 1.00 | 101.88 | | C |
| ATOM | 849 | CB | SER B 1 | −1.921 | −25.273 | 25.050 | 1.00 | 98.08 | | C |
| ATOM | 850 | OG | SER B 1 | −2.578 | −26.089 | 24.089 | 1.00 | 102.28 | | O |
| ATOM | 851 | C | SER B 1 | 0.521 | −24.784 | 25.291 | 1.00 | 97.18 | | C |
| ATOM | 852 | O | SER B 1 | 1.470 | −24.920 | 24.518 | 1.00 | 97.53 | | O |
| ATOM | 853 | N | VAL B 2 | 0.363 | −23.721 | 26.068 | 1.00 | 95.24 | | N |
| ATOM | 854 | CA | VAL B 2 | 1.298 | −22.609 | 26.053 | 1.00 | 90.10 | | C |
| ATOM | 855 | CB | VAL B 2 | 0.544 | −21.265 | 25.957 | 1.00 | 91.49 | | C |
| ATOM | 856 | CG1 | VAL B 2 | −0.568 | −21.208 | 27.007 | 1.00 | 93.18 | | C |
| ATOM | 857 | CG2 | VAL B 2 | 1.507 | −20.099 | 26.091 | 1.00 | 88.83 | | C |
| ATOM | 858 | C | VAL B 2 | 2.196 | −22.664 | 27.285 | 1.00 | 91.82 | | C |
| ATOM | 859 | O | VAL B 2 | 1.712 | −22.725 | 28.410 | 1.00 | 95.00 | | O |
| ATOM | 860 | N | ILE B 3 | 3.504 | −22.619 | 27.050 | 1.00 | 75.97 | | N |
| ATOM | 861 | CA | ILE B 3 | 4.518 | −23.056 | 28.012 | 1.00 | 69.74 | | C |
| ATOM | 862 | CB | ILE B 3 | 5.629 | −23.779 | 27.249 | 1.00 | 69.49 | | C |
| ATOM | 863 | CG1 | ILE B 3 | 5.104 | −25.083 | 26.668 | 1.00 | 67.00 | | C |
| ATOM | 864 | CD1 | ILE B 3 | 6.057 | −25.728 | 25.717 | 1.00 | 64.77 | | C |
| ATOM | 865 | CG2 | ILE B 3 | 6.829 | −24.023 | 28.142 | 1.00 | 77.43 | | C |
| ATOM | 866 | C | ILE B 3 | 5.229 | −21.946 | 28.786 | 1.00 | 71.35 | | C |
| ATOM | 867 | O | ILE B 3 | 5.940 | −21.134 | 28.187 | 1.00 | 70.42 | | O |
| ATOM | 868 | N | GLU B 4 | 5.096 | −21.928 | 30.112 | 1.00 | 74.48 | | N |
| ATOM | 869 | CA | GLU B 4 | 5.899 | −20.991 | 30.881 | 1.00 | 77.86 | | C |
| ATOM | 870 | CB | GLU B 4 | 5.808 | −21.218 | 32.389 | 1.00 | 81.96 | | C |
| ATOM | 871 | CG | GLU B 4 | 6.914 | −20.455 | 33.138 | 1.00 | 85.95 | | C |

| ATOM | 872 | CD | GLU | B | 4 | 6.794 | -20.518 | 34.664 | 1.00 | 97.96 | C |
|---|---|---|---|---|---|---|---|---|---|---|---|
| ATOM | 873 | OE1 | GLU | B | 4 | 5.676 | -20.806 | 35.157 | 1.00 | 90.44 | O |
| ATOM | 874 | OE2 | GLU | B | 4 | 7.815 | -20.264 | 35.363 | 1.00 | 74.71 | O |
| ATOM | 875 | C | GLU | B | 4 | 7.334 | -21.178 | 30.424 | 1.00 | 79.85 | C |
| ATOM | 876 | O | GLU | B | 4 | 7.807 | -22.304 | 30.274 | 1.00 | 86.78 | O |
| ATOM | 877 | N | ILE | B | 5 | 8.020 | -20.072 | 30.176 | 1.00 | 59.31 | N |
| ATOM | 878 | CA | ILE | B | 5 | 9.414 | -20.119 | 29.768 | 1.00 | 60.43 | C |
| ATOM | 879 | CB | ILE | B | 5 | 9.588 | -19.668 | 28.303 | 1.00 | 59.16 | C |
| ATOM | 880 | CG1 | ILE | B | 5 | 9.065 | -20.730 | 27.335 | 1.00 | 53.06 | C |
| ATOM | 881 | CD1 | ILE | B | 5 | 9.234 | -20.316 | 25.872 | 1.00 | 41.17 | C |
| ATOM | 882 | CG2 | ILE | B | 5 | 11.043 | -19.349 | 27.988 | 1.00 | 55.00 | C |
| ATOM | 883 | C | ILE | B | 5 | 10.230 | -19.216 | 30.691 | 1.00 | 57.22 | C |
| ATOM | 884 | O | ILE | B | 5 | 9.790 | -18.123 | 31.051 | 1.00 | 43.81 | O |
| ATOM | 885 | N | ASN | B | 6 | 11.420 | -19.680 | 31.066 | 1.00 | 69.05 | N |
| ATOM | 886 | CA | ASN | B | 6 | 12.307 | -18.951 | 31.976 | 1.00 | 66.62 | C |
| ATOM | 887 | CB | ASN | B | 6 | 12.015 | -19.335 | 33.432 | 1.00 | 63.10 | C |
| ATOM | 888 | CG | ASN | B | 6 | 11.629 | -20.793 | 33.583 | 1.00 | 66.21 | C |
| ATOM | 889 | OD1 | ASN | B | 6 | 12.439 | -21.687 | 33.348 | 1.00 | 73.63 | O |
| ATOM | 890 | ND2 | ASN | B | 6 | 10.389 | -21.039 | 33.989 | 1.00 | 72.15 | N |
| ATOM | 891 | C | ASN | B | 6 | 13.773 | -19.199 | 31.645 | 1.00 | 65.85 | C |
| ATOM | 892 | O | ASN | B | 6 | 14.100 | -20.171 | 30.962 | 1.00 | 64.29 | O |
| ATOM | 893 | N | ASP | B | 7 | 14.645 | -18.322 | 32.136 | 1.00 | 86.42 | N |
| ATOM | 894 | CA | ASP | B | 7 | 16.083 | -18.444 | 31.902 | 1.00 | 98.58 | C |
| ATOM | 895 | CB | ASP | B | 7 | 16.890 | -17.596 | 32.900 | 1.00 | 87.77 | C |
| ATOM | 896 | CG | ASP | B | 7 | 16.026 | -16.648 | 33.719 | 1.00 | 91.48 | C |
| ATOM | 897 | OD1 | ASP | B | 7 | 15.779 | -15.512 | 33.245 | 1.00 | 86.27 | O |
| ATOM | 898 | OD2 | ASP | B | 7 | 15.617 | -17.035 | 34.845 | 1.00 | 86.15 | O |
| ATOM | 899 | C | ASP | B | 7 | 16.564 | -19.900 | 31.961 | 1.00 | 102.54 | C |
| ATOM | 900 | O | ASP | B | 7 | 17.521 | -20.282 | 31.279 | 1.00 | 95.68 | O |
| ATOM | 901 | N | GLU | B | 8 | 15.887 | -20.703 | 32.775 | 1.00 | 93.37 | N |
| ATOM | 902 | CA | GLU | B | 8 | 16.221 | -22.109 | 32.938 | 1.00 | 90.14 | C |
| ATOM | 903 | CB | GLU | B | 8 | 15.522 | -22.661 | 34.173 | 1.00 | 98.10 | C |
| ATOM | 904 | CG | GLU | B | 8 | 16.004 | -22.044 | 35.468 | 1.00 | 112.15 | C |
| ATOM | 905 | CD | GLU | B | 8 | 15.239 | -22.557 | 36.667 | 1.00 | 124.79 | C |
| ATOM | 906 | OE1 | GLU | B | 8 | 14.001 | -22.707 | 36.557 | 1.00 | 114.62 | O |
| ATOM | 907 | OE2 | GLU | B | 8 | 15.879 | -22.815 | 37.712 | 1.00 | 137.10 | O |
| ATOM | 908 | C | GLU | B | 8 | 15.860 | -22.959 | 31.730 | 1.00 | 92.49 | C |
| ATOM | 909 | O | GLU | B | 8 | 16.725 | -23.590 | 31.133 | 1.00 | 96.60 | O |
| ATOM | 910 | N | ASN | B | 9 | 14.581 | -22.978 | 31.372 | 1.00 | 91.20 | N |
| ATOM | 911 | CA | ASN | B | 9 | 14.114 | -23.859 | 30.303 | 1.00 | 93.39 | C |
| ATOM | 912 | CB | ASN | B | 9 | 12.707 | -24.396 | 30.609 | 1.00 | 89.12 | C |
| ATOM | 913 | CG | ASN | B | 9 | 11.639 | -23.308 | 30.575 | 1.00 | 94.54 | C |
| ATOM | 914 | OD1 | ASN | B | 9 | 11.928 | -22.130 | 30.805 | 1.00 | 91.22 | O |
| ATOM | 915 | ND2 | ASN | B | 9 | 10.397 | -23.701 | 30.286 | 1.00 | 88.21 | N |
| ATOM | 916 | C | ASN | B | 9 | 14.146 | -23.222 | 28.917 | 1.00 | 95.07 | C |
| ATOM | 917 | O | ASN | B | 9 | 13.634 | -23.792 | 27.957 | 1.00 | 99.72 | O |
| ATOM | 918 | N | PHE | B | 10 | 14.765 | -22.051 | 28.805 | 1.00 | 81.25 | N |
| ATOM | 919 | CA | PHE | B | 10 | 14.684 | -21.284 | 27.560 | 1.00 | 78.84 | C |
| ATOM | 920 | CB | PHE | B | 10 | 15.344 | -19.918 | 27.685 | 1.00 | 71.60 | C |
| ATOM | 921 | CG | PHE | B | 10 | 15.207 | -19.089 | 26.452 | 1.00 | 68.18 | C |
| ATOM | 922 | CD1 | PHE | B | 10 | 13.967 | -18.577 | 26.093 | 1.00 | 76.57 | C |
| ATOM | 923 | CE1 | PHE | B | 10 | 13.808 | -17.809 | 24.944 | 1.00 | 67.39 | C |
| ATOM | 924 | CZ | PHE | B | 10 | 14.898 | -17.548 | 24.129 | 1.00 | 66.06 | C |
| ATOM | 925 | CE2 | PHE | B | 10 | 16.152 | -18.058 | 24.472 | 1.00 | 81.01 | C |
| ATOM | 926 | CD2 | PHE | B | 10 | 16.299 | -18.830 | 25.637 | 1.00 | 79.21 | C |
| ATOM | 927 | C | PHE | B | 10 | 15.265 | -21.989 | 26.352 | 1.00 | 86.33 | C |
| ATOM | 928 | O | PHE | B | 10 | 14.567 | -22.192 | 25.364 | 1.00 | 81.53 | O |
| ATOM | 929 | N | ASP | B | 11 | 16.548 | -22.336 | 26.436 | 1.00 | 100.81 | N |
| ATOM | 930 | CA | ASP | B | 11 | 17.284 | -22.965 | 25.337 | 1.00 | 99.39 | C |
| ATOM | 931 | CB | ASP | B | 11 | 18.611 | -23.503 | 25.848 | 1.00 | 101.34 | C |
| ATOM | 932 | CG | ASP | B | 11 | 18.432 | -24.567 | 26.912 | 1.00 | 110.25 | C |
| ATOM | 933 | OD2 | ASP | B | 11 | 19.357 | -25.396 | 27.070 | 1.00 | 111.74 | O |
| ATOM | 934 | OD1 | ASP | B | 11 | 17.372 | -24.574 | 27.588 | 1.00 | 100.69 | O |

```
ATOM    935  C    ASP B  11      16.533 -24.074  24.588  1.00106.68           C
ATOM    936  O    ASP B  11      17.053 -24.623  23.616  1.00108.36           O
ATOM    937  N    GLU B  12      15.328 -24.407  25.052  1.00 96.91           N
ATOM    938  CA   GLU B  12      14.396 -25.249  24.301  1.00 99.82           C
ATOM    939  CB   GLU B  12      13.186 -25.596  25.157  1.00 94.57           C
ATOM    940  CG   GLU B  12      12.264 -24.406  25.382  1.00 94.67           C
ATOM    941  CD   GLU B  12      10.992 -24.774  26.123  1.00 91.32           C
ATOM    942  OE1  GLU B  12      10.058 -25.299  25.476  1.00 90.27           O
ATOM    943  OE2  GLU B  12      10.924 -24.524  27.349  1.00 85.22           O
ATOM    944  C    GLU B  12      13.909 -24.532  23.035  1.00 99.22           C
ATOM    945  O    GLU B  12      12.918 -24.922  22.423  1.00 88.70           O
ATOM    946  N    VAL B  13      14.598 -23.460  22.667  1.00108.98           N
ATOM    947  CA   VAL B  13      14.391 -22.831  21.373  1.00112.79           C
ATOM    948  CB   VAL B  13      14.660 -21.308  21.414  1.00 99.59           C
ATOM    949  CG1  VAL B  13      13.493 -20.565  22.055  1.00 81.14           C
ATOM    950  CG2  VAL B  13      15.967 -21.015  22.148  1.00 98.96           C
ATOM    951  C    VAL B  13      15.346 -23.481  20.370  1.00124.68           C
ATOM    952  O    VAL B  13      14.929 -23.972  19.314  1.00119.14           O
ATOM    953  N    ILE B  14      16.627 -23.500  20.731  1.00169.10           N
ATOM    954  CA   ILE B  14      17.688 -23.980  19.851  1.00172.43           C
ATOM    955  CB   ILE B  14      19.076 -23.486  20.335  1.00174.56           C
ATOM    956  CG1  ILE B  14      20.126 -23.628  19.224  1.00174.32           C
ATOM    957  CD1  ILE B  14      20.735 -22.301  18.771  1.00162.43           C
ATOM    958  CG2  ILE B  14      19.476 -24.191  21.631  1.00170.25           C
ATOM    959  C    ILE B  14      17.693 -25.501  19.785  1.00171.95           C
ATOM    960  O    ILE B  14      18.152 -26.097  18.808  1.00172.20           O
ATOM    961  N    LYS B  15      17.176 -26.123  20.836  1.00117.60           N
ATOM    962  CA   LYS B  15      17.145 -27.569  20.913  1.00118.01           C
ATOM    963  CB   LYS B  15      17.407 -28.011  22.346  1.00114.54           C
ATOM    964  CG   LYS B  15      18.072 -29.358  22.442  1.00107.96           C
ATOM    965  CD   LYS B  15      18.619 -29.593  23.833  1.00100.90           C
ATOM    966  CE   LYS B  15      18.017 -30.839  24.449  1.00 91.85           C
ATOM    967  NZ   LYS B  15      18.863 -31.357  25.549  1.00 85.25           N
ATOM    968  C    LYS B  15      15.800 -28.087  20.431  1.00110.32           C
ATOM    969  O    LYS B  15      15.615 -29.289  20.245  1.00111.28           O
ATOM    970  N    LYS B  16      14.863 -27.169  20.230  1.00139.88           N
ATOM    971  CA   LYS B  16      13.550 -27.529  19.723  1.00140.00           C
ATOM    972  CB   LYS B  16      12.636 -26.304  19.654  1.00138.11           C
ATOM    973  CG   LYS B  16      11.186 -26.586  20.016  1.00125.86           C
ATOM    974  CD   LYS B  16      11.081 -27.192  21.405  1.00130.26           C
ATOM    975  CE   LYS B  16       9.635 -27.294  21.865  1.00126.28           C
ATOM    976  NZ   LYS B  16       9.514 -27.922  23.213  1.00119.19           N
ATOM    977  C    LYS B  16      13.700 -28.155  18.346  1.00136.26           C
ATOM    978  O    LYS B  16      14.334 -29.195  18.205  1.00141.73           O
ATOM    979  N    ASP B  17      13.122 -27.515  17.333  1.00109.86           N
ATOM    980  CA   ASP B  17      13.114 -28.057  15.980  1.00105.60           C
ATOM    981  CB   ASP B  17      12.647 -29.517  16.006  1.00109.33           C
ATOM    982  CG   ASP B  17      11.488 -29.756  16.983  1.00114.87           C
ATOM    983  OD1  ASP B  17      11.755 -30.089  18.160  1.00122.12           O
ATOM    984  OD2  ASP B  17      10.310 -29.633  16.573  1.00108.52           O
ATOM    985  C    ASP B  17      12.184 -27.249  15.092  1.00108.34           C
ATOM    986  O    ASP B  17      12.600 -26.585  14.140  1.00106.04           O
ATOM    987  N    LYS B  18      10.906 -27.332  15.427  1.00111.78           N
ATOM    988  CA   LYS B  18       9.853 -26.655  14.696  1.00 99.36           C
ATOM    989  CB   LYS B  18       8.592 -27.510  14.707  1.00 80.93           C
ATOM    990  CG   LYS B  18       7.321 -26.758  14.777  1.00 84.98           C
ATOM    991  CD   LYS B  18       6.200 -27.725  14.499  1.00 95.91           C
ATOM    992  CE   LYS B  18       5.179 -27.154  13.532  1.00 96.56           C
ATOM    993  NZ   LYS B  18       5.667 -26.386  12.308  1.00 83.38           N
ATOM    994  C    LYS B  18       9.623 -25.278  15.314  1.00 95.51           C
ATOM    995  O    LYS B  18      10.154 -24.972  16.389  1.00 93.84           O
ATOM    996  N    VAL B  19       8.857 -24.447  14.616  1.00 81.27           N
ATOM    997  CA   VAL B  19       8.782 -23.024  14.917  1.00 79.25           C
```

```
ATOM    998  CB   VAL B  19       8.163 -22.241  13.743  1.00 80.30           C
ATOM    999  CG1  VAL B  19       6.661 -22.296  13.803  1.00 75.87           C
ATOM   1000  CG2  VAL B  19       8.635 -20.801  13.766  1.00 82.15           C
ATOM   1001  C    VAL B  19       8.077 -22.673  16.232  1.00 80.13           C
ATOM   1002  O    VAL B  19       6.993 -23.192  16.560  1.00 72.36           O
ATOM   1003  N    VAL B  20       8.712 -21.756  16.959  1.00 74.87           N
ATOM   1004  CA   VAL B  20       8.283 -21.384  18.294  1.00 66.27           C
ATOM   1005  CB   VAL B  20       9.371 -21.662  19.314  1.00 66.59           C
ATOM   1006  CG1  VAL B  20       8.972 -21.117  20.666  1.00 64.65           C
ATOM   1007  CG2  VAL B  20       9.625 -23.143  19.398  1.00 75.54           C
ATOM   1008  C    VAL B  20       7.877 -19.920  18.384  1.00 62.49           C
ATOM   1009  O    VAL B  20       8.608 -19.012  17.966  1.00 55.20           O
ATOM   1010  N    VAL B  21       6.695 -19.715  18.953  1.00 53.78           N
ATOM   1011  CA   VAL B  21       6.098 -18.401  19.096  1.00 49.56           C
ATOM   1012  CB   VAL B  21       4.649 -18.468  18.615  1.00 46.98           C
ATOM   1013  CG1  VAL B  21       3.974 -17.100  18.697  1.00 48.40           C
ATOM   1014  CG2  VAL B  21       4.626 -18.988  17.203  1.00 49.53           C
ATOM   1015  C    VAL B  21       6.151 -17.962  20.561  1.00 46.23           C
ATOM   1016  O    VAL B  21       5.605 -18.642  21.426  1.00 48.59           O
ATOM   1017  N    VAL B  22       6.796 -16.831  20.843  1.00 50.79           N
ATOM   1018  CA   VAL B  22       7.003 -16.405  22.234  1.00 55.45           C
ATOM   1019  CB   VAL B  22       8.513 -16.390  22.612  1.00 51.44           C
ATOM   1020  CG1  VAL B  22       8.699 -15.963  24.051  1.00 37.20           C
ATOM   1021  CG2  VAL B  22       9.143 -17.749  22.383  1.00 51.37           C
ATOM   1022  C    VAL B  22       6.395 -15.047  22.605  1.00 49.52           C
ATOM   1023  O    VAL B  22       6.863 -14.001  22.146  1.00 49.62           O
ATOM   1024  N    ASP B  23       5.387 -15.076  23.474  1.00 46.70           N
ATOM   1025  CA   ASP B  23       4.746 -13.867  23.988  1.00 46.13           C
ATOM   1026  CB   ASP B  23       3.273 -14.163  24.328  1.00 50.01           C
ATOM   1027  CG   ASP B  23       2.434 -12.894  24.565  1.00 66.21           C
ATOM   1028  OD1  ASP B  23       2.999 -11.786  24.740  1.00 68.81           O
ATOM   1029  OD2  ASP B  23       1.186 -13.015  24.579  1.00 73.34           O
ATOM   1030  C    ASP B  23       5.456 -13.328  25.232  1.00 47.10           C
ATOM   1031  O    ASP B  23       5.420 -13.933  26.314  1.00 43.31           O
ATOM   1032  N    PHE B  24       6.078 -12.170  25.083  1.00 47.25           N
ATOM   1033  CA   PHE B  24       6.665 -11.475  26.217  1.00 44.68           C
ATOM   1034  CB   PHE B  24       7.865 -10.651  25.748  1.00 49.34           C
ATOM   1035  CG   PHE B  24       9.039 -11.485  25.331  1.00 48.06           C
ATOM   1036  CD2  PHE B  24      10.181 -11.526  26.104  1.00 51.49           C
ATOM   1037  CE2  PHE B  24      11.264 -12.311  25.730  1.00 48.77           C
ATOM   1038  CZ   PHE B  24      11.210 -13.052  24.581  1.00 51.16           C
ATOM   1039  CE1  PHE B  24      10.075 -13.022  23.795  1.00 53.41           C
ATOM   1040  CD1  PHE B  24       8.995 -12.242  24.171  1.00 51.72           C
ATOM   1041  C    PHE B  24       5.643 -10.581  26.914  1.00 46.41           C
ATOM   1042  O    PHE B  24       5.231  -9.551  26.385  1.00 52.44           O
ATOM   1043  N    TRP B  25       5.251 -10.958  28.122  1.00 49.55           N
ATOM   1044  CA   TRP B  25       4.166 -10.248  28.810  1.00 50.21           C
ATOM   1045  CB   TRP B  25       2.917 -11.126  28.853  1.00 38.06           C
ATOM   1046  CG   TRP B  25       3.125 -12.338  29.694  1.00 36.57           C
ATOM   1047  CD1  TRP B  25       3.812 -13.467  29.352  1.00 43.51           C
ATOM   1048  NE1  TRP B  25       3.794 -14.368  30.385  1.00 47.39           N
ATOM   1049  CE2  TRP B  25       3.104 -13.823  31.434  1.00 53.97           C
ATOM   1050  CD2  TRP B  25       2.659 -12.541  31.032  1.00 49.97           C
ATOM   1051  CE3  TRP B  25       1.918 -11.762  31.933  1.00 41.36           C
ATOM   1052  CZ3  TRP B  25       1.644 -12.284  33.178  1.00 41.49           C
ATOM   1053  CH2  TRP B  25       2.102 -13.568  33.551  1.00 33.48           C
ATOM   1054  CZ2  TRP B  25       2.824 -14.345  32.699  1.00 36.37           C
ATOM   1055  C    TRP B  25       4.520  -9.803  30.230  1.00 47.79           C
ATOM   1056  O    TRP B  25       5.602 -10.078  30.722  1.00 42.92           O
ATOM   1057  N    ALA B  26       3.581  -9.119  30.875  1.00 46.23           N
ATOM   1058  CA   ALA B  26       3.747  -8.649  32.243  1.00 46.32           C
ATOM   1059  CB   ALA B  26       4.668  -7.423  32.285  1.00 46.20           C
ATOM   1060  C    ALA B  26       2.402  -8.328  32.878  1.00 49.68           C
```

```
ATOM   1061  O    ALA B  26      1.490   -7.832  32.202  1.00  49.52          O
ATOM   1062  N    GLU B  27      2.284   -8.601  34.176  1.00  55.10          N
ATOM   1063  CA   GLU B  27      1.053   -8.320  34.910  1.00  47.35          C
ATOM   1064  CB   GLU B  27      1.115   -8.847  36.360  1.00  47.60          C
ATOM   1065  CG   GLU B  27      1.030  -10.402  36.512  1.00  50.82          C
ATOM   1066  CD   GLU B  27      2.314  -11.069  37.075  1.00  69.56          C
ATOM   1067  OE1  GLU B  27      3.443  -10.633  36.735  1.00  73.38          O
ATOM   1068  OE2  GLU B  27      2.195  -12.043  37.861  1.00  59.42          O
ATOM   1069  C    GLU B  27      0.650   -6.837  34.837  1.00  48.74          C
ATOM   1070  O    GLU B  27     -0.537   -6.525  34.872  1.00  57.81          O
ATOM   1071  N    TRP B  28      1.613   -5.926  34.696  1.00  42.43          N
ATOM   1072  CA   TRP B  28      1.281   -4.495  34.609  1.00  46.07          C
ATOM   1073  CB   TRP B  28      2.445   -3.603  35.044  1.00  47.45          C
ATOM   1074  CG   TRP B  28      3.780   -4.015  34.526  1.00  53.22          C
ATOM   1075  CD1  TRP B  28      4.767   -4.644  35.225  1.00  58.40          C
ATOM   1076  NE1  TRP B  28      5.862   -4.846  34.422  1.00  57.59          N
ATOM   1077  CE2  TRP B  28      5.594   -4.343  33.180  1.00  54.43          C
ATOM   1078  CD2  TRP B  28      4.296   -3.801  33.208  1.00  52.87          C
ATOM   1079  CE3  TRP B  28      3.781   -3.223  32.047  1.00  58.66          C
ATOM   1080  CZ3  TRP B  28      4.569   -3.205  30.910  1.00  56.83          C
ATOM   1081  CH2  TRP B  28      5.859   -3.740  30.921  1.00  54.72          C
ATOM   1082  CZ2  TRP B  28      6.382   -4.312  32.040  1.00  56.31          C
ATOM   1083  C    TRP B  28      0.801   -4.050  33.230  1.00  44.67          C
ATOM   1084  O    TRP B  28      0.530   -2.878  33.000  1.00  40.25          O
ATOM   1085  N    CYS B  29      0.683   -4.994  32.318  1.00  41.27          N
ATOM   1086  CA   CYS B  29      0.408   -4.660  30.942  1.00  47.77          C
ATOM   1087  CB   CYS B  29      1.359   -5.461  30.050  1.00  49.44          C
ATOM   1088  SG   CYS B  29      0.923   -5.573  28.315  1.00  54.33          S
ATOM   1089  C    CYS B  29     -1.060   -4.929  30.579  1.00  46.98          C
ATOM   1090  O    CYS B  29     -1.463   -6.083  30.396  1.00  47.42          O
ATOM   1091  N    GLY B  30     -1.853   -3.862  30.478  1.00  32.22          N
ATOM   1092  CA   GLY B  30     -3.271   -3.982  30.144  1.00  39.33          C
ATOM   1093  C    GLY B  30     -3.574   -4.867  28.940  1.00  41.54          C
ATOM   1094  O    GLY B  30     -4.165   -5.936  29.115  1.00  39.21          O
ATOM   1095  N    PRO B  31     -3.150   -4.445  27.721  1.00  39.76          N
ATOM   1096  CA   PRO B  31     -3.418   -5.185  26.472  1.00  35.11          C
ATOM   1097  CB   PRO B  31     -2.670   -4.393  25.384  1.00  30.68          C
ATOM   1098  CG   PRO B  31     -1.772   -3.406  26.103  1.00  43.17          C
ATOM   1099  CD   PRO B  31     -2.306   -3.248  27.513  1.00  40.78          C
ATOM   1100  C    PRO B  31     -2.882   -6.598  26.518  1.00  41.34          C
ATOM   1101  O    PRO B  31     -3.345   -7.476  25.782  1.00  44.92          O
ATOM   1102  N    CYS B  32     -1.892   -6.827  27.368  1.00  43.72          N
ATOM   1103  CA   CYS B  32     -1.439   -8.194  27.587  1.00  46.16          C
ATOM   1104  CB   CYS B  32     -0.264   -8.224  28.572  1.00  41.98          C
ATOM   1105  SG   CYS B  32      1.277   -7.564  27.865  1.00  44.15          S
ATOM   1106  C    CYS B  32     -2.579   -9.148  28.004  1.00  47.57          C
ATOM   1107  O    CYS B  32     -2.584  -10.320  27.628  1.00  43.74          O
ATOM   1108  N    ARG B  33     -3.552   -8.631  28.752  1.00  61.53          N
ATOM   1109  CA   ARG B  33     -4.694   -9.434  29.205  1.00  71.74          C
ATOM   1110  CB   ARG B  33     -5.498   -8.694  30.285  1.00  73.19          C
ATOM   1111  CG   ARG B  33     -4.725   -8.433  31.573  1.00  59.71          C
ATOM   1112  CD   ARG B  33     -5.361   -7.276  32.360  1.00  73.59          C
ATOM   1113  NE   ARG B  33     -4.756   -6.965  33.667  1.00  88.65          N
ATOM   1114  CZ   ARG B  33     -3.522   -7.286  34.070  1.00  78.95          C
ATOM   1115  NH1  ARG B  33     -2.674   -7.949  33.280  1.00  74.46          N
ATOM   1116  NH2  ARG B  33     -3.129   -6.924  35.285  1.00  71.42          N
ATOM   1117  C    ARG B  33     -5.627   -9.914  28.071  1.00  68.14          C
ATOM   1118  O    ARG B  33     -6.235  -10.976  28.174  1.00  73.53          O
ATOM   1119  N    MET B  34     -5.726   -9.158  26.982  1.00  59.03          N
ATOM   1120  CA   MET B  34     -6.558   -9.590  25.852  1.00  65.04          C
ATOM   1121  CB   MET B  34     -6.949   -8.396  24.991  1.00  59.79          C
ATOM   1122  CG   MET B  34     -6.418   -7.075  25.521  1.00  63.46          C
ATOM   1123  SD   MET B  34     -6.419   -5.810  24.228  1.00  85.43          S
```

| ATOM | 1124 | CE  | MET | B | 34 | -8.103  | -5.956  | 23.641 | 1.00 | 51.55 | C |
| ATOM | 1125 | C   | MET | B | 34 | -5.866  | -10.628 | 24.986 | 1.00 | 59.71 | C |
| ATOM | 1126 | O   | MET | B | 34 | -6.519  | -11.439 | 24.339 | 1.00 | 59.98 | O |
| ATOM | 1127 | N   | ILE | B | 35 | -4.538  | -10.585 | 24.973 | 1.00 | 49.33 | N |
| ATOM | 1128 | CA  | ILE | B | 35 | -3.744  | -11.481 | 24.134 | 1.00 | 53.69 | C |
| ATOM | 1129 | CB  | ILE | B | 35 | -2.370  | -10.866 | 23.776 | 1.00 | 49.08 | C |
| ATOM | 1130 | CG1 | ILE | B | 35 | -2.531  | -9.542  | 23.043 | 1.00 | 51.33 | C |
| ATOM | 1131 | CD1 | ILE | B | 35 | -1.306  | -8.598  | 23.213 | 1.00 | 51.09 | C |
| ATOM | 1132 | CG2 | ILE | B | 35 | -1.566  | -11.812 | 22.933 | 1.00 | 49.18 | C |
| ATOM | 1133 | C   | ILE | B | 35 | -3.498  | -12.835 | 24.802 | 1.00 | 56.23 | C |
| ATOM | 1134 | O   | ILE | B | 35 | -3.266  | -13.842 | 24.132 | 1.00 | 57.30 | O |
| ATOM | 1135 | N   | ALA | B | 36 | -3.524  | -12.859 | 26.126 | 1.00 | 53.64 | N |
| ATOM | 1136 | CA  | ALA | B | 36 | -3.303  | -14.117 | 26.840 | 1.00 | 55.57 | C |
| ATOM | 1137 | CB  | ALA | B | 36 | -3.463  | -13.963 | 28.361 | 1.00 | 47.65 | C |
| ATOM | 1138 | C   | ALA | B | 36 | -4.226  | -15.221 | 26.321 | 1.00 | 56.96 | C |
| ATOM | 1139 | O   | ALA | B | 36 | -3.747  | -16.286 | 25.916 | 1.00 | 56.63 | O |
| ATOM | 1140 | N   | PRO | B | 37 | -5.554  | -14.975 | 26.345 | 1.00 | 64.12 | N |
| ATOM | 1141 | CA  | PRO | B | 37 | -6.522  | -15.985 | 25.884 | 1.00 | 62.85 | C |
| ATOM | 1142 | C   | PRO | B | 37 | -6.298  | -16.368 | 24.438 | 1.00 | 58.27 | C |
| ATOM | 1143 | O   | PRO | B | 37 | -6.281  | -17.546 | 24.089 | 1.00 | 64.00 | O |
| ATOM | 1144 | CB  | PRO | B | 37 | -7.874  | -15.275 | 26.017 | 1.00 | 56.53 | C |
| ATOM | 1145 | CG  | PRO | B | 37 | -7.541  | -13.803 | 26.125 | 1.00 | 64.70 | C |
| ATOM | 1146 | CD  | PRO | B | 37 | -6.231  | -13.772 | 26.862 | 1.00 | 58.90 | C |
| ATOM | 1147 | N   | ILE | B | 38 | -6.122  | -15.368 | 23.594 | 1.00 | 49.38 | N |
| ATOM | 1148 | CA  | ILE | B | 38 | -5.946  | -15.628 | 22.172 | 1.00 | 50.32 | C |
| ATOM | 1149 | CB  | ILE | B | 38 | -5.780  | -14.307 | 21.404 | 1.00 | 50.26 | C |
| ATOM | 1150 | CG1 | ILE | B | 38 | -7.080  | -13.502 | 21.547 | 1.00 | 48.34 | C |
| ATOM | 1151 | CD1 | ILE | B | 38 | -7.072  | -12.139 | 20.896 | 1.00 | 46.09 | C |
| ATOM | 1152 | CG2 | ILE | B | 38 | -5.445  | -14.558 | 19.960 | 1.00 | 47.52 | C |
| ATOM | 1153 | C   | ILE | B | 38 | -4.817  | -16.614 | 21.888 | 1.00 | 55.58 | C |
| ATOM | 1154 | O   | ILE | B | 38 | -5.057  | -17.657 | 21.290 | 1.00 | 60.38 | O |
| ATOM | 1155 | N   | ILE | B | 39 | -3.597  | -16.297 | 22.327 | 1.00 | 60.95 | N |
| ATOM | 1156 | CA  | ILE | B | 39 | -2.441  | -17.192 | 22.145 | 1.00 | 54.32 | C |
| ATOM | 1157 | CB  | ILE | B | 39 | -1.226  | -16.736 | 22.973 | 1.00 | 60.90 | C |
| ATOM | 1158 | CG1 | ILE | B | 39 | -0.316  | -15.853 | 22.126 | 1.00 | 58.32 | C |
| ATOM | 1159 | CD1 | ILE | B | 39 | -1.041  | -14.921 | 21.228 | 1.00 | 57.66 | C |
| ATOM | 1160 | CG2 | ILE | B | 39 | -0.414  | -17.936 | 23.474 | 1.00 | 55.24 | C |
| ATOM | 1161 | C   | ILE | B | 39 | -2.772  | -18.614 | 22.544 | 1.00 | 55.84 | C |
| ATOM | 1162 | O   | ILE | B | 39 | -2.341  | -19.556 | 21.891 | 1.00 | 56.53 | O |
| ATOM | 1163 | N   | GLU | B | 40 | -3.524  | -18.755 | 23.633 | 1.00 | 67.89 | N |
| ATOM | 1164 | CA  | GLU | B | 40 | -4.045  | -20.047 | 24.069 | 1.00 | 71.72 | C |
| ATOM | 1165 | CB  | GLU | B | 40 | -4.896  | -19.884 | 25.323 | 1.00 | 69.49 | C |
| ATOM | 1166 | CG  | GLU | B | 40 | -4.114  | -19.907 | 26.602 | 1.00 | 76.37 | C |
| ATOM | 1167 | CD  | GLU | B | 40 | -4.923  | -19.363 | 27.744 | 1.00 | 85.01 | C |
| ATOM | 1168 | OE2 | GLU | B | 40 | -4.316  | -19.017 | 28.783 | 1.00 | 97.30 | O |
| ATOM | 1169 | OE1 | GLU | B | 40 | -6.164  | -19.269 | 27.588 | 1.00 | 80.90 | O |
| ATOM | 1170 | C   | GLU | B | 40 | -4.891  | -20.711 | 22.998 | 1.00 | 73.56 | C |
| ATOM | 1171 | O   | GLU | B | 40 | -4.647  | -21.867 | 22.657 | 1.00 | 69.17 | O |
| ATOM | 1172 | N   | GLU | B | 41 | -5.901  | -19.986 | 22.503 | 1.00 | 77.03 | N |
| ATOM | 1173 | CA  | GLU | B | 41 | -6.799  | -20.497 | 21.468 | 1.00 | 74.03 | C |
| ATOM | 1174 | CB  | GLU | B | 41 | -7.797  | -19.418 | 21.003 | 1.00 | 67.29 | C |
| ATOM | 1175 | CG  | GLU | B | 41 | -8.783  | -18.942 | 22.095 | 1.00 | 74.98 | C |
| ATOM | 1176 | CD  | GLU | B | 41 | -9.725  | -17.840 | 21.604 | 1.00 | 77.61 | C |
| ATOM | 1177 | OE1 | GLU | B | 41 | -10.750 | -17.566 | 22.275 | 1.00 | 72.53 | O |
| ATOM | 1178 | OE2 | GLU | B | 41 | -9.430  | -17.239 | 20.544 | 1.00 | 68.23 | O |
| ATOM | 1179 | C   | GLU | B | 41 | -5.963  | -21.019 | 20.306 | 1.00 | 80.18 | C |
| ATOM | 1180 | O   | GLU | B | 41 | -6.243  | -22.091 | 19.762 | 1.00 | 86.25 | O |
| ATOM | 1181 | N   | LEU | B | 42 | -4.916  | -20.273 | 19.954 | 1.00 | 58.10 | N |
| ATOM | 1182 | CA  | LEU | B | 42 | -4.032  | -20.665 | 18.865 | 1.00 | 61.89 | C |
| ATOM | 1183 | CB  | LEU | B | 42 | -3.186  | -19.481 | 18.397 | 1.00 | 65.15 | C |
| ATOM | 1184 | CG  | LEU | B | 42 | -3.908  | -18.392 | 17.605 | 1.00 | 70.91 | C |
| ATOM | 1185 | CD1 | LEU | B | 42 | -2.932  | -17.298 | 17.166 | 1.00 | 67.89 | C |
| ATOM | 1186 | CD2 | LEU | B | 42 | -4.583  | -19.007 | 16.408 | 1.00 | 69.23 | C |

```
ATOM   1187  C    LEU B  42     -3.131 -21.839  19.247  1.00 65.88           C
ATOM   1188  O    LEU B  42     -2.709 -22.608  18.392  1.00 69.34           O
ATOM   1189  N    ALA B  43     -2.830 -21.977  20.531  1.00 92.93           N
ATOM   1190  CA   ALA B  43     -2.002 -23.090  20.977  1.00 96.49           C
ATOM   1191  CB   ALA B  43     -1.586 -22.917  22.437  1.00 87.63           C
ATOM   1192  C    ALA B  43     -2.769 -24.388  20.783  1.00 98.90           C
ATOM   1193  O    ALA B  43     -2.179 -25.451  20.589  1.00 99.34           O
ATOM   1194  N    GLU B  44     -4.093 -24.287  20.830  1.00 94.24           N
ATOM   1195  CA   GLU B  44     -4.959 -25.441  20.640  1.00 94.43           C
ATOM   1196  CB   GLU B  44     -6.255 -25.275  21.441  1.00 91.68           C
ATOM   1197  CG   GLU B  44     -6.018 -25.073  22.942  1.00102.06           C
ATOM   1198  CD   GLU B  44     -7.241 -25.394  23.793  1.00113.53           C
ATOM   1199  OE1  GLU B  44     -8.382 -25.204  23.313  1.00111.24           O
ATOM   1200  OE2  GLU B  44     -7.056 -25.849  24.943  1.00111.95           O
ATOM   1201  C    GLU B  44     -5.234 -25.642  19.155  1.00 95.11           C
ATOM   1202  O    GLU B  44     -5.150 -26.756  18.637  1.00 88.62           O
ATOM   1203  N    GLU B  45     -5.542 -24.549  18.469  1.00 94.20           N
ATOM   1204  CA   GLU B  45     -5.684 -24.583  17.023  1.00 91.51           C
ATOM   1205  CB   GLU B  45     -5.832 -23.165  16.472  1.00 88.51           C
ATOM   1206  CG   GLU B  45     -6.013 -23.099  14.968  1.00 91.15           C
ATOM   1207  CD   GLU B  45     -6.631 -21.788  14.502  1.00 92.61           C
ATOM   1208  OE1  GLU B  45     -7.329 -21.127  15.303  1.00 86.51           O
ATOM   1209  OE2  GLU B  45     -6.420 -21.420  13.326  1.00102.52           O
ATOM   1210  C    GLU B  45     -4.480 -25.294  16.393  1.00 92.91           C
ATOM   1211  O    GLU B  45     -4.578 -26.455  15.995  1.00 94.92           O
ATOM   1212  N    TYR B  46     -3.341 -24.610  16.336  1.00 99.92           N
ATOM   1213  CA   TYR B  46     -2.123 -25.177  15.758  1.00102.82           C
ATOM   1214  CB   TYR B  46     -1.180 -24.062  15.334  1.00104.93           C
ATOM   1215  CG   TYR B  46     -1.800 -23.002  14.476  1.00 97.94           C
ATOM   1216  CD2  TYR B  46     -1.707 -23.070  13.103  1.00102.67           C
ATOM   1217  CE2  TYR B  46     -2.261 -22.102  12.305  1.00109.54           C
ATOM   1218  CZ   TYR B  46     -2.914 -21.034  12.878  1.00104.52           C
ATOM   1219  OH   TYR B  46     -3.465 -20.069  12.065  1.00103.24           O
ATOM   1220  CE1  TYR B  46     -3.013 -20.938  14.252  1.00 98.32           C
ATOM   1221  CD1  TYR B  46     -2.454 -21.921  15.039  1.00 96.54           C
ATOM   1222  C    TYR B  46     -1.333 -26.075  16.700  1.00107.17           C
ATOM   1223  O    TYR B  46     -0.105 -25.973  16.753  1.00103.47           O
ATOM   1224  N    ALA B  47     -2.016 -26.950  17.432  1.00111.29           N
ATOM   1225  CA   ALA B  47     -1.338 -27.823  18.390  1.00113.07           C
ATOM   1226  CB   ALA B  47     -2.307 -28.307  19.459  1.00108.38           C
ATOM   1227  C    ALA B  47     -0.653 -29.011  17.706  1.00110.68           C
ATOM   1228  O    ALA B  47     -1.286 -29.773  16.974  1.00103.96           O
ATOM   1229  N    GLY B  48      0.646 -29.159  17.952  1.00 86.03           N
ATOM   1230  CA   GLY B  48      1.429 -30.205  17.319  1.00 83.47           C
ATOM   1231  C    GLY B  48      2.157 -29.693  16.095  1.00 81.29           C
ATOM   1232  O    GLY B  48      3.154 -30.275  15.670  1.00 77.77           O
ATOM   1233  N    LYS B  49      1.658 -28.597  15.529  1.00 90.65           N
ATOM   1234  CA   LYS B  49      2.282 -27.995  14.354  1.00 94.47           C
ATOM   1235  CB   LYS B  49      1.284 -27.871  13.197  1.00 95.48           C
ATOM   1236  CG   LYS B  49      0.179 -28.885  13.222  1.00 95.02           C
ATOM   1237  CD   LYS B  49     -1.025 -28.370  12.483  1.00 95.05           C
ATOM   1238  CE   LYS B  49     -2.260 -28.636  13.311  1.00 99.74           C
ATOM   1239  NZ   LYS B  49     -1.981 -28.402  14.764  1.00 94.62           N
ATOM   1240  C    LYS B  49      2.871 -26.608  14.611  1.00 88.60           C
ATOM   1241  O    LYS B  49      3.048 -25.841  13.673  1.00 91.68           O
ATOM   1242  N    VAL B  50      3.180 -26.282  15.858  1.00 77.93           N
ATOM   1243  CA   VAL B  50      3.825 -25.013  16.198  1.00 77.88           C
ATOM   1244  CB   VAL B  50      3.130 -23.762  15.600  1.00 82.64           C
ATOM   1245  CG1  VAL B  50      3.147 -22.611  16.607  1.00 72.07           C
ATOM   1246  CG2  VAL B  50      3.797 -23.327  14.302  1.00 77.07           C
ATOM   1247  C    VAL B  50      3.723 -24.895  17.681  1.00 72.44           C
ATOM   1248  O    VAL B  50      2.640 -25.095  18.240  1.00 68.36           O
ATOM   1249  N    VAL B  51      4.848 -24.560  18.308  1.00 69.10           N
```

```
ATOM   1250   CA   VAL   B   51      4.933  -24.515   19.762   1.00   71.66        C
ATOM   1251   CB   VAL   B   51      6.173  -25.296   20.270   1.00   74.70        C
ATOM   1252   CG1  VAL   B   51      7.223  -25.401   19.167   1.00   72.04        C
ATOM   1253   CG2  VAL   B   51      6.750  -24.662   21.525   1.00   66.82        C
ATOM   1254   C    VAL   B   51      4.899  -23.081   20.293   1.00   68.23        C
ATOM   1255   O    VAL   B   51      5.461  -22.158   19.682   1.00   62.46        O
ATOM   1256   N    PHE   B   52      4.222  -22.912   21.427   1.00   84.28        N
ATOM   1257   CA   PHE   B   52      3.961  -21.597   21.998   1.00   80.51        C
ATOM   1258   CB   PHE   B   52      2.461  -21.344   22.068   1.00   74.73        C
ATOM   1259   CG   PHE   B   52      1.790  -21.304   20.750   1.00   75.32        C
ATOM   1260   CD2  PHE   B   52      1.763  -20.133   20.017   1.00   80.85        C
ATOM   1261   CE2  PHE   B   52      1.126  -20.077   18.785   1.00   83.29        C
ATOM   1262   CZ   PHE   B   52      0.502  -21.209   18.281   1.00   86.94        C
ATOM   1263   CE1  PHE   B   52      0.522  -22.385   19.013   1.00   91.21        C
ATOM   1264   CD1  PHE   B   52      1.164  -22.428   20.243   1.00   85.14        C
ATOM   1265   C    PHE   B   52      4.470  -21.462   23.416   1.00   75.60        C
ATOM   1266   O    PHE   B   52      4.026  -22.182   24.312   1.00   77.16        O
ATOM   1267   N    GLY   B   53      5.363  -20.507   23.634   1.00   71.47        N
ATOM   1268   CA   GLY   B   53      5.771  -20.169   24.986   1.00   70.87        C
ATOM   1269   C    GLY   B   53      5.553  -18.705   25.325   1.00   67.68        C
ATOM   1270   O    GLY   B   53      5.697  -17.835   24.464   1.00   71.11        O
ATOM   1271   N    LYS   B   54      5.187  -18.434   26.575   1.00   57.51        N
ATOM   1272   CA   LYS   B   54      5.186  -17.068   27.103   1.00   50.75        C
ATOM   1273   CB   LYS   B   54      3.859  -16.751   27.790   1.00   47.07        C
ATOM   1274   CG   LYS   B   54      3.507  -17.711   28.918   1.00   57.82        C
ATOM   1275   CD   LYS   B   54      2.023  -17.635   29.279   1.00   62.01        C
ATOM   1276   CE   LYS   B   54      1.623  -18.735   30.251   1.00   68.17        C
ATOM   1277   NZ   LYS   B   54      0.148  -18.757   30.487   1.00   76.46        N
ATOM   1278   C    LYS   B   54      6.313  -16.904   28.103   1.00   43.13        C
ATOM   1279   O    LYS   B   54      6.665  -17.846   28.820   1.00   44.19        O
ATOM   1280   N    VAL   B   55      6.871  -15.703   28.161   1.00   35.64        N
ATOM   1281   CA   VAL   B   55      7.829  -15.380   29.214   1.00   33.56        C
ATOM   1282   CB   VAL   B   55      9.323  -15.269   28.710   1.00   35.61        C
ATOM   1283   CG1  VAL   B   55      9.461  -15.659   27.268   1.00   38.04        C
ATOM   1284   CG2  VAL   B   55      9.911  -13.879   28.938   1.00   30.88        C
ATOM   1285   C    VAL   B   55      7.412  -14.131   29.980   1.00   35.37        C
ATOM   1286   O    VAL   B   55      7.279  -13.061   29.399   1.00   39.78        O
ATOM   1287   N    ASN   B   56      7.157  -14.270   31.277   1.00   43.17        N
ATOM   1288   CA   ASN   B   56      6.908  -13.095   32.096   1.00   43.41        C
ATOM   1289   CB   ASN   B   56      6.518  -13.464   33.528   1.00   40.96        C
ATOM   1290   CG   ASN   B   56      5.988  -12.281   34.331   1.00   44.32        C
ATOM   1291   OD1  ASN   B   56      6.453  -11.149   34.200   1.00   46.25        O
ATOM   1292   ND2  ASN   B   56      5.012  -12.552   35.184   1.00   46.57        N
ATOM   1293   C    ASN   B   56      8.198  -12.315   32.071   1.00   39.09        C
ATOM   1294   O    ASN   B   56      9.255  -12.852   32.338   1.00   46.22        O
ATOM   1295   N    VAL   B   57      8.102  -11.050   31.707   1.00   40.11        N
ATOM   1296   CA   VAL   B   57      9.252  -10.197   31.483   1.00   38.23        C
ATOM   1297   CB   VAL   B   57      8.809   -8.957   30.656   1.00   41.82        C
ATOM   1298   CG1  VAL   B   57      8.891   -7.670   31.477   1.00   48.41        C
ATOM   1299   CG2  VAL   B   57      9.587   -8.842   29.386   1.00   37.48        C
ATOM   1300   C    VAL   B   57      9.869   -9.768   32.813   1.00   43.99        C
ATOM   1301   O    VAL   B   57     11.073   -9.584   32.908   1.00   43.97        O
ATOM   1302   N    ASP   B   58      9.032   -9.624   33.836   1.00   39.39        N
ATOM   1303   CA   ASP   B   58      9.470   -9.129   35.136   1.00   44.62        C
ATOM   1304   CB   ASP   B   58      8.272   -8.663   35.962   1.00   44.16        C
ATOM   1305   CG   ASP   B   58      7.940   -7.199   35.728   1.00   55.90        C
ATOM   1306   OD1  ASP   B   58      8.867   -6.431   35.388   1.00   60.79        O
ATOM   1307   OD2  ASP   B   58      6.760   -6.819   35.888   1.00   58.41        O
ATOM   1308   C    ASP   B   58     10.259  -10.174   35.917   1.00   50.25        C
ATOM   1309   O    ASP   B   58     11.089   -9.827   36.753   1.00   41.44        O
ATOM   1310   N    GLU   B   59      9.988  -11.441   35.611   1.00   60.17        N
ATOM   1311   CA   GLU   B   59     10.555  -12.592   36.296   1.00   56.54        C
ATOM   1312   CB   GLU   B   59      9.434  -13.592   36.618   1.00   58.67        C
```

```
ATOM   1313  CG   GLU B  59      8.463 -13.102  37.702  1.00 59.69          C
ATOM   1314  CD   GLU B  59      7.190 -13.951  37.829  1.00 74.62          C
ATOM   1315  OE1  GLU B  59      7.067 -14.972  37.100  1.00 59.26          O
ATOM   1316  OE2  GLU B  59      6.310 -13.581  38.664  1.00 77.74          O
ATOM   1317  C    GLU B  59     11.647 -13.251  35.458  1.00 63.18          C
ATOM   1318  O    GLU B  59     12.380 -14.121  35.928  1.00 67.55          O
ATOM   1319  N    ASN B  60     11.744 -12.826  34.204  1.00 46.77          N
ATOM   1320  CA   ASN B  60     12.788 -13.294  33.309  1.00 44.58          C
ATOM   1321  CB   ASN B  60     12.245 -14.349  32.351  1.00 42.72          C
ATOM   1322  CG   ASN B  60     11.783 -15.592  33.069  1.00 50.38          O
ATOM   1323  OD1  ASN B  60     12.597 -16.333  33.604  1.00 53.96          O
ATOM   1324  ND2  ASN B  60     10.472 -15.832  33.084  1.00 45.59          N
ATOM   1325  C    ASN B  60     13.399 -12.115  32.548  1.00 53.55          C
ATOM   1326  O    ASN B  60     13.521 -12.131  31.307  1.00 53.82          O
ATOM   1327  N    PRO B  61     13.822 -11.098  33.304  1.00 53.19          N
ATOM   1328  CA   PRO B  61     14.252  -9.815  32.752  1.00 54.72          C
ATOM   1329  CB   PRO B  61     14.613  -9.009  34.010  1.00 44.21          C
ATOM   1330  CG   PRO B  61     15.069 -10.042  34.976  1.00 51.64          C
ATOM   1331  CD   PRO B  61     14.228 -11.261  34.713  1.00 57.31          C
ATOM   1332  C    PRO B  61     15.472  -9.976  31.848  1.00 63.18          C
ATOM   1333  O    PRO B  61     15.664  -9.162  30.939  1.00 65.32          O
ATOM   1334  N    GLU B  62     16.275 -11.011  32.091  1.00 58.07          N
ATOM   1335  CA   GLU B  62     17.520 -11.184  31.364  1.00 53.43          C
ATOM   1336  CB   GLU B  62     18.441 -12.206  32.040  1.00 60.80          C
ATOM   1337  CG   GLU B  62     19.451 -11.591  33.014  1.00 64.64          C
ATOM   1338  CD   GLU B  62     19.140 -11.933  34.463  1.00 76.08          C
ATOM   1339  OE1  GLU B  62     18.060 -12.522  34.716  1.00 85.98          O
ATOM   1340  OE2  GLU B  62     19.970 -11.625  35.345  1.00 70.48          O
ATOM   1341  C    GLU B  62     17.234 -11.593  29.943  1.00 55.62          C
ATOM   1342  O    GLU B  62     17.930 -11.174  29.022  1.00 56.09          O
ATOM   1343  N    ILE B  63     16.204 -12.409  29.759  1.00 42.81          N
ATOM   1344  CA   ILE B  63     15.777 -12.736  28.402  1.00 43.39          C
ATOM   1345  CB   ILE B  63     14.720 -13.819  28.382  1.00 42.88          C
ATOM   1346  CG1  ILE B  63     15.267 -15.100  29.009  1.00 46.88          C
ATOM   1347  CD1  ILE B  63     14.198 -16.106  29.375  1.00 42.53          C
ATOM   1348  CG2  ILE B  63     14.260 -14.074  26.948  1.00 49.11          C
ATOM   1349  C    ILE B  63     15.259 -11.509  27.638  1.00 42.65          C
ATOM   1350  O    ILE B  63     15.710 -11.234  26.528  1.00 43.67          O
ATOM   1351  N    ALA B  64     14.324 -10.766  28.219  1.00 65.39          N
ATOM   1352  CA   ALA B  64     13.843  -9.552  27.563  1.00 69.64          C
ATOM   1353  CB   ALA B  64     12.933  -8.756  28.503  1.00 60.36          C
ATOM   1354  C    ALA B  64     15.032  -8.701  27.107  1.00 66.52          C
ATOM   1355  O    ALA B  64     15.119  -8.296  25.936  1.00 61.59          O
ATOM   1356  N    ALA B  65     15.949  -8.475  28.048  1.00 67.05          N
ATOM   1357  CA   ALA B  65     17.155  -7.667  27.848  1.00 68.07          C
ATOM   1358  CB   ALA B  65     17.865  -7.438  29.164  1.00 57.43          C
ATOM   1359  C    ALA B  65     18.129  -8.252  26.834  1.00 73.63          C
ATOM   1360  O    ALA B  65     18.877  -7.512  26.195  1.00 76.90          O
ATOM   1361  N    LYS B  66     18.132  -9.575  26.697  1.00 67.41          N
ATOM   1362  CA   LYS B  66     19.007 -10.231  25.729  1.00 63.61          C
ATOM   1363  CB   LYS B  66     19.112 -11.731  26.007  1.00 62.32          C
ATOM   1364  CG   LYS B  66     19.873 -12.483  24.941  1.00 66.41          C
ATOM   1365  CD   LYS B  66     19.660 -13.985  25.044  1.00 68.88          C
ATOM   1366  CE   LYS B  66     20.333 -14.712  23.878  1.00 74.39          C
ATOM   1367  NZ   LYS B  66     21.815 -14.444  23.763  1.00 69.21          N
ATOM   1368  C    LYS B  66     18.528 -10.014  24.298  1.00 62.69          C
ATOM   1369  O    LYS B  66     19.335  -9.996  23.372  1.00 66.63          O
ATOM   1370  N    TYR B  67     17.218  -9.842  24.121  1.00 57.10          N
ATOM   1371  CA   TYR B  67     16.618  -9.795  22.783  1.00 43.01          C
ATOM   1372  CB   TYR B  67     15.536 -10.869  22.661  1.00 46.50          C
ATOM   1373  CG   TYR B  67     16.097 -12.259  22.482  1.00 54.72          C
ATOM   1374  CD1  TYR B  67     16.707 -12.620  21.288  1.00 60.24          C
ATOM   1375  CE1  TYR B  67     17.244 -13.883  21.104  1.00 59.07          C
```

| ATOM | 1376 | CZ | TYR | B | 67 | 17.162 | -14.809 | 22.119 | 1.00 | 59.32 | C |
| ATOM | 1377 | OH | TYR | B | 67 | 17.693 | -16.055 | 21.902 | 1.00 | 58.97 | O |
| ATOM | 1378 | CE2 | TYR | B | 67 | 16.546 | -14.485 | 23.317 | 1.00 | 54.78 | C |
| ATOM | 1379 | CD2 | TYR | B | 67 | 16.021 | -13.210 | 23.494 | 1.00 | 46.66 | C |
| ATOM | 1380 | C | TYR | B | 67 | 16.077 | -8.425 | 22.366 | 1.00 | 41.54 | C |
| ATOM | 1381 | O | TYR | B | 67 | 15.415 | -8.305 | 21.339 | 1.00 | 51.20 | O |
| ATOM | 1382 | N | GLY | B | 68 | 16.348 | -7.391 | 23.158 | 1.00 | 49.99 | N |
| ATOM | 1383 | CA | GLY | B | 68 | 15.949 | -6.042 | 22.772 | 1.00 | 50.67 | C |
| ATOM | 1384 | C | GLY | B | 68 | 14.503 | -5.683 | 23.084 | 1.00 | 46.34 | C |
| ATOM | 1385 | O | GLY | B | 68 | 14.074 | -4.548 | 22.886 | 1.00 | 51.01 | O |
| ATOM | 1386 | N | ILE | B | 69 | 13.770 | -6.666 | 23.594 | 1.00 | 39.72 | N |
| ATOM | 1387 | CA | ILE | B | 69 | 12.362 | -6.545 | 23.930 | 1.00 | 44.17 | C |
| ATOM | 1388 | CB | ILE | B | 69 | 11.764 | -7.927 | 24.174 | 1.00 | 39.48 | C |
| ATOM | 1389 | CG1 | ILE | B | 69 | 12.041 | -8.809 | 22.960 | 1.00 | 33.21 | C |
| ATOM | 1390 | CD1 | ILE | B | 69 | 11.710 | -10.224 | 23.186 | 1.00 | 42.31 | C |
| ATOM | 1391 | CG2 | ILE | B | 69 | 10.267 | -7.814 | 24.485 | 1.00 | 36.65 | C |
| ATOM | 1392 | C | ILE | B | 69 | 12.103 | -5.670 | 25.152 | 1.00 | 47.36 | C |
| ATOM | 1393 | O | ILE | B | 69 | 11.912 | -6.166 | 26.272 | 1.00 | 48.29 | O |
| ATOM | 1394 | N | MET | B | 70 | 12.082 | -4.364 | 24.917 | 1.00 | 36.20 | N |
| ATOM | 1395 | CA | MET | B | 70 | 11.853 | -3.391 | 25.965 | 1.00 | 37.27 | C |
| ATOM | 1396 | CB | MET | B | 70 | 12.869 | -2.263 | 25.829 | 1.00 | 40.10 | C |
| ATOM | 1397 | CG | MET | B | 70 | 14.223 | -2.611 | 26.409 | 1.00 | 45.93 | C |
| ATOM | 1398 | SD | MET | B | 70 | 14.030 | -3.287 | 28.078 | 1.00 | 50.06 | S |
| ATOM | 1399 | CE | MET | B | 70 | 14.320 | -5.052 | 27.827 | 1.00 | 43.96 | C |
| ATOM | 1400 | C | MET | B | 70 | 10.445 | -2.818 | 25.891 | 1.00 | 47.22 | C |
| ATOM | 1401 | O | MET | B | 70 | 10.148 | -1.800 | 26.496 | 1.00 | 45.24 | O |
| ATOM | 1402 | N | SER | B | 71 | 9.580 | -3.486 | 25.145 | 1.00 | 35.71 | N |
| ATOM | 1403 | CA | SER | B | 71 | 8.271 | -2.959 | 24.869 | 1.00 | 38.89 | C |
| ATOM | 1404 | CB | SER | B | 71 | 8.330 | -2.079 | 23.614 | 1.00 | 50.71 | C |
| ATOM | 1405 | OG | SER | B | 71 | 7.230 | -2.312 | 22.747 | 1.00 | 40.16 | O |
| ATOM | 1406 | C | SER | B | 71 | 7.307 | -4.118 | 24.694 | 1.00 | 37.69 | C |
| ATOM | 1407 | O | SER | B | 71 | 7.499 | -4.977 | 23.843 | 1.00 | 33.26 | O |
| ATOM | 1408 | N | ILE | B | 72 | 6.281 | -4.161 | 25.530 | 1.00 | 39.92 | N |
| ATOM | 1409 | CA | ILE | B | 72 | 5.363 | -5.284 | 25.484 | 1.00 | 42.55 | C |
| ATOM | 1410 | CB | ILE | B | 72 | 5.526 | -6.245 | 26.700 | 1.00 | 43.71 | C |
| ATOM | 1411 | CG1 | ILE | B | 72 | 5.012 | -5.585 | 27.971 | 1.00 | 47.17 | C |
| ATOM | 1412 | CD1 | ILE | B | 72 | 4.395 | -6.576 | 28.971 | 1.00 | 48.94 | C |
| ATOM | 1413 | CG2 | ILE | B | 72 | 6.985 | -6.712 | 26.853 | 1.00 | 35.49 | C |
| ATOM | 1414 | C | ILE | B | 72 | 3.919 | -4.825 | 25.369 | 1.00 | 39.41 | C |
| ATOM | 1415 | O | ILE | B | 72 | 3.569 | -3.721 | 25.792 | 1.00 | 31.99 | O |
| ATOM | 1416 | N | PRO | B | 73 | 3.073 | -5.677 | 24.783 | 1.00 | 41.55 | N |
| ATOM | 1417 | CA | PRO | B | 73 | 3.464 | -7.010 | 24.299 | 1.00 | 46.92 | C |
| ATOM | 1418 | CB | PRO | B | 73 | 2.130 | -7.628 | 23.886 | 1.00 | 39.32 | C |
| ATOM | 1419 | CG | PRO | B | 73 | 1.323 | -6.446 | 23.478 | 1.00 | 43.11 | C |
| ATOM | 1420 | CD | PRO | B | 73 | 1.669 | -5.379 | 24.482 | 1.00 | 32.04 | C |
| ATOM | 1421 | C | PRO | B | 73 | 4.405 | -7.025 | 23.089 | 1.00 | 47.78 | C |
| ATOM | 1422 | O | PRO | B | 73 | 4.523 | -6.055 | 22.333 | 1.00 | 43.58 | O |
| ATOM | 1423 | N | THR | B | 74 | 5.087 | -8.150 | 22.927 | 1.00 | 47.82 | N |
| ATOM | 1424 | CA | THR | B | 74 | 5.666 | -8.476 | 21.654 | 1.00 | 48.72 | C |
| ATOM | 1425 | CB | THR | B | 74 | 7.144 | -8.121 | 21.564 | 1.00 | 54.30 | C |
| ATOM | 1426 | OG1 | THR | B | 74 | 7.354 | -6.788 | 22.040 | 1.00 | 48.35 | O |
| ATOM | 1427 | CG2 | THR | B | 74 | 7.618 | -8.243 | 20.106 | 1.00 | 53.29 | C |
| ATOM | 1428 | C | THR | B | 74 | 5.521 | -9.963 | 21.460 | 1.00 | 54.24 | C |
| ATOM | 1429 | O | THR | B | 74 | 5.196 | -10.689 | 22.397 | 1.00 | 59.23 | O |
| ATOM | 1430 | N | LEU | B | 75 | 5.734 | -10.397 | 20.224 | 1.00 | 45.57 | N |
| ATOM | 1431 | CA | LEU | B | 75 | 5.930 | -11.803 | 19.917 | 1.00 | 50.43 | C |
| ATOM | 1432 | C | LEU | B | 75 | 7.283 | -11.981 | 19.270 | 1.00 | 44.61 | C |
| ATOM | 1433 | O | LEU | B | 75 | 7.724 | -11.120 | 18.510 | 1.00 | 45.35 | O |
| ATOM | 1434 | CB | LEU | B | 75 | 4.888 | -12.267 | 18.928 | 1.00 | 46.56 | C |
| ATOM | 1435 | CG | LEU | B | 75 | 3.470 | -12.336 | 19.431 | 1.00 | 46.27 | C |
| ATOM | 1436 | CD1 | LEU | B | 75 | 2.586 | -12.744 | 18.240 | 1.00 | 36.57 | C |
| ATOM | 1437 | CD2 | LEU | B | 75 | 3.459 | -13.363 | 20.549 | 1.00 | 42.31 | C |
| ATOM | 1438 | N | LEU | B | 76 | 7.938 | -13.096 | 19.555 | 1.00 | 46.08 | N |

```
ATOM   1439   CA   LEU B 76      9.137 -13.451  18.811  1.00  45.22          C
ATOM   1440   CB   LEU B 76     10.377 -13.493  19.700  1.00  43.70          C
ATOM   1441   CG   LEU B 76     10.947 -12.105  19.986  1.00  50.29          C
ATOM   1442   CD1  LEU B 76     12.311 -12.227  20.664  1.00  51.08          C
ATOM   1443   CD2  LEU B 76     11.026 -11.285  18.700  1.00  38.02          C
ATOM   1444   C    LEU B 76      8.935 -14.785  18.156  1.00  45.16          C
ATOM   1445   O    LEU B 76      8.422 -15.723  18.760  1.00  51.28          O
ATOM   1446   N    PHE B 77      9.349 -14.873  16.908  1.00  51.28          N
ATOM   1447   CA   PHE B 77      9.280 -16.137  16.214  1.00  58.35          C
ATOM   1448   CB   PHE B 77      8.692 -15.964  14.813  1.00  63.75          C
ATOM   1449   CG   PHE B 77      7.233 -15.617  14.819  1.00  57.56          C
ATOM   1450   CD2  PHE B 77      6.792 -14.433  15.396  1.00  58.07          C
ATOM   1451   CE2  PHE B 77      5.441 -14.108  15.424  1.00  60.94          C
ATOM   1452   CZ   PHE B 77      4.514 -14.973  14.867  1.00  63.33          C
ATOM   1453   CE1  PHE B 77      4.945 -16.158  14.293  1.00  72.32          C
ATOM   1454   CD1  PHE B 77      6.302 -16.479  14.281  1.00  66.36          C
ATOM   1455   C    PHE B 77     10.664 -16.711  16.167  1.00  53.90          C
ATOM   1456   O    PHE B 77     11.620 -16.027  15.815  1.00  53.13          O
ATOM   1457   N    PHE B 78     10.752 -17.968  16.571  1.00  61.37          N
ATOM   1458   CA   PHE B 78     11.994 -18.715  16.550  1.00  64.37          C
ATOM   1459   CB   PHE B 78     12.331 -19.201  17.957  1.00  58.40          C
ATOM   1460   CG   PHE B 78     12.809 -18.108  18.870  1.00  64.52          C
ATOM   1461   CD1  PHE B 78     14.169 -17.846  19.011  1.00  68.90          C
ATOM   1462   CE1  PHE B 78     14.628 -16.824  19.846  1.00  43.26          C
ATOM   1463   CZ   PHE B 78     13.725 -16.053  20.550  1.00  45.14          C
ATOM   1464   CE2  PHE B 78     12.356 -16.296  20.414  1.00  50.61          C
ATOM   1465   CD2  PHE B 78     11.906 -17.321  19.574  1.00  52.92          C
ATOM   1466   C    PHE B 78     11.843 -19.897  15.599  1.00  74.30          C
ATOM   1467   O    PHE B 78     10.903 -20.690  15.729  1.00  72.40          O
ATOM   1468   N    LYS B 79     12.759 -19.981  14.631  1.00  79.99          N
ATOM   1469   CA   LYS B 79     12.861 -21.108  13.705  1.00  81.26          C
ATOM   1470   CB   LYS B 79     12.570 -20.658  12.264  1.00  77.17          C
ATOM   1471   CG   LYS B 79     12.658 -21.748  11.197  1.00  82.29          C
ATOM   1472   CD   LYS B 79     11.640 -22.858  11.430  1.00  85.56          C
ATOM   1473   CE   LYS B 79     11.744 -23.951  10.380  1.00  73.31          C
ATOM   1474   NZ   LYS B 79     11.107 -25.184  10.896  1.00  70.08          N
ATOM   1475   C    LYS B 79     14.274 -21.667  13.814  1.00  87.80          C
ATOM   1476   O    LYS B 79     15.226 -21.041  13.348  1.00  89.09          O
ATOM   1477   N    ASN B 80     14.403 -22.822  14.464  1.00  92.98          N
ATOM   1478   CA   ASN B 80     15.688 -23.519  14.617  1.00  92.12          C
ATOM   1479   CB   ASN B 80     16.262 -23.918  13.254  1.00  91.38          C
ATOM   1480   CG   ASN B 80     15.234 -24.597  12.367  1.00  89.52          C
ATOM   1481   OD1  ASN B 80     14.184 -25.043  12.838  1.00  91.37          O
ATOM   1482   ND2  ASN B 80     15.529 -24.673  11.072  1.00  92.24          N
ATOM   1483   C    ASN B 80     16.750 -22.781  15.426  1.00  89.02          C
ATOM   1484   O    ASN B 80     17.902 -22.701  15.014  1.00  84.72          O
ATOM   1485   N    GLY B 81     16.357 -22.253  16.579  1.00  82.82          N
ATOM   1486   CA   GLY B 81     17.293 -21.642  17.505  1.00  85.38          C
ATOM   1487   C    GLY B 81     17.628 -20.189  17.201  1.00  78.57          C
ATOM   1488   O    GLY B 81     18.407 -19.565  17.921  1.00  70.97          O
ATOM   1489   N   ALYS B 82     17.026 -19.650  16.144  1.00  96.32          N
ATOM   1490   CA  ALYS B 82     17.306 -18.286  15.724  1.00  96.56          C
ATOM   1491   CB  ALYS B 82     18.232 -18.294  14.505  1.00 100.32          C
ATOM   1492   CG  ALYS B 82     19.627 -18.860  14.794  1.00 101.24          C
ATOM   1493   CD  ALYS B 82     20.232 -19.551  13.572  1.00 107.58          C
ATOM   1494   CE  ALYS B 82     19.888 -21.037  13.538  1.00 107.81          C
ATOM   1495   NZ  ALYS B 82     20.635 -21.806  14.591  1.00 108.32          N
ATOM   1496   C   ALYS B 82     16.026 -17.510  15.434  1.00  89.39          C
ATOM   1497   O   ALYS B 82     15.122 -18.010  14.778  1.00  88.12          O
ATOM   1498   N   BLYS B 82     17.046 -19.647  16.138  0.00  96.38          N
ATOM   1499   CA  BLYS B 82     17.317 -18.269  15.756  0.00  96.47          C
ATOM   1500   CB  BLYS B 82     18.274 -18.216  14.562  0.00 100.17          C
ATOM   1501   CG  BLYS B 82     19.566 -18.985  14.772  0.00 101.64          C
```

| ATOM | 1502 | CD | BLYS | B | 82 | 20.476 | -18.274 | 15.762 | 0.00 | 102.96 | C |
| ATOM | 1503 | CE | BLYS | B | 82 | 21.117 | -17.046 | 15.130 | 0.00 | 105.77 | C |
| ATOM | 1504 | NZ | BLYS | B | 82 | 22.212 | -16.489 | 15.975 | 0.00 | 112.72 | N |
| ATOM | 1505 | C | BLYS | B | 82 | 16.030 | -17.512 | 15.450 | 0.00 | 89.32 | C |
| ATOM | 1506 | O | BLYS | B | 82 | 15.128 | -18.022 | 14.796 | 0.00 | 88.08 | O |
| ATOM | 1507 | N | VAL | B | 83 | 15.962 | -16.281 | 15.940 | 1.00 | 70.50 | N |
| ATOM | 1508 | CA | VAL | B | 83 | 14.815 | -15.406 | 15.716 | 1.00 | 68.94 | C |
| ATOM | 1509 | CB | VAL | B | 83 | 14.990 | -14.073 | 16.466 | 1.00 | 65.36 | C |
| ATOM | 1510 | CG1 | VAL | B | 83 | 14.127 | -12.994 | 15.850 | 1.00 | 63.19 | C |
| ATOM | 1511 | CG2 | VAL | B | 83 | 14.644 | -14.262 | 17.926 | 1.00 | 66.41 | C |
| ATOM | 1512 | C | VAL | B | 83 | 14.555 | -15.090 | 14.240 | 1.00 | 56.18 | C |
| ATOM | 1513 | O | VAL | B | 83 | 15.390 | -14.471 | 13.568 | 1.00 | 79.05 | O |
| ATOM | 1514 | N | VAL | B | 84 | 13.381 | -15.487 | 13.753 | 1.00 | 86.32 | N |
| ATOM | 1515 | CA | VAL | B | 84 | 13.001 | -15.233 | 12.364 | 1.00 | 85.25 | C |
| ATOM | 1516 | CB | VAL | B | 84 | 12.349 | -16.475 | 11.709 | 1.00 | 89.52 | C |
| ATOM | 1517 | CG1 | VAL | B | 84 | 13.157 | -17.703 | 12.043 | 1.00 | 79.83 | C |
| ATOM | 1518 | CG2 | VAL | B | 84 | 10.910 | -16.647 | 12.167 | 1.00 | 86.11 | C |
| ATOM | 1519 | C | VAL | B | 84 | 12.080 | -14.017 | 12.190 | 1.00 | 85.53 | C |
| ATOM | 1520 | O | VAL | B | 84 | 12.040 | -13.410 | 11.119 | 1.00 | 78.29 | O |
| ATOM | 1521 | N | ASP | B | 85 | 11.351 | -13.655 | 13.240 | 1.00 | 78.64 | N |
| ATOM | 1522 | CA | ASP | B | 85 | 10.361 | -12.587 | 13.124 | 1.00 | 66.82 | C |
| ATOM | 1523 | CB | ASP | B | 85 | 9.117 | -13.117 | 12.383 | 1.00 | 74.43 | C |
| ATOM | 1524 | CG | ASP | B | 85 | 8.340 | -12.017 | 11.679 | 1.00 | 83.29 | C |
| ATOM | 1525 | OD1 | ASP | B | 85 | 8.954 | -11.001 | 11.273 | 1.00 | 82.00 | O |
| ATOM | 1526 | OD2 | ASP | B | 85 | 7.108 | -12.172 | 11.518 | 1.00 | 70.23 | O |
| ATOM | 1527 | C | ASP | B | 85 | 9.986 | -11.986 | 14.492 | 1.00 | 65.42 | C |
| ATOM | 1528 | O | ASP | B | 85 | 9.995 | -12.691 | 15.499 | 1.00 | 57.50 | O |
| ATOM | 1529 | N | GLN | B | 86 | 9.642 | -10.696 | 14.504 | 1.00 | 56.17 | N |
| ATOM | 1530 | CA | GLN | B | 86 | 9.254 | -9.969 | 15.723 | 1.00 | 45.97 | C |
| ATOM | 1531 | CB | GLN | B | 86 | 10.466 | -9.149 | 16.249 | 1.00 | 48.87 | C |
| ATOM | 1532 | CG | GLN | B | 86 | 10.167 | -7.937 | 17.144 | 1.00 | 68.16 | C |
| ATOM | 1533 | CD | GLN | B | 86 | 11.376 | -7.540 | 18.002 | 1.00 | 76.49 | C |
| ATOM | 1534 | OE1 | GLN | B | 86 | 12.137 | -8.402 | 18.441 | 1.00 | 64.10 | O |
| ATOM | 1535 | NE2 | GLN | B | 86 | 11.556 | -6.239 | 18.240 | 1.00 | 51.41 | N |
| ATOM | 1536 | C | GLN | B | 86 | 7.992 | -9.092 | 15.467 | 1.00 | 42.63 | C |
| ATOM | 1537 | O | GLN | B | 86 | 7.943 | -8.326 | 14.497 | 1.00 | 51.41 | O |
| ATOM | 1538 | N | LEU | B | 87 | 6.959 | -9.226 | 16.306 | 1.00 | 48.41 | N |
| ATOM | 1539 | CA | LEU | B | 87 | 5.706 | -8.461 | 16.110 | 1.00 | 41.88 | C |
| ATOM | 1540 | CB | LEU | B | 87 | 4.523 | -9.363 | 15.714 | 1.00 | 49.21 | C |
| ATOM | 1541 | CG | LEU | B | 87 | 4.505 | -10.087 | 14.366 | 1.00 | 55.08 | C |
| ATOM | 1542 | CD1 | LEU | B | 87 | 5.682 | -11.018 | 14.257 | 1.00 | 47.17 | C |
| ATOM | 1543 | CD2 | LEU | B | 87 | 3.184 | -10.883 | 14.172 | 1.00 | 46.76 | C |
| ATOM | 1544 | C | LEU | B | 87 | 5.345 | -7.659 | 17.355 | 1.00 | 46.15 | C |
| ATOM | 1545 | O | LEU | B | 87 | 4.829 | -8.205 | 18.338 | 1.00 | 51.16 | O |
| ATOM | 1546 | N | VAL | B | 88 | 5.630 | -6.362 | 17.296 | 1.00 | 49.53 | N |
| ATOM | 1547 | CA | VAL | B | 88 | 5.438 | -5.443 | 18.416 | 1.00 | 44.56 | C |
| ATOM | 1548 | CB | VAL | B | 88 | 6.321 | -4.184 | 18.232 | 1.00 | 44.38 | C |
| ATOM | 1549 | CG1 | VAL | B | 88 | 6.031 | -3.144 | 19.314 | 1.00 | 43.01 | C |
| ATOM | 1550 | CG2 | VAL | B | 88 | 7.774 | -4.576 | 18.232 | 1.00 | 41.29 | C |
| ATOM | 1551 | C | VAL | B | 88 | 3.981 | -5.007 | 18.557 | 1.00 | 54.63 | C |
| ATOM | 1552 | O | VAL | B | 88 | 3.337 | -4.642 | 17.577 | 1.00 | 35.82 | O |
| ATOM | 1553 | N | GLY | B | 89 | 3.467 | -5.043 | 19.779 | 1.00 | 36.29 | N |
| ATOM | 1554 | CA | GLY | B | 89 | 2.135 | -4.538 | 20.068 | 1.00 | 39.63 | C |
| ATOM | 1555 | C | GLY | B | 89 | 1.015 | -5.494 | 19.681 | 1.00 | 36.00 | C |
| ATOM | 1556 | O | GLY | B | 89 | 1.171 | -6.319 | 18.767 | 1.00 | 35.32 | O |
| ATOM | 1557 | N | ALA | B | 90 | -0.120 | -5.392 | 20.380 | 1.00 | 41.64 | N |
| ATOM | 1558 | CA | ALA | B | 90 | -1.260 | -6.303 | 20.148 | 1.00 | 34.39 | C |
| ATOM | 1559 | CB | ALA | B | 90 | -2.455 | -5.918 | 21.030 | 1.00 | 34.93 | C |
| ATOM | 1560 | C | ALA | B | 90 | -1.678 | -6.358 | 18.678 | 1.00 | 26.67 | C |
| ATOM | 1561 | O | ALA | B | 90 | -1.876 | -5.318 | 18.054 | 1.00 | 49.75 | O |
| ATOM | 1562 | N | ARG | B | 91 | -1.785 | -7.570 | 18.137 | 1.00 | 55.13 | N |
| ATOM | 1563 | CA | ARG | B | 91 | -2.217 | -7.799 | 16.749 | 1.00 | 55.02 | C |
| ATOM | 1564 | CB | ARG | B | 91 | -1.115 | -8.505 | 15.943 | 1.00 | 55.02 | C |

```
ATOM   1565   CG    ARG B  91      0.259   -7.871   16.020   1.00  51.47           C
ATOM   1566   CD    ARG B  91      0.237   -6.436   15.522   1.00  57.35           C
ATOM   1567   NE    ARG B  91      1.565   -5.832   15.564   1.00  56.80           N
ATOM   1568   CZ    ARG B  91      2.509   -6.021   14.646   1.00  59.60           C
ATOM   1569   NH1   ARG B  91      2.261   -6.792   13.597   1.00  61.33           N
ATOM   1570   NH2   ARG B  91      3.701   -5.436   14.774   1.00  47.41           N
ATOM   1571   C     ARG B  91     -3.461   -8.688   16.726   1.00  53.06           C
ATOM   1572   O     ARG B  91     -3.599   -9.590   17.550   1.00  61.54           O
ATOM   1573   N     PRO B  92     -4.366   -8.461   15.767   1.00  55.10           N
ATOM   1574   CA    PRO B  92     -5.576   -9.306   15.674   1.00  57.39           C
ATOM   1575   CB    PRO B  92     -6.317   -8.735   14.465   1.00  55.36           C
ATOM   1576   CG    PRO B  92     -5.243   -7.993   13.669   1.00  52.14           C
ATOM   1577   CD    PRO B  92     -4.338   -7.417   14.729   1.00  52.97           C
ATOM   1578   C     PRO B  92     -5.273  -10.779   15.438   1.00  51.84           C
ATOM   1579   O     PRO B  92     -4.349  -11.107   14.718   1.00  56.79           O
ATOM   1580   N     LYS B  93     -6.057  -11.661   16.035   1.00  61.95           N
ATOM   1581   CA    LYS B  93     -5.827  -13.094   15.890   1.00  66.27           C
ATOM   1582   CB    LYS B  93     -6.964  -13.877   16.547   1.00  69.50           C
ATOM   1583   CG    LYS B  93     -6.745  -15.383   16.631   1.00  69.49           C
ATOM   1584   CD    LYS B  93     -7.968  -16.096   17.229   1.00  73.18           C
ATOM   1585   CE    LYS B  93     -7.735  -17.608   17.337   1.00  79.39           C
ATOM   1586   NZ    LYS B  93     -8.948  -18.393   17.739   1.00  71.25           N
ATOM   1587   C     LYS B  93     -5.647  -13.546   14.435   1.00  67.82           C
ATOM   1588   O     LYS B  93     -4.878  -14.459   14.160   1.00  72.13           O
ATOM   1589   N     GLU B  94     -6.348  -12.916   13.501   1.00  60.92           N
ATOM   1590   CA    GLU B  94     -6.267  -13.338   12.098   1.00  61.01           C
ATOM   1591   CB    GLU B  94     -7.475  -12.841   11.281   1.00  63.11           C
ATOM   1592   CG    GLU B  94     -8.238  -11.668   11.911   1.00  64.32           C
ATOM   1593   CD    GLU B  94     -9.408  -12.111   12.758   1.00  64.58           C
ATOM   1594   OE1   GLU B  94    -10.372  -12.632   12.168   1.00  58.04           O
ATOM   1595   OE2   GLU B  94     -9.358  -11.951   14.003   1.00  70.15           O
ATOM   1596   C     GLU B  94     -4.951  -12.941   11.416   1.00  56.16           C
ATOM   1597   O     GLU B  94     -4.429  -13.680   10.567   1.00  50.41           O
ATOM   1598   N     ALA B  95     -4.416  -11.779   11.781   1.00  52.48           N
ATOM   1599   CA    ALA B  95     -3.101  -11.362   11.275   1.00  52.05           C
ATOM   1600   CB    ALA B  95     -2.791   -9.928   11.684   1.00  45.22           C
ATOM   1601   C     ALA B  95     -1.959  -12.308   11.676   1.00  53.20           C
ATOM   1602   O     ALA B  95     -1.036  -12.540   10.889   1.00  55.60           O
ATOM   1603   N     LEU B  96     -2.005  -12.850   12.891   1.00  49.28           N
ATOM   1604   CA    LEU B  96     -1.002  -13.850   13.251   1.00  62.57           C
ATOM   1605   CB    LEU B  96     -0.451  -13.711   14.690   1.00  63.49           C
ATOM   1606   CG    LEU B  96     -1.244  -13.315   15.936   1.00  60.05           C
ATOM   1607   CD1   LEU B  96     -1.449  -11.799   16.044   1.00  55.99           C
ATOM   1608   CD2   LEU B  96     -2.559  -14.075   16.000   1.00  66.28           C
ATOM   1609   C     LEU B  96     -1.362  -15.300   12.887   1.00  61.46           C
ATOM   1610   O     LEU B  96     -0.651  -16.235   13.234   1.00  64.83           O
ATOM   1611   N     LYS B  97     -2.451  -15.489   12.164   1.00  69.90           N
ATOM   1612   CA    LYS B  97     -2.617  -16.744   11.436   1.00  73.60           C
ATOM   1613   CB    LYS B  97     -4.095  -17.100   11.251   1.00  77.04           C
ATOM   1614   CG    LYS B  97     -4.855  -17.368   12.544   1.00  73.67           C
ATOM   1615   CD    LYS B  97     -6.207  -18.029   12.241   1.00  82.49           C
ATOM   1616   CE    LYS B  97     -7.336  -17.457   13.108   1.00  87.82           C
ATOM   1617   NZ    LYS B  97     -7.424  -15.958   13.027   1.00  74.66           N
ATOM   1618   C     LYS B  97     -1.872  -16.682   10.076   1.00  67.25           C
ATOM   1619   O     LYS B  97     -1.305  -17.680    9.635   1.00  71.22           O
ATOM   1620   N     GLU B  98     -1.853  -15.510    9.432   1.00  57.34           N
ATOM   1621   CA    GLU B  98     -1.103  -15.317    8.182   1.00  59.76           C
ATOM   1622   CB    GLU B  98     -1.276  -13.900    7.627   1.00  53.73           C
ATOM   1623   CG    GLU B  98     -2.497  -13.687    6.759   1.00  72.23           C
ATOM   1624   CD    GLU B  98     -2.493  -14.478    5.440   1.00  71.43           C
ATOM   1625   OE1   GLU B  98     -1.579  -14.273    4.587   1.00  54.72           O
ATOM   1626   OE2   GLU B  98     -3.439  -15.282    5.255   1.00  67.60           O
ATOM   1627   C     GLU B  98      0.380  -15.588    8.365   1.00  70.75           C
```

EP 2 593 472 B1

```
ATOM   1628  O    GLU B  98      0.967 -16.361    7.612  1.00 72.12           O
ATOM   1629  N    ARG B  99      0.998 -14.925    9.339  1.00 62.62           N
ATOM   1630  CA   ARG B  99      2.368 -15.264    9.675  1.00 59.67           C
ATOM   1631  CB   ARG B  99      2.970 -14.245   10.631  1.00 58.28           C
ATOM   1632  CG   ARG B  99      3.313 -12.925    9.977  1.00 59.63           C
ATOM   1633  CD   ARG B  99      2.693 -11.770   10.732  1.00 60.38           C
ATOM   1634  NE   ARG B  99      2.944 -10.481   10.095  1.00 60.28           N
ATOM   1635  CZ   ARG B  99      4.158 -10.011    9.826  1.00 65.01           C
ATOM   1636  NH1  ARG B  99      5.232 -10.732   10.129  1.00 60.06           N
ATOM   1637  NH2  ARG B  99      4.297  -8.822    9.250  1.00 77.41           N
ATOM   1638  C    ARG B  99      2.270 -16.624   10.322  1.00 63.68           C
ATOM   1639  O    ARG B  99      1.264 -16.924   10.947  1.00 61.27           O
ATOM   1640  N    ILE B 100      3.312 -17.430   10.163  1.00 75.54           N
ATOM   1641  CA   ILE B 100      3.314 -18.854   10.526  1.00 84.64           C
ATOM   1642  CB   ILE B 100      2.458 -19.234   11.786  1.00 86.09           C
ATOM   1643  CG1  ILE B 100      0.982 -19.437   11.437  1.00 82.58           C
ATOM   1644  CD1  ILE B 100      0.107 -19.504   12.665  1.00 82.56           C
ATOM   1645  CG2  ILE B 100      2.668 -18.254   12.948  1.00 77.23           C
ATOM   1646  C    ILE B 100      2.930 -19.736    9.340  1.00 89.48           C
ATOM   1647  O    ILE B 100      3.314 -20.899    9.286  1.00 93.34           O
ATOM   1648  N    LYS B 101      2.175 -19.190    8.392  1.00 76.24           N
ATOM   1649  CA   LYS B 101      2.011 -19.864    7.108  1.00 71.99           C
ATOM   1650  CB   LYS B 101      0.917 -19.204    6.277  1.00 75.08           C
ATOM   1651  CG   LYS B 101     -0.443 -19.751    6.599  1.00 76.84           C
ATOM   1652  CD   LYS B 101     -0.503 -20.028    8.091  1.00 81.30           C
ATOM   1653  CE   LYS B 101     -1.750 -20.793    8.483  1.00 87.45           C
ATOM   1654  NZ   LYS B 101     -1.690 -21.131    9.929  1.00 80.07           N
ATOM   1655  C    LYS B 101      3.335 -19.807    6.373  1.00 67.99           C
ATOM   1656  O    LYS B 101      3.847 -20.826    5.901  1.00 65.13           O
ATOM   1657  N    LYS B 102      3.884 -18.600    6.289  1.00 70.37           N
ATOM   1658  CA   LYS B 102      5.244 -18.413    5.819  1.00 75.89           C
ATOM   1659  CB   LYS B 102      5.739 -17.007    6.167  1.00 73.74           C
ATOM   1660  CG   LYS B 102      7.236 -16.782    5.935  1.00 81.11           C
ATOM   1661  CD   LYS B 102      7.551 -16.428    4.482  1.00 79.05           C
ATOM   1662  C    LYS B 102      6.143 -19.451    6.479  1.00 80.78           C
ATOM   1663  O    LYS B 102      7.019 -20.026    5.828  1.00 80.87           O
ATOM   1664  N    TYR B 103      5.890 -19.705    7.765  1.00 84.47           N
ATOM   1665  CA   TYR B 103      6.774 -20.519    8.607  1.00 87.95           C
ATOM   1666  CB   TYR B 103      7.049 -19.803    9.944  1.00 84.27           C
ATOM   1667  CG   TYR B 103      7.702 -18.456    9.750  1.00 84.36           C
ATOM   1668  CD2  TYR B 103      9.056 -18.361    9.471  1.00 86.66           C
ATOM   1669  CE2  TYR B 103      9.661 -17.135    9.261  1.00 93.19           C
ATOM   1670  CZ   TYR B 103      8.907 -15.982    9.326  1.00 88.00           C
ATOM   1671  OH   TYR B 103      9.517 -14.765    9.123  1.00 87.49           O
ATOM   1672  CE1  TYR B 103      7.556 -16.048    9.596  1.00 78.13           C
ATOM   1673  CD1  TYR B 103      6.960 -17.284    9.801  1.00 83.04           C
ATOM   1674  C    TYR B 103      6.282 -21.946    8.857  1.00 87.16           C
ATOM   1675  O    TYR B 103      6.984 -22.756    9.458  1.00 88.52           O
ATOM   1676  N    LEU B 104      5.078 -22.262    8.403  1.00 87.34           N
ATOM   1677  CA   LEU B 104      4.589 -23.622    8.560  1.00 93.65           C
ATOM   1678  CB   LEU B 104      3.057 -23.683    8.499  1.00 93.72           C
ATOM   1679  CG   LEU B 104      2.354 -24.161    9.780  1.00 93.51           C
ATOM   1680  CD1  LEU B 104      2.397 -23.100   10.870  1.00 90.69           C
ATOM   1681  CD2  LEU B 104      0.912 -24.574    9.513  1.00 88.83           C
ATOM   1682  C    LEU B 104      5.222 -24.504    7.487  1.00100.89           C
ATOM   1683  O    LEU B 104      5.289 -25.729    7.616  1.00 98.15           O
ATOM   1684  OXT  LEU B 104      5.696 -24.000    6.464  1.00 98.63           O
TER
ATOM   1685  N    SER C   1     21.966   8.837   -1.633  1.00 64.26           N
ATOM   1686  CA   SER C   1     21.381   7.576   -1.190  1.00 81.80           C
ATOM   1687  CB   SER C   1     21.451   6.533   -2.312  1.00 80.09           C
ATOM   1688  OG   SER C   1     20.604   5.427   -2.053  1.00 85.01           O
ATOM   1689  C    SER C   1     22.071   7.057    0.085  1.00 90.63           C
```

66

| ATOM | 1690 | O | SER C | 1 | 21.822 | 7.561 | 1.185 | 1.00 85.12 | O |
|------|------|-----|-------|---|--------|-------|-------|------------|---|
| ATOM | 1691 | N | VAL C | 2 | 22.937 | 6.057 | -0.063 | 1.00 74.46 | N |
| ATOM | 1692 | CA | VAL C | 2 | 23.580 | 5.430 | 1.090 | 1.00 65.71 | C |
| ATOM | 1693 | CB | VAL C | 2 | 23.432 | 3.898 | 1.073 | 1.00 67.40 | C |
| ATOM | 1694 | CG1 | VAL C | 2 | 24.017 | 3.311 | 2.338 | 1.00 67.52 | C |
| ATOM | 1695 | CG2 | VAL C | 2 | 21.980 | 3.511 | 0.956 | 1.00 75.97 | C |
| ATOM | 1696 | C | VAL C | 2 | 25.059 | 5.802 | 1.217 | 1.00 70.44 | C |
| ATOM | 1697 | O | VAL C | 2 | 25.943 | 5.130 | 0.672 | 1.00 68.67 | O |
| ATOM | 1698 | N | ILE C | 3 | 25.314 | 6.883 | 1.942 | 1.00 73.24 | N |
| ATOM | 1699 | CA | ILE C | 3 | 26.670 | 7.308 | 2.242 | 1.00 73.46 | C |
| ATOM | 1700 | CB | ILE C | 3 | 26.652 | 8.357 | 3.351 | 1.00 82.71 | C |
| ATOM | 1701 | CG1 | ILE C | 3 | 25.635 | 9.455 | 3.015 | 1.00 79.03 | C |
| ATOM | 1702 | CD1 | ILE C | 3 | 25.171 | 10.266 | 4.227 | 1.00 79.41 | C |
| ATOM | 1703 | CG2 | ILE C | 3 | 28.061 | 8.899 | 3.599 | 1.00 75.14 | C |
| ATOM | 1704 | C | ILE C | 3 | 27.548 | 6.131 | 2.694 | 1.00 71.31 | C |
| ATOM | 1705 | O | ILE C | 3 | 27.119 | 5.266 | 3.454 | 1.00 68.26 | O |
| ATOM | 1706 | N | GLU C | 4 | 28.779 | 6.080 | 2.211 | 1.00 84.63 | N |
| ATOM | 1707 | CA | GLU C | 4 | 29.697 | 5.069 | 2.707 | 1.00 85.17 | C |
| ATOM | 1708 | CB | GLU C | 4 | 30.568 | 4.517 | 1.590 | 1.00 77.58 | C |
| ATOM | 1709 | CG | GLU C | 4 | 31.487 | 3.393 | 2.057 | 1.00 92.86 | C |
| ATOM | 1710 | CD | GLU C | 4 | 30.829 | 2.020 | 2.021 | 1.00 99.67 | C |
| ATOM | 1711 | OE1 | GLU C | 4 | 30.276 | 1.657 | 0.958 | 1.00110.67 | O |
| ATOM | 1712 | OE2 | GLU C | 4 | 30.877 | 1.305 | 3.053 | 1.00 85.60 | O |
| ATOM | 1713 | C | GLU C | 4 | 30.554 | 5.717 | 3.787 | 1.00 79.62 | C |
| ATOM | 1714 | O | GLU C | 4 | 31.275 | 6.681 | 3.517 | 1.00 71.18 | O |
| ATOM | 1715 | N | ILE C | 5 | 30.458 | 5.206 | 5.012 | 1.00 60.08 | N |
| ATOM | 1716 | CA | ILE C | 5 | 31.066 | 5.895 | 6.147 | 1.00 65.03 | C |
| ATOM | 1717 | CB | ILE C | 5 | 30.122 | 5.993 | 7.369 | 1.00 58.57 | C |
| ATOM | 1718 | CG1 | ILE C | 5 | 28.756 | 6.525 | 6.964 | 1.00 63.17 | C |
| ATOM | 1719 | CD1 | ILE C | 5 | 27.832 | 6.822 | 8.140 | 1.00 57.48 | C |
| ATOM | 1720 | CG2 | ILE C | 5 | 30.710 | 6.922 | 8.410 | 1.00 66.63 | C |
| ATOM | 1721 | C | ILE C | 5 | 32.391 | 5.273 | 6.588 | 1.00 66.19 | C |
| ATOM | 1722 | O | ILE C | 5 | 32.621 | 4.059 | 6.451 | 1.00 63.25 | O |
| ATOM | 1723 | N | ASN C | 6 | 33.251 | 6.129 | 7.127 | 1.00 58.23 | N |
| ATOM | 1724 | CA | ASN C | 6 | 34.556 | 5.723 | 7.620 | 1.00 60.85 | C |
| ATOM | 1725 | CB | ASN C | 6 | 35.507 | 5.411 | 6.455 | 1.00 65.81 | C |
| ATOM | 1726 | CG | ASN C | 6 | 36.063 | 6.665 | 5.776 | 1.00 67.62 | C |
| ATOM | 1727 | OD1 | ASN C | 6 | 35.831 | 7.795 | 6.218 | 1.00 68.37 | O |
| ATOM | 1728 | ND2 | ASN C | 6 | 36.826 | 6.458 | 4.697 | 1.00 59.28 | N |
| ATOM | 1729 | C | ASN C | 6 | 35.180 | 6.750 | 8.569 | 1.00 64.42 | C |
| ATOM | 1730 | O | ASN C | 6 | 34.545 | 7.757 | 8.923 | 1.00 57.88 | O |
| ATOM | 1731 | N | ASP C | 7 | 36.425 | 6.481 | 8.967 | 1.00 85.22 | N |
| ATOM | 1732 | CA | ASP C | 7 | 37.148 | 7.292 | 9.945 | 1.00 81.70 | C |
| ATOM | 1733 | CB | ASP C | 7 | 38.371 | 6.530 | 10.465 | 1.00 84.40 | C |
| ATOM | 1734 | CG | ASP C | 7 | 37.996 | 5.325 | 11.328 | 1.00 93.67 | C |
| ATOM | 1735 | OD1 | ASP C | 7 | 37.969 | 5.452 | 12.583 | 1.00 87.03 | O |
| ATOM | 1736 | OD2 | ASP C | 7 | 37.733 | 4.247 | 10.744 | 1.00 87.15 | O |
| ATOM | 1737 | C | ASP C | 7 | 37.599 | 8.631 | 9.382 | 1.00 88.94 | C |
| ATOM | 1738 | O | ASP C | 7 | 38.773 | 8.816 | 9.074 | 1.00100.71 | O |
| ATOM | 1739 | N | GLU C | 8 | 36.667 | 9.566 | 9.268 | 1.00 88.93 | N |
| ATOM | 1740 | CA | GLU C | 8 | 36.950 | 10.896 | 8.739 | 1.00 95.99 | C |
| ATOM | 1741 | CB | GLU C | 8 | 37.713 | 10.828 | 7.414 | 1.00 91.81 | C |
| ATOM | 1742 | CG | GLU C | 8 | 37.948 | 12.208 | 6.798 | 1.00 96.51 | C |
| ATOM | 1743 | CD | GLU C | 8 | 38.331 | 12.160 | 5.330 | 1.00113.79 | C |
| ATOM | 1744 | OE1 | GLU C | 8 | 37.847 | 11.253 | 4.614 | 1.00111.15 | O |
| ATOM | 1745 | OE2 | GLU C | 8 | 39.115 | 13.037 | 4.897 | 1.00116.45 | O |
| ATOM | 1746 | C | GLU C | 8 | 35.628 | 11.598 | 8.506 | 1.00 86.39 | C |
| ATOM | 1747 | O | GLU C | 8 | 35.493 | 12.804 | 8.713 | 1.00 85.44 | O |
| ATOM | 1748 | N | ASN C | 9 | 34.647 | 10.829 | 8.061 | 1.00 64.70 | N |
| ATOM | 1749 | CA | ASN C | 9 | 33.329 | 11.385 | 7.842 | 1.00 70.05 | C |
| ATOM | 1750 | CB | ASN C | 9 | 32.788 | 11.019 | 6.447 | 1.00 63.47 | C |
| ATOM | 1751 | CG | ASN C | 9 | 32.600 | 9.517 | 6.248 | 1.00 69.21 | C |
| ATOM | 1752 | OD1 | ASN C | 9 | 33.166 | 8.709 | 6.980 | 1.00 80.45 | O |

| ATOM | 1753 | ND2 | ASN | C | 9  | 31.803 | 9.143  | 5.251  | 1.00 61.36  | N |
|------|------|-----|-----|---|----|--------|--------|--------|-------------|---|
| ATOM | 1754 | C   | ASN | C | 9  | 32.373 | 10.977 | 8.955  | 1.00 78.58  | C |
| ATOM | 1755 | O   | ASN | C | 9  | 31.351 | 11.623 | 9.177  | 1.00 81.76  | O |
| ATOM | 1756 | N   | PHE | C | 10 | 32.719 | 9.916  | 9.676  | 1.00 79.97  | N |
| ATOM | 1757 | CA  | PHE | C | 10 | 31.831 | 9.413  | 10.721 | 1.00 82.16  | C |
| ATOM | 1758 | CB  | PHE | C | 10 | 32.482 | 8.266  | 11.501 | 1.00 76.49  | C |
| ATOM | 1759 | CG  | PHE | C | 10 | 31.534 | 7.556  | 12.432 | 1.00 59.53  | C |
| ATOM | 1760 | CD2 | PHE | C | 10 | 31.114 | 6.266  | 12.156 | 1.00 64.35  | C |
| ATOM | 1761 | CE2 | PHE | C | 10 | 30.232 | 5.608  | 13.000 | 1.00 57.56  | C |
| ATOM | 1762 | CZ  | PHE | C | 10 | 29.768 | 6.239  | 14.138 | 1.00 59.92  | C |
| ATOM | 1763 | CE1 | PHE | C | 10 | 30.184 | 7.529  | 14.433 | 1.00 62.62  | C |
| ATOM | 1764 | CD1 | PHE | C | 10 | 31.061 | 8.180  | 13.576 | 1.00 67.80  | C |
| ATOM | 1765 | C   | PHE | C | 10 | 31.400 | 10.517 | 11.691 | 1.00 83.27  | C |
| ATOM | 1766 | O   | PHE | C | 10 | 30.318 | 10.456 | 12.268 | 1.00 75.76  | O |
| ATOM | 1767 | N   | ASP | C | 11 | 32.243 | 11.524 | 11.886 | 1.00 91.76  | N |
| ATOM | 1768 | CA  | ASP | C | 11 | 31.886 | 12.570 | 12.833 | 1.00 98.24  | C |
| ATOM | 1769 | CB  | ASP | C | 11 | 33.019 | 13.579 | 13.003 | 1.00101.70  | C |
| ATOM | 1770 | CG  | ASP | C | 11 | 32.930 | 14.322 | 14.327 | 1.00107.93  | C |
| ATOM | 1771 | OD1 | ASP | C | 11 | 32.228 | 13.814 | 15.236 | 1.00 98.14  | O |
| ATOM | 1772 | OD2 | ASP | C | 11 | 33.558 | 15.400 | 14.460 | 1.00106.54  | O |
| ATOM | 1773 | C   | ASP | C | 11 | 30.579 | 13.263 | 12.441 | 1.00 96.84  | C |
| ATOM | 1774 | O   | ASP | C | 11 | 29.764 | 13.612 | 13.305 | 1.00 89.24  | O |
| ATOM | 1775 | N   | GLU | C | 12 | 30.395 | 13.461 | 11.137 | 1.00 94.36  | N |
| ATOM | 1776 | CA  | GLU | C | 12 | 29.145 | 13.979 | 10.585 | 1.00 95.64  | C |
| ATOM | 1777 | CB  | GLU | C | 12 | 29.106 | 13.718 | 9.084  | 1.00 85.23  | C |
| ATOM | 1778 | CG  | GLU | C | 12 | 27.724 | 13.374 | 8.563  | 1.00 94.75  | C |
| ATOM | 1779 | CD  | GLU | C | 12 | 27.229 | 12.014 | 9.029  | 1.00 94.48  | C |
| ATOM | 1780 | OE1 | GLU | C | 12 | 27.980 | 11.024 | 8.882  | 1.00 87.80  | O |
| ATOM | 1781 | OE2 | GLU | C | 12 | 26.089 | 11.939 | 9.544  | 1.00 96.99  | O |
| ATOM | 1782 | C   | GLU | C | 12 | 27.897 | 13.362 | 11.239 | 1.00 97.26  | C |
| ATOM | 1783 | O   | GLU | C | 12 | 26.827 | 13.974 | 11.250 | 1.00 94.53  | O |
| ATOM | 1784 | N   | VAL | C | 13 | 28.047 | 12.143 | 11.762 | 1.00107.14  | N |
| ATOM | 1785 | CA  | VAL | C | 13 | 26.959 | 11.400 | 12.392 | 1.00 97.21  | C |
| ATOM | 1786 | CB  | VAL | C | 13 | 27.451 | 10.068 | 12.988 | 1.00 87.18  | C |
| ATOM | 1787 | CG1 | VAL | C | 13 | 26.384 | 9.462  | 13.857 | 1.00 88.32  | C |
| ATOM | 1788 | CG2 | VAL | C | 13 | 27.823 | 9.101  | 11.892 | 1.00 87.20  | C |
| ATOM | 1789 | C   | VAL | C | 13 | 26.270 | 12.200 | 13.486 | 1.00100.00  | C |
| ATOM | 1790 | O   | VAL | C | 13 | 25.054 | 12.382 | 13.457 | 1.00107.71  | O |
| ATOM | 1791 | N   | ILE | C | 14 | 27.044 | 12.663 | 14.460 | 1.00132.86  | N |
| ATOM | 1792 | CA  | ILE | C | 14 | 26.496 | 13.520 | 15.499 | 1.00138.62  | C |
| ATOM | 1793 | CB  | ILE | C | 14 | 27.522 | 13.822 | 16.620 | 1.00144.97  | C |
| ATOM | 1794 | CG1 | ILE | C | 14 | 28.830 | 14.337 | 16.016 | 1.00142.59  | C |
| ATOM | 1795 | CD1 | ILE | C | 14 | 29.745 | 15.006 | 17.011 | 1.00140.56  | C |
| ATOM | 1796 | CG2 | ILE | C | 14 | 27.766 | 12.584 | 17.492 | 1.00136.12  | C |
| ATOM | 1797 | C   | ILE | C | 14 | 26.048 | 14.826 | 14.857 | 1.00139.06  | C |
| ATOM | 1798 | O   | ILE | C | 14 | 24.910 | 15.263 | 15.039 | 1.00132.27  | O |
| ATOM | 1799 | N   | LYS | C | 15 | 26.943 | 15.423 | 14.074 | 1.00 97.81  | N |
| ATOM | 1800 | CA  | LYS | C | 15 | 26.711 | 16.745 | 13.494 | 1.00103.23  | C |
| ATOM | 1801 | CB  | LYS | C | 15 | 28.042 | 17.378 | 13.092 | 1.00 97.70  | C |
| ATOM | 1802 | CG  | LYS | C | 15 | 28.726 | 18.091 | 14.228 | 1.00 88.92  | C |
| ATOM | 1803 | CD  | LYS | C | 15 | 27.880 | 19.264 | 14.702 | 1.00 93.45  | C |
| ATOM | 1804 | CE  | LYS | C | 15 | 27.496 | 20.173 | 13.544 | 1.00 90.28  | C |
| ATOM | 1805 | NZ  | LYS | C | 15 | 26.459 | 21.167 | 13.936 | 1.00 77.54  | N |
| ATOM | 1806 | C   | LYS | C | 15 | 25.725 | 16.810 | 12.318 | 1.00100.88  | C |
| ATOM | 1807 | O   | LYS | C | 15 | 26.009 | 17.447 | 11.300 | 1.00 98.28  | O |
| ATOM | 1808 | N   | LYS | C | 16 | 24.570 | 16.167 | 12.474 | 1.00122.08  | N |
| ATOM | 1809 | CA  | LYS | C | 16 | 23.475 | 16.292 | 11.514 | 1.00124.81  | C |
| ATOM | 1810 | CB  | LYS | C | 16 | 23.221 | 14.967 | 10.788 | 1.00120.60  | C |
| ATOM | 1811 | CG  | LYS | C | 16 | 24.247 | 14.684 | 9.693  | 1.00118.99  | C |
| ATOM | 1812 | CD  | LYS | C | 16 | 24.728 | 16.007 | 9.078  | 1.00123.36  | C |
| ATOM | 1813 | CE  | LYS | C | 16 | 25.646 | 15.823 | 7.871  | 1.00114.55  | C |
| ATOM | 1814 | NZ  | LYS | C | 16 | 26.325 | 17.101 | 7.487  | 1.00103.42  | N |
| ATOM | 1815 | C   | LYS | C | 16 | 22.202 | 16.837 | 12.163 | 1.00124.26  | C |

| ATOM | 1816 | O   | LYS | C | 16 | 22.176 | 17.988 | 12.608 | 1.00122.97 | O |
|------|------|-----|-----|---|----|--------|--------|--------|------------|---|
| ATOM | 1817 | N   | ASP | C | 17 | 21.158 | 16.014 | 12.224 | 1.00125.26 | N |
| ATOM | 1818 | CA  | ASP | C | 17 | 19.882 | 16.444 | 12.796 | 1.00126.53 | C |
| ATOM | 1819 | CB  | ASP | C | 17 | 19.398 | 17.726 | 12.120 | 1.00126.59 | C |
| ATOM | 1820 | CG  | ASP | C | 17 | 19.161 | 17.542 | 10.636 | 1.00127.49 | C |
| ATOM | 1821 | OD1 | ASP | C | 17 | 19.875 | 16.727 | 10.016 | 1.00127.15 | O |
| ATOM | 1822 | OD2 | ASP | C | 17 | 18.259 | 18.207 | 10.091 | 1.00129.53 | O |
| ATOM | 1823 | C   | ASP | C | 17 | 18.810 | 15.373 | 12.635 | 1.00126.23 | C |
| ATOM | 1824 | O   | ASP | C | 17 | 18.091 | 15.041 | 13.580 | 1.00118.87 | O |
| ATOM | 1825 | N   | LYS | C | 18 | 18.705 | 14.844 | 11.422 | 1.00127.53 | N |
| ATOM | 1826 | CA  | LYS | C | 18 | 17.726 | 13.818 | 11.115 | 1.00118.25 | C |
| ATOM | 1827 | CB  | LYS | C | 18 | 17.571 | 13.694 | 9.604  | 1.00116.09 | C |
| ATOM | 1828 | CG  | LYS | C | 18 | 16.161 | 13.927 | 9.103  | 1.00111.92 | C |
| ATOM | 1829 | CD  | LYS | C | 18 | 16.214 | 14.379 | 7.670  | 1.00109.70 | C |
| ATOM | 1830 | CE  | LYS | C | 18 | 17.228 | 13.543 | 6.909  | 1.00105.74 | C |
| ATOM | 1831 | NZ  | LYS | C | 18 | 17.451 | 14.052 | 5.526  | 1.00110.32 | N |
| ATOM | 1832 | C   | LYS | C | 18 | 18.152 | 12.479 | 11.698 | 1.00115.56 | C |
| ATOM | 1833 | O   | LYS | C | 18 | 19.312 | 12.284 | 12.069 | 1.00112.51 | O |
| ATOM | 1834 | N   | VAL | C | 19 | 17.208 | 11.554 | 11.778 | 1.00 86.34 | N |
| ATOM | 1835 | CA  | VAL | C | 19 | 17.535 | 10.209 | 12.211 | 1.00 81.95 | C |
| ATOM | 1836 | CB  | VAL | C | 19 | 16.287 | 9.337  | 12.355 | 1.00 78.41 | C |
| ATOM | 1837 | CG1 | VAL | C | 19 | 16.644 | 8.005  | 13.008 | 1.00 67.56 | C |
| ATOM | 1838 | CG2 | VAL | C | 19 | 15.240 | 10.067 | 13.164 | 1.00 86.51 | C |
| ATOM | 1839 | C   | VAL | C | 19 | 18.482 | 9.548  | 11.214 | 1.00 74.85 | C |
| ATOM | 1840 | O   | VAL | C | 19 | 18.073 | 9.121  | 10.132 | 1.00 69.37 | O |
| ATOM | 1841 | N   | VAL | C | 20 | 19.756 | 9.472  | 11.584 | 1.00 77.40 | N |
| ATOM | 1842 | CA  | VAL | C | 20 | 20.704 | 8.687  | 10.811 | 1.00 64.56 | C |
| ATOM | 1843 | CB  | VAL | C | 20 | 22.115 | 9.250  | 10.884 | 1.00 53.98 | C |
| ATOM | 1844 | CG1 | VAL | C | 20 | 22.361 | 9.920  | 12.233 | 1.00 66.30 | C |
| ATOM | 1845 | CG2 | VAL | C | 20 | 23.103 | 8.149  | 10.599 | 1.00 53.87 | C |
| ATOM | 1846 | C   | VAL | C | 20 | 20.717 | 7.233  | 11.255 | 1.00 59.30 | C |
| ATOM | 1847 | O   | VAL | C | 20 | 20.784 | 6.934  | 12.452 | 1.00 62.03 | O |
| ATOM | 1848 | N   | VAL | C | 21 | 20.622 | 6.336  | 10.278 | 1.00 53.04 | N |
| ATOM | 1849 | CA  | VAL | C | 21 | 20.697 | 4.902  | 10.519 | 1.00 48.71 | C |
| ATOM | 1850 | CB  | VAL | C | 21 | 19.419 | 4.158  | 10.045 | 1.00 44.74 | C |
| ATOM | 1851 | CG1 | VAL | C | 21 | 19.078 | 4.518  | 8.620  | 1.00 53.87 | C |
| ATOM | 1852 | CG2 | VAL | C | 21 | 19.592 | 2.658  | 10.168 | 1.00 48.21 | C |
| ATOM | 1853 | C   | VAL | C | 21 | 21.955 | 4.341  | 9.838  | 1.00 55.50 | C |
| ATOM | 1854 | O   | VAL | C | 21 | 22.075 | 4.333  | 8.606  | 1.00 54.24 | O |
| ATOM | 1855 | N   | VAL | C | 22 | 22.902 | 3.897  | 10.661 | 1.00 59.83 | N |
| ATOM | 1856 | CA  | VAL | C | 22 | 24.165 | 3.357  | 10.196 | 1.00 48.17 | C |
| ATOM | 1857 | CB  | VAL | C | 22 | 25.306 | 3.844  | 11.064 | 1.00 48.09 | C |
| ATOM | 1858 | CG1 | VAL | C | 22 | 26.587 | 3.876  | 10.246 | 1.00 51.91 | C |
| ATOM | 1859 | CG2 | VAL | C | 22 | 24.985 | 5.227  | 11.608 | 1.00 55.30 | C |
| ATOM | 1860 | C   | VAL | C | 22 | 24.148 | 1.833  | 10.232 | 1.00 49.83 | C |
| ATOM | 1861 | O   | VAL | C | 22 | 23.703 | 1.218  | 11.202 | 1.00 56.58 | O |
| ATOM | 1862 | N   | ASP | C | 23 | 24.649 | 1.224  | 9.172  | 1.00 50.94 | N |
| ATOM | 1863 | CA  | ASP | C | 23 | 24.622 | -0.222 | 9.038  | 1.00 55.85 | C |
| ATOM | 1864 | CB  | ASP | C | 23 | 23.868 | -0.595 | 7.748  | 1.00 59.63 | C |
| ATOM | 1865 | CG  | ASP | C | 23 | 24.357 | -1.891 | 7.111  | 1.00 70.01 | C |
| ATOM | 1866 | OD1 | ASP | C | 23 | 24.894 | -1.818 | 5.984  | 1.00 78.00 | O |
| ATOM | 1867 | OD2 | ASP | C | 23 | 24.186 | -2.980 | 7.703  | 1.00 62.63 | O |
| ATOM | 1868 | C   | ASP | C | 23 | 26.063 | -0.739 | 9.078  | 1.00 55.02 | C |
| ATOM | 1869 | O   | ASP | C | 23 | 26.978 | -0.058 | 8.619  | 1.00 53.05 | O |
| ATOM | 1870 | N   | PHE | C | 24 | 26.265 | -1.918 | 9.657  | 1.00 49.12 | N |
| ATOM | 1871 | CA  | PHE | C | 24 | 27.611 | -2.449 | 9.860  | 1.00 54.08 | C |
| ATOM | 1872 | CB  | PHE | C | 24 | 27.910 | -2.630 | 11.352 | 1.00 47.86 | C |
| ATOM | 1873 | CG  | PHE | C | 24 | 28.182 | -1.331 | 12.082 | 1.00 49.85 | C |
| ATOM | 1874 | CD2 | PHE | C | 24 | 27.133 | -0.548 | 12.556 | 1.00 43.22 | C |
| ATOM | 1875 | CE2 | PHE | C | 24 | 27.369 | 0.643  | 13.214 | 1.00 45.41 | C |
| ATOM | 1876 | CZ  | PHE | C | 24 | 28.689 | 1.077  | 13.418 | 1.00 48.74 | C |
| ATOM | 1877 | CE1 | PHE | C | 24 | 29.744 | 0.306  | 12.949 | 1.00 37.27 | C |
| ATOM | 1878 | CD1 | PHE | C | 24 | 29.486 | -0.894 | 12.282 | 1.00 42.49 | C |

```
ATOM   1879  C    PHE C  24      27.741   -3.773    9.140  1.00  56.04           C
ATOM   1880  O    PHE C  24      27.046   -4.727    9.473  1.00  54.54           O
ATOM   1881  N    TRP C  25      28.642   -3.837    8.165  1.00  55.66           N
ATOM   1882  CA   TRP C  25      28.610   -4.928    7.198  1.00  55.80           C
ATOM   1883  CB   TRP C  25      27.767   -4.505    6.003  1.00  58.74           C
ATOM   1884  CG   TRP C  25      28.465   -3.451    5.175  1.00  60.49           C
ATOM   1885  CD1  TRP C  25      28.636   -2.129    5.496  1.00  64.26           C
ATOM   1886  NE1  TRP C  25      29.338   -1.488    4.496  1.00  61.51           N
ATOM   1887  CE2  TRP C  25      29.630   -2.387    3.513  1.00  53.51           C
ATOM   1888  CD2  TRP C  25      29.107   -3.640    3.902  1.00  52.48           C
ATOM   1889  CE3  TRP C  25      29.280   -4.742    3.061  1.00  59.06           C
ATOM   1890  CZ3  TRP C  25      29.966   -4.566    1.866  1.00  61.55           C
ATOM   1891  CH2  TRP C  25      30.476   -3.311    1.505  1.00  62.77           C
ATOM   1892  CZ2  TRP C  25      30.320   -2.214    2.312  1.00  59.11           C
ATOM   1893  C    TRP C  25      29.972   -5.310    6.675  1.00  57.07           C
ATOM   1894  O    TRP C  25      30.980   -4.660    6.967  1.00  52.56           O
ATOM   1895  N    ALA C  26      29.969   -6.353    5.855  1.00  55.09           N
ATOM   1896  CA   ALA C  26      31.176   -6.844    5.211  1.00  63.91           C
ATOM   1897  CB   ALA C  26      31.991   -7.686    6.183  1.00  58.13           C
ATOM   1898  C    ALA C  26      30.818   -7.656    3.974  1.00  65.29           C
ATOM   1899  O    ALA C  26      29.841   -8.406    3.982  1.00  59.41           O
ATOM   1900  N    GLU C  27      31.629   -7.508    2.928  1.00  71.48           N
ATOM   1901  CA   GLU C  27      31.403   -8.157    1.644  1.00  61.11           C
ATOM   1902  CB   GLU C  27      32.582   -7.872    0.711  1.00  70.23           C
ATOM   1903  CG   GLU C  27      32.220   -7.821   -0.772  1.00  85.45           C
ATOM   1904  CD   GLU C  27      31.488   -6.541   -1.176  1.00  88.32           C
ATOM   1905  OE1  GLU C  27      31.859   -5.447   -0.697  1.00  83.38           O
ATOM   1906  OE2  GLU C  27      30.540   -6.633   -1.988  1.00  95.63           O
ATOM   1907  C    GLU C  27      31.162   -9.668    1.736  1.00  70.29           C
ATOM   1908  O    GLU C  27      30.540  -10.251    0.849  1.00  85.86           O
ATOM   1909  N    TRP C  28      31.634  -10.308    2.799  1.00  79.45           N
ATOM   1910  CA   TRP C  28      31.536  -11.765    2.891  1.00  82.42           C
ATOM   1911  CB   TRP C  28      32.756  -12.333    3.621  1.00  82.22           C
ATOM   1912  CG   TRP C  28      32.769  -12.008    5.086  1.00  82.11           C
ATOM   1913  CD1  TRP C  28      31.969  -12.547    6.063  1.00  82.39           C
ATOM   1914  NE1  TRP C  28      32.276  -11.993    7.284  1.00  81.81           N
ATOM   1915  CE2  TRP C  28      33.280  -11.096    7.118  1.00  73.76           C
ATOM   1916  CD2  TRP C  28      33.627  -11.068    5.744  1.00  79.24           C
ATOM   1917  CE3  TRP C  28      34.640  -10.224    5.305  1.00  79.15           C
ATOM   1918  CZ3  TRP C  28      35.280   -9.435    6.233  1.00  83.88           C
ATOM   1919  CH2  TRP C  28      34.924   -9.473    7.592  1.00  87.39           C
ATOM   1920  CZ2  TRP C  28      33.928  -10.291    8.047  1.00  70.20           C
ATOM   1921  C    TRP C  28      30.255  -12.260    3.575  1.00  84.57           C
ATOM   1922  O    TRP C  28      29.825  -13.398    3.365  1.00  89.52           O
ATOM   1923  N    CYS C  29      29.664  -11.404    4.405  1.00  66.24           N
ATOM   1924  CA   CYS C  29      28.488  -11.755    5.210  1.00  68.33           C
ATOM   1925  CB   CYS C  29      28.270  -10.673    6.284  1.00  71.12           C
ATOM   1926  SG   CYS C  29      26.883  -10.861    7.466  1.00  64.13           S
ATOM   1927  C    CYS C  29      27.231  -11.922    4.346  1.00  72.44           C
ATOM   1928  O    CYS C  29      26.824  -10.996    3.644  1.00  77.46           O
ATOM   1929  N    GLY C  30      26.619  -13.100    4.407  1.00  85.61           N
ATOM   1930  CA   GLY C  30      25.427  -13.389    3.625  1.00  86.62           C
ATOM   1931  C    GLY C  30      24.290  -12.417    3.880  1.00  87.50           C
ATOM   1932  O    GLY C  30      23.877  -11.685    2.971  1.00  85.78           O
ATOM   1933  N    PRO C  31      23.764  -12.414    5.120  1.00  62.79           N
ATOM   1934  CA   PRO C  31      22.663  -11.525    5.515  1.00  54.32           C
ATOM   1935  CB   PRO C  31      22.524  -11.786    7.013  1.00  43.61           C
ATOM   1936  CG   PRO C  31      22.960  -13.198    7.174  1.00  47.24           C
ATOM   1937  CD   PRO C  31      24.074  -13.401    6.169  1.00  53.87           C
ATOM   1938  C    PRO C  31      23.001  -10.069    5.278  1.00  60.54           C
ATOM   1939  O    PRO C  31      22.108   -9.263    5.037  1.00  66.01           O
ATOM   1940  N    CYS C  32      24.278   -9.730    5.352  1.00  68.34           N
ATOM   1941  CA   CYS C  32      24.673   -8.344    5.186  1.00  74.16           C
```

| ATOM | 1942 | CB | CYS C | 32 | 26.145 | -8.150 | 5.563 | 1.00 | 68.53 | C |
| ATOM | 1943 | SG | CYS C | 32 | 26.568 | -8.861 | 7.178 | 1.00 | 64.47 | S |
| ATOM | 1944 | C | CYS C | 32 | 24.422 | -8.012 | 3.733 | 1.00 | 74.06 | C |
| ATOM | 1945 | O | CYS C | 32 | 24.146 | -6.862 | 3.380 | 1.00 | 75.00 | O |
| ATOM | 1946 | N | ARG C | 33 | 24.495 | -9.046 | 2.897 | 1.00 | 57.32 | N |
| ATOM | 1947 | CA | ARG C | 33 | 24.183 | -8.909 | 1.483 | 1.00 | 68.62 | C |
| ATOM | 1948 | CB | ARG C | 33 | 24.930 | -9.955 | 0.656 | 1.00 | 63.33 | C |
| ATOM | 1949 | CG | ARG C | 33 | 26.180 | -9.427 | -0.031 | 1.00 | 52.87 | C |
| ATOM | 1950 | CD | ARG C | 33 | 27.163 | -10.567 | -0.262 | 1.00 | 46.97 | C |
| ATOM | 1951 | NE | ARG C | 33 | 26.458 | -11.845 | -0.244 | 1.00 | 61.48 | N |
| ATOM | 1952 | CZ | ARG C | 33 | 27.033 | -13.031 | -0.060 | 1.00 | 68.09 | C |
| ATOM | 1953 | NH1 | ARG C | 33 | 28.346 | -13.124 | 0.132 | 1.00 | 72.02 | N |
| ATOM | 1954 | NH2 | ARG C | 33 | 26.290 | -14.133 | -0.069 | 1.00 | 70.32 | N |
| ATOM | 1955 | C | ARG C | 33 | 22.680 | -9.005 | 1.232 | 1.00 | 70.27 | C |
| ATOM | 1956 | O | ARG C | 33 | 22.152 | -8.293 | 0.385 | 1.00 | 61.48 | O |
| ATOM | 1957 | N | MET C | 34 | 21.992 | -9.874 | 1.976 | 1.00 | 104.03 | N |
| ATOM | 1958 | CA | MET C | 34 | 20.550 | -10.060 | 1.783 | 1.00 | 104.88 | C |
| ATOM | 1959 | CB | MET C | 34 | 20.071 | -11.406 | 2.340 | 1.00 | 97.45 | C |
| ATOM | 1960 | CG | MET C | 34 | 19.071 | -12.113 | 1.413 | 1.00 | 115.64 | C |
| ATOM | 1961 | SD | MET C | 34 | 17.985 | -13.340 | 2.195 | 1.00 | 147.26 | S |
| ATOM | 1962 | CE | MET C | 34 | 16.736 | -12.316 | 2.992 | 1.00 | 106.94 | C |
| ATOM | 1963 | C | MET C | 34 | 19.727 | -8.930 | 2.391 | 1.00 | 101.70 | C |
| ATOM | 1964 | O | MET C | 34 | 18.544 | -9.092 | 2.667 | 1.00 | 99.87 | O |
| ATOM | 1965 | N | ILE C | 35 | 20.360 | -7.784 | 2.603 | 1.00 | 74.35 | N |
| ATOM | 1966 | CA | ILE C | 35 | 19.678 | -6.627 | 3.170 | 1.00 | 69.12 | C |
| ATOM | 1967 | CB | ILE C | 35 | 19.783 | -6.582 | 4.701 | 1.00 | 66.74 | C |
| ATOM | 1968 | CG1 | ILE C | 35 | 21.187 | -6.164 | 5.136 | 1.00 | 74.90 | C |
| ATOM | 1969 | CD1 | ILE C | 35 | 21.366 | -6.105 | 6.644 | 1.00 | 55.25 | C |
| ATOM | 1970 | CG2 | ILE C | 35 | 19.431 | -7.925 | 5.324 | 1.00 | 71.04 | C |
| ATOM | 1971 | C | ILE C | 35 | 20.295 | -5.367 | 2.611 | 1.00 | 70.92 | C |
| ATOM | 1972 | O | ILE C | 35 | 19.731 | -4.282 | 2.731 | 1.00 | 71.07 | O |
| ATOM | 1973 | N | ALA C | 36 | 21.471 | -5.512 | 2.011 | 1.00 | 69.67 | N |
| ATOM | 1974 | CA | ALA C | 36 | 22.057 | -4.393 | 1.295 | 1.00 | 79.08 | C |
| ATOM | 1975 | CB | ALA C | 36 | 23.270 | -4.825 | 0.476 | 1.00 | 75.52 | C |
| ATOM | 1976 | C | ALA C | 36 | 20.972 | -3.774 | 0.408 | 1.00 | 81.00 | C |
| ATOM | 1977 | O | ALA C | 36 | 20.647 | -2.594 | 0.564 | 1.00 | 80.69 | O |
| ATOM | 1978 | N | PRO C | 37 | 20.378 | -4.576 | -0.497 | 1.00 | 80.07 | N |
| ATOM | 1979 | CA | PRO C | 37 | 19.283 | -4.083 | -1.344 | 1.00 | 87.49 | C |
| ATOM | 1980 | CB | PRO C | 37 | 18.774 | -5.358 | -2.044 | 1.00 | 84.80 | C |
| ATOM | 1981 | CG | PRO C | 37 | 19.318 | -6.500 | -1.241 | 1.00 | 82.18 | C |
| ATOM | 1982 | CD | PRO C | 37 | 20.643 | -6.003 | -0.746 | 1.00 | 81.04 | C |
| ATOM | 1983 | C | PRO C | 37 | 18.142 | -3.407 | -0.561 | 1.00 | 79.73 | C |
| ATOM | 1984 | O | PRO C | 37 | 17.813 | -2.252 | -0.868 | 1.00 | 69.36 | O |
| ATOM | 1985 | N | ILE C | 38 | 17.553 | -4.109 | 0.412 | 1.00 | 61.47 | N |
| ATOM | 1986 | CA | ILE C | 38 | 16.492 | -3.532 | 1.254 | 1.00 | 61.66 | C |
| ATOM | 1987 | CB | ILE C | 38 | 16.168 | -4.415 | 2.458 | 1.00 | 53.33 | C |
| ATOM | 1988 | CG1 | ILE C | 38 | 15.565 | -5.750 | 2.031 | 1.00 | 34.59 | C |
| ATOM | 1989 | CD1 | ILE C | 38 | 16.601 | -6.761 | 1.640 | 1.00 | 55.36 | C |
| ATOM | 1990 | CG2 | ILE C | 38 | 15.227 | -3.673 | 3.410 | 1.00 | 66.48 | C |
| ATOM | 1991 | C | ILE C | 38 | 16.813 | -2.143 | 1.833 | 1.00 | 69.25 | C |
| ATOM | 1992 | O | ILE C | 38 | 16.019 | -1.205 | 1.723 | 1.00 | 66.90 | O |
| ATOM | 1993 | N | ILE C | 39 | 17.960 | -2.020 | 2.490 | 1.00 | 80.75 | N |
| ATOM | 1994 | CA | ILE C | 39 | 18.367 | -0.725 | 3.010 | 1.00 | 79.60 | C |
| ATOM | 1995 | CB | ILE C | 39 | 19.772 | -0.784 | 3.651 | 1.00 | 85.93 | C |
| ATOM | 1996 | CG1 | ILE C | 39 | 19.838 | -1.919 | 4.674 | 1.00 | 84.47 | C |
| ATOM | 1997 | CD1 | ILE C | 39 | 18.688 | -1.916 | 5.653 | 1.00 | 83.32 | C |
| ATOM | 1998 | CG2 | ILE C | 39 | 20.146 | 0.557 | 4.293 | 1.00 | 70.33 | C |
| ATOM | 1999 | C | ILE C | 39 | 18.348 | 0.287 | 1.870 | 1.00 | 75.91 | C |
| ATOM | 2000 | O | ILE C | 39 | 17.867 | 1.402 | 2.027 | 1.00 | 73.40 | O |
| ATOM | 2001 | N | GLU C | 40 | 18.852 | -0.112 | 0.710 | 1.00 | 126.73 | N |
| ATOM | 2002 | CA | GLU C | 40 | 18.983 | 0.821 | -0.403 | 1.00 | 136.48 | C |
| ATOM | 2003 | CB | GLU C | 40 | 19.900 | 0.252 | -1.490 | 1.00 | 138.06 | C |
| ATOM | 2004 | CG | GLU C | 40 | 21.377 | 0.652 | -1.356 | 1.00 | 142.34 | C |

```
ATOM   2005  CD   GLU C  40      22.119   -0.097   -0.255  1.00155.00           C
ATOM   2006  OE1  GLU C  40      21.568   -0.244    0.858  1.00146.66           O
ATOM   2007  OE2  GLU C  40      23.263   -0.537   -0.508  1.00150.93           O
ATOM   2008  C    GLU C  40      17.632    1.253   -0.983  1.00136.96           C
ATOM   2009  O    GLU C  40      17.514    2.345   -1.545  1.00134.09           O
ATOM   2010  N    GLU C  41      16.623    0.393   -0.850  1.00107.75           N
ATOM   2011  CA   GLU C  41      15.249    0.761   -1.193  1.00105.66           C
ATOM   2012  CB   GLU C  41      14.312   -0.452   -1.138  1.00102.74           C
ATOM   2013  CG   GLU C  41      14.639   -1.618   -2.056  1.00 99.38           C
ATOM   2014  CD   GLU C  41      13.728   -2.816   -1.796  1.00109.61           C
ATOM   2015  OE1  GLU C  41      13.994   -3.910   -2.343  1.00110.54           O
ATOM   2016  OE2  GLU C  41      12.745   -2.663   -1.034  1.00111.88           O
ATOM   2017  C    GLU C  41      14.761    1.771   -0.170  1.00106.25           C
ATOM   2018  O    GLU C  41      14.408    2.902   -0.504  1.00100.33           O
ATOM   2019  N    LEU C  42      14.754    1.339    1.089  1.00 88.35           N
ATOM   2020  CA   LEU C  42      14.297    2.167    2.192  1.00 80.06           C
ATOM   2021  CB   LEU C  42      14.401    1.407    3.516  1.00 83.80           C
ATOM   2022  CG   LEU C  42      13.336    0.331    3.790  1.00 93.85           C
ATOM   2023  CD1  LEU C  42      13.274   -0.718    2.678  1.00 92.86           C
ATOM   2024  CD2  LEU C  42      13.549   -0.338    5.163  1.00 88.25           C
ATOM   2025  C    LEU C  42      15.100    3.462    2.218  1.00 79.12           C
ATOM   2026  O    LEU C  42      14.741    4.422    2.904  1.00 79.02           O
ATOM   2027  N    ALA C  43      16.171    3.490    1.431  1.00103.95           N
ATOM   2028  CA   ALA C  43      17.008    4.674    1.306  1.00103.40           C
ATOM   2029  CB   ALA C  43      18.368    4.307    0.730  1.00100.87           C
ATOM   2030  C    ALA C  43      16.331    5.760    0.467  1.00109.15           C
ATOM   2031  O    ALA C  43      16.618    6.949    0.639  1.00107.59           O
ATOM   2032  N    GLU C  44      15.431    5.358   -0.429  1.00 96.88           N
ATOM   2033  CA   GLU C  44      14.668    6.327   -1.212  1.00 94.95           C
ATOM   2034  CB   GLU C  44      14.508    5.881   -2.674  1.00103.63           C
ATOM   2035  CG   GLU C  44      13.945    4.466   -2.880  1.00110.41           C
ATOM   2036  CD   GLU C  44      12.438    4.440   -3.155  1.00111.87           C
ATOM   2037  OE2  GLU C  44      11.666    4.056   -2.245  1.00112.81           O
ATOM   2038  OE1  GLU C  44      12.023    4.782   -4.285  1.00102.44           O
ATOM   2039  C    GLU C  44      13.310    6.600   -0.573  1.00 94.85           C
ATOM   2040  O    GLU C  44      12.957    7.756   -0.332  1.00 91.64           O
ATOM   2041  N    GLU C  45      12.560    5.536   -0.286  1.00 75.83           N
ATOM   2042  CA   GLU C  45      11.252    5.672    0.339  1.00 80.99           C
ATOM   2043  CB   GLU C  45      10.699    4.297    0.747  1.00 86.09           C
ATOM   2044  CG   GLU C  45       9.165    4.183    0.775  1.00 90.28           C
ATOM   2045  CD   GLU C  45       8.522    4.812    2.011  1.00 91.99           C
ATOM   2046  OE2  GLU C  45       8.039    5.965    1.916  1.00 88.00           O
ATOM   2047  OE1  GLU C  45       8.499    4.156    3.078  1.00 87.14           O
ATOM   2048  C    GLU C  45      11.409    6.604    1.542  1.00 85.18           C
ATOM   2049  O    GLU C  45      10.460    7.264    1.975  1.00 84.68           O
ATOM   2050  N    TYR C  46      12.627    6.652    2.072  1.00 93.10           N
ATOM   2051  CA   TYR C  46      12.976    7.593    3.121  1.00 87.83           C
ATOM   2052  CB   TYR C  46      13.237    6.859    4.429  1.00 86.56           C
ATOM   2053  CG   TYR C  46      12.061    6.025    4.870  1.00 88.69           C
ATOM   2054  CD1  TYR C  46      10.902    6.623    5.343  1.00 84.82           C
ATOM   2055  CE1  TYR C  46       9.817    5.864    5.741  1.00 86.73           C
ATOM   2056  CZ   TYR C  46       9.885    4.486    5.663  1.00 93.03           C
ATOM   2057  OH   TYR C  46       8.812    3.715    6.058  1.00 88.79           O
ATOM   2058  CE2  TYR C  46      11.027    3.871    5.190  1.00 90.00           C
ATOM   2059  CD2  TYR C  46      12.102    4.639    4.795  1.00 87.39           C
ATOM   2060  C    TYR C  46      14.191    8.379    2.690  1.00 82.88           C
ATOM   2061  O    TYR C  46      15.289    8.168    3.184  1.00 85.71           O
ATOM   2062  N    ALA C  47      13.984    9.260    1.723  1.00 73.83           N
ATOM   2063  CA   ALA C  47      15.049   10.116    1.240  1.00 73.14           C
ATOM   2064  CB   ALA C  47      15.120   10.074   -0.263  1.00 75.07           C
ATOM   2065  C    ALA C  47      14.736   11.516    1.710  1.00 75.13           C
ATOM   2066  O    ALA C  47      13.572   11.919    1.741  1.00 75.12           O
ATOM   2067  N    GLY C  48      15.769   12.264    2.077  1.00 62.09           N
```

72

| ATOM | 2068 | CA | GLY | C | 48 | 15.565 | 13.583 | 2.649 | 1.00 | 61.50 | C |
|------|------|-----|-----|---|----|--------|--------|-------|------|--------|---|
| ATOM | 2069 | C | GLY | C | 48 | 14.788 | 13.520 | 3.958 | 1.00 | 61.88 | C |
| ATOM | 2070 | O | GLY | C | 48 | 14.425 | 14.549 | 4.527 | 1.00 | 59.02 | O |
| ATOM | 2071 | N | LYS | C | 49 | 14.526 | 12.311 | 4.440 | 1.00 | 89.30 | N |
| ATOM | 2072 | CA | LYS | C | 49 | 13.863 | 12.150 | 5.729 | 1.00 | 98.01 | C |
| ATOM | 2073 | CB | LYS | C | 49 | 12.453 | 11.579 | 5.549 | 1.00 | 97.68 | C |
| ATOM | 2074 | CG | LYS | C | 49 | 12.325 | 10.486 | 4.496 | 1.00 | 101.42 | C |
| ATOM | 2075 | CD | LYS | C | 49 | 10.855 | 10.151 | 4.254 | 1.00 | 98.99 | C |
| ATOM | 2076 | CE | LYS | C | 49 | 10.048 | 11.409 | 3.952 | 1.00 | 90.65 | C |
| ATOM | 2077 | NZ | LYS | C | 49 | 8.692 | 11.354 | 4.552 | 1.00 | 85.11 | N |
| ATOM | 2078 | C | LYS | C | 49 | 14.682 | 11.299 | 6.702 | 1.00 | 100.75 | C |
| ATOM | 2079 | O | LYS | C | 49 | 14.617 | 11.486 | 7.920 | 1.00 | 97.70 | O |
| ATOM | 2080 | N | VAL | C | 50 | 15.450 | 10.365 | 6.152 | 1.00 | 99.20 | N |
| ATOM | 2081 | CA | VAL | C | 50 | 16.301 | 9.508 | 6.959 | 1.00 | 93.53 | C |
| ATOM | 2082 | CB | VAL | C | 50 | 15.601 | 8.195 | 7.325 | 1.00 | 98.91 | C |
| ATOM | 2083 | CG1 | VAL | C | 50 | 16.601 | 7.222 | 7.945 | 1.00 | 94.88 | C |
| ATOM | 2084 | CG2 | VAL | C | 50 | 14.422 | 8.456 | 8.269 | 1.00 | 101.35 | C |
| ATOM | 2085 | C | VAL | C | 50 | 17.595 | 9.179 | 6.237 | 1.00 | 90.14 | C |
| ATOM | 2086 | O | VAL | C | 50 | 17.589 | 8.888 | 5.047 | 1.00 | 95.63 | O |
| ATOM | 2087 | N | VAL | C | 51 | 18.705 | 9.220 | 6.964 | 1.00 | 62.53 | N |
| ATOM | 2088 | CA | VAL | C | 51 | 20.001 | 8.911 | 6.371 | 1.00 | 59.94 | C |
| ATOM | 2089 | CB | VAL | C | 51 | 21.131 | 9.680 | 7.036 | 1.00 | 46.71 | C |
| ATOM | 2090 | CG1 | VAL | C | 51 | 22.427 | 9.368 | 6.320 | 1.00 | 52.06 | C |
| ATOM | 2091 | CG2 | VAL | C | 51 | 20.834 | 11.179 | 7.035 | 1.00 | 43.27 | C |
| ATOM | 2092 | C | VAL | C | 51 | 20.344 | 7.449 | 6.529 | 1.00 | 54.52 | C |
| ATOM | 2093 | O | VAL | C | 51 | 20.038 | 6.857 | 7.547 | 1.00 | 55.20 | O |
| ATOM | 2094 | N | PHE | C | 52 | 20.977 | 6.866 | 5.521 | 1.00 | 66.58 | N |
| ATOM | 2095 | CA | PHE | C | 52 | 21.508 | 5.523 | 5.657 | 1.00 | 67.85 | C |
| ATOM | 2096 | CB | PHE | C | 52 | 20.813 | 4.553 | 4.710 | 1.00 | 65.84 | C |
| ATOM | 2097 | CG | PHE | C | 52 | 19.361 | 4.348 | 5.017 | 1.00 | 73.14 | C |
| ATOM | 2098 | CD2 | PHE | C | 52 | 18.925 | 3.185 | 5.626 | 1.00 | 77.89 | C |
| ATOM | 2099 | CE2 | PHE | C | 52 | 17.574 | 2.992 | 5.909 | 1.00 | 84.94 | C |
| ATOM | 2100 | CZ | PHE | C | 52 | 16.650 | 3.975 | 5.582 | 1.00 | 81.32 | C |
| ATOM | 2101 | CE1 | PHE | C | 52 | 17.081 | 5.142 | 4.978 | 1.00 | 78.53 | C |
| ATOM | 2102 | CD1 | PHE | C | 52 | 18.425 | 5.322 | 4.694 | 1.00 | 76.89 | C |
| ATOM | 2103 | C | PHE | C | 52 | 23.001 | 5.550 | 5.385 | 1.00 | 75.88 | C |
| ATOM | 2104 | O | PHE | C | 52 | 23.433 | 5.726 | 4.243 | 1.00 | 74.55 | O |
| ATOM | 2105 | N | GLY | C | 53 | 23.789 | 5.410 | 6.446 | 1.00 | 69.77 | N |
| ATOM | 2106 | CA | GLY | C | 53 | 25.224 | 5.278 | 6.304 | 1.00 | 59.28 | C |
| ATOM | 2107 | C | GLY | C | 53 | 25.518 | 3.801 | 6.340 | 1.00 | 52.61 | C |
| ATOM | 2108 | O | GLY | C | 53 | 24.671 | 2.997 | 6.705 | 1.00 | 58.22 | O |
| ATOM | 2109 | N | LYS | C | 54 | 26.712 | 3.422 | 5.941 | 1.00 | 51.28 | N |
| ATOM | 2110 | CA | LYS | C | 54 | 27.121 | 2.067 | 6.211 | 1.00 | 54.13 | C |
| ATOM | 2111 | CB | LYS | C | 54 | 26.710 | 1.124 | 5.097 | 1.00 | 48.13 | C |
| ATOM | 2112 | CG | LYS | C | 54 | 27.343 | 1.434 | 3.781 | 1.00 | 50.56 | C |
| ATOM | 2113 | CD | LYS | C | 54 | 26.991 | 0.357 | 2.778 | 1.00 | 53.71 | C |
| ATOM | 2114 | CE | LYS | C | 54 | 27.604 | 0.675 | 1.418 | 1.00 | 70.19 | C |
| ATOM | 2115 | NZ | LYS | C | 54 | 27.612 | 2.155 | 1.143 | 1.00 | 78.27 | N |
| ATOM | 2116 | C | LYS | C | 54 | 28.610 | 2.021 | 6.486 | 1.00 | 52.48 | C |
| ATOM | 2117 | O | LYS | C | 54 | 29.383 | 2.870 | 6.020 | 1.00 | 54.45 | O |
| ATOM | 2118 | N | VAL | C | 55 | 28.996 | 1.032 | 7.278 | 1.00 | 42.53 | N |
| ATOM | 2119 | CA | VAL | C | 55 | 30.330 | 1.011 | 7.842 | 1.00 | 42.46 | C |
| ATOM | 2120 | CB | VAL | C | 55 | 30.322 | 1.252 | 9.357 | 1.00 | 30.49 | C |
| ATOM | 2121 | CG1 | VAL | C | 55 | 31.648 | 0.827 | 9.962 | 1.00 | 39.78 | C |
| ATOM | 2122 | CG2 | VAL | C | 55 | 30.063 | 2.716 | 9.644 | 1.00 | 30.77 | C |
| ATOM | 2123 | C | VAL | C | 55 | 30.853 | -0.348 | 7.596 | 1.00 | 39.90 | C |
| ATOM | 2124 | O | VAL | C | 55 | 30.352 | -1.302 | 8.186 | 1.00 | 41.46 | O |
| ATOM | 2125 | N | ASN | C | 56 | 31.827 | -0.439 | 6.691 | 1.00 | 46.31 | N |
| ATOM | 2126 | CA | ASN | C | 56 | 32.483 | -1.708 | 6.423 | 1.00 | 52.50 | C |
| ATOM | 2127 | CB | ASN | C | 56 | 33.275 | -1.682 | 5.115 | 1.00 | 45.91 | C |
| ATOM | 2128 | CG | ASN | C | 56 | 33.782 | -3.056 | 4.739 | 1.00 | 56.04 | C |
| ATOM | 2129 | OD1 | ASN | C | 56 | 34.504 | -3.671 | 5.529 | 1.00 | 59.69 | O |
| ATOM | 2130 | ND2 | ASN | C | 56 | 33.364 | -3.579 | 3.563 | 1.00 | 45.20 | N |

| ATOM | 2131 | C   | ASN | C | 56 | 33.411 | -1.995 | 7.588  | 1.00 | 50.45 | C |
|------|------|-----|-----|---|----|--------|--------|--------|------|-------|---|
| ATOM | 2132 | O   | ASN | C | 56 | 34.331 | -1.200 | 7.852  | 1.00 | 41.60 | O |
| ATOM | 2133 | N   | VAL | C | 57 | 33.164 | -3.110 | 8.284  | 1.00 | 39.33 | N |
| ATOM | 2134 | CA  | VAL | C | 57 | 33.858 | -3.388 | 9.547  | 1.00 | 47.47 | C |
| ATOM | 2135 | CB  | VAL | C | 57 | 33.145 | -4.439 | 10.415 | 1.00 | 43.33 | C |
| ATOM | 2136 | CG1 | VAL | C | 57 | 31.744 | -3.936 | 10.879 | 1.00 | 47.81 | C |
| ATOM | 2137 | CG2 | VAL | C | 57 | 33.073 | -5.762 | 9.666  | 1.00 | 38.58 | C |
| ATOM | 2138 | C   | VAL | C | 57 | 35.278 | -3.881 | 9.328  | 1.00 | 56.89 | C |
| ATOM | 2139 | O   | VAL | C | 57 | 36.016 | -4.107 | 10.295 | 1.00 | 58.38 | O |
| ATOM | 2140 | N   | ASP | C | 58 | 35.646 | -4.061 | 8.061  | 1.00 | 55.13 | N |
| ATOM | 2141 | CA  | ASP | C | 58 | 36.979 | -4.514 | 7.689  | 1.00 | 54.15 | C |
| ATOM | 2142 | CB  | ASP | C | 58 | 36.939 | -5.180 | 6.309  | 1.00 | 59.24 | C |
| ATOM | 2143 | CG  | ASP | C | 58 | 36.766 | -6.690 | 6.376  | 1.00 | 64.76 | C |
| ATOM | 2144 | OD1 | ASP | C | 58 | 36.534 | -7.230 | 7.482  | 1.00 | 54.14 | O |
| ATOM | 2145 | OD2 | ASP | C | 58 | 36.843 | -7.332 | 5.298  | 1.00 | 63.35 | O |
| ATOM | 2146 | C   | ASP | C | 58 | 37.952 | -3.351 | 7.635  | 1.00 | 52.58 | C |
| ATOM | 2147 | O   | ASP | C | 58 | 39.093 | -3.458 | 8.071  | 1.00 | 57.81 | O |
| ATOM | 2148 | N   | GLU | C | 59 | 37.491 | -2.248 | 7.073  | 1.00 | 51.55 | N |
| ATOM | 2149 | CA  | GLU | C | 59 | 38.357 | -1.119 | 6.834  | 1.00 | 52.89 | C |
| ATOM | 2150 | CB  | GLU | C | 59 | 38.110 | -0.502 | 5.450  | 1.00 | 59.39 | C |
| ATOM | 2151 | CG  | GLU | C | 59 | 38.312 | -1.475 | 4.290  | 1.00 | 69.54 | C |
| ATOM | 2152 | CD  | GLU | C | 59 | 39.691 | -2.136 | 4.300  | 1.00 | 74.29 | C |
| ATOM | 2153 | OE2 | GLU | C | 59 | 39.746 | -3.392 | 4.355  | 1.00 | 64.82 | O |
| ATOM | 2154 | OE1 | GLU | C | 59 | 40.710 | -1.401 | 4.259  | 1.00 | 76.06 | O |
| ATOM | 2155 | C   | GLU | C | 59 | 38.133 | -0.100 | 7.913  | 1.00 | 52.19 | C |
| ATOM | 2156 | O   | GLU | C | 59 | 38.734 | 0.972  | 7.910  | 1.00 | 67.23 | O |
| ATOM | 2157 | N   | ASN | C | 60 | 37.243 | -0.422 | 8.836  | 1.00 | 43.23 | N |
| ATOM | 2158 | CA  | ASN | C | 60 | 37.122 | 0.381  | 10.041 | 1.00 | 42.67 | C |
| ATOM | 2159 | CB  | ASN | C | 60 | 36.126 | 1.552  | 9.892  | 1.00 | 39.53 | C |
| ATOM | 2160 | CG  | ASN | C | 60 | 36.145 | 2.198  | 8.495  | 1.00 | 49.81 | C |
| ATOM | 2161 | OD1 | ASN | C | 60 | 36.509 | 3.369  | 8.337  | 1.00 | 49.02 | O |
| ATOM | 2162 | ND2 | ASN | C | 60 | 35.703 | 1.440  | 7.484  | 1.00 | 43.51 | N |
| ATOM | 2163 | C   | ASN | C | 60 | 36.716 | -0.506 | 11.196 | 1.00 | 41.06 | C |
| ATOM | 2164 | O   | ASN | C | 60 | 35.657 | -0.292 | 11.786 | 1.00 | 43.11 | O |
| ATOM | 2165 | N   | PRO | C | 61 | 37.562 | -1.489 | 11.531 | 1.00 | 43.68 | N |
| ATOM | 2166 | CA  | PRO | C | 61 | 37.363 | -2.368 | 12.693 | 1.00 | 40.85 | C |
| ATOM | 2167 | CB  | PRO | C | 61 | 38.539 | -3.344 | 12.601 | 1.00 | 46.90 | C |
| ATOM | 2168 | CG  | PRO | C | 61 | 39.596 | -2.600 | 11.856 | 1.00 | 48.05 | C |
| ATOM | 2169 | CD  | PRO | C | 61 | 38.884 | -1.675 | 10.902 | 1.00 | 52.02 | C |
| ATOM | 2170 | C   | PRO | C | 61 | 37.380 | -1.608 | 14.022 | 1.00 | 40.26 | C |
| ATOM | 2171 | O   | PRO | C | 61 | 36.805 | -2.060 | 15.019 | 1.00 | 42.23 | O |
| ATOM | 2172 | N   | GLU | C | 62 | 38.011 | -0.446 | 14.041 | 1.00 | 37.89 | N |
| ATOM | 2173 | CA  | GLU | C | 62 | 38.011 | 0.345  | 15.263 | 1.00 | 41.07 | C |
| ATOM | 2174 | CB  | GLU | C | 62 | 39.132 | 1.387  | 15.286 | 1.00 | 45.15 | C |
| ATOM | 2175 | CG  | GLU | C | 62 | 39.009 | 2.467  | 14.234 | 1.00 | 53.87 | C |
| ATOM | 2176 | CD  | GLU | C | 62 | 39.409 | 1.974  | 12.842 | 1.00 | 66.10 | C |
| ATOM | 2177 | OE1 | GLU | C | 62 | 40.147 | 0.964  | 12.743 | 1.00 | 62.85 | O |
| ATOM | 2178 | OE2 | GLU | C | 62 | 38.977 | 2.588  | 11.842 | 1.00 | 67.46 | O |
| ATOM | 2179 | C   | GLU | C | 62 | 36.691 | 1.023  | 15.570 | 1.00 | 49.39 | C |
| ATOM | 2180 | O   | GLU | C | 62 | 36.336 | 1.148  | 16.733 | 1.00 | 52.80 | O |
| ATOM | 2181 | N   | ILE | C | 63 | 35.965 | 1.475  | 14.549 | 1.00 | 50.07 | N |
| ATOM | 2182 | CA  | ILE | C | 63 | 34.678 | 2.132  | 14.783 | 1.00 | 47.59 | C |
| ATOM | 2183 | CB  | ILE | C | 63 | 34.108 | 2.714  | 13.484 | 1.00 | 56.25 | C |
| ATOM | 2184 | CG1 | ILE | C | 63 | 35.209 | 3.411  | 12.690 | 1.00 | 54.63 | C |
| ATOM | 2185 | CD1 | ILE | C | 63 | 34.690 | 4.469  | 11.743 | 1.00 | 58.91 | C |
| ATOM | 2186 | CG2 | ILE | C | 63 | 32.957 | 3.666  | 13.774 | 1.00 | 48.01 | C |
| ATOM | 2187 | C   | ILE | C | 63 | 33.689 | 1.119  | 15.353 | 1.00 | 42.05 | C |
| ATOM | 2188 | O   | ILE | C | 63 | 32.973 | 1.380  | 16.329 | 1.00 | 45.48 | O |
| ATOM | 2189 | N   | ALA | C | 64 | 33.664 | -0.052 | 14.735 | 1.00 | 37.78 | N |
| ATOM | 2190 | CA  | ALA | C | 64 | 32.924 | -1.171 | 15.274 | 1.00 | 38.10 | C |
| ATOM | 2191 | CB  | ALA | C | 64 | 33.054 | -2.362 | 14.352 | 1.00 | 30.52 | C |
| ATOM | 2192 | C   | ALA | C | 64 | 33.377 | -1.530 | 16.705 | 1.00 | 41.18 | C |
| ATOM | 2193 | O   | ALA | C | 64 | 32.528 | -1.823 | 17.550 | 1.00 | 37.48 | O |

```
ATOM   2194  N    ALA C  65      34.695  -1.512  16.983  1.00 37.79           N
ATOM   2195  CA   ALA C  65      35.183  -1.780  18.364  1.00 39.04           C
ATOM   2196  CB   ALA C  65      36.719  -1.648  18.516  1.00 33.90           C
ATOM   2197  C    ALA C  65      34.510  -0.821  19.317  1.00 35.16           C
ATOM   2198  O    ALA C  65      34.026  -1.247  20.357  1.00 33.90           O
ATOM   2199  N    LYS C  66      34.466   0.461  18.928  1.00 39.82           N
ATOM   2200  C    LYS C  66      32.459   1.277  20.199  1.00 47.86           C
ATOM   2201  O    LYS C  66      32.122   1.455  21.360  1.00 47.09           O
ATOM   2202  CA  ALYS C  66      33.911   1.539  19.757  1.00 42.27           C
ATOM   2203  CB  ALYS C  66      34.012   2.868  19.002  1.00 43.14           C
ATOM   2204  CG  ALYS C  66      33.833   4.146  19.831  1.00 43.31           C
ATOM   2205  CD  ALYS C  66      34.353   5.346  19.026  1.00 45.93           C
ATOM   2206  CE  ALYS C  66      34.414   6.633  19.820  1.00 44.79           C
ATOM   2207  NZ  ALYS C  66      33.119   6.876  20.459  1.00 52.79           N
ATOM   2208  CA  BLYS C  66      33.902   1.532  19.753  0.00 42.47           C
ATOM   2209  CB  BLYS C  66      34.011   2.870  19.011  0.00 43.32           C
ATOM   2210  CG  BLYS C  66      33.579   4.098  19.805  0.00 43.74           C
ATOM   2211  CD  BLYS C  66      34.001   5.381  19.089  0.00 46.14           C
ATOM   2212  CE  BLYS C  66      33.703   6.622  19.919  0.00 46.58           C
ATOM   2213  NZ  BLYS C  66      34.420   7.830  19.412  0.00 45.62           N
ATOM   2214  N    TYR C  67      31.604   0.855  19.270  1.00 41.70           N
ATOM   2215  CA   TYR C  67      30.190   0.604  19.574  1.00 42.10           C
ATOM   2216  CB   TYR C  67      29.282   1.101  18.413  1.00 40.46           C
ATOM   2217  CG   TYR C  67      29.451   2.577  18.193  1.00 36.76           C
ATOM   2218  CD1  TYR C  67      28.753   3.495  18.958  1.00 37.38           C
ATOM   2219  CE1  TYR C  67      28.948   4.848  18.793  1.00 33.25           C
ATOM   2220  CZ   TYR C  67      29.859   5.280  17.871  1.00 36.05           C
ATOM   2221  OH   TYR C  67      30.087   6.614  17.698  1.00 52.57           O
ATOM   2222  CE2  TYR C  67      30.546   4.399  17.104  1.00 34.03           C
ATOM   2223  CD2  TYR C  67      30.349   3.054  17.270  1.00 35.02           C
ATOM   2224  C    TYR C  67      29.953  -0.876  19.850  1.00 44.78           C
ATOM   2225  O    TYR C  67      28.871  -1.408  19.599  1.00 39.42           O
ATOM   2226  N    GLY C  68      30.979  -1.552  20.345  1.00 40.15           N
ATOM   2227  CA   GLY C  68      30.867  -2.983  20.570  1.00 39.35           C
ATOM   2228  C    GLY C  68      29.913  -3.666  19.601  1.00 43.60           C
ATOM   2229  O    GLY C  68      28.904  -4.214  19.998  1.00 44.26           O
ATOM   2230  N    ILE C  69      30.222  -3.643  18.313  1.00 46.69           N
ATOM   2231  CA   ILE C  69      29.421  -4.419  17.379  1.00 45.48           C
ATOM   2232  CB   ILE C  69      29.466  -3.866  15.954  1.00 44.44           C
ATOM   2233  CG1  ILE C  69      29.130  -2.383  15.957  1.00 41.83           C
ATOM   2234  CD1  ILE C  69      27.781  -2.109  16.539  1.00 42.63           C
ATOM   2235  CG2  ILE C  69      28.476  -4.608  15.087  1.00 34.26           C
ATOM   2236  C    ILE C  69      29.888  -5.861  17.379  1.00 43.90           C
ATOM   2237  O    ILE C  69      30.832  -6.223  16.679  1.00 43.22           O
ATOM   2238  N    MET C  70      29.208  -6.688  18.159  1.00 46.82           N
ATOM   2239  CA   MET C  70      29.577  -8.093  18.267  1.00 46.35           C
ATOM   2240  CB   MET C  70      29.263  -8.610  19.668  1.00 42.63           C
ATOM   2241  CG   MET C  70      29.900  -7.765  20.758  1.00 54.97           C
ATOM   2242  SD   MET C  70      31.665  -7.406  20.509  1.00 56.66           S
ATOM   2243  CE   MET C  70      32.323  -9.066  20.516  1.00 57.88           C
ATOM   2244  C    MET C  70      28.967  -9.015  17.218  1.00 50.60           C
ATOM   2245  O    MET C  70      29.147 -10.228  17.289  1.00 54.74           O
ATOM   2246  N    SER C  71      28.241  -8.451  16.257  1.00 58.73           N
ATOM   2247  CA   SER C  71      27.664  -9.254  15.177  1.00 60.33           C
ATOM   2248  CB   SER C  71      26.575 -10.184  15.702  1.00 59.93           C
ATOM   2249  OG   SER C  71      25.440  -9.431  16.077  1.00 72.31           O
ATOM   2250  C    SER C  71      27.094  -8.417  14.034  1.00 62.52           C
ATOM   2251  O    SER C  71      26.563  -7.327  14.241  1.00 55.88           O
ATOM   2252  N    ILE C  72      27.210  -8.951  12.824  1.00 55.25           N
ATOM   2253  CA   ILE C  72      26.654  -8.316  11.641  1.00 54.91           C
ATOM   2254  CB   ILE C  72      27.767  -7.872  10.664  1.00 49.06           C
ATOM   2255  CG1  ILE C  72      28.837  -8.958  10.551  1.00 49.61           C
ATOM   2256  CD1  ILE C  72      29.856  -8.744   9.459  1.00 47.30           C
```

| ATOM | 2257 | CG2 | ILE | C | 72 | 28.408 | -6.590 | 11.139 | 1.00 | 50.20 | C |
|------|------|-----|-----|---|----|--------|--------|--------|------|-------|---|
| ATOM | 2258 | C | ILE | C | 72 | 25.647 | -9.248 | 10.951 | 1.00 | 55.19 | C |
| ATOM | 2259 | O | ILE | C | 72 | 25.835 | -10.463 | 10.896 | 1.00 | 52.21 | O |
| ATOM | 2260 | N | PRO | C | 73 | 24.559 | -8.673 | 10.431 | 1.00 | 71.29 | N |
| ATOM | 2261 | CA | PRO | C | 73 | 24.412 | -7.221 | 10.465 | 1.00 | 64.21 | C |
| ATOM | 2262 | CB | PRO | C | 73 | 23.318 | -6.968 | 9.439 | 1.00 | 63.93 | C |
| ATOM | 2263 | CG | PRO | C | 73 | 22.439 | -8.194 | 9.563 | 1.00 | 68.11 | C |
| ATOM | 2264 | CD | PRO | C | 73 | 23.340 | -9.344 | 9.944 | 1.00 | 71.62 | C |
| ATOM | 2265 | C | PRO | C | 73 | 23.915 | -6.762 | 11.811 | 1.00 | 54.18 | C |
| ATOM | 2266 | O | PRO | C | 73 | 23.480 | -7.548 | 12.663 | 1.00 | 56.58 | O |
| ATOM | 2267 | N | THR | C | 74 | 23.976 | -5.455 | 11.974 | 1.00 | 33.44 | N |
| ATOM | 2268 | CA | THR | C | 74 | 23.498 | -4.782 | 13.157 | 1.00 | 41.33 | C |
| ATOM | 2269 | CB | THR | C | 74 | 24.619 | -4.446 | 14.221 | 1.00 | 45.94 | C |
| ATOM | 2270 | OG1 | THR | C | 74 | 25.076 | -5.618 | 14.911 | 1.00 | 36.18 | O |
| ATOM | 2271 | CG2 | THR | C | 74 | 24.090 | -3.437 | 15.253 | 1.00 | 41.31 | C |
| ATOM | 2272 | C | THR | C | 74 | 23.170 | -3.466 | 12.551 | 1.00 | 42.64 | C |
| ATOM | 2273 | O | THR | C | 74 | 24.043 | -2.808 | 11.989 | 1.00 | 42.88 | O |
| ATOM | 2274 | N | LEU | C | 75 | 21.923 | -3.060 | 12.650 | 1.00 | 60.43 | N |
| ATOM | 2275 | CA | LEU | C | 75 | 21.596 | -1.726 | 12.236 | 1.00 | 61.04 | C |
| ATOM | 2276 | C | LEU | C | 75 | 21.813 | -0.873 | 13.457 | 1.00 | 58.74 | C |
| ATOM | 2277 | O | LEU | C | 75 | 21.518 | -1.308 | 14.573 | 1.00 | 59.77 | O |
| ATOM | 2278 | CB | LEU | C | 75 | 20.134 | -1.672 | 11.833 | 1.00 | 72.15 | C |
| ATOM | 2279 | CG | LEU | C | 75 | 19.867 | -0.895 | 10.557 | 1.00 | 74.20 | C |
| ATOM | 2280 | CD1 | LEU | C | 75 | 20.118 | -1.816 | 9.365 | 1.00 | 64.99 | C |
| ATOM | 2281 | CD2 | LEU | C | 75 | 18.437 | -0.371 | 10.597 | 1.00 | 75.12 | C |
| ATOM | 2282 | N | LEU | C | 76 | 22.324 | 0.336 | 13.268 | 1.00 | 54.05 | N |
| ATOM | 2283 | CA | LEU | C | 76 | 22.394 | 1.280 | 14.385 | 1.00 | 62.87 | C |
| ATOM | 2284 | CB | LEU | C | 76 | 23.845 | 1.610 | 14.763 | 1.00 | 66.29 | C |
| ATOM | 2285 | CG | LEU | C | 76 | 24.281 | 1.300 | 16.194 | 1.00 | 60.93 | C |
| ATOM | 2286 | CD1 | LEU | C | 76 | 24.068 | -0.186 | 16.467 | 1.00 | 53.52 | C |
| ATOM | 2287 | CD2 | LEU | C | 76 | 25.744 | 1.741 | 16.466 | 1.00 | 46.80 | C |
| ATOM | 2288 | C | LEU | C | 76 | 21.631 | 2.562 | 14.075 | 1.00 | 69.02 | C |
| ATOM | 2289 | O | LEU | C | 76 | 21.692 | 3.093 | 12.958 | 1.00 | 61.39 | O |
| ATOM | 2290 | N | PHE | C | 77 | 20.913 | 3.057 | 15.079 | 1.00 | 66.79 | N |
| ATOM | 2291 | CA | PHE | C | 77 | 20.167 | 4.297 | 14.929 | 1.00 | 57.13 | C |
| ATOM | 2292 | CB | PHE | C | 77 | 18.717 | 4.110 | 15.350 | 1.00 | 55.21 | C |
| ATOM | 2293 | CG | PHE | C | 77 | 17.980 | 3.113 | 14.529 | 1.00 | 51.17 | C |
| ATOM | 2294 | CD2 | PHE | C | 77 | 16.923 | 3.511 | 13.723 | 1.00 | 48.29 | C |
| ATOM | 2295 | CE2 | PHE | C | 77 | 16.235 | 2.595 | 12.966 | 1.00 | 55.06 | C |
| ATOM | 2296 | CZ | PHE | C | 77 | 16.596 | 1.248 | 13.006 | 1.00 | 63.34 | C |
| ATOM | 2297 | CE1 | PHE | C | 77 | 17.645 | 0.847 | 13.814 | 1.00 | 64.55 | C |
| ATOM | 2298 | CD1 | PHE | C | 77 | 18.331 | 1.780 | 14.569 | 1.00 | 52.63 | C |
| ATOM | 2299 | C | PHE | C | 77 | 20.761 | 5.391 | 15.770 | 1.00 | 53.08 | C |
| ATOM | 2300 | O | PHE | C | 77 | 20.617 | 5.386 | 16.985 | 1.00 | 55.40 | O |
| ATOM | 2301 | N | PHE | C | 78 | 21.424 | 6.335 | 15.122 | 1.00 | 54.56 | N |
| ATOM | 2302 | CA | PHE | C | 78 | 21.810 | 7.552 | 15.810 | 1.00 | 60.77 | C |
| ATOM | 2303 | CB | PHE | C | 78 | 23.136 | 8.093 | 15.266 | 1.00 | 63.12 | C |
| ATOM | 2304 | CG | PHE | C | 78 | 24.307 | 7.182 | 15.498 | 1.00 | 57.80 | C |
| ATOM | 2305 | CD2 | PHE | C | 78 | 25.465 | 7.668 | 16.081 | 1.00 | 49.21 | C |
| ATOM | 2306 | CE2 | PHE | C | 78 | 26.563 | 6.837 | 16.285 | 1.00 | 49.15 | C |
| ATOM | 2307 | CZ | PHE | C | 78 | 26.492 | 5.498 | 15.906 | 1.00 | 55.56 | C |
| ATOM | 2308 | CE1 | PHE | C | 78 | 25.330 | 5.005 | 15.317 | 1.00 | 56.96 | C |
| ATOM | 2309 | CD1 | PHE | C | 78 | 24.253 | 5.844 | 15.116 | 1.00 | 53.92 | C |
| ATOM | 2310 | C | PHE | C | 78 | 20.708 | 8.596 | 15.638 | 1.00 | 71.43 | C |
| ATOM | 2311 | O | PHE | C | 78 | 20.014 | 8.628 | 14.611 | 1.00 | 61.31 | O |
| ATOM | 2312 | N | LYS | C | 79 | 20.545 | 9.433 | 16.659 | 1.00 | 96.54 | N |
| ATOM | 2313 | CA | LYS | C | 79 | 19.738 | 10.642 | 16.563 | 1.00 | 102.07 | C |
| ATOM | 2314 | CB | LYS | C | 79 | 18.312 | 10.412 | 17.067 | 1.00 | 89.55 | C |
| ATOM | 2315 | CG | LYS | C | 79 | 17.298 | 11.471 | 16.613 | 1.00 | 99.43 | C |
| ATOM | 2316 | CD | LYS | C | 79 | 15.880 | 10.887 | 16.609 | 1.00 | 102.79 | C |
| ATOM | 2317 | CE | LYS | C | 79 | 14.818 | 11.883 | 16.152 | 1.00 | 96.69 | C |
| ATOM | 2318 | NZ | LYS | C | 79 | 13.456 | 11.267 | 16.207 | 1.00 | 87.00 | N |
| ATOM | 2319 | C | LYS | C | 79 | 20.435 | 11.673 | 17.420 | 1.00 | 105.44 | C |

| ATOM | 2320 | O   | LYS | C | 79 | 20.819 | 11.380 | 18.552 | 1.00 | 109.55 | O |
|------|------|-----|-----|---|----|--------|--------|--------|------|--------|---|
| ATOM | 2321 | N   | ASN | C | 80 | 20.619 | 12.868 | 16.877 | 1.00 | 80.61  | N |
| ATOM | 2322 | CA  | ASN | C | 80 | 21.246 | 13.951 | 17.624 | 1.00 | 86.90  | C |
| ATOM | 2323 | CB  | ASN | C | 80 | 20.345 | 14.403 | 18.783 | 1.00 | 88.32  | C |
| ATOM | 2324 | CG  | ASN | C | 80 | 18.944 | 14.779 | 18.320 | 1.00 | 83.69  | C |
| ATOM | 2325 | OD1 | ASN | C | 80 | 18.758 | 15.267 | 17.204 | 1.00 | 85.52  | O |
| ATOM | 2326 | ND2 | ASN | C | 80 | 17.951 | 14.548 | 19.175 | 1.00 | 79.77  | N |
| ATOM | 2327 | C   | ASN | C | 80 | 22.652 | 13.617 | 18.122 | 1.00 | 82.58  | C |
| ATOM | 2328 | O   | ASN | C | 80 | 23.239 | 14.375 | 18.890 | 1.00 | 83.49  | O |
| ATOM | 2329 | N   | GLY | C | 81 | 23.192 | 12.488 | 17.671 | 1.00 | 92.00  | N |
| ATOM | 2330 | CA  | GLY | C | 81 | 24.546 | 12.093 | 18.025 | 1.00 | 92.08  | C |
| ATOM | 2331 | C   | GLY | C | 81 | 24.603 | 11.146 | 19.212 | 1.00 | 80.18  | C |
| ATOM | 2332 | O   | GLY | C | 81 | 25.162 | 11.482 | 20.249 | 1.00 | 80.20  | O |
| ATOM | 2333 | N   | LYS | C | 82 | 24.032 | 9.959  | 19.041 | 1.00 | 67.40  | N |
| ATOM | 2334 | CA  | LYS | C | 82 | 23.853 | 9.004  | 20.120 | 1.00 | 67.23  | C |
| ATOM | 2335 | CB  | LYS | C | 82 | 23.504 | 9.732  | 21.413 | 1.00 | 79.38  | C |
| ATOM | 2336 | CG  | LYS | C | 82 | 22.227 | 10.565 | 21.322 | 1.00 | 85.22  | C |
| ATOM | 2337 | CD  | LYS | C | 82 | 22.245 | 11.686 | 22.346 | 1.00 | 81.09  | C |
| ATOM | 2338 | CE  | LYS | C | 82 | 20.935 | 11.772 | 23.092 | 1.00 | 77.88  | C |
| ATOM | 2339 | NZ  | LYS | C | 82 | 21.084 | 12.697 | 24.249 | 1.00 | 79.59  | N |
| ATOM | 2340 | C   | LYS | C | 82 | 22.741 | 8.015  | 19.779 | 1.00 | 66.98  | C |
| ATOM | 2341 | O   | LYS | C | 82 | 21.649 | 8.398  | 19.351 | 1.00 | 68.27  | O |
| ATOM | 2342 | N   | VAL | C | 83 | 23.020 | 6.738  | 19.998 | 1.00 | 73.07  | N |
| ATOM | 2343 | CA  | VAL | C | 83 | 22.073 | 5.669  | 19.695 | 1.00 | 67.65  | C |
| ATOM | 2344 | CB  | VAL | C | 83 | 22.686 | 4.305  | 20.055 | 1.00 | 67.41  | C |
| ATOM | 2345 | CG1 | VAL | C | 83 | 21.844 | 3.159  | 19.497 | 1.00 | 70.44  | C |
| ATOM | 2346 | CG2 | VAL | C | 83 | 24.108 | 4.235  | 19.506 | 1.00 | 57.20  | C |
| ATOM | 2347 | C   | VAL | C | 83 | 20.706 | 5.841  | 20.372 | 1.00 | 67.71  | C |
| ATOM | 2348 | O   | VAL | C | 83 | 20.551 | 6.646  | 21.289 | 1.00 | 73.66  | O |
| ATOM | 2349 | N   | VAL | C | 84 | 19.714 | 5.100  | 19.885 | 1.00 | 64.50  | N |
| ATOM | 2350 | CA  | VAL | C | 84 | 18.371 | 5.087  | 20.469 | 1.00 | 65.66  | C |
| ATOM | 2351 | CB  | VAL | C | 84 | 17.510 | 6.236  | 19.941 | 1.00 | 64.78  | C |
| ATOM | 2352 | CG1 | VAL | C | 84 | 17.964 | 7.557  | 20.542 | 1.00 | 64.18  | C |
| ATOM | 2353 | CG2 | VAL | C | 84 | 17.568 | 6.272  | 18.426 | 1.00 | 63.80  | C |
| ATOM | 2354 | C   | VAL | C | 84 | 17.670 | 3.779  | 20.126 | 1.00 | 66.30  | C |
| ATOM | 2355 | O   | VAL | C | 84 | 16.612 | 3.460  | 20.674 | 1.00 | 60.94  | O |
| ATOM | 2356 | N   | ASP | C | 85 | 18.262 | 3.049  | 19.183 | 1.00 | 60.89  | N |
| ATOM | 2357 | CA  | ASP | C | 85 | 17.813 | 1.704  | 18.842 | 1.00 | 56.32  | C |
| ATOM | 2358 | CB  | ASP | C | 85 | 16.472 | 1.724  | 18.112 | 1.00 | 58.62  | C |
| ATOM | 2359 | CG  | ASP | C | 85 | 15.565 | 0.565  | 18.522 | 1.00 | 63.73  | C |
| ATOM | 2360 | OD1 | ASP | C | 85 | 15.840 | -0.583 | 18.105 | 1.00 | 64.51  | O |
| ATOM | 2361 | OD2 | ASP | C | 85 | 14.574 | 0.797  | 19.251 | 1.00 | 62.49  | O |
| ATOM | 2362 | C   | ASP | C | 85 | 18.864 | 0.947  | 18.039 | 1.00 | 50.54  | C |
| ATOM | 2363 | O   | ASP | C | 85 | 19.985 | 1.423  | 17.855 | 1.00 | 55.36  | O |
| ATOM | 2364 | N   | GLN | C | 86 | 18.480 | -0.214 | 17.533 | 1.00 | 63.88  | N |
| ATOM | 2365 | CA  | GLN | C | 86 | 19.462 | -1.243 | 17.284 | 1.00 | 71.44  | C |
| ATOM | 2366 | CB  | GLN | C | 86 | 20.174 | -1.500 | 18.612 | 1.00 | 58.82  | C |
| ATOM | 2367 | CG  | GLN | C | 86 | 21.501 | -2.210 | 18.562 | 1.00 | 67.38  | C |
| ATOM | 2368 | CD  | GLN | C | 86 | 22.218 | -2.091 | 19.898 | 1.00 | 73.25  | C |
| ATOM | 2369 | OE1 | GLN | C | 86 | 21.575 | -1.845 | 20.926 | 1.00 | 66.33  | O |
| ATOM | 2370 | NE2 | GLN | C | 86 | 23.550 | -2.238 | 19.890 | 1.00 | 61.66  | N |
| ATOM | 2371 | C   | GLN | C | 86 | 18.804 | -2.533 | 16.809 | 1.00 | 71.58  | C |
| ATOM | 2372 | O   | GLN | C | 86 | 18.269 | -3.304 | 17.606 | 1.00 | 79.93  | O |
| ATOM | 2373 | N   | LEU | C | 87 | 18.844 | -2.792 | 15.518 | 1.00 | 55.92  | N |
| ATOM | 2374 | CA  | LEU | C | 87 | 18.318 | -4.069 | 15.058 | 1.00 | 67.42  | C |
| ATOM | 2375 | CB  | LEU | C | 87 | 17.546 | -3.934 | 13.743 | 1.00 | 66.95  | C |
| ATOM | 2376 | CG  | LEU | C | 87 | 16.131 | -3.434 | 14.008 | 1.00 | 75.43  | C |
| ATOM | 2377 | CD1 | LEU | C | 87 | 15.605 | -4.039 | 15.321 | 1.00 | 69.09  | C |
| ATOM | 2378 | CD2 | LEU | C | 87 | 16.120 | -1.918 | 14.070 | 1.00 | 72.19  | C |
| ATOM | 2379 | C   | LEU | C | 87 | 19.420 | -5.099 | 14.940 | 1.00 | 64.87  | C |
| ATOM | 2380 | O   | LEU | C | 87 | 19.917 | -5.368 | 13.849 | 1.00 | 66.46  | O |
| ATOM | 2381 | N   | VAL | C | 88 | 19.800 | -5.679 | 16.068 | 1.00 | 58.03  | N |
| ATOM | 2382 | CA  | VAL | C | 88 | 20.835 | -6.701 | 16.055 | 1.00 | 62.36  | C |

| | | | | | | | | | | | |
|---|---|---|---|---|---|---|---|---|---|---|---|
| ATOM | 2383 | CB | VAL | C | 88 | 21.331 | -7.009 | 17.472 | 1.00 | 63.43 | C |
| ATOM | 2384 | CG1 | VAL | C | 88 | 22.245 | -8.222 | 17.458 | 1.00 | 65.75 | C |
| ATOM | 2385 | CG2 | VAL | C | 88 | 22.038 | -5.789 | 18.051 | 1.00 | 54.87 | C |
| ATOM | 2386 | C | VAL | C | 88 | 20.364 | -7.984 | 15.366 | 1.00 | 66.90 | C |
| ATOM | 2387 | O | VAL | C | 88 | 19.698 | -8.830 | 15.973 | 1.00 | 68.64 | O |
| ATOM | 2388 | N | GLY | C | 89 | 20.724 | -8.128 | 14.097 | 1.00 | 56.20 | N |
| ATOM | 2389 | CA | GLY | C | 89 | 20.304 | -9.274 | 13.315 | 1.00 | 59.05 | C |
| ATOM | 2390 | C | GLY | C | 89 | 19.596 | -8.846 | 12.044 | 1.00 | 65.04 | C |
| ATOM | 2391 | O | GLY | C | 89 | 18.898 | -7.830 | 12.038 | 1.00 | 68.50 | O |
| ATOM | 2392 | N | ALA | C | 90 | 19.781 | -9.610 | 10.967 | 1.00 | 89.45 | N |
| ATOM | 2393 | CA | ALA | C | 90 | 19.118 | -9.317 | 9.698 | 1.00 | 94.03 | C |
| ATOM | 2394 | CB | ALA | C | 90 | 19.652 | -10.191 | 8.581 | 1.00 | 87.47 | C |
| ATOM | 2395 | C | ALA | C | 90 | 17.638 | -9.532 | 9.860 | 1.00 | 93.83 | C |
| ATOM | 2396 | O | ALA | C | 90 | 17.206 | -10.385 | 10.640 | 1.00 | 90.85 | O |
| ATOM | 2397 | N | ARG | C | 91 | 16.864 | -8.754 | 9.116 | 1.00 | 91.05 | N |
| ATOM | 2398 | CA | ARG | C | 91 | 15.414 | -8.786 | 9.231 | 1.00 | 97.66 | C |
| ATOM | 2399 | CB | ARG | C | 91 | 14.958 | -7.824 | 10.333 | 1.00 | 86.56 | C |
| ATOM | 2400 | CG | ARG | C | 91 | 15.382 | -8.299 | 11.705 | 1.00 | 81.24 | C |
| ATOM | 2401 | CD | ARG | C | 91 | 15.044 | -7.317 | 12.809 | 1.00 | 87.65 | C |
| ATOM | 2402 | NE | ARG | C | 91 | 14.751 | -8.024 | 14.058 | 1.00 | 93.55 | N |
| ATOM | 2403 | CZ | ARG | C | 91 | 15.608 | -8.811 | 14.708 | 1.00 | 87.81 | C |
| ATOM | 2404 | NH1 | ARG | C | 91 | 16.830 | -9.008 | 14.240 | 1.00 | 81.53 | N |
| ATOM | 2405 | NH2 | ARG | C | 91 | 15.241 | -9.405 | 15.833 | 1.00 | 85.17 | N |
| ATOM | 2406 | C | ARG | C | 91 | 14.755 | -8.447 | 7.902 | 1.00 | 98.31 | C |
| ATOM | 2407 | O | ARG | C | 91 | 15.388 | -7.858 | 7.020 | 1.00 | 98.13 | O |
| ATOM | 2408 | N | PRO | C | 92 | 13.485 | -8.844 | 7.749 | 1.00 | 77.00 | N |
| ATOM | 2409 | CA | PRO | C | 92 | 12.648 | -8.524 | 6.580 | 1.00 | 77.89 | C |
| ATOM | 2410 | CB | PRO | C | 92 | 11.404 | -9.396 | 6.784 | 1.00 | 83.19 | C |
| ATOM | 2411 | CG | PRO | C | 92 | 11.399 | -9.744 | 8.261 | 1.00 | 79.58 | C |
| ATOM | 2412 | CD | PRO | C | 92 | 12.830 | -9.774 | 8.688 | 1.00 | 72.09 | C |
| ATOM | 2413 | C | PRO | C | 92 | 12.269 | -7.035 | 6.475 | 1.00 | 78.19 | C |
| ATOM | 2414 | O | PRO | C | 92 | 12.329 | -6.304 | 7.473 | 1.00 | 75.10 | O |
| ATOM | 2415 | N | LYS | C | 93 | 11.859 | -6.604 | 5.279 | 1.00 | 84.79 | N |
| ATOM | 2416 | CA | LYS | C | 93 | 11.629 | -5.186 | 4.993 | 1.00 | 79.09 | C |
| ATOM | 2417 | CB | LYS | C | 93 | 11.009 | -4.999 | 3.612 | 1.00 | 76.22 | C |
| ATOM | 2418 | CG | LYS | C | 93 | 11.088 | -3.551 | 3.138 | 1.00 | 89.03 | C |
| ATOM | 2419 | CD | LYS | C | 93 | 10.760 | -3.395 | 1.660 | 1.00 | 94.28 | C |
| ATOM | 2420 | CE | LYS | C | 93 | 9.254 | -3.314 | 1.426 | 1.00 | 98.15 | C |
| ATOM | 2421 | NZ | LYS | C | 93 | 8.894 | -3.173 | -0.027 | 1.00 | 85.12 | N |
| ATOM | 2422 | C | LYS | C | 93 | 10.814 | -4.389 | 6.027 | 1.00 | 83.56 | C |
| ATOM | 2423 | O | LYS | C | 93 | 11.316 | -3.420 | 6.599 | 1.00 | 81.20 | O |
| ATOM | 2424 | N | GLU | C | 94 | 9.561 | -4.772 | 6.254 | 1.00 | 112.68 | N |
| ATOM | 2425 | CA | GLU | C | 94 | 8.685 | -3.975 | 7.118 | 1.00 | 117.30 | C |
| ATOM | 2426 | CB | GLU | C | 94 | 7.211 | -4.308 | 6.879 | 1.00 | 115.74 | C |
| ATOM | 2427 | CG | GLU | C | 94 | 6.864 | -5.755 | 7.163 | 1.00 | 123.33 | C |
| ATOM | 2428 | CD | GLU | C | 94 | 7.563 | -6.714 | 6.214 | 1.00 | 125.67 | C |
| ATOM | 2429 | OE1 | GLU | C | 94 | 7.525 | -6.465 | 4.989 | 1.00 | 115.14 | O |
| ATOM | 2430 | OE2 | GLU | C | 94 | 8.150 | -7.709 | 6.693 | 1.00 | 125.63 | O |
| ATOM | 2431 | C | GLU | C | 94 | 9.025 | -4.088 | 8.604 | 1.00 | 111.21 | C |
| ATOM | 2432 | O | GLU | C | 94 | 8.791 | -3.145 | 9.365 | 1.00 | 102.74 | O |
| ATOM | 2433 | N | ALA | C | 95 | 9.555 | -5.240 | 9.015 | 1.00 | 121.83 | N |
| ATOM | 2434 | CA | ALA | C | 95 | 10.100 | -5.383 | 10.362 | 1.00 | 121.78 | C |
| ATOM | 2435 | CB | ALA | C | 95 | 10.894 | -6.679 | 10.487 | 1.00 | 110.98 | C |
| ATOM | 2436 | C | ALA | C | 95 | 11.006 | -4.186 | 10.552 | 1.00 | 112.84 | C |
| ATOM | 2437 | O | ALA | C | 95 | 10.872 | -3.406 | 11.500 | 1.00 | 103.60 | O |
| ATOM | 2438 | N | LEU | C | 96 | 11.915 | -4.040 | 9.597 | 1.00 | 70.09 | N |
| ATOM | 2439 | CA | LEU | C | 96 | 12.801 | -2.901 | 9.538 | 1.00 | 76.22 | C |
| ATOM | 2440 | CB | LEU | C | 96 | 13.767 | -3.062 | 8.368 | 1.00 | 80.81 | C |
| ATOM | 2441 | CG | LEU | C | 96 | 14.833 | -1.971 | 8.271 | 1.00 | 87.57 | C |
| ATOM | 2442 | CD1 | LEU | C | 96 | 15.520 | -1.812 | 9.617 | 1.00 | 87.54 | C |
| ATOM | 2443 | CD2 | LEU | C | 96 | 15.842 | -2.295 | 7.181 | 1.00 | 90.71 | C |
| ATOM | 2444 | C | LEU | C | 96 | 12.019 | -1.602 | 9.390 | 1.00 | 79.52 | C |
| ATOM | 2445 | O | LEU | C | 96 | 12.221 | -0.658 | 10.161 | 1.00 | 80.63 | O |

78

```
ATOM   2446  N   LYS C  97   11.128  -1.563   8.400  1.00  75.39          N
ATOM   2447  CA  LYS C  97   10.359  -0.365   8.095  1.00  72.02          C
ATOM   2448  CB  LYS C  97    9.346  -0.643   6.969  1.00  84.24          C
ATOM   2449  CG  LYS C  97    8.993   0.578   6.085  1.00  89.21          C
ATOM   2450  CD  LYS C  97    7.952   0.259   4.996  1.00  84.81          C
ATOM   2451  CE  LYS C  97    8.520  -0.643   3.898  1.00  85.94          C
ATOM   2452  NZ  LYS C  97    7.553  -0.880   2.786  1.00  79.83          N
ATOM   2453  C   LYS C  97    9.665   0.165   9.352  1.00  70.03          C
ATOM   2454  O   LYS C  97    9.637   1.371   9.592  1.00  72.61          O
ATOM   2455  N   GLU C  98    9.135  -0.738  10.168  1.00  65.07          N
ATOM   2456  CA  GLU C  98    8.432  -0.353  11.391  1.00  69.66          C
ATOM   2457  CB  GLU C  98    7.873  -1.586  12.103  1.00  77.27          C
ATOM   2458  CG  GLU C  98    6.680  -2.248  11.435  1.00  68.98          C
ATOM   2459  CD  GLU C  98    6.339  -3.583  12.081  1.00  73.97          C
ATOM   2460  OE1 GLU C  98    5.920  -4.511  11.348  1.00  77.99          O
ATOM   2461  OE2 GLU C  98    6.493  -3.704  13.322  1.00  67.60          O
ATOM   2462  C   GLU C  98    9.264   0.459  12.395  1.00  78.44          C
ATOM   2463  O   GLU C  98    8.808   1.506  12.868  1.00  74.73          O
ATOM   2464  N   ARG C  99   10.456  -0.020  12.755  1.00  80.01          N
ATOM   2465  CA  ARG C  99   11.254   0.717  13.736  1.00  77.38          C
ATOM   2466  CB  ARG C  99   12.474  -0.082  14.218  1.00  76.38          C
ATOM   2467  CG  ARG C  99   12.195  -0.839  15.525  1.00  78.22          C
ATOM   2468  CD  ARG C  99   13.385  -1.623  16.087  1.00  81.36          C
ATOM   2469  NE  ARG C  99   12.983  -2.400  17.267  1.00  81.78          N
ATOM   2470  CZ  ARG C  99   13.817  -3.048  18.082  1.00  90.20          C
ATOM   2471  NH1 ARG C  99   15.127  -3.023  17.858  1.00  85.19          N
ATOM   2472  NH2 ARG C  99   13.339  -3.720  19.132  1.00  81.47          N
ATOM   2473  C   ARG C  99   11.643   2.076  13.178  1.00  78.21          C
ATOM   2474  O   ARG C  99   11.768   3.049  13.913  1.00  77.49          O
ATOM   2475  N   ILE C 100   11.789   2.145  11.861  1.00  74.35          N
ATOM   2476  CA  ILE C 100   12.104   3.405  11.207  1.00  81.30          C
ATOM   2477  CB  ILE C 100   12.514   3.205   9.740  1.00  82.84          C
ATOM   2478  CG1 ILE C 100   13.518   2.060   9.630  1.00  72.62          C
ATOM   2479  CD1 ILE C 100   13.975   1.791   8.229  1.00  87.35          C
ATOM   2480  CG2 ILE C 100   13.092   4.496   9.163  1.00  79.94          C
ATOM   2481  C   ILE C 100   10.915   4.345  11.270  1.00  73.62          C
ATOM   2482  O   ILE C 100   11.071   5.510  11.617  1.00  80.26          O
ATOM   2483  N   LYS C 101    9.731   3.844  10.940  1.00  64.40          N
ATOM   2484  CA  LYS C 101    8.511   4.653  11.057  1.00  75.53          C
ATOM   2485  CB  LYS C 101    7.246   3.831  10.740  1.00  67.79          C
ATOM   2486  CG  LYS C 101    7.078   3.459   9.272  1.00  67.50          C
ATOM   2487  CD  LYS C 101    5.949   2.469   9.086  1.00  58.98          C
ATOM   2488  CE  LYS C 101    4.622   3.046   9.589  1.00  66.40          C
ATOM   2489  NZ  LYS C 101    3.469   2.100   9.390  1.00  52.86          N
ATOM   2490  C   LYS C 101    8.404   5.273  12.443  1.00  72.34          C
ATOM   2491  O   LYS C 101    7.765   6.308  12.630  1.00  60.93          O
ATOM   2492  N   LYS C 102    9.048   4.624  13.407  1.00  72.88          N
ATOM   2493  CA  LYS C 102    9.014   5.059  14.792  1.00  68.92          C
ATOM   2494  CB  LYS C 102    9.533   3.933  15.698  1.00  71.58          C
ATOM   2495  CG  LYS C 102    9.698   4.294  17.163  1.00  70.95          C
ATOM   2496  CD  LYS C 102    8.504   5.081  17.685  1.00  81.71          C
ATOM   2497  C   LYS C 102    9.819   6.348  14.973  1.00  72.63          C
ATOM   2498  O   LYS C 102    9.405   7.255  15.699  1.00  71.22          O
ATOM   2499  N   TYR C 103   10.950   6.440  14.277  1.00  79.31          N
ATOM   2500  CA  TYR C 103   11.862   7.568  14.437  1.00  77.29          C
ATOM   2501  CB  TYR C 103   13.303   7.067  14.395  1.00  70.64          C
ATOM   2502  CG  TYR C 103   13.513   5.986  15.424  1.00  65.24          C
ATOM   2503  CD1 TYR C 103   13.483   6.281  16.776  1.00  67.49          C
ATOM   2504  CE1 TYR C 103   13.642   5.293  17.726  1.00  68.13          C
ATOM   2505  CZ  TYR C 103   13.827   3.984  17.327  1.00  68.01          C
ATOM   2506  OH  TYR C 103   13.983   2.996  18.273  1.00  63.25          O
ATOM   2507  CE2 TYR C 103   13.856   3.666  15.988  1.00  62.76          C
ATOM   2508  CD2 TYR C 103   13.688   4.667  15.048  1.00  66.94          C
```

```
ATOM   2509  C    TYR C 103      11.610    8.598  13.371  1.00 75.21          C
ATOM   2510  O    TYR C 103      12.472    9.416  13.069  1.00 80.48          O
ATOM   2511  N    LEU C 104      10.390    8.570  12.848  1.00122.70          N
ATOM   2512  CA   LEU C 104      10.020    9.265  11.621  1.00125.91          C
ATOM   2513  CB   LEU C 104       8.844    8.527  10.978  1.00123.71          C
ATOM   2514  CG   LEU C 104       8.698    8.470   9.460  1.00129.05          C
ATOM   2515  CD1  LEU C 104       9.878    7.732   8.854  1.00128.54          C
ATOM   2516  CD2  LEU C 104       7.382    7.790   9.089  1.00128.89          C
ATOM   2517  C    LEU C 104       9.643   10.727  11.856  1.00135.85          C
ATOM   2518  O    LEU C 104      10.391   11.644  11.513  1.00130.81          O
ATOM   2519  OXT  LEU C 104       8.575   11.034  12.391  1.00137.62          O
TER
HETATM 2520  O    HOH S   1      25.811    2.360  24.963  1.00 45.86          O
HETATM 2521  O    HOH S   2       7.173   -5.978  14.925  1.00 38.83          O
HETATM 2522  O    HOH S   3       9.962    0.261  27.317  1.00 39.71          O
HETATM 2523  O    HOH S   4      24.011  -15.969  22.835  1.00 47.78          O
HETATM 2524  O    HOH S   5       2.695    0.019  35.519  1.00 61.10          O
TER
END
```

## References

Adam, G. and M. Delbruck, Structural Chemistry and Molecular Biology, ed. A. Rich and N. Davidson. 1968, New York: W. H. Freeman and Co. 198-215.

Ainavarapu, S.R., Wiita, A.P., Huang, H.H. & Fernandez, J.M. A single-molecule assay to directly identify solvent-accessible disulfide bonds and probe their effect on protein folding. J Am Chem Soc 130, 436-7 (2008).

Alegre-Cebollada J, Perez-Jimenez R, Kosuri P, Fernandez JM., Single-molecule force spectroscopy approach to enzyme catalysis, J Biol Chem, 285(25), 18961-6 (2010).

Arner, E.S. and A. Holmgren, Physiological functions of thioredoxin and thioredoxin reductase. Eur J Biochem, 2000. 267(20): p. 6102-9.

Avval, F.Z. and A. Holmgren, Molecular mechanisms of thioredoxin and glutaredoxin as hydrogen donors for Mammalian s phase ribonucleotide reductase. J Biol Chem, 2009. 284(13): p. 8233-40.

Baker-Austin, C. & Dopson, M., Life in acid: pH Homeostasis in acidophiles. Trends Microbiol 15, 165-71 (2007).

Benner, S.A., Sassi, S.O. & Gaucher, E.A. Molecular paleoscience: systems biology from the past. Adv Enzymol Relat Areas Mol Biol 75, 1-132, xi (2007).

Berg, O.G. and C. Blomberg, Association Kinetics with Coupled Diffusion .3. Ionic-Strength Dependence of Lac Repressor-Operator Association. Biophysical Chemistry, 1978. 8(4): p. 271-280.

Berg, O.G., R.B. Winter, and P.H. Vonhippel, Diffusion-Driven Mechanisms of Protein Translocation on Nucleic-Acids .1. Models and Theory. Biochemistry, 1981. 20(24): p. 6929-6948.

Beynon, R.J. and J.S. Bond, Proteolytic enzymes: a practical approach. 2001, New York: Oxford University Press.

Boussau, B., Blanquart, S., Necsulea, A., Lartillot, N. & Gouy, M. Parallel adaptations to high temperatures in the Archaean eon. Nature 456, 942-5 (2008).

Capitani, G., Markovic-Housley, Z., DelVal, G., Morris, M., Jansonius, J.N. & Schurmann, P., Crystal structures of two functionally different thioredoxins in spinach chloroplasts.. J Mol Biol, 2000. 302: p. 135-154.

Carvalho, A.T., et al., Mechanism of thioredoxin-catalyzed disulfide reduction. Activation of the buried thiol and role of the variable active-site residues. J Phys Chem B, 2008. 112(8): p. 2511-23.

Cecconi, C., et al., Protein-DNA chimeras for single molecule mechanical folding studies with the optical tweezers. Eur Biophys J, 2008. 37(6): p. 729-38.

Chang, B.S., Jonsson, K., Kazmi, M.A., Donoghue, M.J. & Sakmar, T.P. Recreating a functional ancestral archosaur visual pigment. Mol Biol Evol 19, 1483-9 (2002).

Chivers, P.T. and R.T. Raines, General acid/base catalysis in the active site of Escherichia coli thioredoxin. Biochemistry, 1997. 36(50): p. 15810-6.

Chivers, P.T., M.C. Laboissiere, and R.T. Raines, The CXXC motif: imperatives for the formation of native disulfide bonds in the cell. EMBO J, 1996. 15(11): p. 2659-67.

Cipriano, D.J. and S.D. Dunn, Tethering polypeptides through bifunctional PEG cross-linking agents to probe protein function: application to ATP synthase. Proteins, 2008. 73(2): p. 458-67.

Corey, D.R., Synthesis of oligonucleotide-peptide and oligonucleotide-protein conjugates. Methods Mol Biol, 2004. 283: p. 197-206.

Crankshaw, M.W. and G.A. Grant, Modification of cysteine. Curr Protoc Protein Sci, 2001. Chapter 15: p. Unit15 1.

del Rio, A., et al., Stretching single talin rod molecules activates vinculin binding. Science, 2009. 323(5914): p. 638-41.

Dyson, H.J. et al., Effects of buried charged groups on cysteine thiol ionization and reactivity in Escherichia coli thioredoxin: structural and functional characterization of mutants of Asp 26 and Lys 57. Biochemistry 36, 2622-36 (1997).

Edgar, R.C. MUSCLE: multiple sequence alignment with high accuracy and high throughput. Nucleic Acids Res 32, 1792-7 (2004).

Fernandez, J.M. & Li, H. Force-clamp spectroscopy monitors the folding trajectory of a single protein. Science 303, 1674-8 (2004).

Florin, E.L. et al. Sensing Specific Molecular-Interactions with the Atomic-Force Microscope. Biosensors & Bioelectronics 10, 895-901 (1995).

Frey, P.A. and A.D. Hegeman, Enzymatic reaction mechanisms. 2007, Oxford: Oxford University Press.

Garcia-Manyes, S., et al., Direct observation of an ensemble of stable collapsed states in the mechanical folding of ubiquitin. Proceedings of the National Academy of Sciences of the United States of America, 2009. 106(26): p. 10534-10539.

Garcia-Manyes, S., L. Dougan, and J.M. Fernandez, Osmolyte-induced separation of the mechanical folding phases of ubiquitin. Proceedings of the National Academy of Sciences of the United States of America, 2009. 106(26): p. 10540-10545.

Garcia-Manyes, S., Liang, J., Szoszkiewicz, R., Kuo, T.L. & Fernandez, J.M. Force-activated reactivity switch in a bimolecular chemical reaction. Nature Chemistry 1, 236-242 (2009).

Gaucher, E.A., et al., Inferring the palaeoenvironment of ancient bacteria on the basis of resurrected proteins. Nature, 2003. 425(6955): p. 285-8.

Gaucher, E.A., Govindarajan, S. & Ganesh, O.K. Palaeotemperature trend for Precambrian life inferred from resurrected proteins. Nature 451, 704-7 (2008).

Gaucher, E.A., Thomson, J.M., Burgan, M.F. & Benner, S.A. Inferring the palaeoenvironment of ancient bacteria on the basis of resurrected proteins. Nature 425, 285-8 (2003).

Godoy-Ruiz, R. et al. Natural selection for kinetic stability is a likely origin of correlations between mutational effects on protein energetics and frequencies of amino acid occurrences in sequence alignments. J Mol Biol 362, 966-78 (2006).

Gogarten-Boekels, M. Hilario, E. & Gogarten J.P., The effects of heavy meteorite bombardment on the early evolution-the emergence fo the three domains of life. Orig. Life Evol. Biosph., 25: 251-264 (1995).

Gorman, J., et al., Dynamic basis for one-dimensional DNA scanning by the mismatch repair complex Msh2-Msh6. Mol Cell, 2007. 28(3): p. 359-70.

Green, N.S., E. Reisler, and K.N. Houk, Quantitative evaluation of the lengths of homobifunctional protein cross-linking reagents used as molecular rulers. Protein Sci, 2001. 10(7): p. 1293-304.

Halford, S.E. and J.F. Marko, How do site-specific DNA-binding proteins find their targets? Nucleic Acids Res, 2004. 32(10): p. 3040-52.

Hall, B.G. Simple and accurate estimation of ancestral protein sequences. Proc Natl Acad Sci U S A 103, 5431-6 (2006).

Hedges, S.B. & Kumar, S. The Timetree of Life, xxi, 551 p. (Oxford University Press, Oxford, 2009).

Holmgren, A. Thioredoxin. Annu Rev Biochem 54, 237-71 (1985).

Holmgren, A., Reduction of disulfides by thioredoxin. Exceptional reactivity of insulin and suggested functions of thioredoxin in mechanism of hormone action. J Biol Chem, 1979. 254(18): p. 9113-9.

Holmgren, A., Thioredoxin and glutaredoxin systems. J Biol Chem, 1989. 264(24): p. 13963-6.

Holmgren, A., Thioredoxin. Annu Rev Biochem, 1985. 54: p. 237-71.

Holmgren, A., Tryptophan fluorescence study of conformational transitions of the oxidized and reduced form of thioredoxin. J Biol Chem, 1972. 247(7): p. 1992-8.

Holmgren, A., Thioredoxin catalyzes the reduction of insulin disulfides by dithiothreitol and dihydrolipoamide. J Biol Chem 254, 9627-32 (1979)

Ibarra-Molero, B., Loladze, V.V., Makhatadze, G.I. & Sanchez-Ruiz, J.M. Thermal versus guanidine-induced unfolding of ubiquitin. An analysis in terms of the contributions from charge-charge interactions to protein stability. Biochemistry 38, 8138-49 (1999).

Ji, T.H., Bifunctional reagents. Methods Enzymol, 1983. 91: p. 580-609.

Katti, S.K., D.M. LeMaster, and H. Eklund, Crystal structure of thioredoxin from Escherichia coli at 1.68 A resolution. J Mol Biol, 1990. 212(1): p. 167-84.

Kaganman, I., Resurrected Enzymes, Research Highlights, Nature Methods, 8:452 (2011).

Kice, J.L. Nucleophilic Substitution at Different Oxidation States of Sulfur. in Progress in Inorganic Chemistry (ed. Edwards, J.O.) 147-206 (2007).

Knauth, L.P. & Lowe, D.R. High Archean climatic temperature inferred from oxygen isotope geochemistry of cherts in the 3.5 Ga Swaziland Supergroup, South Africa. Geol. Soc. Am. Bull. 115, 566-580 (2003).

Koti Ainavarapu, S.R., Wiita, A.P., Dougan, L., Uggerud, E. & Fernandez, J.M. Single-molecule force spectroscopy measurements of bond elongation during a bimolecular reaction. J Am Chem Soc 130, 6479-87 (2008).

Kumar, J.K., S. Tabor, and C.C. Richardson, Proteomic analysis of thioredoxin-targeted proteins in Escherichia coli. Proc Natl Acad Sci U S A, 2004. 101(11): p. 3759-64.

LaMantia, M.L. and W.J. Lennarz, The essential function of yeast protein disulfide isomerase does not reside in its isomerase activity. Cell, 1993. 74(5): p. 899-908.

Lancelin, J.M., Guilhaudis, L., Krimm, I., Blackledge, M.J., Marion, D. & Jacquot, J.P., NMR structures of thioredoxin m from the green alga Chlamydomonas reinhardtii. Proteins 2000. 41: p. 334-349

Liang, J. & Fernandez, J.M. Mechanochemistry: One Bond at a Time. ACS Nano (2009).

Liberles, D.A. Ancestral sequence reconstruction, xiii, 252 p. (Oxford University Press, Oxford; New York, 2007).

Light, A. and H. Janska, Enterokinase (enteropeptidase): comparative aspects. Trends Biochem Sci, 1989. 14(3): p. 110-2.

Lillig, C.H. and A. Holmgren, Thioredoxin and related molecules--from biology to health and disease. Antioxid Redox Signal, 2007. 9(1): p. 25-47.

Liu, R.C., et al., Mechanical Characterization of Protein L in the Low-Force Regime by Electromagnetic Tweezers/Evanescent Nanometry. Biophysical Journal, 2009. 96(9): p. 3810-3821.

Lopez-Otin, C. and J.S. Bond, Proteases: multifunctional enzymes in life and disease. J Biol Chem, 2008. 283(45): p. 30433-7.

Lu, D., et al., Crystal structure of enteropeptidase light chain complexed with an analog of the trypsinogen activation peptide. J Mol Biol, 1999. 292(2): p. 361-73.

Maeda, K., et al., Structural basis for target protein recognition by the protein disulfide reductase thioredoxin. Structure, 2006. 14(11): p. 1701-7.

Martin, J.L., Thioredoxin--a fold for all reasons. Structure, 1995. 3(3): p. 245-50.

Matthias, L.J. and P.J. Hogg, Redox control on the cell surface: implications for HIV-1 entry. Antioxid Redox Signal, 2003. 5(1): p. 133-8.

Matthias, L.J., et al., Disulfide exchange in domain 2 of CD4 is required for entry of HIV-1. Nat Immunol, 2002. 3(8): p. 727-32.

Menzel, U. & Gottschalk, G., The internal pH of Acetobacterium wieringae and Acetobacter aceti during growth and production of acetic acid. Archives of Microbiology 143, 47-51 (1985).

Milanesi, L., et al., A method for the reversible trapping of proteins in non-native conformations. Biochemistry, 2008. 47(51): p. 13620-34.

Ming, H. et al., Crystal structure of thioredoxin domain of ST2123 from thermophilic archaea Sulfolobus tokodaii strain7. Proteins 69, 204-8 (2007)

Mustacich, D. and G. Powis, Thioredoxin reductase. Biochem J, 2000. 346 Pt 1: p. 1-8.

Nelder, J.A. & Mead, R. A Simplex-Method for Function Minimization. Computer Journal 7, 308-313 (1965).

Nisbet, E.G. & Sleep, N.H. The habitat and nature of early life. Nature 409, 1083-91 (2001).

Peregrin-Alvarez, J.M., Tsoka, S. & Ouzounis, C.A. The phylogenetic extent of metabolic enzymes and pathways. Genome Res 13, 422-7 (2003).

Perez-Jimenez, R. et al. Diversity of chemical mechanisms in thioredoxin catalysis revealed by single-molecule force spectroscopy. Nat Struct Mol Biol 16, 890-6 (2009).

Perez-Jimenez, R. et al. Force-clamp spectroscopy detects residue co-evoulation in enzyme catalysis. J Biol Chem., 283, 27121-27129 (2009).

Perez-Jimenez, R. et al. Single-molecule paleoenzymology probes the chemistry of resurrected enzymes., Nat Struct Mol Biol. 2011 May;18(5):592-6. Epub 2011 Apr 3.

Peterson, F.C., Lytle, B.L., Sampath, S., Vinarov, D., Tyler, E., Shahan, M., Markley, J.L., Volkman, B.F., Solution structure of thioredoxin h1 from Arabidopsis thaliana. Protein Sci. , 2005. 14: p. 2195-2200

Pollock, D.D. & Chang, B.S.W. in Ancestral sequence reconstruction, pages 85-94 (ed. Liberles. D.A., Oxford University Press, Oxford ; New York, 2007).

Powis, G. and W.R. Montfort, Properties and biological activities of thioredoxins. Annu Rev Biophys Biomol Struct, 2001. 30: p. 421-55.

Qin, J., Clore, G.M., Kennedy, W.M., Huth, J.R. & Gronenborn, A.M. , Solution structure of human thioredoxin in a mixed disulfide intermediate complex with its target peptide from the transcription factor NF kappa B. Structure, 1995. 3: p. 289-297

Qin, J., Clore, G.M., Kennedy, W.P., Kuszewski, J. & Gronenborn, A.M. , The solution structure of human thioredoxin complexed with its target from Ref-1 reveals peptide chain reversal. Structure 1996. 4: p. 613-620

Riggs, A.D., Bourgeoi.S, and M. Cohn, Lac Repressor-Operator Interaction .3. Kinetic Studies. Journal of Molecular Biology, 1970. 53(3): p. 401-&.

Russell, M.J. & Hall, A.J. The emergence of life from iron monosulphide bubbles at a submarine hydrothermal redox and pH front. J Geol Soc Lond 154, 377-402 (1997).

Sarkar, A., R.B. Robertson, and J.M. Fernandez, Simultaneous atomic force microscope and fluorescence measurements of protein unfolding using a calibrated evanescent wave. Proc Natl Acad Sci U S A, 2004. 101(35): p. 12882-6.

Schulte, M. The Emergence of Life on Earth. Oceanography 20, 42-49 (2007).

Smeets, A., Evrard, C., Landtmeters, M., Marchand, C., Knoops, B. & Declercq, J.P., Crystal structures of oxidized and reduced forms of human mitochondrial thioredoxin 2.. Protein Sci. , 2005. 14: p. 2610-2621

Stanford, N.P., et al., One- and three-dimensional pathways for proteins to reach specific DNA sites. Embo J, 2000. 19(23): p. 6546-57.

Starks, C.M., Francois, J.A., MacArthur, K.M., Heard, B.Z. & Kappock, T.J. Atomic-resolution crystal structure of thioredoxin from the acidophilic bacterium Acetobacter aceti. Protein Sci 16, 92-8 (2007)

Suarez, M. et al., Using multi-objective computational design to extend protein promiscuity. Biophys Chem 147, 13-9 (2010)

Tachibana, C. and T.H. Stevens, The yeast EUG1 gene encodes an endoplasmic reticulum protein that is functionally related to protein disulfide isomerase. Mol Cell Biol, 1992. 12(10): p. 4601-11.

Thomson, J.M. et al. Resurrecting ancestral alcohol dehydrogenases from yeast. Nat Genet 37, 630-5 (2005).

Thornton, J.W. Resurrecting ancient genes: experimental analysis of extinct molecules. Nat Rev Genet 5, 366-75 (2004).

Thornton, J.W., Need, E. & Crews, D. Resurrecting the ancestral steroid receptor: ancient origin of estrogen signaling. Science 301, 1714-7 (2003).

Turk, B., Targeting proteases: successes, failures and future prospects. Nat Rev Drug Discov, 2006. 5(9): p. 785-99.

von Hippel, P.H. and O.G. Berg, Facilitated target location in biological systems. J Biol Chem, 1989. 264(2): p. 675-8.

Walker, B. and J.F. Lynas, Strategies for the inhibition of serine proteases. Cell Mol Life Sci, 2001. 58(4): p. 596-624.

Walker, J.C.G. Possible Limits on the Composition of the Archean Ocean. Nature 302, 518-520 (1983).

Wiita, A.P. et al. Probing the chemistry of thioredoxin catalysis with force. Nature 450, 124-7 (2007).

Wiita, A.P., Ainavarapu, S.R., Huang, H.H. & Fernandez, J.M. Force-dependent chemical kinetics of disulfide bond reduction observed with single-molecule techniques. Proc Natl Acad Sci U S A 103, 7222-7 (2006).

Wiita, A.P., et al., Probing the chemistry of thioredoxin catalysis with force. Nature, 2007. 450(7166): p. 124-7.

Williams, C.H., et al., Thioredoxin reductase two modes of catalysis have evolved. Eur J Biochem, 2000. 267(20): p. 6110-7.

Windle, H.J., Fox, A., Ni Eidhin, D. & Kelleher, D., The thioredoxin system of Helicobacter pylori. J Biol Chem 275, 5081-9 (2000)

Wynn, R. and F.M. Richards, Chemical modification of protein thiols: formation of mixed disulfides. Methods Enzymol, 1995. 251: p. 351-6.

Xu, S.Z., et al., TRPC channel activation by extracellular thioredoxin. Nature, 2008. 451(7174): p. 69-72.

Yang, Z., Kumar, S. & Nei, M. A new method of inference of ancestral nucleotide and amino acid sequences. Genetics 141, 1641-50 (1995).

Yang, Z.H. PAML: a program package for phylogenetic analysis by maximum likelihood. Comput Appl Biosci 13, 555-556 (1997).

Zalatan, J.G. & Herschlag, D., The far reaches of enzymology. Nat. Chem. Biol., 5:516-520 (2009).

**Claims**

1. An isolated polypeptide having a sequence of SEQ ID NO: 1, or a variant of SEQ ID NO: 1 having at least about 75% identity to SEQ ID NO: 1, wherein the polypeptide has a rate constant for disulfide reduction at pH 5.0 that is greater than human thioredoxin.

2. The isolated polypeptide of claim 1, wherein the rate constant is measured by single molecule force-spectroscopy.

3. The isolated polypeptide of claim 1, wherein the sequence does not have 100% identity with any extant polypeptide.

4. The isolated polypeptide of claim 1, wherein the variant has at least about 85.5%, at least about 90.5%, at least about 92.5%, at least about 95%, about 96%, about 96.5%, about 97%, about 97.5%, about 98%, about 98.5%, about 99%, about 99.5% or about 99.9% amino acid sequence identity to SEQ ID NO: 1.

5. The isolated polypeptide of claim 1, wherein the polypeptide is labeled or wherein the polypeptide is chemically modified.

6. The isolated polypeptide of claim 1, wherein the polypeptide is labeled and the label is colorimetric, radioactive, chemiluminescent, or fluorescent.

7. The isolated polypeptide of claim 1, wherein the polypeptide is chemically modified, and the chemical modification comprises covalent modification of an amino acid, the covalent modification optionally comprises methylation, acetylation, phosphorylation, ubiquitination, sumoylation, citrullination, or ADP ribosylation.

8. An isolated antibody that specifically binds to a polypeptide of SEQ ID NO: 1.

9. An isolated nucleic acid comprising a nucleic acid sequence which encodes the polypeptide of claim 1, the sequence optionally optimized for expression in a mammalian expression system or in a bacterial expression system such as E. coli, the isolated nucleic acid being optionally operably linked to one or more control sequences that direct the production of the polypeptide in a suitable expression host.

10. A recombinant expression vector or recombinant host cell comprising the nucleic acid of claim 9.

11. A method for producing the polypeptide of claim 1, the method comprising cultivating a host cell comprising a nucleic acid construct comprising a polynucleotide encoding the polypeptide under conditions suitable for production of the polypeptide; and recovering the polypeptide.

**Patentansprüche**

1. Isoliertes Polypeptid, das eine Sequenz von SEQ ID NR.: 1 oder eine Variante von SEQ ID NR.: 1 mit mindestens etwa 75% Identität zu SEQ ID NR.: 1 aufweist, wobei das Polypeptid eine Geschwindigkeitskonstante zur Disulfid-Reduktion bei pH 5,0 aufweist, die größer als Human-Thioredoxin ist.

2. Isoliertes Polypeptid nach Anspruch 1, wobei die Geschwindigkeitskonstante durch Einzelmolekülkraftspektroskopie gemessen wird.

3. Isoliertes Polypeptid nach Anspruch 1, wobei die Sequenz keine 100% Identität mit beliebigem bestehendem Polypeptid aufweist.

4. Isoliertes Polypeptid nach Anspruch 1, wobei die Variante mindestens etwa 85,5%, mindestens etwa 90,5%, mindestens etwa 92,5%, mindestens etwa 95%, etwa 96%, etwa 96,5%, etwa 97%, etwa 97,5%, etwa 98%, etwa 98,5%, etwa 99%, etwa 99,5% oder etwa 99,9% Aminosäure-Sequenzidentität zu SEQ ID NR.: 1 aufweist.

5. Isoliertes Polypeptid nach Anspruch 1, wobei das Polypeptid markiert ist oder wobei das Polypeptid chemisch modifiziert ist.

6. Isoliertes Polypeptid nach Anspruch 1, wobei das Polypeptid markiert ist und die Markierung kolorimetrisch, radioaktiv, chemilumineszierend oder fluoreszierend ist.

7. Isoliertes Polypeptid nach Anspruch 1, wobei das Polypeptid chemisch modifiziert ist und die chemische Modifizierung eine kovalente Modifizierung einer Aminosäure umfasst, wobei die kovalente Modifizierung gegebenenfalls Methylierung, Acetylierung, Phosphorylierung, Ubiquitinierung, Sumoylierung, Citrullinierung oder ADP-Ribosylierung umfasst.

8. Isolierter Antikörper, der an ein Polypeptid von SEQ ID NR.: 1 spezifisch bindet.

9. Isolierte Nukleinsäure, umfassend eine Nukleinsäure-Sequenz, die für das Polypeptid nach Anspruch 1 kodiert, wobei die Sequenz gegebenenfalls zur Expression in einem Säuger-Expressionssystem oder in einem bakteriellen Expressionssystem, wie E. coli, optimiert ist, wobei die isolierte Nukleinsäure gegebenenfalls mit einer oder mehreren Kontroll-Sequenzen funktionsfähig verbunden ist, die die Erzeugung des Polypeptids in einem geeigneten Expressionswirt lenken.

10. Rekombinanter Expressionsvektor oder rekombinante Wirtszelle, umfassend die Nukleinsäure nach Anspruch 9.

11. Verfahren zur Herstellung des Polypeptids nach Anspruch 1, wobei das Verfahren Kultivieren einer Wirtszelle, umfassend ein Nukleinsäure-Konstrukt, umfassend ein Polynukleotid, das für das Polypeptid unter zur Erzeugung des Polypeptids geeigneten Bedingungen kodiert; und Gewinnen des Polypeptids umfasst.

**Revendications**

1. Polypeptide isolé ayant une séquence SEQ ID NO: 1, ou un variant de SEQ ID NO: 1 ayant au moins environ 75% d'identité avec SEQ ID NO: 1, dans lequel le polypeptide présente une constante de vitesse pour la réduction du disulfure à pH 5 qui est plus élevée que pour la thiorédoxine humaine.

2. Polypeptide isolé selon la revendication 1, dans lequel la constante de vitesse est mesurée par spectroscopie de force à une seule molécule.

3. Polypeptide isolé selon la revendication 1, dans lequel la séquence n'a pas une identité de 100% avec tout polypeptide existant.

4. Polypeptide isolé selon la revendication 1, dans lequel le variant a au moins environ 85,5%, au moins environ 90,5%, au moins environ 92,5%, au moins environ 95%, environ 96%, environ 96,5%, environ 97%, environ 97,5%, environ 98%, environ 98,5%, environ 99%, environ 99,5% ou environ 99,9% d'identité de séquence d'acides aminés avec SEQ ID NO: 1.

5. Polypeptide isolé selon la revendication 1, dans lequel le polypeptide est marqué ou dans lequel le polypeptide est chimiquement modifié.

6. Polypeptide isolé selon la revendication 1, dans lequel le polypeptide est marqué et le marqueur est colorimétrique, radioactif, chimioluminescent, ou fluorescent.

7. Polypeptide isolé selon la revendication 1, dans lequel le polypeptide est chimiquement modifié, et la modification chimique comprend une modification covalente d'un acide aminé, la modification covalente comprend optionnellement une méthylation, une acétylation, une phosphorylation, une ubiquitination, une sumoylation, une citrullination ou une ribosylation de l'ADP.

8. Anticorps isolé qui se lie spécifiquement à un polypeptide de SEQ ID NO: 1.

9. Acide nucléique isolé comprenant une séquence d'acide nucléique qui code pour le polypeptide de la revendication 1, la séquence est optionnellement optimisée pour l'expression dans un système d'expression de mammifère ou dans un système d'expression bactérien tel que E. coli, l'acide nucléique isolé étant lié optionnellement de manière fonctionnelle à une ou plusieurs séquences de contrôle qui dirigent la production du polypeptide dans un hôte d'expression approprié.

10. Vecteur d'expression recombinant ou cellule hôte recombinante comprenant l'acide nucléique selon la revendication 9.

11. Procédé pour produire le polypeptide selon la revendication 1, le procédé comprenant une culture d'une cellule hôte comprenant une construction d'acide nucléique comprenant un polynucléotide codant pour le polypeptide dans des conditions appropriées pour la production du polypeptide; et une récupération du polypeptide.

**FIGURE 1**

**A**

C32  groove

**B**

**FIGURE 2**

**FIGURE 3**

FIGURE 4

FIGURE 5

**FIGURE 6**

**FIGURE 7**

**FIGURE 8**

FIGURE 9

$$A_\sigma = \{\text{Ala, Val, Leu, Ile, Pro}\ldots\}$$

$$P(A_\sigma | A_a) = \frac{P(A_a | A_\sigma) P(A_\sigma)}{P(A_a)}$$

| | |
|---|---|
| LBCA Trx | ~MSVIEINDENFEEEVLKS-DKPVLVDFWAPWCGPCRMIAPIIEELAEEYEGKVK |
| LACA Trx | ~MSVVQLNDENFEDEVIKRN-NKVVVVDFWAEWCGPCRMIAPIIEELAKEYAGKVV |
| AECA Trx | ~MSVIEINDENFDEVIKRS-DKVVVVDFWAEWCGPCRMIAPIIEELAEEYAGKVV |
| LGPCA Trx | ~MSIIHVTDDSFDQDVLKA-DKPVLVDFWAEWCGPCRMIAPILDEIAEEYEGKLK |
| LECA Trx | ~MVIQVTNKEEFEAILSEA-DKLVVVDFFATWCGPCKMIAPFFEELSEEYPDKVV |
| LPBCA Trx | ~MSVIEVTDENFEQEVLKS-DKPVLVDFWAPWCGPCRMIAPIIEELAKEYEGKVK |
| LAFCA Trx | ~MVIQVTNKDEFESILSEA-DKLVVVDFTATWCGPCKMIAPKFEELSEEYPDNVV |
| E.coli Trx | SDKIIHLTDDSFDTDVLKA-DGAILVDFWAEWCGPCKMIAPILDEIADEYQGKLT |
| Human TRX | ~MVKQIESKTAFQEALDAAGDKLVVVDFSATWCGPCKMIKPFFHSLSEKYS-NVI |
| | ...  *:  :    :  :;*** * *****;** *  :.,:.,:* :: |

| | |
|---|---|
| LBCA Trx | FAKVNVDENPETAAKYGIMSIPTLLLFKNGEVVDKLVGARPKEALKERIEKHL~ |
| LACA Trx | FGKLNVDENPEIAAKYGIMSIPTLLFKNGKVVDQLVGAMPKEALKERIKKYL~ |
| AECA Trx | FGKVNVDENPEIAAKYGIMSIPTLLFKNGKVVDQLVGARPKEALKERIKKYL~ |
| LGPCA Trx | VAKVNIDENPETAAKYGIRGIPTIMLFKNGEVAATKVGALSKSQLKEFLDANL~ |
| LECA Trx | FIKVDVDEVPDVAAKYGITSMPTFKFFKNGKKVDELVGAN~QEKLKQMILKHAP |
| LPBCA Trx | VVKVNVDENPNTAAQYGIRSIPTLLLFKNGQVVDELVGAQPKEALKERIDKHL~ |
| LAFCA Trx | FLKVDVDEVEDVAARYGISAMPTFQFFKNGKKVDELTGAN~QEKLKAMIKKHAA |
| E.coli Trx | VAKVNIDQNPGTAPKYGIRGIPTLLLFKNGEVAATKVGALSKGQLKEFLDANLA |
| Human TRX | FLEVDVDDCQDVASECEVKCTPTFQFFKKGQKVGEFSGAN~KEKLEATINELV~ |
| | .  :::;*:     *,:  :   **;  ;**;*:  ,    **  :  *;  : |

**FIGURE 10**

**FIGURE 11**

FIGURE 12

**FIGURE 13**

**FIGURE 14**

**FIGURE 15**

**FIGURE 16**

**FIGURE 17**

**FIGURE 18**

EP 2 593 472 B1

**FIGURE 19**

**FIGURE 20**

**FIGURE 21**

**FIGURE 22**

**FIGURE 23**

**FIGURE 24**

**FIGURE 25**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 61364640 A
- US 1144084 W

- WO 0221139 A2

### Non-patent literature cited in the description

- **PEREZ- JIMENEZ et al.** *Nat Struct Mol Biol.,* 2011, vol. 18 (5), 592-6
- **PEREZ- JIMENEZ et al.** *Nat Struct Mol Biol,* 2009, vol. 16, 890-6
- **ALEGRE-CEBOLLADA et al.** *J Biol Chem,* 2010, vol. 285 (25), 18961-6
- **ZALATAN et al.** *Nat. Chem. Biol.,* 2009, vol. 5, 516-520
- **THORNTON.** *Science,* 2003, vol. 301, 1714-7
- **THOMSON et al.** *Nat Genet,* 2005, vol. 37, 630-5
- **BOUSSAU et al.** *Nature,* 2008, vol. 456, 942-5
- **CHANG et al.** *Mol Biol Evol,* 2002, vol. 19, 1483-9
- **BENNER et al.** *Adv Enzymol Relat Areas Mol Biol,* 2007, vol. 75 (xi), 1-132
- **THORNTON.** *Nat Rev Genet,* 2004, vol. 5, 366-75
- **GAUCHER et al.** *Nature,* 2003, vol. 425, 285-8
- **NISBET ; SLEEP.** *Nature,* 2001, vol. 409, 1083-91
- **HEDGES ; KUMAR.** The Timetree of life. Oxford University Press, 2009, vol. xxi, 551
- **PEREZ-JIMENEZ et al.** *Nat Struct Mol Biol,* 2009, vol. 16, 890-6
- **LIBERIES.** Ancestral sequence reconstruction. Oxford University Press, 2007, vol. xiii, 252
- **LIBERLES.** Ancestral sequence reconstruction. Oxford University Press, 2007, vol. xiii, 252
- **HALL.** *Proc Natl Acad Sci U S A,* 2006, vol. 103, 5431-6
- **POLLOCK ; CHANG.** Ancestral sequence reconstruction. Oxford University Press, 2007, 85-94
- **GAUCHER et al.** *Nature,* 2008, vol. 451, 704-7
- **GAUCHER et al.** *Nature,* 2008, vol. 451 (7179), 704-U2

- **GAUCHER et al.** *Nature,* 2003, vol. 425 (6955), 285-8
- **KNAUTH et al.** *Geo. Soc. Am. Bull.,* 2003, vol. 115, 566-580
- **SCHULTE, M.** *Oceanography,* 2007, vol. 20, 42-49
- **GOGARTEN-BOEKELS et al.** *Orig. Life Evol. Biosph.,* 1995, vol. 25, 251-264
- **PEREGRIN- ALVAREZ et al.** *Genome Res,* 2003, vol. 13, 422-7
- **EDGAR.** *Nucleic Acids Res,* 2004, vol. 32, 1792-7
- Current Protocols in Molecular Biology. 1987
- **SMITH ; WATERMAN.** *Adv. Appl. Math.,* 1981, vol. 2, 482
- **NEEDLEMAN ; WUNSCH.** *J. Mol. Biol.,* 1970, vol. 48, 443
- **PEARSON ; LIPMAN.** *Proc. Natl. Acad. Sci. USA,* 1988, vol. 85, 2444
- **FENG ; DOOLITTLE.** *J. Mol. Evol.,* 1987, vol. 35, 351-60
- **HIGGINS ; SHARP.** *Gene,* 1988, vol. 73, 237-44
- *CABIOS,* 1989, vol. 5, 151-53
- **AUSUBEL et al.** Current Protocols in Molecular Biology. John Wiley and Sons, 1996
- **CREIGHTON.** Proteins. W. H. Freeman and Company, 1984
- **LAI.** *Microbiol. Rev.,* 1992, vol. 56, 61-79
- **LEE et al.** *J. Virol.,* 1999, vol. 73, 11-18
- **GAO et al.** *J. Virol.,* 2005, vol. 79, 1154-1163
- **HIGGINS ; SHARP.** *Gene,* 1988, vol. 73, 237-244
- **LOGVINOFFET.** *Proc. Natl. Acad. Sci. USA,* 2004, vol. 101, 10149-10154
- **MINK et al.** *Virology,* 1994, vol. 200, 246-255
- **YANG.** *Comput Appl Biosci,* 1997, vol. 13, 555-556
- **HOLMGREN.** *Thioredoxin. Annu Rev Biochem,* 1985, vol. 54, 237-71
- **AINAVARAPU et al.** *Journal of the American Chemical Society,* 2008, vol. 130 (20), 6479-6487

- **WIITA, A.P. et al.** *Nature,* 2007, vol. 450 (7166), 124-7
- **WIITA et al.** *Nature,* 2007, vol. 450, 124-7
- **ARNER ; HOLMGREN.** *Eur J Biochem,* 2000, vol. 267 (20), 6102-9
- **KUMAR et al.** *Proc Natl Acad Sci USA,* 2004, vol. 101 (11), 3759-64
- **POWIS ; MONTFORT.** *Annu Rev Biophys Biomol Struct,* 2001, vol. 30, 421-55
- **LILLIG ; HOLMGREN.** *Antioxid Redox Signal,* 2007, vol. 9 (1), 25-47
- **XU et al.** TRPC channel activation by extracellular thioredoxin. *Nature,* 2008, vol. 451 (7174), 69-72
- **HOLMGREN, A.** Thioredoxin and glutaredoxin systems. *J Biol Chem,* 1989, vol. 264 (24), 13963-6
- **MATTHIAS et al.** *Nat Immunol,* 2002, vol. 3 (8), 727-32
- **MATTHIAS ; HOGG.** *Antioxid Redox Signal,* 2003, vol. 5 (1), 133-8
- **AVVAL ; HOLMGREN.** *J Biol Chem,* 2009, vol. 284 (13), 8233-40
- **CARVALHO et al.** *J Phys Chem B,* 2008, vol. 112 (8), 2511-23
- **CHIVERS ; RAINES.** *Biochemistry,* 1997, vol. 36 (50), 15810-6
- **WILLIAMS et al.** *Eur J Biochem,* 2000, vol. 267 (20), 6110-7
- **MUSTACICH ; POWIS.** *Biochem J,* 2000, vol. 346, 1-8
- **PEREZ-JIMENEZ et al.** *Nature Structural & Molecular Biology,* 2009, vol. 16 (8), 890-U120
- **CAPITANI et al.** *J Mol Biol,* 2000, vol. 302, 135-154
- **QIN et al.** *Structure,* 1995, vol. 3, 289-297
- **PETERSON et al.** *Protein Sci.,* 2005, vol. 14, 2195-2200
- **SMEETS et al.** *Protein Sci.,* 2005, vol. 14, 2610-2621
- **LANCELIN et al.** *Proteins,* 2000, vol. 41, 334-349
- **KATTI et al.** *J Mol Biol,* 1990, vol. 212 (1), 167-84
- **QIN et al.** *Structure,* 1996, vol. 4, 613-620
- **MAEDA et al.** *Structure,* 2006, vol. 14 (11), 1701-10
- **PEREZ- JIMENEZ et al.** *Nature Structural & Molecular Biology,* 2009, vol. 16 (8), 890-U120
- **BENOIST ; CHAMBON.** *Nature,* 1981, vol. 290, 304-10
- **YAMAMOTO et al.** *Cell,* 1980, vol. 22, 787-97
- **WAGNER et al.** *Proc. Natl. Acad. Sci. USA,* 1981, vol. 78, 1441-45
- **BRINSTER et al.** *Nature,* 1982, vol. 296, 39-42
- **VILLA-KOMAROFF et al.** *Proc. Natl. Acad. Sci. USA,* 1978, vol. 75, 3727-31
- **DEBOER et al.** *Proc. Natl. Acad. Sci. USA,* 1983, vol. 80, 21-25
- **GARDNER et al.** *Nucl. Acids Res.,* 1981, vol. 9, 2871-88
- **HERRERA-ESTRELLA et al.** *Nature,* 1984, vol. 310, 115-20
- **TYMMS.** In vitro Transcription and Translation Protocols: Methods in Molecular Biology. Garland Publishing, 1995, vol. 37
- **WALKER.** Protein Protocols on CD-ROM. Human Press, 1998
- **HUNKAPILLER et al.** *Nature,* 1984, vol. 310, 105-11
- **STEWART ; YOUNG.** Solid Phase Peptide Synthesis. Pierce Chemical Co, 1984
- **HOLMGREN.** *J Biol Chem,* 1979, vol. 254 (18), 9113-9
- **HOLMGREN.** *J Biol Chem,* 1979, vol. 254 (19), 9627-32
- **HOLMGREN.** Annu Rev Biochem. *Thioredoxin,* 1985, vol. 54, 237-71
- **HOLMGREN.** *J Biol Chem,* 1972, vol. 247 (7), 1992-8
- **SARKAR et al.** *Proc Natl Acad Sci USA,* 2004, vol. 101 (35), 12882-6
- **LIU et al.** *Biophysical Journal,* 2009, vol. 96 (9), 3810-3821
- **DEL RIO et al.** *Science,* 2009, vol. 323 (5914), 638-41
- **BEYNON ; BOND.** Proteolytic enzymes: a practical approach. Oxford University Press, 2001
- **LOPEZ-OTIN ; BOND.** *J Biol Chem,* 2008, vol. 283 (45), 30433-7
- **TURK.** *Nat Rev Drug Discov,* 2006, vol. 5 (9), 785-99
- **WALKER ; LYNAS.** *Cell Mol Life Sci,* 2001, vol. 58 (4), 596-624
- **ADAM ; DELBRUCK.** Structural Chemistry and Molecular Biology. W. H. Freeman and Co, 1968, 198-215
- **HIPPEL ; BERG.** *J Biol Chem,* 1989, vol. 264 (2), 675-8
- **GORMAN et al.** *Mol Cell,* 2007, vol. 28 (3), 359-70
- **STANFORD et al.** *Embo J,* 2000, vol. 19 (23), 6546-57
- **RIGGS et al.** Lac Repressor-Operator Interaction .3. Kinetic Studies. *Journal of Molecular Biology,* 1970, vol. 53 (3), 401-7
- **HALFORD et al.** *Nucleic Acids Res,* 2004, vol. 32 (10), 3040-52
- **BERG ; BLOMBERG.** *Biophysical Chemistry,* 1978, vol. 8 (4), 271-280

- **BERG et al.** *Biochemistry,* 1981, vol. 20 (24), 6929-6948
- **STANFORD et al.** *Embo Journal,* 2000, vol. 19 (23), 6546-6557
- **HALFORD et al.** *Nucleic Acids Research,* 2004, vol. 32 (10), 3040-3052
- **CREIGHTON.** Protein Structure and Molecular Properties. W. H. Freeman and Company, 1993
- **WOLD.** Posttranslational Covalent Modification of Proteins. Academic Press, 1983, 1-12
- **SEIFTER et al.** *Meth. Enzymol.,* 1990, vol. 182, 626-646
- **RATTAN et al.** *Ann. N. Y Acad. Sci.,* 1992, vol. 663, 48-62
- Posttranslational Modifications. **KRISHNA, R. G. ; F. WOLD.** Proteins--Analysis and Design. Academic Press, 1998, vol. 1, 121-206
- Methods in Enzymo logy. 1990, vol. 193, 647-660
- Post-translational modifications of proteins. **R. G. KRISHNA ; F. WOLD.** Methods in Protein Sequence Analysis. Plenum Press, 1993, 167-172
- **COOPER et al.** GlycoSuiteDB: a new curated relational database of glycoprotein glycan structures and their biological sources. *Nucleic Acids Res.,* 2001, vol. 29, 332-335
- **GUPTA et al.** O-GLYCBASE version 4.0: a revised database of O-glycosylated proteins. *Nucleic Acids Research,* 1999, vol. 27, 370-372
- **KREEGIPUU et al.** PhosphoBase, a database of phosphorylation sites: release 2.O. *Nucleic Acids Res,* 1999, vol. 27 (1), 237-239
- **LEWIS et al.** *Nucl. Acids Res.,* 1990, vol. 18, 3439
- **BOHNSACK et al.** *Meth. Mol. Biol.,* 1996, vol. 57, 1
- **VAVRA et al.** *Promega Notes,* 1996, vol. 58, 30
- **DENG et al.** *Anal. Biochem.,* 1992, vol. 200, 81
- **KUNKEL et al.** *Proc. Natl. Acad. Sci. USA,* 1985, vol. 82, 488
- **KUNKE et al.** *Meth. Enzymol.,* 1987, vol. 154, 367
- **TAYLOR et al.** *Nucl. Acids Res.,* 1985, vol. 13, 8764
- **NAKAMAYE et al.** *Nucl. Acids Res.,* 1986, vol. 14, 9679
- **HIGUCHI et al.** *Nucl. Acids Res.,* 1988, vol. 16, 7351
- **SHIMADA et al.** *Meth. Mol. Biol.,* 1996, vol. 57, 157
- **HO et al.** *Gene,* 1989, vol. 77, 51
- **HORTON et al.** *Gene,* 1989, vol. 77, 61
- **SARKAR et al.** *BioTechniques,* 1990, vol. 8, 404
- **SAMBROOK et al.** Molecular Cloning: A Laboratory Manual. Cold Spring Harbor Laboratory, 2001

- **YANG et al.** *Genetics,* 1995, vol. 141, 1641-50
- **KNAUTH ; LOWE.** *Geol. Soc. Am. Bull.,* 2003, vol. 115, 566-580
- **SCHULTE, M.** The Emergence of Life on Earth. *Oceanography,* 2007, vol. 20, 42-49
- **GODOY-RUIZ et al.** *J Mol Biol,* 2006, vol. 362, 966-78
- **KICE et al.** Progress in Inorganic Chemistry. 2007, 147-206
- **WIITA et al.** *Proc Natl Acad Sci U S A,* 2006, vol. 103, 7222-7
- **KOTI AINAVARAPU et al.** *J Am Chem Soc,* 2008, vol. 130, 6479-87
- **GARCIA-MANYES et al.** *Nature Chemistry,* 2009, vol. 1, 236-242
- **LIANG ; FERNANDEZ.** Mechanochemistry: One Bond at a Time. *ACS Nano,* 2009
- **AINAVARAPU et al.** *J Am Chem Soc,* 2008, vol. 130, 436-7
- **WALKER.** *Nature,* 1983, vol. 302, 518-520
- **RUSSELL ; HALL.** *J Geol Soc Lond,* 1997, vol. 154, 377-402
- **DYSON, H.J. et al.** *Biochemistry,* 1997, vol. 36, 2622-36
- **IBARRA-MOLERO et al.** *Biochemistry,* 1999, vol. 38, 8138-49
- **FERNANDEZ ; LI.** *Science,* 2004, vol. 303, 1674-8
- **FLORIN et al.** *Biosensors & Bioelectronics,* 1995, vol. 10, 895-901
- **NELDER ; MEAD.** *Computer Journal,* 1965, vol. 7, 308-313
- **FREY ; HEGEMAN.** Enzymatic reaction mechanisms. Oxford University Press, 2007
- **WYNN et al.** *Methods Enzymol,* 1995, vol. 251, 351-6
- **CRANKSHAW ; GRANT.** Curr Protoc Protein Sci. 2001
- **COREY.** *Methods Mol Biol,* 2004, vol. 283, 197-206
- **JI.** *Methods Enzymol,* 1983, vol. 91, 580-609
- **CECCONI et al.** *Eur Biophys J,* 2008, vol. 37 (6), 729-38
- **GREEN et al.** *Protein Sci,* 2001, vol. 10 (7), 1293-304
- **MILANESI et al.** *Biochemistry,* 2008, vol. 47 (51), 13620-34
- **CIPRIANO et al.** *Proteins,* 2008, vol. 73 (2), 458-67
- **LIGHT ; H. JANSKA.** *Trends Biochem Sci,* 1989, vol. 14 (3), 110-2
- **LU et al.** *J Mol Biol,* 1999, vol. 292 (2), 361-73
- **WIITA et al.** *Proc Natl Acad Sci U S A,* 2006, vol. 103 (19), 7222-7
- **MARTIN.** *Structure,* 1995, vol. 3 (3), 245-50
- **CHIVERS et al.** *EMBO J,* 1996, vol. 15 (11), 2659-67

- **TACHIBANA et al.** *Mol Cell Biol,* 1992, vol. 12 (10), 4601-11
- **LAMANTIA et al.** *Cell,* 1993, vol. 74 (5), 899-908
- **GARCIA-MANYES et al.** *PNAS,* 2009, vol. 106 (26), 10534-10539
- **GARCIA-MANYES et al.** *PNAS,* 2009, vol. 106 (26), 10540-10545
- **HOLMGREN et al.** *J Biol Chem,* 1979, vol. 254, 9113-9
- **SUAREZ, M. et al.** *Biophys Chem,* 2010, vol. 147, 13-9
- **HOLMGREN, A.** *J Biol Chem,* 1979, vol. 254, 9627-32
- **XU, S.Z. et al.** *Nature,* 2008, vol. 451, 69-72
- **WINDLE, H.J. ; FOX, A. ; NI EIDHIN, D. ; KELLE-HER, D.** *J Biol Chem,* 2000, vol. 275, 5081-9
- **MING, H. et al.** *Proteins,* 2007, vol. 69, 204-8
- **BAKER-AUSTIN, C. ; DOPSON, M.** *Trends Microbiol,* 2007, vol. 15, 165-71
- **STARKS, C.M. ; FRANCOIS, J.A. ; MACARTHUR, K.M. ; HEARD, B.Z. ; KAPPOCK, T.J.** *Protein Sci,* 2007, vol. 16, 92-8
- **MENZEL, U. ; GOTTSCHALK, G.** *Archives of Microbiology,* 1985, vol. 143, 47-51
- **PEREZ-JIMENEZ et al.** *J. Biol. Chem.,* 2008, vol. 283, 27121-27129
- **ADAM, G. ; M. DELBRUCK.** Structural Chemistry and Molecular Biology. W. H. Freeman and Co, 1968, 198-215
- **AINAVARAPU, S.R. ; WIITA, A.P. ; HUANG, H.H. ; FERNANDEZ, J.M.** A single-molecule assay to directly identify solvent-accessible disulfide bonds and probe their effect on protein folding. *J Am Chem Soc,* 2008, vol. 130, 436-7
- **ALEGRE-CEBOLLADA J ; PEREZ-JIMENEZ R ; KOSURI P ; FERNANDEZ JM.** Single-molecule force spectroscopy approach to enzyme catalysis. *J Biol Chem,* 2010, vol. 285 (25), 18961-6
- **ARNER, E.S. ; A. HOLMGREN.** Physiological functions of thioredoxin and thioredoxin reductase. *Eur J Biochem,* 2000, vol. 267 (20), 6102-9
- **AVVAL, F.Z. ; A. HOLMGREN.** Molecular mechanisms of thioredoxin and glutaredoxin as hydrogen donors for Mammalian s phase ribonucleotide reductase. *J Biol Chem,* 2009, vol. 284 (13), 8233-40
- **BAKER-AUSTIN, C. ; DOPSON, M.** Life in acid: pH Homeostasis in acidophiles. *Trends Microbiol,* 2007, vol. 15, 165-71
- **BENNER, S.A. ; SASSI, S.O. ; GAUCHER, E.A.** Molecular paleoscience: systems biology from the past. *Adv Enzymol Relat Areas Mol Biol,* 2007, vol. 75 (xi), 1-132
- **BERG, O.G. ; C. BLOMBERG.** Association Kinetics with Coupled Diffusion .3. Ionic-Strength Dependence of Lac Repressor-Operator Association. *Biophysical Chemistry,* 1978, vol. 8 (4), 271-280
- **BERG, O.G. ; R.B. WINTER ; P.H. VONHIPPEL.** Diffusion-Driven Mechanisms of Protein Translocation on Nucleic-Acids .1. Models and Theory. *Biochemistry,* 1981, vol. 20 (24), 6929-6948
- **BEYNON, R.J. ; J.S. BOND.** Proteolytic enzymes: a practical approach. Oxford University Press, 2001
- **BOUSSAU, B. ; BLANQUART, S. ; NECSULEA, A. ; LARTILLOT, N. ; GOUY, M.** Parallel adaptations to high temperatures in the Archaean eon. *Nature,* 2008, vol. 456, 942-5
- **CAPITANI, G. ; MARKOVIC-HOUSLEY, Z. ; DEL-VAL, G. ; MORRIS, M. ; JANSONIUS, J.N. ; SCHURMANN, P.** Crystal structures of two functionally different thioredoxins in spinach chloroplasts. *J Mol Biol,* 2000, vol. 302, 135-154
- **CARVALHO, A.T. et al.** Mechanism of thioredoxin-catalyzed disulfide reduction. Activation of the buried thiol and role of the variable active-site residues. *J Phys Chem B,* 2008, vol. 112 (8), 2511-23
- **CECCONI, C. et al.** Protein-DNA chimeras for single molecule mechanical folding studies with the optical tweezers. *Eur Biophys J,* 2008, vol. 37 (6), 729-38
- **CHANG, B.S. ; JONSSON, K. ; KAZMI, M.A. ; DONOGHUE, M.J. ; SAKMAR, T.P.** Recreating a functional ancestral archosaur visual pigment. *Mol Biol Evol,* 2002, vol. 19, 1483-9
- **CHIVERS, P.T. ; R.T. RAINES.** General acid/base catalysis in the active site of Escherichia coli thioredoxin. *Biochemistry,* 1997, vol. 36 (50), 15810-6
- **CHIVERS, P.T. ; M.C. LABOISSIERE ; R.T. RAINES.** The CXXC motif: imperatives for the formation of native disulfide bonds in the cell. *EMBO J,* 1996, vol. 15 (11), 2659-67
- **CIPRIANO, D.J. ; S.D. DUNN.** Tethering polypeptides through bifunctional PEG cross-linking agents to probe protein function: application to ATP synthase. *Proteins,* 2008, vol. 73 (2), 458-67
- **COREY, D.R.** Synthesis of oligonucleotide-peptide and oligonucleotide-protein conjugates. *Methods Mol Biol,* 2004, vol. 283, 197-206
- Modification of cysteine. **CRANKSHAW, M.W. ; G.A. GRANT.** Curr Protoc Protein Sci. 2001
- **DEL RIO, A. et al.** Stretching single talin rod molecules activates vinculin binding. *Science,* 2009, vol. 323 (5914), 638-41
- **DYSON, H.J. et al.** Effects of buried charged groups on cysteine thiol ionization and reactivity in Escherichia coli thioredoxin: structural and functional characterization of mutants of Asp 26 and Lys 57. *Biochemistry,* 1997, vol. 36, 2622-36
- **EDGAR, R.C.** MUSCLE: multiple sequence alignment with high accuracy and high throughput. *Nucleic Acids Res,* 2004, vol. 32, 1792-7

- **FERNANDEZ, J.M. ; LI, H.** Force-clamp spectroscopy monitors the folding trajectory of a single protein. *Science,* 2004, vol. 303, 1674-8
- **FLORIN, E.L. et al.** Sensing Specific Molecular-Interactions with the Atomic-Force Microscope. *Biosensors & Bioelectronics,* 1995, vol. 10, 895-901
- **FREY, P.A. ; A.D. HEGEMAN.** Enzymatic reaction mechanisms. Oxford University Press, 2007
- **GARCIA-MANYES, S. et al.** Direct observation of an ensemble of stable collapsed states in the mechanical folding of ubiquitin. *Proceedings of the National Academy of Sciences of the United States of America,* 2009, vol. 106 (26), 10534-10539
- **GARCIA-MANYES, S. ; L. DOUGAN ; J.M. FERNANDEZ.** Osmolyte-induced separation of the mechanical folding phases of ubiquitin. *Proceedings of the National Academy of Sciences of the United States of America,* 2009, vol. 106 (26), 10540-10545
- **GARCIA-MANYES, S. ; LIANG, J. ; SZOSZKIEWICZ, R. ; KUO, T.L. ; FERNANDEZ, J.M.** Force-activated reactivity switch in a bimolecular chemical reaction. *Nature Chemistry,* 2009, vol. 1, 236-242
- **GAUCHER, E.A. et al.** Inferring the palaeoenvironment of ancient bacteria on the basis of resurrected proteins. *Nature,* 2003, vol. 425 (6955), 285-8
- **GAUCHER, E.A. ; GOVINDARAJAN, S. ; GANESH, O.K.** Palaeotemperature trend for Precambrian life inferred from resurrected proteins. *Nature,* 2008, vol. 451, 704-7
- **GAUCHER, E.A. ; THOMSON, J.M. ; BURGAN, M.F. ; BENNER, S.A.** Inferring the palaeoenvironment of ancient bacteria on the basis of resurrected proteins. *Nature,* 2003, vol. 425, 285-8
- **GODOY-RUIZ, R. et al.** Natural selection for kinetic stability is a likely origin of correlations between mutational effects on protein energetics and frequencies of amino acid occurrences in sequence alignments. *J Mol Biol,* 2006, vol. 362, 966-78
- **GOGARTEN-BOEKELS, M. ; HILARIO, E. ; GOGARTEN J.P.** The effects of heavy meteorite bombardment on the early evolution-the emergence fo the three domains of life. *Orig. Life Evol. Biosph.,* 1995, vol. 25, 251-264
- **GORMAN, J. et al.** Dynamic basis for one-dimensional DNA scanning by the mismatch repair complex Msh2-Msh6. *Mol Cell,* 2007, vol. 28 (3), 359-70
- **GREEN, N.S. ; E. REISLER ; K.N. HOUK.** Quantitative evaluation of the lengths of homobifunctional protein cross-linking reagents used as molecular rulers. *Protein Sci,* 2001, vol. 10 (7), 1293-304
- **HALFORD, S.E. ; J.F. MARKO.** How do site-specific DNA-binding proteins find their targets?. *Nucleic Acids Res,* 2004, vol. 32 (10), 3040-52
- **HALL, B.G.** Simple and accurate estimation of ancestral protein sequences. *Proc Natl Acad Sci U S A,* 2006, vol. 103, 5431-6
- **HEDGES, S.B. ; KUMAR, S.** The Timetree of Life. Oxford University Press, 2009, vol. xxi, 551
- **HOLMGREN, A.** Annu Rev Biochem. *Thioredoxin,* 1985, vol. 54, 237-71
- **HOLMGREN, A.** Reduction of disulfides by thioredoxin. Exceptional reactivity of insulin and suggested functions of thioredoxin in mechanism of hormone action. *J Biol Chem,* 1979, vol. 254 (18), 9113-9
- **HOLMGREN, A.** *Thioredoxin. Annu Rev Biochem,* 1985, vol. 54, 237-71
- **HOLMGREN, A.** Tryptophan fluorescence study of conformational transitions of the oxidized and reduced form of thioredoxin. *J Biol Chem,* 1972, vol. 247 (7), 1992-8
- **HOLMGREN, A.** Thioredoxin catalyzes the reduction of insulin disulfides by dithiothreitol and dihydrolipoamide. *J Biol Chem,* 1979, vol. 254, 9627-32
- **IBARRA-MOLERO, B. ; LOLADZE, V.V. ; MAKHATADZE, G.I. ; SANCHEZ-RUIZ, J.M.** Thermal versus guanidine-induced unfolding of ubiquitin. An analysis in terms of the contributions from charge-charge interactions to protein stability. *Biochemistry,* 1999, vol. 38, 8138-49
- **JI, T.H.** Bifunctional reagents. *Methods Enzymol,* 1983, vol. 91, 580-609
- **KATTI, S.K. ; D.M. LEMASTER ; H. EKLUND.** Crystal structure of thioredoxin from Escherichia coli at 1.68 A resolution. *J Mol Biol,* 1990, vol. 212 (1), 167-84
- **KAGANMAN, I.** Resurrected Enzymes, Research Highlights. *Nature Methods,* 2011, vol. 8, 452
- Nucleophilic Substitution at Different Oxidation States of Sulfur. **KICE, J.L.** Progress in Inorganic Chemistry. 2007, 147-206
- **KNAUTH, L.P. ; LOWE, D.R.** High Archean climatic temperature inferred from oxygen isotope geochemistry of cherts in the 3.5 Ga Swaziland Supergroup. *South Africa. Geol. Soc. Am. Bull.,* 2003, vol. 115, 566-580
- **KOTI AINAVARAPU, S.R. ; WIITA, A.P. ; DOUGAN, L. ; UGGERUD, E. ; FERNANDEZ, J.M.** Single-molecule force spectroscopy measurements of bond elongation during a bimolecular reaction. *J Am Chem Soc,* 2008, vol. 130, 6479-87
- **KUMAR, J.K. ; S. TABOR ; C.C. RICHARDSON.** Proteomic analysis of thioredoxin-targeted proteins in Escherichia coli. *Proc Natl Acad Sci U S A,* 2004, vol. 101 (11), 3759-64
- **LAMANTIA, M.L. ; W.J. LENNARZ.** The essential function of yeast protein disulfide isomerase does not reside in its isomerase activity. *Cell,* 1993, vol. 74 (5), 899-908

- **LANCELIN, J.M. ; GUILHAUDIS, L. ; KRIMM, I. ; BLACKLEDGE, M.J. ; MARION, D. ; JACQUOT, J.P.** NMR structures of thioredoxin m from the green alga Chlamydomonas reinhardtii. *Proteins,* 2000, vol. 41, 334-349
- **LIANG, J. ; FERNANDEZ, J.M.** Mechanochemistry: One Bond at a Time. *ACS Nano,* 2009
- **LIBERLES, D.A.** Ancestral sequence reconstruction. Oxford University Press, 2007, vol. xiii, 252
- **LIGHT, A. ; H. JANSKA.** Enterokinase (enteropeptidase): comparative aspects. *Trends Biochem Sci,* 1989, vol. 14 (3), 110-2
- **LILLIG, C.H. ; A. HOLMGREN.** Thioredoxin and related molecules--from biology to health and disease. *Antioxid Redox Signal,* 2007, vol. 9 (1), 25-47
- **LIU, R.C. et al.** Mechanical Characterization of Protein L in the Low-Force Regime by Electromagnetic Tweezers/Evanescent Nanometry. *Biophysical Journal,* 2009, vol. 96 (9), 3810-3821
- **LOPEZ-OTIN, C. ; J.S. BOND.** Proteases: multifunctional enzymes in life and disease. *J Biol Chem,* 2008, vol. 283 (45), 30433-7
- **LU, D. et al.** Crystal structure of enteropeptidase light chain complexed with an analog of the trypsinogen activation peptide. *J Mol Biol,* 1999, vol. 292 (2), 361-73
- **MAEDA, K. et al.** Structural basis for target protein recognition by the protein disulfide reductase thioredoxin. *Structure,* 2006, vol. 14 (11), 1701-7
- **MARTIN, J.L.** Thioredoxin--a fold for all reasons. *Structure,* 1995, vol. 3 (3), 245-50
- **MATTHIAS, L.J. ; P.J. HOGG.** Redox control on the cell surface: implications for HIV-1 entry. *Antioxid Redox Signal,* 2003, vol. 5 (1), 133-8
- **MATTHIAS, L.J. et al.** Disulfide exchange in domain 2 of CD4 is required for entry of HIV-1. *Nat Immunol,* 2002, vol. 3 (8), 727-32
- **MENZEL, U. ; GOTTSCHALK, G.** The internal pH of Acetobaceterium wieringae and Acetobacter aceti during growth and production of acetic acid. *Archives of Microbiology,* 1985, vol. 143, 47-51
- **MILANESI, L. et al.** A method for the reversible trapping of proteins in non-native conformations. *Biochemistry,* 2008, vol. 47 (51), 13620-34
- **MING, H. et al.** Crystal structure of thioredoxin domain of ST2123 from thermophilic archaea Sulfolobus tokodaii strain7. *Proteins,* 2007, vol. 69, 204-8
- **MUSTACICH, D. ; G. POWIS.** Thioredoxin reductase. *Biochem J,* 2000, vol. 346, 1-8
- **NELDER, J.A. ; MEAD, R.** A Simplex-Method for Function Minimization. *Computer Journal,* 1965, vol. 7, 308-313
- **NISBET, E.G. ; SLEEP, N.H.** The habitat and nature of early life. *Nature,* 2001, vol. 409, 1083-91
- **PEREGRIN-ALVAREZ, J.M. ; TSOKA, S. ; OUZOUNIS, C.A.** The phylogenetic extent of metabolic enzymes and pathways. *Genome Res,* 2003, vol. 13, 422-7
- **PEREZ-JIMENEZ, R. et al.** Diversity of chemical mechanisms in thioredoxin catalysis revealed by single-molecule force spectroscopy. *Nat Struct Mol Biol,* 2009, vol. 16, 890-6
- **PEREZ-JIMENEZ, R. et al.** Force-clamp spectroscopy detects residue co-evolution in enzyme catalysis. *J Biol Chem.,* 2009, vol. 283, 27121-27129
- **PEREZ-JIMENEZ, R. et al.** Single-molecule paleoenzymology probes the chemistry of resurrected enzymes. *Nat Struct Mol Biol.,* May 2011, vol. 18 (5), 592-6
- **PETERSON, F.C. ; LYTLE, B.L. ; SAMPATH, S. ; VINAROV, D. ; TYLER, E. ; SHAHAN, M. ; MARKLEY, J.L. ; VOLKMAN, B.F.** Solution structure of thioredoxin h1 from Arabidopsis thaliana. *Protein Sci.,* 2005, vol. 14, 2195-2200
- **POLLOCK, D.D. ; CHANG, B.S.W.** Ancestral sequence reconstruction. Oxford University Press, 2007, 85-94
- **POWIS, G. ; W.R. MONTFORT.** Properties and biological activities of thioredoxins. *Annu Rev Biophys Biomol Struct,* 2001, vol. 30, 421-55
- **QIN, J. ; CLORE, G.M. ; KENNEDY, W.M. ; HUTH, J.R. ; GRONENBORN, A.M.** Solution structure of human thioredoxin in a mixed disulfide intermediate complex with its target peptide from the transcription factor NF kappa B. *Structure,* 1995, vol. 3, 289-297
- **QIN, J. ; CLORE, G.M. ; KENNEDY, W.P. ; KUSZEWSKI, J. ; GRONENBORN, A.M.** The solution structure of human thioredoxin complexed with its target from Ref-1 reveals peptide chain reversal. *Structure,* 1996, vol. 4, 613-620
- **RIGGS, A.D. ; BOURGEOI.S ; M. COHN.** Lac Repressor-Operator Interaction .3. Kinetic Studies. *Journal of Molecular Biology,* 1970, vol. 53 (3), 401
- **RUSSELL, M.J. ; HALL, A.J.** The emergence of life from iron monosulphide bubbles at a submarine hydrothermal redox and pH front. *J Geol Soc Lond,* 1997, vol. 154, 377-402
- **SARKAR, A. ; R.B. ROBERTSON ; J.M. FERNANDEZ.** Simultaneous atomic force microscope and fluorescence measurements of protein unfolding using a calibrated evanescent wave. *Proc Natl Acad Sci U S A,* 2004, vol. 101 (35), 12882-6
- **SCHULTE, M.** The Emergence of Life on Earth. *Oceanography,* 2007, vol. 20, 42-49
- **SMEETS, A. ; EVRARD, C. ; LANDTMETERS, M. ; MARCHAND, C. ; KNOOPS, B. ; DECLERCQ, J.P.** Crystal structures of oxidized and reduced forms of human mitochondrial thioredoxin 2. *Protein Sci.,* 2005, vol. 14, 2610-2621

- **STANFORD, N.P. et al.** One- and three-dimensional pathways for proteins to reach specific DNA sites. *Embo J,* 2000, vol. 19 (23), 6546-57
- **STARKS, C.M. ; FRANCOIS, J.A. ; MACARTHUR, K.M. ; HEARD, B.Z. ; KAPPOCK, T.J.** Atomic-resolution crystal structure of thioredoxin from the acidophilic bacterium Acetobacter aceti. *Protein Sci,* 2007, vol. 16, 92-8
- **SUAREZ, M. et al.** Using multi-objective computational design to extend protein promiscuity. *Biophys Chem,* 2010, vol. 147, 13-9
- **TACHIBANA, C. ; T.H. STEVENS.** The yeast EUG1 gene encodes an endoplasmic reticulum protein that is functionally related to protein disulfide isomerase. *Mol Cell Biol,* 1992, vol. 12 (10), 4601-11
- **THOMSON, J.M. et al.** Resurrecting ancestral alcohol dehydrogenases from yeast. *Nat Genet,* 2005, vol. 37, 630-5
- **THORNTON, J.W.** Resurrecting ancient genes: experimental analysis of extinct molecules. *Nat Rev Genet,* 2004, vol. 5, 366-75
- **THORNTON, J.W. ; NEED, E. ; CREWS, D.** Resurrecting the ancestral steroid receptor: ancient origin of estrogen signaling. *Science,* 2003, vol. 301, 1714-7
- **TURK, B.** Targeting proteases: successes, failures and future prospects. *Nat Rev Drug Discov,* 2006, vol. 5 (9), 785-99
- **HIPPEL, P.H. ; O.G. BERG.** Facilitated target location in biological systems. *J Biol Chem,* 1989, vol. 264 (2), 675-8
- **WALKER, B. ; J.F. LYNAS.** Strategies for the inhibition of serine proteases. *Cell Mol Life Sci,* 2001, vol. 58 (4), 596-624
- **WALKER, J.C.G.** Possible Limits on the Composition of the Archean Ocean. *Nature,* 1983, vol. 302, 518-520
- **WIITA, A.P. et al.** Probing the chemistry of thioredoxin catalysis with force. *Nature,* 2007, vol. 450, 124-7
- **WIITA, A.P. ; AINAVARAPU, S.R. ; HUANG, H.H. ; FERNANDEZ, J.M.** Force-dependent chemical kinetics of disulfide bond reduction observed with single-molecule techniques. *Proc Natl Acad Sci U S A,* 2006, vol. 103, 7222-7
- **WIITA, A.P. et al.** Probing the chemistry of thioredoxin catalysis with force. *Nature,* 2007, vol. 450 (7166), 124-7
- **WILLIAMS, C.H. et al.** Thioredoxin reductase two modes of catalysis have evolved. *Eur J Biochem,* 2000, vol. 267 (20), 6110-7
- **WINDLE, H.J. ; FOX, A. ; NI EIDHIN, D. ; KELLEHER, D.** The thioredoxin system of Helicobacter pylori. *J Biol Chem,* 2000, vol. 275, 5081-9
- **WYNN, R. ; F.M. RICHARDS.** Chemical modification of protein thiols: formation of mixed disulfides. *Methods Enzymol,* 1995, vol. 251, 351-6
- **XU, S.Z. et al.** TRPC channel activation by extracellular thioredoxin. *Nature,* 2008, vol. 451 (7174), 69-72
- **YANG, Z. ; KUMAR, S. ; NEI, M.** A new method of inference of ancestral nucleotide and amino acid sequences. *Genetics,* 1995, vol. 141, 1641-50
- **YANG, Z.H.** PAML: a program package for phylogenetic analysis by maximum likelihood. *Comput Appl Biosci,* 1997, vol. 13, 555-556
- **ZALATAN, J.G. ; HERSCHLAG, D.** The far reaches of enzymology. *Nat. Chem. Biol.,* 2009, vol. 5, 516-520